(19)

**Europäisches Patentamt**
**European Patent Office**
**Office européen des brevets**

(11)  **EP 2 301 587 B1**

(12)  **EUROPEAN PATENT SPECIFICATION**

(45) Date of publication and mention
of the grant of the patent:
**25.06.2014   Bulletin 2014/26**

(51) Int Cl.:
*A61K 49/22* (2006.01)     *C07K 7/08* (2006.01)
*C07K 14/00* (2006.01)     *C07K 14/52* (2006.01)
*C07K 14/71* (2006.01)

(21) Application number: **10185498.2**

(22) Date of filing: **03.03.2003**

(54) **KDR and VEGF/KDR binding peptides and their use in diagnosis**

KDR- und VEGF/KDR-bindende Peptide sowie ihre Verwendung bei der Diagnose und Therapie

Peptides de liaison KDR et VEGF/KDR et leur utilisation pour le diagnostic et la thérapie

(84) Designated Contracting States:
**AT BE BG CH CY CZ DE DK EE ES FI FR GB GR
HU IE IT LI LU MC NL PT RO SE SI SK TR**

(30) Priority: **01.03.2002   US 360851 P
15.01.2003   US 440411 P**

(43) Date of publication of application:
**30.03.2011   Bulletin 2011/13**

(62) Document number(s) of the earlier application(s) in
accordance with Art. 76 EPC:
**08008365.2 / 2 014 310
03711418.8 / 1 572 724**

(73) Proprietors:
 • **Dyax Corp.
Burlington, MA 01803 (US)**
 • **Bracco Suisse SA
6928 Manno (CH)**

(72) Inventors:
 • **Sato, Aaron K.
Richmond, CA 94805 (US)**
 • **Sexton, Daniel J.
Melrose, CA 02176 (US)**
 • **Dransfield, Daniel T.
Hanson, MA 02341 (US)**
 • **Ladner, Robert C.
Ijamsville, MD 21754 (US)**
 • **Arbogast, Christophe
74250 Viuz-en-Sallaz (FR)**
 • **Bussat, Philippe
74800 La Roche-sur-Foron (FR)**
 • **Fan, Hong
Shanghai, PuDong 201203 (CN)**

 • **Linder, Karen E.
Kingston, NJ 08528 (US)**
 • **Marinelli, Edmund R.
Tucson, AZ 85704 (US)**
 • **Nanjappan, Palaniappa
Princeton, NJ 08540 (US)**
 • **Nunn, Adrian D.
Lambertville, NJ 08530 (US)**
 • **Pillai, Radhakrishna K.
Cranbury, NJ 08512 (US)**
 • **Pochon, Sibylle
1256 Troinex (CH)**
 • **Ramalingam, Kondareddiar
Dayton, NJ 08810 (US)**
 • **Shrivastava, Ajay
Princeton, NJ 08540 (US)**
 • **Song, Bo
Princeton, NJ 08540 (US)**
 • **Swenson, Rolf E.
Princeton, NJ 08540 (US)**
 • **Von Wronski, Mathew A.
Geneva 1205 (CH)**
 • **Yan, Feng
Grand-Lancy 1216 (CH)**

(74) Representative: **Krauss, Jan
Boehmert & Boehmert
Pettenkoferstrasse 20-22
80336 München (DE)**

(56) References cited:
**WO-A-01/52875     WO-A-99/58162**

**(Cont. next page)**

- **HAIYAN JIA ET AL: "PEPTIDES ENCODED BY EXON 6 OF VEGF INHIBIT ENDOTHELIAL CELL BIOLOGICAL RESPONSES AND ANGIOGENESIS INDUCED BY VEGF", BIOCHEMICAL AND BIOPHYSICAL RESEARCH COMMUNICATIONS, ACADEMIC PRESS INC. ORLANDO, FL, US, vol. 283, no. 1, 2001, pages 164-173, XP002941058, ISSN: 0006-291X**

**Description**

BACKGROUND OF THE INVENTION

In the developing embryo, the primary vascular network is established by *in situ* differentiation of meso-dermal cells in a process called vasculogenesis. After embryonic vasculogenesis however, it is believed that all subsequent generation of new blood vessels, in the embryo or in adults, is governed by the sprouting or splitting of new capillaries from the pre-existing vasculature in a process called angiogenesis (Pepper, M. et al., 1996. Enzyme Protein, 49:138-162; Risau, W., 1997. Nature, 386:671-674). Angiogenesis is not only involved in embryonic development and normal tissue growth and repair, it is also involved in the female reproductive cycle, establishment and maintenance of pregnancy, and in repair of wounds and fractures. In addition to angiogenesis that takes place in the normal individual, angiogenic events are involved in a number of pathological processes, notably tumor growth and metastasis, and other conditions in which blood vessel proliferation is increased, such as diabetic retinopathy, psoriasis and arthropathies. Angiogenesis is so important in the transition of a tumor from hyperplastic to neoplastic growth, that inhibition of angiogenesis has become an active cancer therapy (Kim, K. et al., 1993. Nature, 362:841-844).

Tumor-induced angiogenesis is thought to depend on the production of pro-angiogenic growth factors by the tumor cells, which overcome other forces that tend to keep existing vessels quiescent and stable (Hanahan, D. and Folkman, J., 1996. Cell, 86:353-364). The best characterized of these pro-angiogenic agents is vascular endothelial growth factor (VEGF) (Neufeld, G. et al., 1999. FASEB J., 13:9-22).

VEGF is produced naturally by a variety of cell types in response to hypoxia and some other stimuli. Many tumors also produce large amounts of VEGF, and/or induce nearby stromal cells to make VEGF (Fukumura, D. et al., 1998. Cell, 94:715-725). VEGF, also referred to as VEGF-A, is synthesized as five different splice isoforms of 121, 145, 165, 189, and 206 amino acids. $VEGF_{121}$ and $VEGF_{165}$ are the main forms produced, particularly in tumors (see, Neufeld, G. *et al.* 1999, *supra*). $VEGF_{121}$ lacks a basic domain encoded by exons 6 and 7 of the VEGF gene and does not bind to heparin or extracellular matrix, unlike $VEGF_{165}$.

VEGF family members act primarily by binding to receptor tyrosine kinases. In general, receptor tyrosine kinases are glycoproteins having an extracellular domain capable of binding one or more specific growth factors, a transmembrane domain (usually an alpha helix), a juxtamembrane domain (where the receptor may be regulated, e.g., by phosphorylation), a tyrosine kinase domain (the catalytic component of the receptor), and a carboxy-terminal tail, which in many receptors is involved in recognition and binding of the substrates for the tyrosine kinase. There are three endothelial cell-specific receptor tyrosine kinases known to bind VEGF: VEGFR-1 (Flt-1), VEGFR-2 (KDR or Flk-1), and VEGFR-3 (Flt4). Flt-1 and KDR have been identified as the primary high affinity VEGF receptors. While Flt-1 has higher affinity for VEGF, KDR displays more abundant endothelial cell expression (Bikfalvi, A. et al., 1991. J. Cell. Physiol., 149:50-59). Moreover, KDR is thought to dominate the angiogenic response and is therefore of greater therapeutic and diagnostic interest (see, Neufeld, G. *et al.* 1999, *supra*). Expression of KDR is highly upregulated in angiogenic vessels, especially in tumors that induce a strong angiogenic response (Veikkola, T. et al., 2000. Cancer Res., 60:203-212).

WO99/58162 discloses ultrasound contrast agents binding to KDR comprising a cyclic peptide. Thus, these ligands are structurally different to the ones according to the present invention.

KDR is made up of 1336 amino acids in its mature form. Because of glycosylation, it migrates on an SDS-PAGE gel with an apparent molecular weight of about 205 kDa. KDR contains seven immunoglobulin-like domains in its extracellular domain, of which the first three are the most important in VEGF binding (Neufeld, G. *et al.* 1999, *supra*). VEGF itself is a homodimer capable of binding to two KDR molecules simultaneously. The result is that two KDR molecules become dimerized upon binding and autophosphorylate, becoming much more active. The increased kinase activity in turn initiates a signaling pathway that mediates the KDR-specific biological effects of VEGF.

From the foregoing, it can be seen that not only is the VEGF binding activity of KDR *in vivo* critical to angiogenesis, but the ability to detect KDR upregulation on endothelial cells or to detect VEGF/KDR binding complexes would be extremely beneficial in detecting or monitoring angiogenesis, with particular diagnostic applications such as detecting malignant tumor growth. It would also be beneficial in therapeutic applications such as targeting tumorcidal agents or angiogenesis inhibitors to a tumor site or targeting KDR, VEGF/KDR, or angiogenesis agonists to a desired site.

SUMMARY OF THE INVENTION

The present invention relates to a compound comprising an optical label according to the appending claims useful for detecting and targeting primary receptors on endothelial cells for vascular endothelial growth factor (VEGF), *i.e.,* vascular endothelial growth factor receptor-2 (VEGFR-2, also known as kinase domain region (KDR) and fetal liver kinase-1 (Flk-1)), and for imaging complexes formed by VEGF and KDR. The involvement of VEGF and KDR in angiogenesis makes the VEGF/KDR and KDR binding polypeptides of the present invention particularly useful for imaging important sites of angiogenesis, e.g., neoplastic tumors.

A group of polypeptides has been discovered that bind to KDR or VEGF/KDR complex (referred to herein as "KDR binding polypeptides" or "KDR binding moieties" and homologues thereof). Such KDR and VEGF/KDR binding polypeptides will concentrate at the sites of angiogenesis, thus providing a means for detecting and imaging sites of active angiogenesis, which may include sites of neoplastic tumor growth. Such KDR and VEGF/KDR binding polypeptides provide novel therapeutics to inhibit or promote, *e.g.,* angiogenesis. The preparation, use and screening of such polypeptides, for example as imaging agents or as fusion partners for KDR or VEGF/KDR-homing therapeutics, is described in detail herein.

In answer to the need for improved materials and methods for detecting, localizing, measuring and possibly inhibiting affecting, e.g., angiogenesis, we have now surprisingly discovered seven families of non-naturally occurring polypeptides that bind specifically to KDR or VEGF/KDR complex. Appropriate labeling of such polypeptides provides detectable imaging agents that can bind, *e.g.,* at high concentration, to KDR-expressing endothelial cells or cells exhibiting VEGF/KDR complexes, providing angiogenesis specific imaging agents. The KDR and VEGF/KDR binding polypeptides of the instant invention can thus be used in the detection and diagnosis of such angiogenesis-related disorders. Conjugation or fusion of such polypeptides with effective agents such as VEGF inhibitors or tumorcidal agents can also be used to treat pathogenic tumors, *e.g.,* by causing the conjugate or fusion to "home" to the site of active angiogenesis, thereby providing an effective means for treating pathogenic conditions associated with angiogenesis.

This invention pertains to at least one KDR and VEGF/KDR binding polypeptide comprising an optical label conjugated directly or via a linker or spacer to said at least one polypeptide, and includes use of a single binding polypeptide as a monomer or in a multimeric or polymeric construct as well as use of more than one binding polypeptide of the invention in multimeric or polymeric constructs. Binding polypeptides according to this invention are useful in any application where binding, detecting or isolating KDR or VEGF/KDR complex, or fragments thereof retaining the polypeptide binding site, is advantageous. A particularly advantageous use of the binding polypeptides disclosed herein is in a method of imaging angiogenesis *in vivo.* The method entails the use of specific binding polypeptides according to the invention for detecting a site of angiogenesis, where the binding polypeptides have been detectably labeled for use as imaging agents, including optical imaging agents.

Another advantageous use of the KDR and VEGF/KDR complex binding polypeptides disclosed herein is to target therapeutic agents, (including compounds capable of providing a therapeutic, radiotherapeutic or cytotoxic effect.) or delivery vehicles for therapeutics (including drugs, genetic material, etc.) to sites of angiogenesis or other tissue expressing KDR.

Constructs comprising two or more KDR or KDR/VEGF binding polypeptides show improved ability to bind the target molecule compared to the corresponding monomeric binding polypeptides. For example, as shown in Experiment 5, tetrameric constructs of KDR binding polypeptides provided herein showed improved ability to bind KDR-transfected 293H cells. Combining two or more binding polypeptides in a single molecular construct appears to improve the avidity of the construct over the monomeric binding polypeptides as shown by a decrease in $K_D$.

In addition, as demonstrated herein, constructs comprising two or more binding polypeptides specific for different epitopes of KDR and/or KDR/VEGF (*e.g.*, "heteromeric" or "heteromultimeric" constructs, see U.S. application number 60/440,201 published as US 2004018974) were made. Constructs comprising two or more binding polypeptides provided herein are expected to block multiple sites on KDR or VEGF/KDR. The heteromeric constructs show superior binding ability over both the corresponding monomers, as well as multimeric constructs comprising multiple copies of the same binding polypeptide. Furthermore, heteromeric constructs comprising two or more binding peptides specific for different epitopes, together with a control peptide were also able to efficiently bind KDR-transfected 293H cells. Thus, inclusion of two or more binding polypeptides that recognize different epitopes further improves the avidity of the construct for the target molecule, as demonstrated by a decrease in $K_D$.

Therefore, the present invention is drawn to constructs comprising two or more binding polypeptides. In one embodiment, the multimeric constructs comprise two or more copies of a single binding polypeptide. In another embodiment, the multimeric constructs of the present invention comprise two or more binding polypeptides, such that at least two of the binding polypeptides in the construct are specific for different epitopes of KDR and/or KDR/VEGF. These constructs are also referred to herein as "heteromeric constructs," "heteromultimers," etc. The constructs of the present invention can also include unrelated, or control peptide. The constructs can include two or more, three or more, or four or more binding polypeptides. Based on the teachings provided herein, one of ordinary skill in the art is able to assemble the binding polypeptides provided herein into multimeric constructs and to select multimeric constructs having improved properties, such as improved ability to bind the target molecule. Such multimeric constructs having improved properties are included in the present invention, as defined in the claims.

Examination of the sequence information and binding data identifies a series of KDR or VEGF/KDR complex binding polypeptides that may form loop structures.

The KDR and VEGF/KDR binding polypeptides described above can optionally have additional amino acids attached at either or both of the N- and C-terminal ends

Another aspect of the present invention relates to modifications of the foregoing polypeptides to provide specific angiogenesis imaging agents by detectably labeling a polypeptide according to the present invention. Such detectable labeling involves conjugation with optical dyes.

These and other aspects of the present invention will become apparent with reference to the following detailed description.

Also, methods of screening polypeptides identified by phage display for their ability to bind to cells expressing the target are described. These methods permit rapid screening of the binding ability of polypeptides, including polypeptides with monomeric affinities that are too low for evaluation in standard cell-binding assays. Additionally, these methods may be used to rapidly assess the stability of the peptides in the presence of serum.

BRIEF DESCRIPTION OF THE DRAWINGS

FIG. 1 (panels A and B) are graphs illustrating the saturation binding curves of binding peptide/neutravidin-HRP complexes. FIG. 1A illustrates the saturation binding curve for SEQ ID NO:5 and SEQ ID NO:31. FIG. 1B illustrates the saturation binding curve for SEQ ID NO:17 and SEQ ID NO:66. All peptides had a C-terminal biotin and JJ spacer.

FIG. 2 is a graph illustrating the binding of peptide/neutravidin-HRP complexes: control (biotinylated with spacer, and SEQ ID NOS:5, 31, 17 and 66) to KDR-transfected and Mock-transfected 293H cells at a single concentration (5.55 nM). All peptides had a C-terminal biotin and JJ spacer.

FIG. 3 is illustrates peptide structures, with and without both spacer (di(8-amino-3,6-dioxaoctanoic acid) "JJ") and biotin tested in Example 5((a) biotinylated SEQ ID NO:5 with a JJ spacer; (b) SEQ ID NO:5 with an N-terminal biotin; (c) biotinylated SEQ ID NO:31 with the JJ spacer (d) biotinylated SEQ ID NO:31).

FIG. 4 is a bar graph illustrating binding of peptide/neutravidin HRP complexes to KDR-transfected and mock-transfected 293H cells at single a concentration (2.78 nM); peptides include (a) control (with spacer); (b) control; (c) biotinylated SEQ ID NO:5 with a JJ spacer; (d) SEQ ID NO:5 with an N-terminal biotin; and (e) biotinylated SEQ ID NO:31 with the JJ spacer; and biotinylated SEQ ID NO:31.

FIG. 5 is a bar graph illustrating specific binding (binding to KDR transfected cells minus binding to Mock transfected cells) of peptide/neutravidin-HRP complexes with and without 40% rat serum. (a) SEQ ID NO:31; (b) SEQ ID NO:5; (c) SEQ ID NO:17; (d) SEQ ID NO:66. Concentration of peptide/avidin HRP solutions was 6.66 nM for (a) and (b), 3.33 nM (c), and 2.22 nM for (d). ). All peptides had a C-terminal biotin and JJ spacer.

FIG. 6 is a bar graph illustrating binding of polypeptide/avidin-HRP solutions (SEQ ID NO:31 and/or SEQ ID NO:5) to mock- and KDR-transfected cells plotted as absorbance at 450 nm. The proportions of control and KDR binding peptides used to form each tetrameric complex are indicated in the legend for each tested multimer.

FIG. 7 is a bar graph illustrating specific binding of a biotinylated SEQ ID NO:5 with a JJ spacer/avidin-HRP complex to KDR transfected cells (background binding to mock-transfected cells subtracted), plotted as absorbance at 450 nm. Increasing concentrations (as indicated in the X axis) of uncomplexed peptides were added to the assay as indicated in the legend. Only free SEQ ID NO:5 was able to decrease the binding of the SEQ ID NO:5 complex to KDR-transfected cells.

FIG. 8 illustrates structures of binding polypeptide sequences tested in Example 6: SEQ ID NOS:31 and 76-80.

FIG. 9 is a bar graph illustrating the binding of fluorescent beads to KDR-transfected and mock-transfected cells. Neutravidin-coated beads with the indicated biotinylated ligands attached were tested for binding to KDR-expressing and non-expressing 293H cells.

FIG. 10 is a bar graph illustrating percent inhibition of $^{125}$I-labeled VEGF binding by binding polypeptides (a) acetylated SEQ ID NO:31 (without the modified C-terminus, "P6", GDSRVCWEDSWGGEVCFRYDP; SEQ ID NO:81); (b) SEQ ID NO:4 (without the modified C-terminus, "P4", AGDSWCSTEYTYCEMIGT; SEQ ID NO:82); (c) biotinylated SEQ ID NO:5 with a JJ spacer; and (d) SEQ ID NO:17 (biotinylated with the JJ spacer), at two concentrations (30 μM and 0.3 μM), to KDR-expressing 293H transfectants.

FIG. 11 depicts chemiluminescent detection on film demonstrating that activated (phosphorylated) KDR was not detected in immunoprecipitates from unstimulated (-V) HUVECs, but was abundant in immunoprecipitates from VEGF-stimulated (+V) HUVECs (upper panel). Reprobing the blot with anti-KDR demonstrated that comparable amounts of total KDR were present in both immunoprecipitates (lower panel).

FIG. 12 depicts chemiluminescent detection on film demonstrating the ability of an anti-KDR antibody (1 μg/mL) to partially block VEGF-mediated phosphorylation.

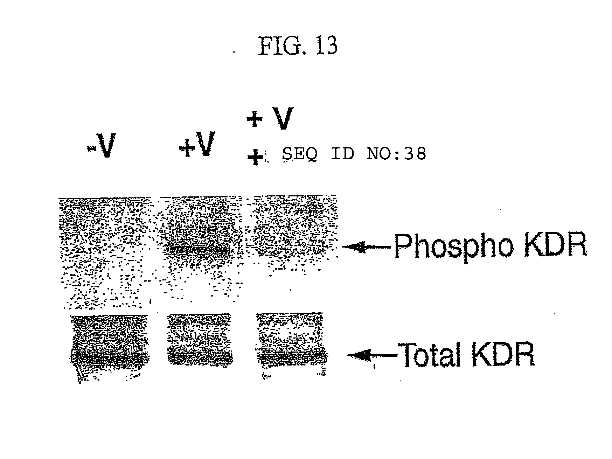

FIG. 13 depicts chemiluminescent detection on film demonstrating the ability of a KDR-binding polypeptide SEQ ID NO:38 (10 μM) to block VEGF-mediated KDR phosphorylation.

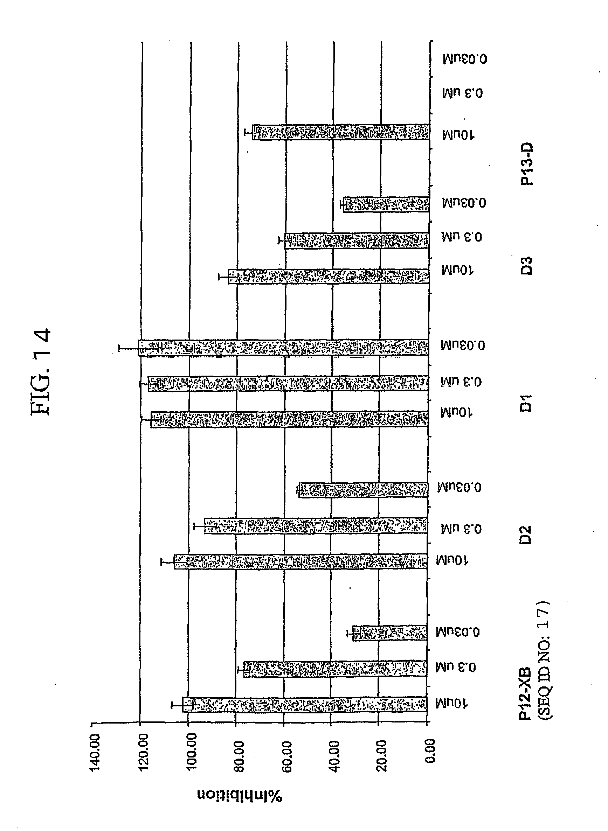

FIG. 14 is a graph showing the percentage inhibition of $^{125}$I-labeled VEGF binding by peptides P12-XB (SEQ ID NO:17) D2, D1, D3, and P13-D (AQDWYYDEILSMADQLRHAFLSGG; SEQ ID NO:83) at three different concentrations (10 μM, 0.3 μM, and 0.03 μM) to KDR-transfected 293H cells. The results are from one experiment carried out in triplicate +/- S.D.

FIG. 15 is a photograph showing the ability of D1 to completely block the VEGF-induced phosphorylation of KDR in HUVECs at 10 nM and the majority of phosphorylation at 1 nM. Reprobing the blot for total KDR (lower panel) demonstrated that the effects of the tested compounds was not due to reduced sample loading. Homodimers composed of the two binding sequences contained in D1 did not interfere with the phosphorylation at up to 100 nM.

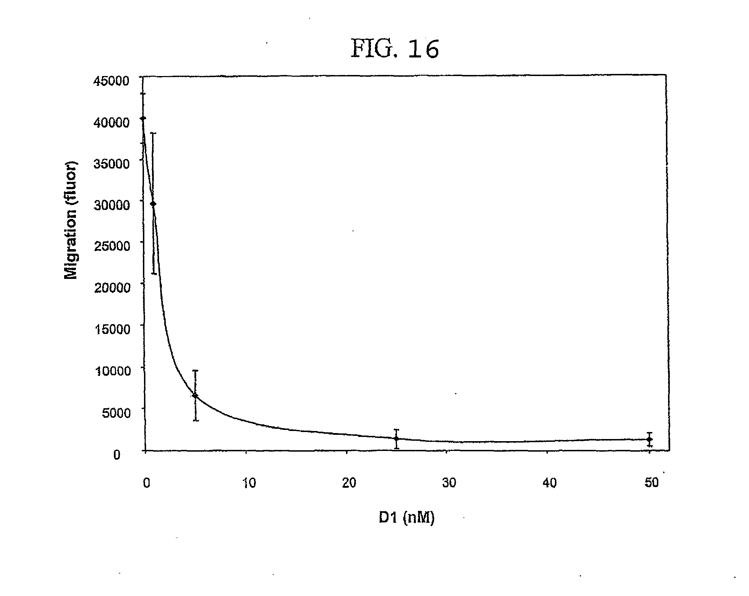

FIG. 16 is a graph showing that D1 potently blocks the migration/invasion of endothelial cells induced by VEGF. Migrating cells were quantitated by fluorescence measurement after staining the migrated cells with a fluorescent dye.

FIG. 17 is a graph of plasma clearance as percent injected dose per mL versus time.

FIG. 18 shows SE-HPLC profiles of plasma from the Superdex peptide column. Top panel, sample injected; followed by 0min, 30min, and 90min. The insert within each panel shows time point, animal number and volume injected for HPLC analysis.

FIG. 19 is a graph showing the results of testing of KDR peptides in HUVEC proliferation assay. A: D6; B: SEQ ID NO:17; C: SEQ ID NO:84 (AEGTGDLHCYFPWVCSLDPGPEGGGK; negative control); F: SEQ ID NO:84; negative control.

FIG. 20 shows the kinetic analysis of D1 (see FIG. 25), binding to murine KDR-Fc. All sensograms are fit to the bivalent analyte model.

FIG. 21 shows the kinetic analysis of D7, a heterodimer of SEQ ID NO:5 and SEQ ID NO:31. All sensograms are fit to the bivalent analyte model.

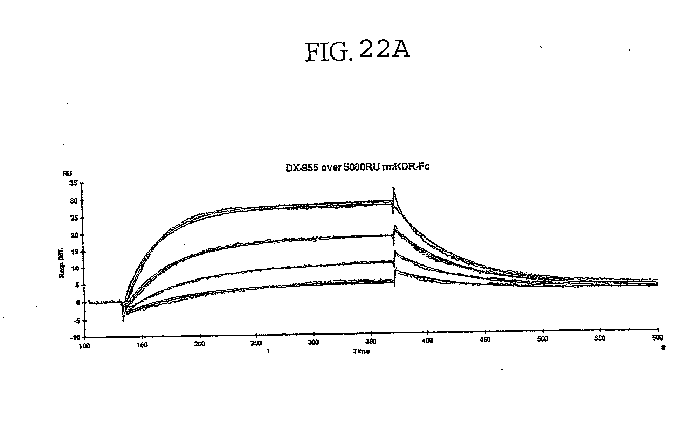

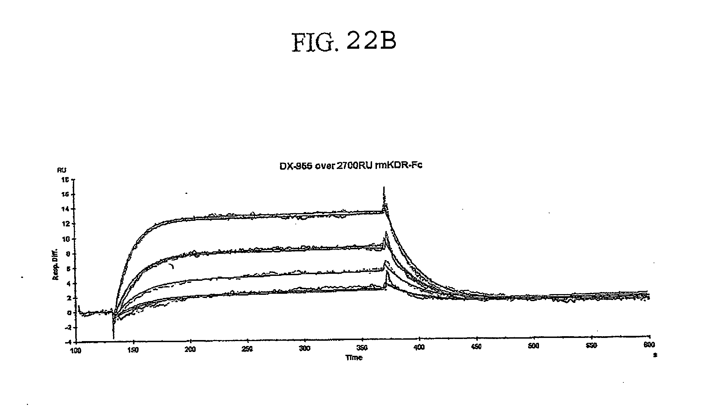

FIG. 22 shows Kinetic analysis of fluorescein labeled SEQ ID NO:17 binding to murine KDR-Fc. All sensograms are fit to the 1:1 Langmuir model.

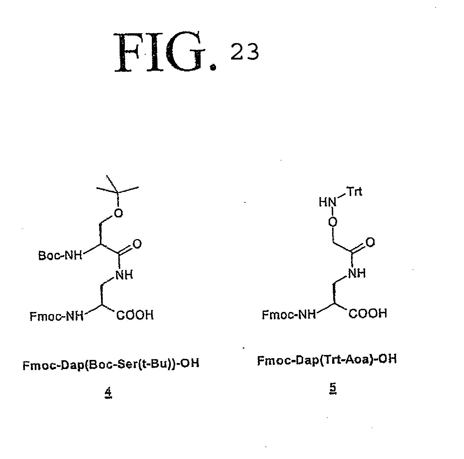

FIG. 23 shows an oxime linker. The amino acids containing an aminoalcohol function (4), and containing an alkoxyamino function (5), are incorporated into the peptide chain, not necessarily at the end of the peptide chain.

FIG. 24 shows phospholipid structures.

FIG. 25 shows dimer 1 (D1; Ac-AGPTWCEDDWYYCWLFGTGGGK(SEQ ID NO:17)[(Biotin-JJK-(O=)C(CH$_2$)$_3$C(=O)-JJ-NH(CH$_2$)$_4$-(S)-CH((Ac-VCWEDSWGGEVCFRYDPGGG(SEQ ID NO:78))-NH)CONH$_2$]-NH$_2$).

FIG. 26 shows dimer 2 (D2; Ac-AGPTWCEDDWYYCWLFGTGGGK(SEQ ID NO:17) [(Biotin-JJK-(O=)C(CH$_2$)$_3$C(=O)-JJ-NH(CH$_2$)$_4$-(S)-CH((Ac-AGPTWCEDDWYYCWLFGTJ(SEQ ID NO:50))-NH)CONH$_2$]-NH$_2$).

FIG. 27 shows dimer 3 (D3; Ac-VCWEDSWGGEVCFRYDPGGGK(SEQ ID NO:49)[(Biotin-JJK-(O=)C(CH$_2$)$_3$C(=O)-JJ-NH(CH$_2$)$_4$-(S)-CH((Ac-VCWEDSWGGEVCFRYDPGGG(SEQ ID NO: 78))-NH)CONH$_2$ ]-NH$_2$).

FIG. 28 shows dimer 4 (D4; Ac-AGPTWCEDDWYYCWLFGTJK(SEQ ID NO:50)[DOTA-JJK-(O=)C(CH$_2$)$_3$C(=O)-JJ-NH(CH$_2$)$_4$-(S)-CH((Ac-VCWEDSWGGEVCFRYDPGGG(SEQ ID NO: 78))-NH)CONH$_2$ ]-NH$_2$).

FIG. 29 shows dimer 5 (D5; Ac-VCWEDSWGGEVCFRYDPGGGK(SEQ ID NO:49) (JJ-C(=O)(CH$_2$)$_3$C(=O)-K-NH(CH$_2$)$_4$-(S)-CH((Ac-AGPTWCEDDWYYCWLFGTGGG(SEQ ID NO:85))-NH)CONH$_2$)-NH$_2$).

FIG. 30 shows dimer 8 (D8; Ac-AQDWYYDEILSMADQLRHAFLSGGGGGK(SEQ ID NO:66){Ac-AQDWYYDEILSMADQLRHAFLSGGGGGK(SEQ ID NO:66)(J-Glut-)-NH$_2$}K(Biotin-JJ)-NH$_2$).

FIG. 31 shows dimer 9 (D9; Ac-AQDWYYDEILSMADQLRHAFLSGGGGGK(SEQ ID NO:66){[Ac-GDSRVCWEDSWGGEVCFRYDPGGGK(SEQ ID NO:31)(JJ-Glut-)]-NH$_2$}K-NH$_2$).

FIG. 32 shows dimer 10 (D10Ac-AGPTWCEDDWYYCWLFGTGGGK(SEQ ID NO:17){[Ac-GDSRVCWEDSWGGEVCFRYDPGGGK(SEQ ID NO:31) (JJ-Glut-NH(CH$_2$)$_4$-(S)-CH(PnAO6-Glut-NH)(C=O)-]-NH$_2$}-NH$_2$).

FIG. 33 shows dimer 11 (D11; Ac-AGPTWCEDDWYYCWLFGTGGGK(SEQ ID NO:17){Ac-VCWEDSWEDSWGGEVCFRYDPGGGK(SEQ ID NO:97)[JJ-Glut-NH(CH$_2$)$_4$-(S)-CH(DOTA-JJ-NH-)(C=O)-]-NH$_2$}-NH$_2$).

FIG. 34 shows dimer 12 (D12; Ac-AGPTWCEDDYCWLFGTGGGK(SEQ ID NO:98){[PnA06-Glut-K(Ac-VCWEDSWGGEVCFRYDPGGGK(SEQ ID NO:49)(-C(=O)CH$_2$(OCH$_2$CH$_2$)$_2$OCH$_2$C(=O)-)-NH$_2$]}-NH$_2$).

FIG. 35 shows dimer 13 (D13; Ac-AGPTWCEDDWYYCWLFGTGGGK(SEQ ID NO:17){Ac-VCWEDSWGGEVCFRYDPGGGK(SEQ ID NO:49)[JJ-Glut-K(BOA)]-NH$_2$}-NH$_2$).

FIG. 36 shows dimer 14 (D14; Ac-AQDWYYDEILSMADQLRHAFLSGGGGGK(SEQ ID NO:66){PnAO6-Glut-K[Ac-GSDRVCWEDSWGGEVCFRYDPGGGK(SEQ ID NO:99) (JJ-Glut)-NH$_2$]}-NH$_2$).

FIG. 37 shows dimer 15 (D15; Ac-AGPTWCEDDWYYCWLFGTGGGK(SEQ ID NO:17){[[Ac-GDSRVCWEDSWGGEVCFRYDPGGGKJJ(SEQ ID NO:31)-Glut]-NH$_2$]-K(PnAO6-Glut)}-NH$_2$).

FIG. 38 shows dimer 16 (D16; Ac-AGPTWCEDDWYYCWLFGTGGGGK(SEQ ID NO:17)(PnAO6-Glut-K [Ac-GDSRVCWEDSWGGEVCFRYDPGGGK(SEQ ID NO:31)[-C(=O)CH$_2$O(CH$_2$CH$_2$O)$_2$CH$_2$C(=O)NH(CH$_2$)$_3$O(CH$_2$CH$_2$O)$_2$(CH$_2$)$_3$NH C(=O)CH$_2$O(CH$_2$CH$_2$O)$_2$CH$_2$C(=O)-]-NH$_2$]}-NH$_2$).

FIG. 39 shows dimer 17 (D17; Ac-AQDWYYDEILJGRGGRGGRGGK(SEQ ID NO:100){K[Ac-VCWEDSWGGEVCFRYDPGGGK(SEQ ID NO:31)(JJ-Glut)-NH$_2$]}-NH$_2$).

FIG. 40 shows dimer 18 (D18; Ac-AGPTWCDYDWEYCWLGTFGGGK(SEQ ID NO: 101){PnAO6-Glut-K[Ac-GVDFRCEWSDWGEVGCRSPDYGGGK (SEQ ID NO:106)(JJ-Glut)-NH$_2$]}-NH$_2$).

FIG. 41 shows dimer 19 (D19; Ac-AGPTWCEDDWYYCWLFGTGGGK(SEQ ID NO:31){Biotin-K[Ac-VCWEDSWG-GEVCFRYDPGGGK(JJ-Glut)-NH$_2$]}-NH$_2$) (SEQ ID NO:49).

FIG. 42 shows dimer 20 (D20; (-JJAGPTWCEDDWYYCWLFGTGGGGK(SEQ ID NO: 102)-NH$_2$)-Glut-VCWEDSWGGEVCFRYDPGGG(SEQ ID NO:78)-NH$_2$).

FIG. 43 shows dimer 21 (D21; [-JJAGPTWCEDDWYYCWLFGTGGGGK(SEQ ID NO: 102)(PnAO6-Glut)-NH$_2$]-Glut-VCWEDSWGGEVCFRYDPGGG(SEQ ID NO:78)-NH$_2$).

FIG. 44 shows dimer 22 (D22; Ac-GDSRVCWEDSWGGEVCFRYDPGGGK(SEQ ID NO:31){JJ-Glut-JJ-AGPTWCEDDWYYCWLFTGGGK(SEQ ID NO:103)-NH$_2$}-NH$_2$)

FIG. 45 shows dimer 23 (D23; Ac-AGPTWCEDDWYYCWLFGTGGGK(SEQ ID NO:17){Ac-VCWEDSWGGEVCFRYDPGGGK(SEQ ID NO:49) [JJ-Glut-K(SATA)]-NH$_2$}-NH$_2$. D23 is also D5 functionalized with the SATA (S-Acetylthioacetyl) group).

FIG. 46 shows dimer 24 (D24; Ac-AGPTWCEDDWYYCWLFGTGGGK(SEQ ID NO:17){SATA-JJK[Ac-VCWEDSWGGEVCFRYDPGGGK(SEQ ID NO:49)(JJ-Glut)-NH$_2$]}-NH$_2$).

FIG. 47 shows dimer 25 (D25; Ac-AGPTWCEDDWYYCWLFGTGGGK(SEQ ID NO: 17){Ac-GDSRVCWEDSWGGEVCFRYDPGGGK(SEQ ID NO:31)[JJ-Glut-NH(CH$_2$)4-(S)-CH(NH$_2$)C(=O)-]-NH$_2$}-NH$_2$).

FIG. 48 shows dimer 26 (D26; AGPTWCEDDWYYCWLFGTGGGK(SEQ ID NO:17){(-Glut-JJ-VCWEDSWGGEVCFRYDPGGG(SEQ ID NO:78)-NH$_2$)-K}-NH$_2$).

FIG. 49 shows dimer 27 (D27; Ac-AGPTWCEDDWYYCWLFGTGGGK(SEQ ID NO:17){Ac-VCWEDSWGGEVCFRYDPGGGK(SEQ ID NO:49)[S(GalNAc(Ac)$_3$-alpha-D)-G-S(GalNAc(Ac)$_3$-alpha-D)-Glut-S(GalNAc(Ac)$_3$-alpha-D)-G-S(GalNAc(Ac)$_3$-alpha-D)-NH(CH$_2$)$_4$-(S)-CH(Biotin-JJNH-)C(=O)-]-NH$_2$}-NN$_2$).

FIG. 50 shows a dimeric binding peptide of the invention.

FIG. 51 shows a dimeric binding peptide of the invention.

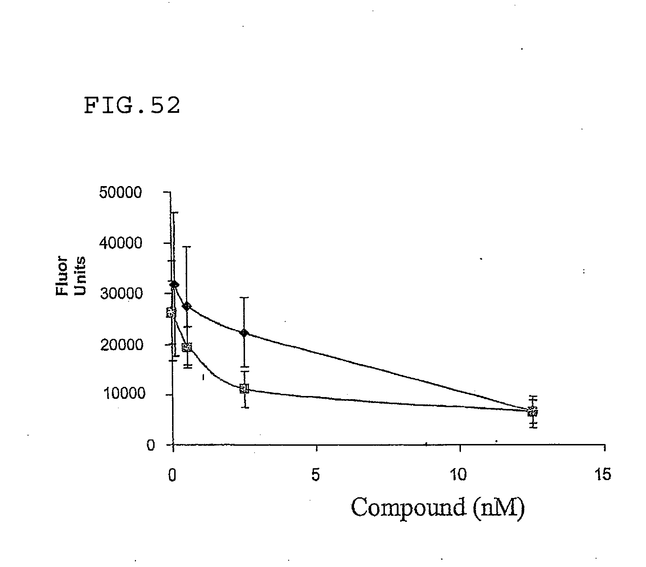

FIG. 52 shows that D26 (squares) with its glycosylation and modified spacer is able to block the effects of VEGF in the migration assay to block VEGF-stimulated migration even more potently than D24 (diamonds), which lacks those chemical modifications.

FIG. 53 shows that Adjunct A enhances the potency of D6 in blocking the biological effects of VEGF in a migration assay with cultured HUVECs. Diamonds: D6 alone at the indicated concentrations. Squares: D6 at the indicated concentrations plus 100nM Adjunct A (constant).

FIG. 54 is a schematic showing Scheme 1 (synthesis of Peptide 2).

FIG. 55 is a schematic showing Scheme 2 (synthesis of Peptide 4).

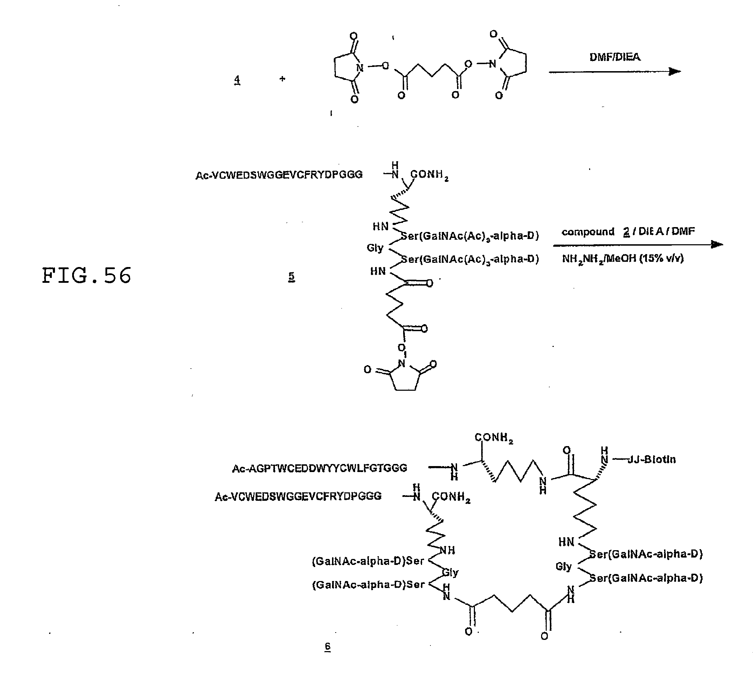

FIG. 56 is a schematic showing Scheme 3 (synthesis of D26).

DEFINITIONS

In the following sections, the term "recombinant" is used to describe non-naturally altered or manipulated nucleic acids, host cells transfected with exogenous nucleic acids, or polypeptides expressed non-naturally, through manipulation of isolated DNA and transformation of host cells. Recombinant is a term that specifically encompasses DNA molecules which have been constructed *in vitro* using genetic engineering techniques, and use of the term "recombinant" as an adjective to describe a molecule, construct, vector, cell, polypeptide or polynucleotide specifically excludes naturally occurring such molecules, constructs, vectors, cells, polypeptides or polynucleotides.

The term "bacteriophage" is defined as a bacterial virus containing a DNA core and a protective shell built up by the aggregation of a number of different protein molecules. The terms "bacteriophage" and "phage" are used herein interchangeably.

The term "polypeptide" is used to refer to a compound of two or more amino acids joined through the main chain (as opposed to side chain) by a peptide amide bond (-C(:O)NH-). The term "peptide" is used interchangeably herein with "polypeptide" but is generally used to refer to polypeptides having fewer than 40, and preferably fewer than 25 amino acids.

The term "binding polypeptide" as used herein refers to any polypeptide capable of forming a binding complex with another molecule. An equivalent term sometimes used herein is "binding moiety". "KDR binding polypeptide" is a polypeptide that forms a complex *in vitro* or *in vivo* with vascular endothelial growth factor receptor-2 (or KDR, Flk-1); "VEGF/KDR complex binding polypeptide" is a polypeptide that forms a complex *in vitro* or *in vivo* with a binding complex formed between vascular endothelial growth factor (VEGF) and KDR, in particular the complex of homodimeric VEGF and one or two KDR molecules that is believed to form at the surface of endothelial cells during angiogenesis. Specific examples of KDR and VEGF/KDR binding polypeptides include but are not limited to the peptides presented in Tables 1-7, *infra,* and include hybrid and chimeric polypeptides incorporating such peptides. Also included within the definition of KDR and VEGF/KDR complex binding polypeptides are polypeptides which are modified or optimized as disclosed herein.

Specific examples of such modifications are discussed in detail *infra,* but include substitution of amino acids for those in the parent polypeptide sequence to optimize properties, obliterate an enzyme cleavage site, etc.; C- or N-terminal amino acid substitutions or elongations, *e.g.,* for the purpose of linking the binding polypeptide to a detectable imaging label or other substrate, examples of which include, e.g., addition of a polyhistidine "tail" in order to assist in purification; truncations; amide bond changes; translocations; retroinverso peptides; peptoids; retroinversopeptoids; the use of N-terminal or C-terminal modifications or linkers, such as polyglycine or polylysine segments; alterations to include functional groups, notably hydrazide ($-NH-NH_2$) functionalities or the C-terminal linker -Gly-Gly-Gly-Lys (SEQ ID NO:18), to assist in immobilization of binding peptides according to this invention on solid supports or for attachment of fluorescent dyes; pharmacokinetic modifications, structural modifications to retain structural features, formation of salts to increase water solubility or ease of formulation, and the like.

In addition to the detectable labels described further herein, other suitable substrates for the binding polypeptides include a tumorcidal agent or enzyme, a liposome (e.g., loaded with a therapeutic agent, an ultrasound appropriate gas, or both), or a solid support, well, plate, bead, tube, slide, filter, or dish. Moreover, dimers or multimers of one or more KDR or VEGF/KDR binding polypeptides may be formed. Such constructs may, for example, exhibit increased ability to bind to KDR. All such modified binding polypeptides are also considered KDR or VEGF/KDR complex binding polypeptides so long as they retain the ability to bind the KDR or VEGF/KDR targets.

"Homologues" of the binding polypeptides described herein may be produced using any of the modification or optimization techniques described herein or known to those skilled in the art. Such homologous polypeptides will be understood to fall within the scope of the present invention and the definition of KDR and VEGF/KDR complex binding polypeptides so long as the substitution, addition, or deletion of amino acids or other such modification does not eliminate its ability to bind either KDR or VEGF/KDR complex. The term "homologous", as used herein, refers to the degree of sequence similarity between two polymers (i.e., polypeptide molecules or nucleic acid molecules). When the same nucleotide or amino acid residue or one with substantially similar properties (i.e., a conservative substitution) occupies a sequence position in the two polymers under comparison, then the polymers are homologous at that position. For example, if the amino acid residues at 60 of 100 amino acid positions in two polypeptide sequences match or are homologous then the two sequences are 60% homologous. The homology percentage figures referred to herein reflect the maximal homology possible between the two polymers, i.e., the percent homology when the two polymers are so aligned as to have the greatest number of matched (homologous) positions. Polypeptide homologues within the scope of the present invention will be at least 70% and preferably greater than 80% homologous to at least one of the KDR or VEGF/KDR binding sequences disclosed herein.

The term "binding" refers to the determination by standard assays, including those described herein, that a binding polypeptide recognizes and binds reversibly to a given target. Such standard assays include, but are not limited to equilibrium dialysis, gel filtration, and the monitoring of spectroscopic changes that result from binding.

The term "specificity" refers to a binding polypeptide having a higher binding affinity for one target over another. The term "KDR specificity" refers to a KDR binding moiety having a higher affinity for KDR over an irrelevant target. The term "VEGF/KDR specificity" refers to a VEGF/KDR complex binding moiety having a higher affinity for a VEGF/KDR complex over an a given target. Binding specificity may be characterized by a dissociation equilibrium constant ($K_D$) or an association equilibrium constant ($K_a$) for the two tested target materials, or can be any measure of relative binding strength. The binding polypeptides according to the present invention are specific for KDR or VEGF/KDR complex and preferably have a $K_D$ for KDR or VEGF/KDR complex that is lower than $10\mu M$, more preferably less than $1.0\mu M$, most preferably less than $0.5\mu M$ or even lower.

The term "patient" as used herein refers to any mammal, especially humans.

The term "pharmaceutically acceptable" carrier or excipient refers to a non-toxic carrier or excipient that may be administered to a patient, together with a compound of this invention, and which does not destroy the biological or pharmacological activity thereof.

The following common abbreviations are used throughout this specification: 9-fluorenylmethyloxycarbonyl (fmoc or Fmoc), 1-hydroxybenzotriazole (HOBt), N,N'-diisopropylcarbodiimide (DIC), acetic anhydride ($Ac_2O$), (4,4-dimethyl-2,6-dioxocyclohex-1-ylidene)-3-methylbutyl (ivDde), trifluoroacetic acid (TFA), Reagent B (TFA:$H_2O$:phenol:triisopropyl-silane, 88:5:5:2), N,N-diisopropylethylamine (DIEA), O-(1H-benzotriazole-1-yl)-N,N,N',N'-tetramethyluronium hexafluorophosphate (HBTU),O-(7-azabenzotriazol-1-yl)-1,1,3,3-tetramethyluronium hexafluorphosphate (HATU), N-hydroxysuccinimide (NHS), solid phase peptide synthesis (SPPS), dimethyl sulfoxide (DMSO), dichloromethane (DCM), dimethylformamide (DMF), and N-methylpyrrolidinone (NMP).

DETAILED DESCRIPTION OF THE INVENTION

The present invention provides to at least one polypeptide comprising an optical label conjugated directly or via a linker or spacer to said at least one polypeptide that is binding to KDR or a complex of VEGF and KDR as defined in the claims. Such binding moieties make possible the efficient detection, imaging and localization of activated endothelial cells exhibiting upregulated KDR expression and binding to VEGF. Such endothelial cells are characteristic of active angiogenesis, and therefore the polypeptides described herein provide a means of detecting, monitoring and localizing sites of angiogenesis. In particular, the binding polypeptides of this invention, when appropriately labeled, are useful for detecting, imaging and localizing tumor-induced angiogenesis. Thus, the binding polypeptides can be used to form a variety of diagnostic agents for diagnosing neoplastic tumor growth or other pathogenic instances of angiogenesis.

Specific labeled KDR and VEGF/KDR complex binding polypeptides according to the present invention were isolated initially by screening of phage display libraries, that is, populations of recombinant bacteriophage transformed to express an exogenous peptide on their surface. In order to isolate new polypeptide binding moieties for a particular target, such as KDR or VEGF/KDR, screening of large peptide libraries, for example using phage display techniques, is especially advantageous, in that very large numbers ($e.g.$, $5 \times 10^9$) of potential binders can be tested and successful binders isolated in a short period of time.

In order to prepare a phage library of displaying polypeptides to screen for binding polypeptides such as KDR or VEGF/KDR complex binding polypeptides, a candidate binding domain is selected to serve as a structural template for the peptides to be displayed in the library. The phage library is made up of a multiplicity of analogues of the parental domain or template. The binding domain template may be a naturally occurring or synthetic protein, or a region or domain of a protein. The binding domain template may be selected based on knowledge of a known interaction between the binding domain template and the binding target, but this is not critical. In fact, it is not essential that the domain selected to act as a template for the library have any affinity for the target at all: Its purpose is to provide a structure from which a multiplicity (library) of similarly structured polypeptides (analogues) can be generated, which multiplicity of analogues will hopefully include one or more analogues that exhibit the desired binding properties (and any other properties screened for).

In selecting the parental binding domain or template on which to base the variegated amino acid sequences of the library, the most important consideration is how the variegated peptide domains will be presented to the target, $i.e.,$ in what conformation the peptide analogues will come into contact with the target. In phage display methodologies, for example, the analogues will be generated by insertion of synthetic DNA encoding the analogues into phage, resulting in display of the analogue on the surfaces of the phage. Such libraries of phage, such as M13 phage, displaying a wide variety of different polypeptides, can be prepared using techniques as described, e.g., in Kay et al., Phage Display of Peptides and Proteins: A Laboratory Manual (Academic Press, Inc., San Diego, 1996) and US 5,223,409 (Ladner et al.).

In isolating the labeled specific polypeptides according to this invention, seven cyclic peptide (or "loop") libraries, designated TN8/IX, TN12/I, and a linear library, designated Lin20, were used. Each library was constructed for expression of diversified polypeptides on M13 phage. The seven libraries having a "TN" designation were designed to display a short, variegated exogenous peptide loop of 6, 7, 8, 9, 10, 12 or 13 amino acids, respectively, on the surface of M13 phage, at the amino terminus of protein III. The libraries are designated TN8/IX (having a potential $2.2 \times 10^{15}$ amino acid sequence diversity), TN12/I (having a sequence diversity of $4.6 \times 10^{19}$), and Lin20 (having a potential $3.8 \times 10^{25}$ amino acid sequence diversity).

The TN8/IX library was constructed to display a single microprotein binding loop contained in a 14-amino acid template. The TN8/IX library utilized a template sequence of $Xaa_1$-$Xaa_2$-$Xaa_3$-Cys-$Xaa_5$-$Xaa_6$-$Xaa_7$-$Xaa_8$-$Xaa_9$-$Xaa_{10}$-Cys-$Xaa_{12}$-$Xaa_{13}$-$Xaa_{14}$. The amino acids at position 1, 2, 3, 5, 6, 7, 8, 9, 10, 12, 13, and 14 in the template were varied to permit any amino acid except cysteine (Cys).

The TN12/I library was constructed to display a single microprotein binding loop contained in an 18-amino acid template. The TN12/I library utilized a template sequence $Xaa_1$-$Xaa_2$-$Xaa_3$-Cys-$Xaa_5$-$Xaa_6$-$Xaa_7$-$Xaa_8$-$Xaa_9$-$Xaa_{10}$-$Xaa_{11}$-$Xaa_{12}$-$Xaa_{13}$-$Xaa_{14}$-Cys-$Xaa_{16}$-$Xaa_{17}$-$Xaa_{18}$. The amino acids at position 1,2, 17, and 18 in the template were varied to permit any amino acid selected from a group of 12 amino acids: A, D, F, G, H, L, N, P, R, S, W, or Y). The amino acids at positions 3, 5, 6, 7, 8, 9, 10, 11, 12, 13, 14, and 16 were varied to permit any amino acid except cysteine (Cys).

The Lin20 library was constructed to display a single linear peptide in a 20-amino acid template. The amino acids at each position in the template were varied to permit any amino acid except cysteine (Cys).

The labeled binding polypeptides provided herein can include additions or truncations in the N- and/or C- termini. Such modified binding polypeptides are expected to bind KDR or VEGF/KDR complex. For example, the -GGGK linker present at the N-terminus of some of the binding polypeptides provided herein is an optional linker. Therefore, polypeptides having the same sequence, except without the terminal -GGGK sequences as far as defined in the claims are also encompassed by the present invention. In addition, binding polypeptides comprising the loop portion of the templates and sequences as described herein are expected to bind KDR and/or VEGF/KDR complex. The loop portion of the templates and sequences includes the sequences between and including the two cysteine residues that are expected to form a disulfide bond, thereby generating a peptide loop structure. Furthermore, the binding polypeptides of the present invention can include additional amino acid residues at the N- and/or C-termini.

The phage display libraries were created by making a designed series of mutations or variations within a coding sequence for the polypeptide template, each mutant sequence encoding a peptide analogue corresponding in overall structure to the template except having one or more amino acid variations in the sequence of the template. The novel variegated (mutated) DNA provides sequence diversity, and each transformant phage displays one variant of the initial template amino acid sequence encoded by the DNA, leading to a phage population (library) displaying a vast number of different but structurally related amino acid sequences.

As indicated previously, the techniques discussed in Kay et al., Phage Display of Peptides and Proteins: A Laboratory Manual (Academic Press, Inc., San Diego, 1996) and US 5,223,409 are particularly useful in preparing a library of potential binders corresponding to the selected parental template. The libraries discussed above were prepared according to such techniques, and they were screened for KDR or VEGF/KDR complex binding polypeptides against an immobilized target, as explained in the examples to follow.

In a typical screen, a phage library is contacted with and allowed to bind the target, or a particular subcomponent thereof. To facilitate separation of binders and non-binders, it is convenient to immobilize the target on a solid support. Phage bearing a target-binding moiety form a complex with the target on the solid support whereas non-binding phage remain in solution and may be washed away with excess buffer. Bound phage are then liberated from the target by changing the buffer to an extreme pH (pH 2 or pH 10), changing the ionic strength of the buffer, adding denaturants, or other known means. To isolate the binding phage exhibiting the polypeptides of the present invention, a protein elution was performed, *i.e.,* some phage were eluted from target using VEGF in solution (competitive elution); and also, very high affinity binding phage that could not be competed off incubating with VEGF overnight were captured by using the phage still bound to substrate for infection of *E.coli* cells.

The recovered phage may then be amplified through infection of bacterial cells and the screening process repeated with the new pool that is now depleted in non-binders and enriched in binders. The recovery of even a few binding phage is sufficient to carry the process to completion. After a few rounds of selection, the gene sequences encoding the binding moieties derived from selected phage clones in the binding pool are determined by conventional methods, described below, revealing the peptide sequence that imparts binding affinity of the phage to the target. When the selection process works, the sequence diversity of the population falls with each round of selection until desirable binders remain. The sequences converge on a small number of related binders, typically 10-50 out of the more than 10 million original candidates from each library. An increase in the number of phage recovered at each round of selection, and of course, the recovery of closely related sequences are good indications that convergence of the library has occurred in a screen. After a set of binding polypeptides is identified, the sequence information may be used to design other secondary phage libraries, biased for members having additional desired properties.

Formation of the disulfide binding loop is advantageous because it leads to increased affinity and specificity for such peptides. However, in serum, the disulfide bond might be opened by free cysteines or other thiol-containing molecules. Thus, it may be useful to modify the cysteine residues to replace the disulfide cross-link with another less reactive linkage. The $-CH_2-S-S-CH_2-$ cross-link has a preferred geometry in which the dihedral bond between sulfurs is close to 90 degrees, but the exact geometry is determined by the context of other side groups and the binding state of the molecule. Preferred modifications of the closing cross-link of the binding loop will preserve the overall bond lengths and angles as much as possible. Suitable such alternative cross-links include thioether linkages such as $-CH_2-S-CH_2-CH_2-$, $-CH_2-CH_2-S-CH_2-$, $-CH_2-CH_2-S-CH_2-CH_2-$; lactam linkages such as $-CH_2-NH-CO-CH_2-$ and $-CH_2-CO-NH-CH_2-$; ether linkages such as $-CH_2-CH_2-O-CH_2-CH_2-$; alkylene bridges such as $-(CH_2)_n-$ (where n = 4, 5, or 6); the linkage $-CH_2-NH-CO-NH-CH_2-$, and similar groups known in the art.

Direct synthesis of the polypeptides of the invention may be accomplished using conventional techniques, including solid-phase peptide synthesis, solution-phase synthesis, etc. Solid-phase synthesis is preferred. See Stewart et al., Solid-Phase Peptide Synthesis (W. H. Freeman Co., San Francisco, 1989); Merrifield, J. Am. Chem. Soc., 85:2149-2154 (1963); Bodanszky and Bodanszky, The Practice of Peptide Synthesis (Springer-Verlag, New York, 1984).

Polypeptides according to the invention may also be prepared commercially by companies providing peptide synthesis as a service (e.g., BACHEM Bioscience, Inc., King of Prussia, PA; Quality Controlled Biochemicals, Inc., Hopkinton, MA). Automated peptide synthesis machines, such as manufactured by Perkin-Elmer Applied Biosystems, also are available.

The polypeptide compound is preferably purified once it has been isolated or synthesized by either chemical or recombinant techniques. For purification purposes, there are many standard methods that may be employed, including reversed-phase high-pressure liquid chromatography (RP-HPLC) using an alkylated silica column such as $C_4$-, $C_8$- or $C_{18}$-silica. A gradient mobile phase of increasing organic content is generally used to achieve purification, for example, acetonitrile in an aqueous buffer, usually containing a small amount of trifluoroacetic acid. Ion-exchange chromatography can also be used to separate peptides based on their charge. The degree of purity of the polypeptide may be determined by various methods, including identification of a major large peak on HPLC. A polypeptide that produces a single peak that is at least 95% of the input material on an HPLC column is preferred. Even more preferable is a polypeptide that produces a single peak that is at least 97%, at least 98%, at least 99% or even 99.5% or more of the input material on an HPLC column.

In order to ensure that the peptide obtained using any of the techniques described above is the desired peptide for use in compositions of the present invention, analysis of the peptide composition may be carried out. Such composition analysis may be conducted using high resolution mass spectrometry to determine the molecular weight of the peptide. Alternatively, the amino acid content of the peptide can be confirmed by hydrolyzing the peptide in aqueous acid, and separating, identifying and quantifying the components of the mixture using HPLC, or an amino acid analyzer. Protein sequenators, which sequentially degrade the peptide and identify the amino acids in order, may also be used to determine the sequence of the peptide.

Labeled KDR or VEGF/KDR complex binding polypeptides according to the present invention also may be produced using recombinant DNA techniques, utilizing nucleic acids (polynucleotides) encoding the polypeptides according to this invention and then expressing them recombinantly, *i.e.,* by manipulating host cells by introduction of exogenous nucleic acid molecules in known ways to cause such host cells to produce the desired KDR or VEGF/KDR complex binding polypeptides. Such procedures are within the capability of those skilled in the art (see Davis et al., Basic Methods in Molecular Biology, (1986)). Recombinant production of short peptides such as those described herein may not be practical in comparison to direct synthesis, however recombinant means of production may be very advantageous where a KDR or VEGF/KDR complex binding moiety of this invention is incorporated in a hybrid polypeptide or fusion protein.

In the practice of the present invention, a determination of the affinity of the KDR or VEGF/KDR complex binding moiety for KDR or VEGF/KDR complex relative to another protein or target is a useful measure, and is referred to as specificity for KDR or VEGF/KDR complex. Standard assays for quantitating binding and determining affinity include equilibrium dialysis, equilibrium binding, gel filtration, or the monitoring of numerous spectroscopic changes (such as a change in fluorescence polarization) that may result from the interaction of the binding moiety and its target. These techniques measure the concentration of bound and free ligand as a function of ligand (or protein) concentration. The concentration of bound polypeptide ([Bound]) is related to the concentration of free polypeptide ([Free]) and the concentration of binding sites for the polypeptide, *i.e.,* on KDR or VEGF/KDR complex, (N), as described in the following equation:

$$[Bound] = N \times [Free]/((1/K_a)+[Free]).$$

A solution of the data to this equation yields the association constant, $K_a$, a quantitative measure of the binding affinity. The association constant, $K_a$ is the reciprocal of the dissociation constant, $K_D$. The $K_D$ is more frequently reported in measurements of affinity. Preferred KDR or VEGF/KDR complex binding polypeptides have a $K_D$ for KDR or VEGF/KDR complex in the range of 1 nanomolar (nM) to 100 micromolar ($\mu$M), which includes $K_D$ values of less than 10 nM, less than 20 nM, less than 40 nM, less than 60 nM, less than 80 nM, less than 1 $\mu$M, less than 5 $\mu$M, less than 10 $\mu$M, less than 20 $\mu$M, less than 40 $\mu$M, less than 60 $\mu$M, and less than 80 $\mu$M.

Where KDR or VEGF/KDR complex binding moieties are employed as imaging agents, other aspects of binding specificity may become more important: Imaging agents operate in a dynamic system in that binding of the imaging agent to the target (KDR or VEGF/KDR complex, *e.g.,* on activated endothelium) may not be in a stable equilibrium state throughout the imaging procedure. For example, when the imaging agent is initially injected, the concentration of imaging agent and of agent-target complex rapidly increases. Shortly after injection, however, the circulating (free) imaging agent starts to clear through the kidneys or liver, and the plasma concentration of imaging agent begins to drop. This drop in the concentration of free imaging agent in the plasma eventually causes the agent-target complex to dissociate. The usefulness of an imaging agent depends on the difference in rate of agent-target dissociation relative to the clearing rate of the agent. Ideally, the dissociation rate will be slow compared to the clearing rate, resulting in a long imaging time during which there is a high concentration of agent-target complex and a low concentration of free imaging agent (background signal) in the plasma.

Quantitative measurement of dissociation rates may be easily performed using several methods known in the art, such as fiber optic fluorimetry (see, e.g., Anderson & Miller, Clin. Chem., 34(7):1417-21 (1988)), surface plasmon resonance (see, Malmborg et al., J. Immunol. Methods, 198(1):51-7 (1996) and Schuck, Current Opinion in Biotechnology, 8:498-502 (1997)), resonant mirror, and grating coupled planar waveguiding (see, *e.g.,* Hutchinson, Molec. Biotechnology, 3:47-54 (1995)). Automated biosensors are commercially available for measuring binding kinetics: BIAcore surface plasmon resonance sensor (Biacore AB, Uppsala SE), IAsys resonant mirror sensor (Fisons Applied Sensor Technology, Cambridge GB), BIOS-1 grated coupled planar waveguiding sensor (Artificial Sensor Instruments, Zurich CH).

**Methods of Screening Polypeptides Identified by Phage Display For Their Ability To Bind To Cells Expressing The Target:**

Furthermore, methods of screening binding polypeptides identified by phage display for their ability to bind to cells expressing the target (and not to cells which do not express the target) are disclosed. These methods address a significant problem associated with screening peptides identified by phage display: frequently the peptides so identified do not have sufficient affinity for the target to be screened against target-expressing cells in conventional assays. However, ascertaining that a particular phage-identified peptide binds to cells that express the target (and does not bind to cells that do not) is a critical piece of information in identifying binding peptides which are potential *in vivo* targeting moieties. The method takes advantage of the increase in affinity and avidity associated with multivalent binding and permit screening of polypeptides with low affinities against target-expressing cells.

The method generally consists of preparation and screening of multimeric constructs including one or more binding polypeptides. For example, polypeptides identified by phage display as binding to a target are biotinylated and complexed with avidin, streptavidin or neutravidin to form tetrameric constructs. These tetrameric constructs are then incubated with cells that express the desired target and cells that do not, and binding of the tetrameric construct is detected. Binding may be detected using any method of detection known in the art. For example, to detect binding the avidin, streptavidin, or neutravidin may be conjugated to a detectable marker (e.g., a radioactive label, a fluorescent label, or an enzymatic label which undergoes a color change, such as HRP (horse radish peroxidase), TMB (tetramethyl benzidine) or alkaline phosphatase).

The biotinylated peptides are preferably complexed with neutravidin-HRP. Neutravidin exhibits lower non-specific binding to molecules than the other alternatives due to the absence of lectin binding carbohydrate moieties and cell adhesion receptor-binding RYD domain in neutravidin. See, Hiller et al., Biochem. J., 248:167-171 (1987); Alon et al., Biochem. Biophys. Res. Commun., 170:1236-41 (1990).

The tetrameric constructs may be screened against cells which naturally express the target or cells which have been engineered via recombinant DNA technologies to express the target (e.g., transfectants, transformants, etc.). If cells which have been transfected to express the target are used, mock transfected cells (*i.e.,* cells transfected without the genetic material encoding the target) may be used as a control.

The tetrameric complexes may optionally be screened in the presence of serum. Thus, the assay may also be used to rapidly evaluate the effect of serum on the binding of peptides to the target.

The methods disclosed herein are particularly useful in preparing and evaluating combinations of distinct binding polypeptides for use in dimeric or multimeric targeting contructs which contain two or more binding polypeptides. Use of biotin/avidin complexes allows for relatively easy preparation of tetrameric constructs containing one to four different binding peptides. Furthermore, it has now been found that affinity and avidity of a targeting construct may be increased by inclusion of two or more targeting moieties which bind to different epitopes on the same target. The screening methods described herein are useful in identifying combinations of binding polypeptides which may have increased affinity when included in such multimeric constructs.

The screening methods described herein may be used to screen KDR and VEGF/KDR complex binding polypeptides identified by phage display, such as those described herein. As described in more detail in Example 5 *infra,* these methods may be used to assess the specific binding of KDR binding polypeptides to cells which express KDR or have been engineered to express KDR. Tetrameric complexes of labeled KDR binding polypeptides of the invention and neutravidin-HRP may be prepared and screened against cells transfected to express KDR as well as mock transfected cells (without any KDR).

As shown in Example 5, the assay may be used to identify KDR binding polypeptides which bind specifically to KDR-expressing cells (and do not bind to cells that do not express KDR) even when the monodentate $K_D$ of the polypeptide is on the order of 200nM-300nM. The assay may be used to screen homotetrameric constructs containing four copies of a single KDR binding polypeptide of the invention as well as heterotetrameric (constructs containing two or more different KDR binding polypeptides. The methods described herein are particularly useful for assessing combinations of KDR binding polypeptides for use in multimeric constructs, particularly constructs containing two or more KDR binding polypeptides which bind to different epitopes of KDR.

The assay may also be used to assess the effect of serum on the KDR binding polypeptides. Indeed, using the screening methods disclosed herein, KDR binding polypeptides, such as SEQ ID NOS: 4 and 31, were identified whose binding is not significantly affected by serum.

**Substitution of Amide Bonds**

A type of modification within the scope of the patent is to substitute the amide bonds within the backbone of the polypeptide. For example, to reduce or eliminate undesired proteolysis, or other degradation pathways which diminish serum stability, resulting in reduced or abolished bioactivity, or to restrict or increase conformational flexibility, it is common to substitute amide bonds within the backbone of the peptides with functionality that mimics the existing conformation or alters the conformation in the manner desired. Such modifications may produce increased binding affinity or improved pharmacokinetic behavior. It is understood that those knowledgeable in the art of peptide synthesis can make the following amide bond-changes for any amide bond connecting two amino acids with the expectation that the resulting peptides could have the same or improved activity: insertion of alpha-N-methylamides or peptide amide backbone thioamides, removal of the carbonyl to produce the cognate secondary amines, replacement of one amino acid with an aza-aminoacid to produce semicarbazone derivatives, and use of E-olefins and substituted E-olefins as amide bond surrogates.

**Modifications To Improve Pharmacokinetic or Pharmacodynamic Properties**

It is also understood that use of the KDR or VEGF/KDR complex binding polypeptide in a particular application may necessitate modifications of the peptide or formulations of the peptide to improve pharmacokinetic and pharmacodynamic behavior. It is expected that the properties of the peptide may be changed by attachment of moieties anticipated to bring about the desired physical or chemical properties. Such moieties may be appended to the peptide using acids or amines, via amide bonds or urea bonds, respectively, to the N- or C-terminus of the peptide, or to the pendant amino group of a suitably located lysine or lysine derivative, 2, 3-diaminopropionic acid, ornithine, or other amino acid in the peptide that possesses a pendant amine group or a pendant alkoxyamine or hydrazine group. The moieties introduced may be groups that are hydrophilic, basic, or nonpolar alkyl or aromatic groups depending on the peptide of interest and the extant requirements for modification of its properties.

**Glycosylation of Amino Acid Residues**

Yet another modification within the scope of the invention is to employ glycosylated amino acid residues (*e.g.* serine, threonine or asparagine residues), singly or in combination in the either the binding moiety (or moieties) or the linker moiety or both. Glycosylation, which may be carried out using standard conditions, can be used to enhance solubility, alter pharmacokinetics and pharmacodynamics or to enhance binding via a specific or non-specific interaction involving the glycosidic moiety. In another approach glycosylated amino acids such as O-(2-acetamido-2-deoxy-3,4,6-tri-O-acetyl-β-D-glucopyranosyl) serine or the analogous threonine derivative (either the D- or L- amino acids) can be incorporated into the peptide during manual or automated solid phase peptide synthesis, or in manual or automated solution phase peptide synthesis. Similarly D- or L-N$^\gamma$-(2-acetamido-2-deoxy-3,4,6-tri-O-acetyl-β-D-glucopyranosyl)-asparagine can be employed. The use of amino acids glycosylated on a pendant oxygen, nitrogen or sulfur function by the agency of suitably functionalized and activated carbohydrate moieties that can be employed in glycosylation is anticipated. Such carbohydrate functions could be monosaccharides, disaccharides or even larger assemblies of oligosaccharides (Kihlberg, Jan. (2000) Glycopeptide synthesis. In: Fmoc Solid Phase Peptide Synthesis - A Practical Approach (Chan, W.C. and White, P.D. Eds) Oxford University Press, New York, NY Chap. 8, pp195-213).

Also anticipated is the appendage of carbohydrate functions to amino acids by means other than glycosylation via activation of a leaving group at the anomeric carbon. Linkage of the amino acid to the glycoside is not limited to the formation of a bond to the anomeric carbon of the carbohydrate function. Instead, linkage of the carbohydrate moiety to the amino acid could be through any suitable, sufficiently reactive oxygen atom, nitrogen atom, carbon atom or other pendant atom of the carbohydrate function via methods employed for formation of C-heteroatom, C-C or heteroatom-heteroatom (examples are S-S, O-N, N-N, P-O, P-N) bonds known in the art.

**Formation of Salts**

It is also within the scope of the invention to form different salts that may increase the water solubility or the ease of formulation of these peptides. These may include, but are not restricted to, N-methylglucamine (meglumine), acetate, oxalates, ascorbates, etc.

1.) Oxime Linker

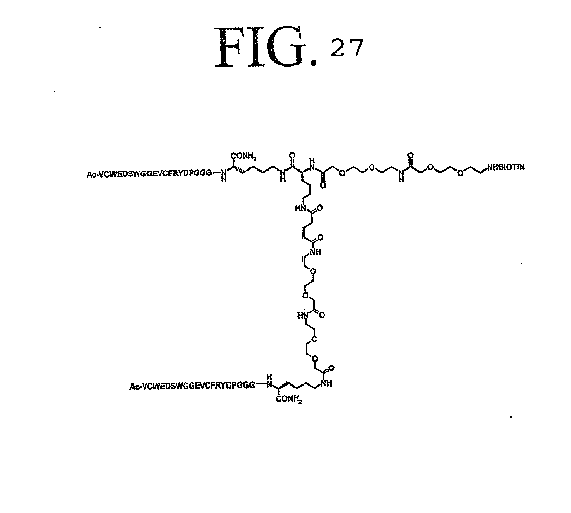

The oxime moiety has been employed as a linker by investigators in a number of contexts. Of the most interest is the work by Mutter et. al. (Wahl and Mutter, Tetrahedron Lett., 37:6861-6864 (1996)). The amino acids 4, containing an aminoalcohol function, and 5, containing an alkoxyamino function, are incorporated into the peptide chain, not necessarily at the end of the peptide chain (FIG. 27). After formation of the peptide the sidechain protecting groups are removed. The aldehyde group is unmasked and an oxime linkage is formed.

2.) Lanthionine Linker

Lanthionines are cyclic sulfides, wherein the disulfide linkage (S-S) is replaced by a carbon-sulfur (C-S) linkage. Thus, the lability to reduction is far lower. Lanthionines have been prepared by a number of methods since 1971.

**Linkers**

Additional modifications within the scope of the invention include introduction of linkers or spacers between the targeting sequence of the KDR or VEGF/KDR complex binding peptide and the detectable label. Use of such linkers/spacers may improve the relevant properties of the binding peptide (*e.g.*, increase serum stability, etc.). These linkers may include, but are not restricted to, substituted or unsubstituted alkyl chains, polyethylene glycol derivatives, amino acid spacers, sugars, or aliphatic or aromatic spacers common in the art. Furthermore, linkers which are combinations of the moieties described above, can also be employed to confer special advantage to the properties of the peptide. Lipid molecules with linkers may be attached to allow formulation of ultrasound bubbles, liposomes or other aggregation based constructs. Such constructs could be employed as agents for targeting and delivery of a diagnostic reporter, a therapeutic agent (e.g., a chemical "warhead" for therapy) or a combination of these.

**Multimeric Constructs of KDR and VEGF/KDR Complex Binding Polypeptides**

Constructs employing dimers, multimers or polymers of one or more VEGF or VEGF/KDR complex binding polypeptides of the invention are also contemplated. Indeed, there is ample literature evidence that the binding of low potency peptides or small molecules can be substantially increased by the formation of dimers and multimers. Thus, dimeric and multimeric constructs (both homogeneous and heterogeneous) are within the scope of the instant invention. Indeed, as discussed in more detail in the Examples, it is within the scope of the present invention to include multiple KDR or VEGF/KDR complex binding polypeptide sequences in a dimeric or multimeric construct. Moreover, as shown in Example 4 *infra,* these constructs may exhibit improved binding compared to a monomeric construct. The polypeptide sequences in the dimeric constructs may be attached at their N- or C- terminus or the N-epsilon nitrogen of a suitably placed lysine moiety (or another function bearing a selectively derivatizable group such as a pendant oxyamino or other nucleophilic group), or may be joined together via one or more linkers employing the appropriate attachment chemistry. This coupling chemistry may include amide, urea, thiourea, oxime, or aminoacetylamide (from chloro- or bromoacetamide derivatives, but is not so limited. For example, any of the following methods may be utilized to prepare dimeric or multimeric constructs of KDR or VEGF/KDR complex binding polypeptides of the invention.

**Method A**

Fully protected KDR-binding peptides can be built up on Ellman-type safety catch resin using automated or manual Fmoc peptide synthesis protocols. Backes et al, J. Am. Chem. Soc., 118(12):3055-56 (1996). Separately, using standard methods known in the art of peptide synthesis, a di-lysine derivative can be constructed on 2-chlorotrityl resin. See, for example, Fields et al, "Principles and Practice of Solid Phase Synthesis" in Synthetic Peptides, A Users Guide, Grant, Ed. (W.H. Freeman Co., New York, 1992), Chapt. 3, pp. 77-183; Barlos et al., "Convergent Peptide Synthesis" in Fmoc Solid Phase Peptide Synthesis, Chan, W.C. and White, P.D., Eds. (Oxford University Press, New York, 2000), Chapt. 9, pp. 215-228. Liberation of this from the 2-chlorotrityl resin without removal of the side-chain protecting groups, activation of the carboxyl group and coupling to any amine-functionalized labeling group provides a di-lysine derivative whose protected pendant nitrogen atoms may be unmasked to give two free amino groups. The prior-mentioned safety-catch resin is activated and the desired N-deprotected labeling group-functionalized di-lysine derivative is added to the activated safety-catch resin. The pendant amino groups are acylated by the carboxy-terminus of the safety-catch resin-bound peptide which is now detached from the resin and an integral part of the di-lysine structure. An excess of the safety-catch resin-bound peptide can be employed to insure complete reaction of the amino groups of the di-lysine construct. Optimization of the ratio of the reacting partners in this scheme optimizes the yield. The protecting groups on the KDR-binding peptides are removed employing trifluoroacetic acid based cleavage protocols.

The synthesis of dimeric and multimeric constructs wherein two or more KDR-binding peptides are present in one construct is easily accomplished. Orthogonal protection schemes (such as an allyloxycarbonyl group on one nitrogen and an Fmoc group on the other, or employing the Fmoc group in conjunction with the iV-Dde protecting group on the other, for example) can be employed to distinguish the pendant nitrogen atoms of the di-lysine derivatives described above. Unmasking of one of the amino groups, followed by reaction of the resulting product with an activated safety-catch resin-bound KDR-binding peptide as described above, provides a di-lysine construct having a single KDR-binding peptide attached. Removal of the second protecting group unmasks the remaining nitrogen. See, also, Mellor et al., "Synthesis of Modified Peptides" in Fmoc Solid Phase Peptide Synthesis, Chan, W.C. and White, P.D., Eds. (Oxford University Press, New York, 2000), Chapt. 6, pp. 169-176. The resulting product may be reacted with a second safety-catch resin bearing another KDR-binding peptide to provide a fully-protected homodimeric construct, which after removal of protecting groups with trifluoroacetic acid, provides the desired material.

**Method B**

A KDR-binding peptide is assembled on a Rink-amide resin by automated or manual peptide coupling methods, usually employing Fmoc peptide synthesis protocols. The peptide may possess a C-terminus or N-terminus functionalized with a linker or a linker-labeling group construct that may possess an additional nucleophilic group such as the $\varepsilon$-amino group of a lysine moiety, for example. Cleavage of the protecting groups is accomplished employing trifluoroacetic acid with appropriate modifiers depending on the nature of the peptide. The fully deprotected peptide is then reacted with a large excess of a bifunctional electrophile such as the commercially available glutaric acid bis-N-hydroxysuccinimide ester (Tyger Scientific, Inc.). The resulting monoamidated, mono-N-hydroxysuccinimidyl ester of glutaric acid is then treated with an additional equivalent of the same peptide, or an equivalent of a different KDR-binding peptide. Purification of the resulting material by HPLC affords the desired homo-dimeric construct bearing a suitable labeling group.

**Method C**

A modular scheme can be employed to prepare dimeric or higher multimeric constructs bearing suitable labeling groups as defined above. In a simple illustration, fmoc-lysine(iV-Dde) Rink amide resin is treated with piperidine to remove the fmoc moiety. Then a labeling function, such as biotin, 5-carboxyfluorescein or N,N-Dimethyl-Gly-Ser(O-t-Bu)-Cys(Acm)-Gly-OH is coupled to the nitrogen atom. The resin is next treated with hydrazine to remove the iV-Dde group. After thorough washing, the resin is treated with cyanuric chloride and a hindered base such as diisopropylethylamine in a suitable solvent such as DMF, NMP or dichloromethane to provide a monofunctionalized dichlorotriazine bound to the resin. Subsequent successive displacement of the remaining chlorine atoms by two equivalents of a KDR-binding peptide provides a resin-bound homo-dimeric labeling group-functionalized construct. Falorni et al., Tetrahedron Lett., 39(41):7607-7610 (1998); Johnson et al., Tetrahedron Lett., 54(16):4097-4106 (1998); Stankova et al., Mol. Diversity, 2(1/2):75-80 (1996). The incoming peptides may be protected or unprotected as the situation warrants. Cleavage of protecting groups is accomplished employing trifluoroacetic acid-based deprotection reagents as described above, and the desired materials are purified by high performance liquid chromatography.

It is understood that in each of these methods lysine derivatives may be serially employed to increase the multiplicity of the multimers. The use of related, more rigid molecules bearing the requisite number of masked, or orthogonally protected nitrogen atoms to act as scaffolds to vary the distance between the KDR-binding peptides, to increase the rigidity of the construct (by constraining the motion and relative positions of the KDR-binding peptides relative to each other and the reporter) is entirely within the scope of methods A-C and all other methods described herein.

**Uses for KDR or VEGF/KDR Complex Binding Polypeptides**

The KDR or VEGF/KDR complex binding moieties according to this invention will be extremely useful for imaging of KDR or VEGF/KDR complex *in vitro* or *in vivo,* and particularly for detection and/or imaging of sites of angiogenesis, in which VEGF and KDR are intimately involved, as explained above.

***In vitro:***

For detection of KDR or VEGF/KDR complex in solution, a binding polypeptide according to the invention can be detectably labeled, e.g., fluorescently labeled, then contacted with the solution, and thereafter formation of a complex between the binding polypeptide and the KDR or VEGF/KDR complex target can be detected. As an example, a fluorescently labeled KDR or VEGF/KDR complex binding peptide may be used for *in vitro* KDR or VEGF/KDR complex detection assays, wherein the peptide is added to a solution to be tested for KDR or VEGF/KDR complex under conditions allowing binding to occur. The complex between the fluorescently labeled KDR or VEGF/KDR complex binding peptide and KDR or VEGF/KDR complex target can be detected and quantified by measuring the increased fluorescence polarization arising from the KDR or VEGF/KDR complex-bound peptide relative to that of the free peptide.

For detection of soluble KDR or VEGF/KDR complex in or from a solution, binding polypeptides of the invention can be immobilized on a solid substrate such as a chromatographic support or other matrix material, then the immobilized binder can be loaded or contacted with the solution under conditions suitable for formation of a binding polypeptide:KDR complex or binding polypeptide:VEGF/KDR complex. The non-binding portion of the solution can be removed and the complex may be detected, or the KDR or VEGF/KDR complex target may be released from the binding moiety at appropriate elution conditions.

The biology of angiogenesis and the roles of VEGF and KDR in initiating and maintaining it have been investigated by many researchers and continues to be an active field for research and development.

*In vivo:*

**Diagnostic Imaging**

A particularly preferred use for the labeled polypeptides according to the present invention is for creating visually readable images of KDR expressing tissue, such as, for example, neoplastic tumors, which require angiogenesis for survival and metastasis, or other sites of angiogenic activity. The KDR and VEGF/KDR complex binding polypeptides disclosed herein may be converted to imaging reagents by conjugating the polypeptides with a label appropriate for diagnostic detection, optionally via a linker. Preferably, a peptide exhibiting much greater specificity for KDR or VEGF/KDR complex than for other serum proteins is conjugated or linked to a label appropriate for the detection methodology to be employed. For example, the KDR or VEGF/KDR complex binding polypeptide may be conjugated with or without a linker to an optical imaging dye.

Suitable linkers can be substituted or unsubstituted alkyl chains, amino acid chains (*e.g.,* polyglycine), polyethylene glycols, polyamides, and other simple polymeric linkers known in the art.

In general, the technique of using a detectably labeled KDR or VEGF/KDR complex binding moiety is based on the premise that the label generates a signal that is detectable outside the patient's body. For example, when the detectably labeled KDR or VEGF/KDR complex binding moiety is administered to the patient in which it is desirable to detect, e.g., angiogenesis, the high affinity of the KDR or VEGF/KDR complex binding moiety for KDR or VEGF/KDR complex causes the binding moiety to bind to the site of angiogenesis and accumulate label at the site of angiogenesis. Sufficient time is allowed for the labeled binding moiety to localize at the site of angiogenesis. The signal generated by the labeled peptide is detected by a scanning device which will vary according to the type of label used, and the signal is then converted to an image of the site of angiogenesis.

**C. Optical Imaging, Sonoluminescence or Photoacoustic Imaging**

In accordance with the present invention, a number of optical parameters may be employed to determine the location of KDR or VEGF/KDR complex with *in vivo* light imaging after injection of the subject with an optically-labeled KDR or VEGF/KDR complex binding polypeptide. Optical parameters to be detected in the preparation of an image may include transmitted radiation, absorption, fluorescent or phosphorescent emission, light reflection, changes in absorbance amplitude or maxima, and elastically scattered radiation. For example, biological tissue is relatively translucent to light in the near infrared (NIR) wavelength range of 650-1000 nm. NIR radiation can penetrate tissue up to several centimeters, permitting the use of the KDR or VEGF/KDR complex binding polypeptides of the present invention for optical imaging of KDR or VEGF/KDR complex *in vivo.*

The KDR or VEGF/KDR complex binding polypeptides may be conjugated with photolabels, such as optical dyes, including organic chromophores or fluorophores, having extensive delocalized ring systems and having absorption or emission maxima in the range of 400-1500 nm. The KDR or VEGF/KDR complex binding polypeptide may alternatively be derivatized with a bioluminescent molecule. The preferred range of absorption maxima for photolabels is between 600 and 1000 nm to minimize interference with the signal from hemoglobin. Preferably, photoabsorption labels have large molar absorptivities, e.g., $> 10^5$ cm$^{-1}$M$^{-1}$, while fluorescent optical dyes will have high quantum yields. Examples of optical dyes include, but are not limited to those described in WO 98/18497, WO 98/18496, WO 98/18495, WO 98/18498, WO 98/53857, WO 96/17628, WO 97/18841, WO 96/23524, WO 98/47538, and references cited therein. The photolabels may be covalently linked directly to the KDR or VEGF/KDR complex binding peptide or linked to the KDR or VEGF/KDR complex binding peptide via a linker, as described previously.

After injection of the optically-labeled KDR or VEGF/KDR complex binding moiety, the patient is scanned with one or more light sources (*e.g.,* a laser) in the wavelength range appropriate for the photolabel employed in the agent. The light used may be monochromatic or polychromatic and continuous or pulsed. Transmitted, scattered, or reflected light is detected via a photodetector tuned to one or multiple wavelengths to determine the location of KDR or VEGF/KDR complex in the subject. Changes in the optical parameter may be monitored over time to detect accumulation of the optically-labeled reagent at the site of angiogenesis. Standard image processing and detecting devices may be used in conjunction with the optical imaging reagents of the present invention.

The optical imaging reagents described above may also be used for acousto-optical or sonoluminescent imaging performed with optically-labeled imaging agents (see, US 5,171,298, WO 98/57666, and references cited therein). In acousto-optical imaging, ultrasound radiation is applied to the subject and affects the optical parameters of the transmitted, emitted, or reflected light. In sonoluminescent imaging, the applied ultrasound actually generates the light detected. Suitable imaging methods using such techniques are described in WO 98/57666.

**Other Therapeutic Applications** (for reference only) The labeled KDR or VEGF/KDR complex binding polypeptides of the present invention can be used to monitor the activity of therapeutic agents such as anti-angiogenic or tumorcidal agents against undesired angiogenesis such as occurs in neoplastic tumors.

Suitable therapeutic agents include, but are not limited to: antineoplastic agents, such as platinum compounds (*e.g.,* spiroplatin, cisplatin, and carboplatin), methotrexate, adriamycin, mitomycin, ansamitocin, bleomycin, cytosine, arabinoside, arabinosyl adenine, mercaptopolylysine, vincristine, busulfan, chlorambucil, melphalan (*e.g.,* PAM, L-PAM, or phenylalanine mustard), mercaptopurine, mitotane, procarbazine hydrochloride, dactinomycin (actinomycin D), daunorubcin hydrochloride, doxorubicin hydrochloride, taxol, mitomycin, plicamycin (mithramycin), aminoglutethimide, estramustine phosphate sodium, flutamide, leuprolide acetate, megestrol acetate, tamoxifen citrate, testoiactone, trilostane, amsacrine (m-AMSA), aparaginase (L-aparaginase), *Erwina* aparaginase, etoposide (VP-16), interferon *cx*-2a, Interferon *cx*-2b, teniposide (VM-26, vinblastine sulfate (VLB), vincristine sulfate, bleomycin sulfate, adriamycin, and arabinosyl; anti-angiogenic agents such as tyrosine kinase inhibitors with activity toward signaling molecules important in angiogenesis and/or tumor growth such as SU5416 and SU6668 (Sugen/Pharmacia & Upjohn), endostatin (EntreMed), angiostatin (EntreMed), Combrestatin (Oxigene), cyclosporine, 5-fluorouracil, vinblastine, doxorubicin, paclitaxel, daunorubcin, immunotoxins; coagulation factors; antivirals such as acyclovir, amantadine azidothymidine (AZT or Zidovudine), ribavirin and vidarabine monohydrate (adenine arahinoside, ara-A); antibiotics, antimalarials, antiprotozoans such as chloroquine, hydroxychloroquine, metroidazole, quinine and meglumine antimonate; anti-inflammatories such as diflunisal, ibuprofen, indomethacin, meclofenamate, mefenamic acid, naproxen, oxyphenbutazone, phenylbutazone, piroxicam, sulindac, tolmetin, aspirin and salicylates.

The binding polypeptides and constructs thereof can be administered by any suitable route. Suitable routes of administration include, but are not limited to, topical application, transdermal, parenteral, gastrointestinal, intravaginal, and transalveolar. Compositions for the desired route of administration can be prepared by any of the methods well known in the pharmaceutical arts, for example, as described in Remington: The Science and Practice of Pharmacy, 20th ed., Lippincott, Williams and Wilkins, 2000.

For topical application, the binding polypeptides can be suspended, for example, in a cream, gel or rinse which allows the polypeptides or constructs to penetrate the skin and enter the blood stream, for systemic delivery, or contact the area of interest, for localized delivery. Compositions suitable for topical application include any pharmaceutically acceptable base in which the polypeptides are at least minimally soluble.

For transdermal administration, the polypeptides can be applied in pharmaceutically acceptable suspension together with a suitable transdermal device or "patch." Examples of suitable transdermal devices for administration of the polypeptides of the present invention are described, for example, in U.S. Patent No. 6,165,458, issued December 26, 2000 to Foldvari, et al*., and U.S. Patent No. 6,274,166B1, issued August 4, 2001 to Sintov, et al.*

For parenteral administration, the polypeptides can be injected intravenously, intramuscularly, intraperitoneally, or subcutaneously. Typically, compositions for intravenous administration are solutions in sterile isotonic aqueous buffer. Other pharmaceutically acceptable carriers include, but are not limited to, sterile water, saline solution, and buffered saline (including buffers like phosphate or acetate), alcohol, vegetable oils, polyethylene glycols, gelatin, lactose, amylose, magnesium stearate, talc, silicic acid, paraffin, etc. Where necessary, the composition may also include a solubilizing agent and a local anaesthetic such as lidocaine to ease pain at the site of the injection, preservatives, stabilizers, wetting agents, emulsifiers, salts, lubricants, *etc.* as long as they do not react deleteriously with the active compounds. Similarly, the composition may comprise conventional excipients, *i.e.* pharmaceutically acceptable organic or inorganic carrier substances suitable for parenteral, enteral or intranasal application which do not deleteriously react with the active compounds. Generally, the ingredients will be supplied either separately or mixed together in unit dosage form, for example, as a dry lyophilized powder or water free concentrate in a hermetically sealed container such as an ampoule or sachette indicating the quantity of active agent in activity units. Where the composition is to be administered by infusion, it can be dispensed with an infusion bottle containing sterile pharmaceutical grade "water for injection" or saline. Where the composition is to be administered by injection, an ampoule of sterile water for injection or saline may be provided so that the ingredients may be mixed prior to administration.

For gastrointestinal and intravaginal administration, the polypeptides can be incorporated into pharmaceutically acceptable powders, pills or liquids for ingestion, and suppositories for rectal or vaginal administration.

For transalveolar, buccal or pulmonary administration, the polypeptides can be suspended in a pharmaceutically acceptable excipient suitable for aerosolization and inhalation or as a mouthwash. Devices suitable for transalveolar administration such as atomizers and vaporizers are also included within the scope of the invention. Suitable formulations for aerosol delivery of polypeptides using buccal or pulmonary routes can be found, for example in U.S. Patent No. 6,312,665B1, issued November 6, 2001 to Pankaj Modi.

In addition, the polypeptides of the present invention can be administered nasally or ocularly, where the polypeptide is suspended in a liquid pharmaceutically acceptable agent suitable for drop wise dosing.

The polypeptides of the present invention can be administered such that the polypeptide is released in the individual over an extended period of time (sustained or controlled release). For example, the polypeptide can be formulated into a composition such that a single administration provides delivery of the polypeptide for at least one week, or over the period of a year or more. Controlled release systems include monolithic or reservoir-type microcapsules, depot implants, osmotic pumps, vesicles, micelles, liposomes, transdermal patches and iontophoretic devices. In one embodiment, the polypeptides of the present invention are encapsulated or admixed in a slowly degrading, non-toxic polymer. Additional formulations suitable for controlled release of the polypeptides provided herein are described in U.S. Patent No. 4,391,797, issued July 5, 1983, to Folkman, et al..

Isolation of KDR or VEGF/KDR complex binding moieties in accordance with this invention will be further illustrated in the following examples. The specific parameters included in the following examples are intended to illustrate the practice of the invention, and they are not presented to in any way limit the scope of the invention as defined in the claims.

EXAMPLES (subject-matter not claimed is present for reference purposes)

*Example 1: Library Screening Against KDR and KDR/VEGF Complex Targets*

Chimeric fusions of Ig Fc region with human KDR (#357-KD-050), murine KDR (#443-KD-050), human VEGFR-1 (#321-FL-050), human VEGFR-3 (#349-F4-050), and human Trail R4 (#633-TR-100) were purchased in carrier-free form (no BSA) from R & D Systems (Minneapolis, MN). Trail R4 Fc is an irrelevant Fc fusion protein with the same Fc fusion region as the target Fc fusion (KDR Fc) and is used to deplete the libraries of Fc binders. $VEGF_{165}$ (#100-20) was purchased in carrier-free form from Peprotech (Rocky Hill, NJ). Protein A Magnetic Beads (#100.02) were purchased from Dynal (Oslo, Norway). Heparin (#H-3393) was purchased from Sigma Chemical Company (St. Louis, MO). A 2-component tetramethyl benzidine (TMB) system was purchased from KPL (Gaithersburg, MD).

In the following procedures, microtiter plates were washed with a Bio-Tek 404 plate washer (Winooski, VT). ELISA signals were read with a Bio-Tek plate reader (Winooski, VT). Agitation of 96-well plates was on a LabQuake shaker (Labindustries, Berkeley, CA).

Eight M13 phage display libraries were prepared for screening against immobilized KDR and VEGF/KDR targets: Cyclic peptide display libraries TN6/VI, TN7/IV, TN8/IX, TN9/IV, TN10/IX, TN12/I, and MTN13/I, and a linear display library, Lin20. The design of these libraries has been described, *supra.*

The DNA encoding the library was synthesized with constant DNA on either side so that the DNA can be PCR amplified using *Taq* DNA polymerase (Perkin-Elmer, Wellesley, MA), cleaved with *Nco*I and *Pst*I, and ligated to similarly cleaved phage display vector. XL1-Blue MFR' *E. coli* cells were transformed with the ligated DNA. All of the libraries were constructed in same manner.

KDR Selection Protocol in the Presence of Heparin

Protein A Magnetic Beads were blocked once with 1X PBS (pH 7.5), 0.01% Tween-20, 0.1% HSA (Blocking Buffer) for 30 minutes at room temperature and then washed five times with 1X PBS (pH 7.5), 0.01% Tween-20, 5 µg/ml heparin (PBSTH Buffer).

The cyclic peptide, or "constrained loop", libraries were pooled for the initial screening into two pools: TN6/VI, TN7/IV and TN8/IX were in one pool; TN9/IV, TN10/IX and TN12/I were in the second pool. The two pooled libraries and the linear library (Lin20) were depleted against Trail R4 Fc fusion (an irrelevant Fc fusion) and then selected against KDR Fc fusion. $10^{11}$ plaque forming units (pfu) from each library per 100 µl PBSTH were pooled together, *e.g.,* 3 pooled libraries would result in a total volume of ~350 µl in PBSTH.

To prepare the irrelevant Fc fusion beads, 500 µl of Trail R4-Fc fusion (0.1µg/µl stock in PBST (no heparin)) were added to 1000 µl of washed, blocked protein A magnetic beads. The fusion was allowed to bind to the beads overnight with agitation at 4°C. The next day, the magnetic beads were washed 5 times with PBSTH. Each phage pool was incubated with 50 µl of Trail R4 Fc fusion beads on a Labquake shaker for 1 hour at room temperature (RT). After incubation, the phage supernatant was removed and incubated with another 50 µl of Trail R4 beads. This was repeated for a total of 5 rounds of depletion, to remove non-specific Fc fusion and bead binding phage from the libraries.

To prepare the KDR target beads, 500 µl of KDR-Fc fusion (0.1 µg/µl stock in PBST (no heparin)) were added to 500 µl of washed, blocked beads. The KDR-Fc fusion was allowed to bind overnight with agitation at 4°C. The next day, the beads were washed 5 times with PBSTH. Each depleted library pool was added to 100 µl of KDR-Fc beads and allowed to incubate on a LabQuake shaker for 1 hour at RT. Beads were then washed as rapidly as possible with 5 X 1 ml PBSTH using a magnetic stand (Promega) to separate the beads from the wash buffer. Phage still bound to beads after the washing were eluted once with 250 µl of VEGF (50 µg/ml, ~1 µM) in PBSTH for 1 hour at RT on a LabQuake shaker. The 1-hour elution was removed and saved. After the first elution, the beads were incubated again with 250 µl of VEGF (50µg/ml, ~1µM) overnight at RT on a LabQuake shaker. The two VEGF elutions were kept separate and a small aliquot taken from each for titering. Each elution was mixed with an aliquot of XL1-Blue MRF' (or other F' cell line) *E. coli* cells which had been chilled on ice after having been grown to mid-logarithmic phase. The remaining beads after VEGF elution were also mixed with cells to amplify the phage still bound to the beads, *i.e.,* KDR-binding phage that had not been competed off by the two VEGF incubations (1-hour and overnight (O/N) elutions). After approximately 15 minutes at room temperature, the phage/cell mixtures were spread onto Bio-Assay Dishes (243 X 243 X 18 mm, Nalge Nunc) containing 250 ml of NZCYM agar with 50 $\mu$g/ml of ampicillin. The plate was incubated overnight at 37°C. The next day, each amplified phage culture was harvested from its respective plate. Over the next day, the input, output and amplified phage cultures were titered for FOI (*i.e.,* Fraction of Input = phage output divided by phage input).

In the first round, each pool yielded three amplified eluates. These eluates were panned for 2-3 more additional rounds of selection using ~$10^{10}$ input phage/round according to the same protocol as described above. For each additional round, the KDR-Fc beads were prepared the night before the round was initiated. For the elution step in subsequent rounds, the amplified elution re-screen on KDR-Fc beads was always eluted in the same manner and all other elutions were treated as washes. For example, for the amplified elution recovered by using the still-bound beads to infect *E. coli,* the 1-hour and overnight VEGF elutions were performed and then discarded as washes. Then the beads were used to again infect *E. coli* and produce the next round amplified elution. Using this procedure, each library pool only yielded three final elutions at the end of the selection. Two pools and one linear library, therefore, yielded a total of 9 final elutions at the end of the selection.

This selection procedure was repeated for all libraries in the absence of heparin in all binding buffers, *i.e.,* substituting PBST (PBS (pH 7.5), 0.01% Tween-20) for PBSTH in all steps.

KDR:VEGF Complex Selection Protocol in the Presence of Heparin

Protein A magnetic beads were blocked once with Blocking Buffer for 30 minutes at room temperature and then washed five times with PBSTH.

Two pools of constrained loop libraries and a linear library (Lin20) were prepared as before and then depleted against KDR Fc fusion alone, instead of Trail-R4 Fc fusion, to remove binders to the receptor without bound VEGF. Once depleted, the libraries were selected against the KDR:VEGF$_{165}$ complex.

To prepare KDR-Fc fusion depletion beads, 1 mL of KDR-Fc fusion (0.1 $\mu$g/$\mu$l stock in PBST (no heparin)) was added to 1 mL of washed, blocked beads. The fusion was allowed to bind overnight with agitation at 4°C. The next day, the beads were washed 5 times with PBSTH. Each phage pool was incubated with 50 $\mu$l of KDR-Fc fusion beads on a LabQuake shaker for 1 hour at RT. After incubation, the phage supernatant was removed and incubated with another 50$\mu$l of KDR-Fc beads. This was repeated for a total of 5 rounds of depletion.

To prepare the KDR:VEGF complex beads, 300 $\mu$l of KDR-Fc fusion beads from above were incubated with 15 $\mu$l of VEGF (1 mg/ml). VEGF was allowed to bind for 1 hour at RT. The beads were washed 5 times with PBSTH. Each depleted library pool was added to 100 $\mu$l of KDR:VEGF complex beads and allowed to incubate on a LabQuake shaker for 1 hour at RT. Beads were then washed as rapidly as possible with 5 $\times$ 1 mL PBSTH using a magnetic stand (Promega) to separate the beads from the wash buffer. To elute the phage still bound after washing, the beads were mixed with cells to amplify the phage still bound to the beads. After approximately 15 minutes at room temperature, the phage/cell mixtures were spread onto Bio-Assay Dishes (243 $\times$ 243 $\times$ 18 mm, Nalge Nunc) containing 250 ml of NZCYM agar with 50 $\mu$g/ml of ampicillin. The plate was incubated overnight at 37°C. The next day, each amplified phage culture was harvested from its respective plate. Over the next day, the input, output and amplified phage cultures were titered for FOI. This selection protocol was repeated for two additional rounds using $10^{10}$ input phage from each amplified elution.

KDR and KDR/VEGF Screening Assay

100 $\mu$l of KDR-Fc fusion or Trail R4-Fc fusion (1$\mu$g/ml) were added to duplicate Immulon II plates, to every well, and allowed to incubate at 4°C overnight. Each plate was washed twice with PBST (PBS, 0.05% Tween-20). The wells were filled to the top with 1X PBS, 1% BSA and allowed to incubate at RT for 2 hours. Each plate was washed once with PBST (PBS, 0.05% Tween-20).

To assess binding to KDR:VEGF complex, another set of KDR plates was prepared as above and then 100 $\mu$l of VEGF (1$\mu$g/ml) in PBST was added to each KDR well and allowed to incubate at RT for 30 minutes. Each plate was then washed with PBST (PBS, 0.05% Tween-20).

Once the plates were prepared, each overnight phage culture was diluted 1:1 (or to $10^{10}$ pfu if using purified phage stock) with PBS, 0.05% Tween-20, 1% BSA. 100 $\mu$l of each diluted culture was added and allowed to incubate at RT for 2-3 hours. Each plate was washed 5 times with PBST. The binding phage were visualized by adding 100 $\mu$l of a 1:10,000 dilution of HRP-anti-M13 antibody conjugate (Pharmacia), diluted in PBST, to each well, then incubating at room temperature for 1 hr. Each plate was washed 7 times with PBST (PBS, 0.05% Tween-20), then the plates were developed with HRP substrate (~10 minutes) and the absorbance signal (630 nm) detected with plate reader.

KDR and VEGF/KDR complex binding phage were recovered, amplified, and the sequences of the display peptides responsible for the binding were determined by standard DNA sequencing methods.

After isolation of KDR and VEGF/KDR complex isolates in initial selection rounds, certain isolates were selected to act as templates for the construction of secondary libraries, from which additional high affinity binding polypeptides were isolated. In a secondary TN8 library, the phage isolate sequence PKWCEEDWYYCMIT (SEQ ID NO:1) was used as a template to construct a library that allowed one-, two-, and three-base mutations to the parent sequence at each variable codon. In a secondary TN12 library, the phage isolate sequence SRVCWEDSWGGEVCFRY (SEQ ID NO:3) was used as a template to construct a library that allowed one-, two-, and three-base mutations to the parent sequence at each variable codon. In a another TN8 secondary library, a recurrent motif from the initial TN8 sequences was kept constant (WVEC---TG-C---) and all of the other codon positions (*i.e.,* at "-") were allowed to vary (all possible 20 amino acids) using NNK codon substitution, where N stands for any nucleotide and K stands for any keto nucleotide (G or T).

Using a method of peptide optimization by soft randomization as described by Fairbrother et al., Biochemistry, 37(51): 17754-17764 (1998), two libraries were prepared based on the SEQ ID NO: 1 and SEQ ID NO:3 sequences. At each residue position, each nucleotide within a particular codon was allowed to evolve by adding fixed amounts of the other three nucleotides that did not correspond to the nucleotide of the parent codon. This nucleotide mixing is accomplished in the synthesis of the template DNA used to make the library. For these libraries, the parent nucleotide within each codon was maintained at 64% for SEQ ID NO:1 and 67% for SEQ ID NO:3, whereas the other nucleotides were added at the remainder frequency divided by three. Since the parent nucleotides are in the majority, the overall consensus sequence for the whole library should still contain the parental sequence. Inspection of individual isolates, however, shows that multiple mutations are possible, thus allowing selection of peptides with improved binding ability compared to the parent sequence.

For the third library, the TN8 motif described above was kept constant and all of the other positions in were allowed to vary with NNK substitution in the template oligonucleotide. To extend the substitution, NNK diversity was also permitted in the two flanking amino acid positions, thus adding variable amino acid positions N-terminal and C-terminal to the display peptide. Unlike the previous two libraries, where the consensus sequence remains the parental sequence, this library was quite diverse in all allowed positions and only resembled the parent motif in the residues that were held constant.

A total of $2 \times 10^{11}$ pfu from each library was used as before, except the elution strategy was changed. Competition elution of bound phage was performed using the parental peptide (50 μM) that was used to make the particular secondary library (*i.e.,* peptides of SEQ ID NOS:21, 88, and 40, respectively). Binding phage were eluted through three steps: (1) elution for 1 hour at room temperature, the eluted phage being used to infect cells for amplification, (2) elution overnight, wherein fresh competition elution peptide was added to the bound phage and incubated at 4°C overnight with mixing, the eluted phage being then used to infect cells for amplification, and (3) the remaining beads (bearing uneluted binding phage) were used to infect cells directly. Three rounds of selections were performed. Plaques were picked from rounds 2 and 3 and analyzed by ELISA and sequencing. KDR positive isolates were assayed further for competition with 50 μM free parent peptide. Those peptides that showed minimal competition with the parent peptide were deemed higher affinity binders and were synthesized.

*Example 2: Peptide Synthesis and Fluorescein Labeling*

Selected KDR or VEGF/KDR complex binding peptides corresponding to positive phage isolates were synthesized on solid phase using 9-fluorenylmethoxycarbonyl protocols and purified by reverse phase chromatography. Peptide masses were confirmed by electrospray mass spectrometry, and peptides were quantified by absorbance at 280 nm. For synthesis, two N-terminal and two C-terminal amino acids from the phage vector sequence from which the peptide was excised were retained and a -Gly-Gly-Gly-Lys-NH$_2$ linker was added to the C-terminus of each peptide. Each peptide was N-terminally acetylated. For peptides with selected lysine residues, these were protected with 1-(4,4-dimethyl-2,6-dioxocyclohex-1-ylidene)-3-methylbutyl (ivDde), which allows selective coupling to the C-terminal lysine, is not removed during peptide cleavage, and can be removed after coupling with 2% hydrazine in DMF or 0.5 M hydroxylamine, pH 8, in water.

Each peptide was labeled with fluorescein on the C-terminal lysine using fluorescein (N-hydroxysuccinimide ester derivative) or fluorescein isothiocyanate (FITC) in DMF, 2% diisopropylethylamine (DIPEA). If the peptide contained an ivDde protected lysine, the reaction was quenched by the addition of 2% hydrazine, which reacts with all free NHS-fluorescein and removes the internal protecting group. For all other peptides, the reaction was quenched by the addition of an equal volume of 0.5 M hydroxylamine, pH 8. The quenched reactions were then diluted with water to less than 10% DMF and then purified using C18 reverse phase chromatography. The peptides were characterized for purity and correct mass on an LC-MS system (HP1100 HPLC with in-line SCIEX AP150 single quadrapole mass spectrometer).

*Example 3: Fluorescence Anisotropy Measurements and BiaCore Assays*

Fluorescence anisotropy measurements were performed in 384-well microplates in a volume of 10 $\mu$l in binding buffer (PBS, 0.01% Tween-20, pH 7.5) using a Tecan Polarion fluorescence polarization plate reader. In some cases, heparin (0.5 $\mu$g/ml) or 10% human serum was added to the binding buffer (data not shown). The concentration of fluorescein labeled peptide was held constant (20 nM) and the concentration of KDR-Fc (or similar target) was varied. Binding mixtures were equilibrated for 10 minutes in the microplate at 30°C before measurement. The observed change in anisotropy was fit to the equation below via nonlinear regression to obtain the apparent $K_D$. This equation (1) assumes that the synthetic peptide and KDR form a reversible complex in solution with 1:1 stoichiometry.

$$r_{obs} = r_{free} + \left(r_{bound} - r_{free}\right)\frac{(K_D + KDR + P) - \sqrt{(K_D + KDR + P)^2 - 4 \cdot KDR \cdot P}}{2 \cdot P} \qquad (1),$$

where $r_{obs}$ is the observed anisotropy, $r_{free}$ is the anisotropy of the free peptide, $r_{bound}$ is the anisotropy of the bound peptide, $K_D$ is the apparent dissociation constant, KDR is the total KDR concentration, and P is the total fluorescein-labeled peptide concentration. $K_D$ was calculated in a direct binding assay ($K_{D,B}$) (see Table 1), and therefore these values represent KDR binding to the fluorescein labeled peptide.

For BiaCore determinations of $K_D$, KDR-Fc(or other protein targets) was crosslinked to the dextran surface of a CM5 sensor chip by the standard amine coupling procedure (0.5 mg/ml solutions diluted 1:20 with 50 mM acetate, pH 6.0, $R_L$ KDR-Fc = 12859). Experiments were performed in HBS-P buffer (0.01 M HEPES, pH 7.4, 0.15 M NaCl, 0.005% polysorbate 20 (v/v)). Peptide solutions quantitated by extinction coefficient were diluted to 400 nM in HBS-P. Serial dilutions were performed to produce 200, 100, 50, and 25 nM solutions. For association, peptides were injected at 20 $\mu$l/min. for 1 minute using the kinject program. Following a 1-minute dissociation, any remaining peptide was stripped from the target surface with a quick injection of 1M NaCl for 25 sec. at 50 $\mu$l/min. All samples were injected in duplicate. Between each peptide series a buffer injection and a non-target binding peptide injection served as additional controls. Sensorgrams were analyzed using the simultaneous $k_a/k_d$ fitting program in the BIAevaluation software 3.1. Apparent $K_D$ by this method is set forth as $BiaK_D$ in Table 1. Unlike the fluorescence anisotropy experiments above, the unlabeled peptide was used for all testing using this assay and therefore, these values represent KDR binding to the unlabeled peptide. Binding affinities determined for the synthesized polypeptides are set forth in Table 1, below. The putative disulfide-constrained cyclic peptide moieties of the polypeptides are underlined.

| Table 1: Binding Affinities for Synthesized Peptides | | | |
|---|---|---|---|
| Sequence | $K_{D,B}$($\mu$M) | $BiaK_D$ ($\mu$M) | SEQ ID NO: |
| | | | |
| TN8 | | | |
| AGDSW<u>CSTEYTYC</u>EMIGTGGGK | >2 | | 4 |
| AGPKW<u>CEEDWYYC</u>MITGTGGGK | 0.28 | 0.027 | 5 |
| AGVWE<u>CAKTFPFC</u>HWFGTGGGK | 2.60 | | 6 |
| AGWVE<u>CWWKSGQC</u>YEFGTGGGK | 1.3 | | 7 |
| AGWIQ<u>CNSITGHC</u>TSGGTGGGK | 0.24 | | 8 |
| AGWIE<u>CYHPDGIC</u>YHFGTGGGK | 0.32 | 0.32 | 9 |
| AGSDW<u>CRVDWYYC</u>WLMGTGGGK | 0.064 | | 10 |
| AGANW<u>CEEDWYYC</u>FITGTGGGK | 0.310 | | 11 |
| AGANW<u>CEEDWYYC</u>WITGTGGGK | 0.097 | | 12 |
| AGPDW<u>CEEDWYYC</u>WITGTGGGK | 0.075 | | 13 |
| AGSNW<u>CEEDWYYC</u>YITGTGGGK | 0.046 | | 14 |
| AGPDW<u>CAADWYYC</u>YITGTGGGK | 0.057 | | 15 |
| AGPEW<u>CEVDWYYC</u>WLLGTGGGK | 0.075 | | 16 |

(continued)

| Table 1: Binding Affinities for Synthesized Peptides | | | |
|---|---|---|---|
| Sequence | $K_{D,B}(\mu M)$ | $BiaK_D (\mu M)$ | SEQ ID NO: |
| AGPTWCEDDWYYCWLFGTGGGK | 0.0032 | 0.079 | 17 |
| AGSKWCEQDWYYCWLLGTGGGK | 0.400 | | 18 |
| AGRNWCEEDWYYCFITGTGGGK | 0.190 | | 19 |
| AGVNWCEEDWYYCWITGTGGGK | 0.260 | | 20 |
| AGANWCEEDWYYCYITGTGGGK | 0.180 | | 21 |
| AGQAWVECYAETGYCWPRSWGTGGGK | 0.71 | | 22 |
| AGQAWIECYAEDGYCWPRSWGTGGGK | 1.40 | | 23 |
| AGVGWVECYQSTGFCYHSRDGTGGGK | 1.30 | | 24 |
| AGDWWVECRVGTGLCYRYDTGTGGGK | 0.93 | | 25 |
| AGDSWVECDAQTGFCYSFLYGTGGGK | 2.30 | | 26 |
| AGERWVECRAETGFCYTWVSGTGGGK | 2.10 | | 27 |
| AGGGWVECRAETGHCQEYRLGTGGGK | 1.60 | | 28 |
| AGVAWVECYQTTGKCYTFRGGTGGGK | ~2 | | 29 |
| AGEGWVECFANTGACFTYPRGTGGGK | 2.10 | | 30 |
| | | | |
| TN12 | | | |
| GDSRVCWEDSWGGEVCFRYDPGGGK | 0.069 | 0.12 | 31 |
| GDDSYCMMNEKGWWNCYLYDPGGGK | 1.3 | | 32 |
| GDPAQCWESNYQGIFFCDNPDPGGGK | 2.3 | | 33 |
| GDGWACAKWPWGGEICQPSDPGGGK | 1.0 | 1.5 | 34 |
| GDSSVCFEYSWGGEVCFRYDPGGGK | 0.058 | | 104 |
| GDSRVCWEYSWGGQICLGYDPGGGK | 0.32 | | 105 |
| | | | |
| Lin20 | | | |
| zIQQVQYQFFLGTPRYEQWDLDKGGK | 1.7 | | 35 |
| AQEPEGYAYWEVITLYHEEDGDGGK | 0.27 | 0.73 | 36 |
| AQAFPRFGGDDYWIQQYLRYTDGGGK | 0.53 | 0.25 | 37 |
| AQGDYVYWEIIELTGATDHTPPGGK | 0.18 | | 38 |
| AQRGDYQEQYWHQQLVEQLKLLGGK | 0.31 | 5.3 | 39 |
| AQRSWYLGPPYYEEWDPIPNGGK | 1.8 | | 40 |
| AQDWYYDEILSMADQLRHAFLSGGGK | | 0.05 | 41 |

For the analysis of those peptides that bind specifically to KDR/VEGF complex, each peptide was tested for binding to the complex in both assays (fluorescence anisotropy/Biacore) as above. In the anisotropy assay, KDR-VEGF complex was formed by mixing together a two fold molar excess of VEGF with KDR-Fc. This mixture was then used in the direct binding titration using a fluorescein labeled peptide as done previously. As a control, each peptide was also tested for binding to KDR and VEGF alone to assess their specificity for complex. Since none of the peptides bound VEGF to any extent, the presence of excess VEGF in the assay should not affect the $K_D$ determination. As shown in Table 2, below, all of the peptides showed a dramatic binding preference, binding for KDR/VEGF complex over VEGF.

Some of them, however, did show some residual binding to free KDR. To confirm the anisotropy results, the unlabeled peptides were tested in Biacore as before, except the chip was saturated with VEGF to form KDR/VEGF complex prior to the injection of the peptides. In the peptides tested, the BiaK$_D$ was within at least 2-fold of the anisotropy measurement.

Table 2: KDR/VEGF Complex Specific Peptides

| SEQ ID NO: | Sequence | $K_D$, B (KDR) | $K_D$, B (VEGF) | $K_D$, B (KDR/ VEGF) | BiaK$_D$ (KDR/ VEGF) |
|---|---|---|---|---|---|
| 42 | AQAPAWTFGTNWRSIQRVDSLTGGGGGK | NB | NB | 0.84 | |
| 43 | AQEGWFRNPQEIMGFGDSWDKPGGGGGK | NB | NB | 1.4 | |

METHODS FOR EXAMPLES 4-10

The following methods were employed in Examples 4-10. The following common abbreviations are used: 9-fluorenylmethyloxycarbonyl (Fmoc), 1-hydroxybenzotriazole (HOBt), N,N'-diisopropylcarbodiimide (DIC), N-methylpyr-rolidinone (NMP), acetic anhydride (Ac$_2$O), (4,4-dimethyl-2,6-dioxocyclohex-1-ylidene)-3-methylbutyl (ivDde), trifluoroacetic acid (TFA), Reagent B (TFA: H$_2$O: phenol: triisopropylsilane 88:5:5:2), diisopropylethylamine (DIEA), O-(1H-benzotriazole-1-yl)-N,N,N',N'-tetramethyluronium hexafluorophosphate (HBTU), O-(7-azabenzotriazol-1-yl)-1,1,3,3-tetramethyluronium hexafluorophosphate (HATU), N-hydroxysuccinimide (NHS), solid phase peptide synthesis (SPPS), dimethyl sulfoxide (DMSO), dichloromethane (DCM), dimethylformamide (DMF), human serum albumin (HSA), and radiochemical purity (RCP).

Method 1 for the ACT 357 MPS and ACT 496 MOS Synthesizers

The peptides were synthesized on NovaSyn TGR (Rink amide) resin (0.2 mmol/g) using the Advanced ChemTech ACT 357 or ACT 496 Synthesizers employing Fmoc peptide synthesis protocols, specifically using, HOBt/DIC as the coupling reagents and NMP as the solvent. The Fmoc was removed by treating the Nova-Syn TGR (Rink amide-available from NovaBiochem, San Diego CA) resin-bound peptide with 25% piperidine in DMF twice (4 min and 10 min). All amino acids were dissolved in NMP (DMF was added when the amino acid was not soluble in pure NMP). The concentration of the amino acid was 0.25M, and the concentrations for HOBt and DIC respectively were 0.5 M. For a 0.04 mmol scale synthesis:

A typical amino acid coupling cycle (not including wash steps) was to dispense piperidine solution (2.4 mL) to each well and mix for 4 min, then empty all wells. NMP (320 $\mu$L), HOBt solution (320$\mu$L, 4 eq), amino acid (640$\mu$L, 4 eq) and DIC (320 $\mu$L, 4 eq) solutions were dispensed to each well. The coupling time was 3h; then the resin was washed. The cycle was repeated for each amino acid. After the last amino acid coupling, the resin-bound peptide was treated with 25% piperidine to remove the Fmoc protecting group. After washing, the resin bound peptide was capped with 1.0M Ac$_2$O (1.2 ml per well) and diisopropylethylamine in DMF, optionally including varying amounts of HOBt in the mixture for 30 min. The resin was washed with methanol and then dichloromethane and dried. Cleavage of the peptides from the resin and side-chain deprotection was accomplished using Reagent B for 4.5 h. The cleavage solutions were collected and the resins were washed with an additional aliquot of Reagent B. The combined solutions were concentrated to dryness. Ether was added to the residue with swirling or stirring to precipitate the peptides. The ether was decanted, and solid was collected. This procedure was repeated 2-3 times to remove impurities. The crude linear peptides were dissolved in DMSO and water mixtures, and purified by HPLC (column: Waters Associates Xterra C18, 19 $\times$ 50 mm; solvents: H$_2$O with 0.1% TFA and CH$_3$CN with 0.1% TFA; UV 220 $\mu$m; Flow rate: 50-60 ml/min). The solutions containing the peptide were lyophilized to give the desired peptides as white fluffy lyophilizates (> 90% purity). The purified linear di-cysteine containing peptides were dissolved in water, mixtures of water-acetonitrile, or mixtures of water-DMSO at concentrations between 0.1 mg/ml and 2.0 mg/ml. The choice of solvent was a function of the solubility of the crude peptide in the solvent. The pH of the solution was adjusted to pH 7.5-8.5 with aqueous ammonia, aqueous ammonium carbonate or aqueous ammonium bicarbonate. The mixture was stirred vigorously in air for 24-48 hrs. In the case of non-DMSO containing solvent systems, the pH of the solution was adjusted to pH 2 with aqueous trifluoroacetic acid. The mixture was lyophilized to provide the crude cyclic disulfide containing peptide. The cyclic disulfide peptide was then dissolved to a volume of 1-2 ml in aqueous (0.1% TFA) containing a minimum of acetonitrile (0.1% TFA). The resulting solution was loaded onto a reverse phase column and the desired compound obtained by a gradient elution of acetonitrile into water, employing a C18, or C8 reverse phase semipreparative or preparative HPLC column. In the case of the DMSO-containing solutions, the solution was diluted until the DMSO concentration was minimal without precipitation of the peptide. The resulting mixture was quickly acidified to pH 2 with dilute trifluoroacetic acid and loaded onto the reverse phase HPLC system and purified as described. Fractions containing the desired materials were pooled and the peptides isolated by lyophilization.

Method 2 for the ACT 357 MPS and ACT 496 MOS Synthesizers

The peptides were synthesized as in Method 1 with the following changes. HBTU/HOBt/DIEA were used as the coupling reagent and NMP as the solvent. A low load (~0.2 mmol/g) Fmoc-GGGK(Boc)-NovSyn-TGR-resin-prepared from the above-described Nova-Syn TGR resin was employed for peptide synthesis on 0.01 mmol scale.
For a 0.01mmol scale synthesis:

After the Fmoc group was removed, a standard coupling procedure used a solution of HOBt (720 µl, 6 eq), amino acid (804µl, 6.6 eq), HBTU (720µl, 6 eq) and DIEA (798 µl, 13.3 eq). The mixture was agitated for 15 min., emptied and the resin washed. After all couplings and after cleavage and purification as above, the solutions containing desired linear peptides were lyophilized to give the peptides (> 90% purity) as white fluffy solids. The crude ether-precipitated linear di-cysteine containing peptides were cyclized by dissolution in water, mixtures of aqueous acetonitrile (0.1% TFA), or aqueous DMSO and adjustment of the pH of the solution to pH 7.5 - 8.5 by addition of aqueous ammonia, aqueous ammonium carbonate, or aqueous ammonium bicarbonate solution. The peptide concentration was between 0.1 and 2.0 mg/ml. The mixture was stirred in air for 24-48 hrs., acidified to a pH 2 with aqueous trifluoroacetic acid, and then purified by preparative reverse phase HPLC employing a gradient of acetonitrile into water. Fractions containing the desired material were pooled and the peptides were isolated by lyophilization.

Method 3 for the ACT 496 MOS Synthesizer

The peptides were synthesized by using an Advanced ChemTech ACT 496 MOS Synthesizer as in method 1. The low load (~0.2 mmol/g) GGGK(Boc)-NovaSyn-TGR resin was employed for peptide synthesis. The coupling solvent was NMP/DMSO 8:2. The synthesis was performed at a 0.02 mmol scale using a coupling time of 3h. The crude linear peptides were further processed as described for Method 1.

Method 4 for the ACT 496 MOS Synthesizer

The peptides were synthesized using method 3 on the ACT 496 with HBTU/DIEA as the coupling reagents, and NMP as the solvent. 2,4,6-collidine as a 1 M solution was used as the base. The low load Fmoc-GGGK(ivDde)-Novsyn-TGR resin (~0.2 mmol/g) was used for peptide synthesis. The coupling time was 30 minutes. The crude linear peptides were further processed as described for Method 1.

Method 5 for the ABI 433A Synthesizer

Synthesis of peptides was carried out on a 0.25 mmol scale using the FastMoc protocol (Applied Biosystems Inc). In each cycle of this protocol, 1.0 mmol of a dry protected amino acid in a cartridge was dissolved in a solution of 0.9 mmol of HBTU, 2 mmol of DIEA, and 0.9 mmol of HOBt in DMF with additional NMP added. The peptides were made using 0.1 mmol ofNovaSyn TGR (Rink amide) resin (resin substitution 0.2 mmol/g). The coupling time in this protocol was 21 min. Fmoc deprotection was carried out with 20% piperidine in NMP. At the end of the last cycle, the synthesized peptide was acetylated using acetic anhydride/DIEA/HOBt/NMP. The peptide resin was washed and dried for further manipulations or cleaved from the resin (using reagent B). Generally, the cleaved peptides were cyclized as in Method 1 before purification.

Method 6: Biotinylation of Resin-Bound Peptides

The peptides were prepared using Method 5. The ivDde protecting group on the C-terminal lysine was selectively removed by treatment with 10% hydrazine in DMF. The resin was then treated with a solution of Biotin-N-hydroxysuccinimidyl ester in DMF in the presence of DIEA. After washing, the resin was dried and cleavage was performed using with Reagent B. The resin was filtered off and the filtrate concentrated to dryness. The biotinylated peptide was dissolved in neat DMSO and treated with DIEA and stirred for 4-6 hours to effect disulfide cyclization. The crude mixture was purified by preparative HPLC.

In a typical experiment, 200 mg of the resin -bound peptide was treated with 10% hydrazine in DMF (2 × 20 mL) and washed with DMF (2 × 20 mL) and then with dichloromethane (1 × 20 mL). The resin was resuspended in DMF (10 mL) and treated with a solution of Biotin-NHS ester (0.2 mmol, 5 equivalents) and DIEA (0.2 mmol) and the resin was mixed with the reagents for 4 h. The completion of the reaction was checked by the ninhydrin test. The peptide was then released from the resin by treatment with Reagent B (10 mL) for 4 h. The resin was filtered off, Reagent B was removed *in vacuo* and the peptide was precipitated by addition of anhydrous ether. The solid formed was collected, washed with ether and dried. The solid was dissolved in anhydrous DMSO and the mixture was adjusted to pH 7.5 with DIEA and stirred for 4-6 h to effect disulfide cyclization. The disulfide cyclization reaction was monitored by analytical HPLC. After completion of the cyclization, the mixture solution was diluted with 25% acetonitrile in water and directly purified by HPLC on a reverse phase C18 column using a gradient of acetonitrile into water (both containing 0.1 % TFA). Fractions were analyzed by analytical HPLC and those containing the pure product were collected and lyophilized to obtain the required biotinylated peptide.

Method 7: Biotinylation of Purified Peptides

The purified peptide (10 mg, prepared by methods 1-5) containing a free amino group was dissolved in anhydrous DMF or DMSO (1 ml) and Biotin-NHS ester (5 equivalents) and DIEA (5 equivalents) were added. The reaction was monitored by HPLC and after the completion of the reaction (1-2 h.), the crude reaction mixture was directly purified by preparative HPLC. Fractions were analyzed by analytical HPLC and those containing the pure product were collected and lyophilized to obtain the required biotinylated peptide.

Method 8: Biotinylation of Resin-Bound Peptides Containing Linkers

In a typical experiment, 400 mg of the resin- containing peptide (made using the ABI 433A Synthesizer and bearing an ivDde-protected lysine) was treated with 10% hydrazine in DMF (2 × 20 ml). The resin was washed with DMF (2 × 20 ml) and DCM (1 × 20 ml). The resin was resuspended in DMF (10 ml) and treated with Fmoc-aminodioxaoctanoic acid (0.4 mmol), HOBt (0.4 mmol), DIC (0.4 mmol), DIEA (0.8 mmol) with mixing for 4 h. After the reaction, the resin was washed with DMF (2 × 10 ml) and with DCM (1 × 10 ml). The resin was then treated with 20% piperidine in DMF (2 × 15 ml) for 10 min. each time. The resin was washed and the coupling with Fmoc-diaminodioxaoctanoic acid and removal of the Fmoc protecting group were repeated once more. The resulting resin, containing a peptide with a free amino group, was treated with a solution of Biotin-NHS ester (0.4 mmol, 5 equivalents) and DIEA (0.4 mmol, 5 equivalents) in DMF for 2 hours. The peptide-resin was washed and dried as described previously and then treated with reagent B (20 mL) for 4h. The mixture was filtered, and the filtrate concentrated to dryness. The residue was stirred with ether to produce a solid that was collected, washed with ether and dried. The solid was dissolved in anhydrous DMSO and the pH adjusted to 7.5 with DIEA. The mixture was stirred for 4-6 hr to effect the disulfide cyclization reaction, which was monitored by analytical HPLC. After the completion of the cyclization, the DMSO solution was diluted with 25% acetonitrile in water and applied directly to a reverse phase C-18 column. Purification was effected using a gradient of acetonitrile into water (both containing 0.1 % TFA). Fractions were analyzed by analytical HPLC and those containing the pure product were collected and lyophilized to provide the required biotinylated peptide.

Method 9: Formation of 5-Carboxyfluorescein-Labeled Peptides

Peptide-resin obtained via Method 5, containing an ivDde protecting group on the epsilon nitrogen of lysine, was mixed with a solution of hydrazine in DMF (10% hydrazine/DMF, 2 × 10 ml, 10 min) to remove the ivDde group. The epsilon nitrogen of the lysine was labeled with fluorescein-5-isothiocyanate (0.12 mmol) and diisopropylethylamine (0.12 mmol) in DMF. The mixture was agitated for 12 h (fluorescein-containing compounds were protected from light). The resin was then washed with DMF (3 × 10 mL) and twice with $CH_2Cl_2$ (10 mL) and dried under nitrogen for 1h. The peptide was cleaved from the resin using reagent B for 4h and the solution collected by filtration. The volatiles were removed under reduced pressure and the residue was dried under vacuum. The peptide was precipitated with ether, collected and the precipitate was dried under a stream of nitrogen. The precipitate was added to water (1 mg/ml) and the pH of the mixture was adjusted to 8 with 10% aqueous meglumine. Cyclization of the peptide was carried out for 48 h and the solution was freeze-dried. The crude cyclic peptide was dissolved in water and purified by RP-HPLC on a $C_{18}$ column with a linear gradient of acetonitrile into water (both phases contained 0.1%TFA). Fractions containing the pure product were collected and freeze-dried. The peptides were characterized by ES-MS and the purity was determined by RP-HPLC (linear gradient of acetonitrile into water/0.1% TFA).

Method 11: Formation of Mercaptoacetylated Peptides

Using S-Acetylthioglycolic acid N-Hydoxysuccinimide Ester

S-acetylthioglycolic acid N-hydroxysuccinimide ester (SATA) (0.0055mmol) was added to a solution of a peptide (0.005 mmol, obtained from Methods 1-5 with a free amine) in DMF (0.25 mL) and the reaction mixture was stirred at ambient temperature for 6 h. The volatiles were removed under vacuum and the residue was purified by preparative HPLC using acetonitrile-water containing 0.1%TFA. Fractions containing the pure product were collected and freeze-dried to yield the mercaptoacetylated peptide. The mercaptoacetylated peptide was characterized by ESI-MS and the purity was determined by reverse phase HPLC analysis employing a linear gradient of acetonitrile into water (both containing 0.1% TFA).

Method 12: Formation of Mercaptoacetylated Peptides using S-Acetylthioglycolic acid

Purified peptides from method 5, after disulfide cyclization, was coupled with S-acetylthioglycolic acid(1.5-10 eq.)/HOBt (1.5-10 eq.)/DIC (1.5-10 eq.) in NMP for 2-16 hours at room temperature. The mixture was then purified by preparative HPLC; the fractions containing pure peptide were combined and lyophilized. In the case of compounds with another lysine protected by an ivDde group, the deprotection reaction employed 2% hydrazine in DMSO for 3h at room temperature. Purification of the reaction mixture afforded pure peptide.

In the case when a preparing a compound with S-acetylthioglycolic acid coupled to two aminodioxaoctanoic acid groups and the peptide, the purified peptide from method 5 (having a free amino group, was coupled to $AcSCH_2CO-(NH-CH_2-CH_2-O-CH_2-CH_2-O-CH_2-CO)_2-OH$ (30 eq.)/HOBt (30 eq.)/DIC (30 eq.) in NMP for 40 hours at room temperature. The mixture was purified and the ivDde group was removed. A second purification gave the final product as a white lyophilizate.

Alternatively Fmoc aminodioxaoctanoic acid was coupled twice successively to the peptide (produced by method 5) followed by Fmoc removal and coupling to S-acetylthioglycolic acid.

Method 13: Preparation of Homo and Heterodimers

The required purified peptides were prepared by SPPS using Method 5. To prepare homodimers, half of the peptide needed to prepare the dimer was dissolved in DMF and treated with 10 equivalents of glutaric acid bis N-hydroxysuccinimidyl ester. The progress of the reaction was monitored by HPLC analysis and mass spectroscopy. At completion of the reaction, The volatiles were removed *in vacuo* and the residue was washed with ethyl acetate to remove unreacted bis-NHS ester. The residue was dried, re-dissolved in anhydrous DMF and treated with another half portion of the peptide in the presence of 2 equivalents of DIEA. The reaction was allowed to proceed for 24 h. This mixture was applied directly to a Waters Associates C-18 XTerra reverse phase HPLC column and purified by elution with a linear gradient of acetonitrile into water (both containing 0.1% TFA).

In the case of heterodimers, one of the monomers was reacted with the bis NHS ester of glutaric acid and after washing off the excess of bis NHS ester, the second peptide was added in the presence of DIEA. After the reaction, the mixture was purified by preparative HPLC.

*Example 4: Preparation of KDR and VEGF/KDR Complex Binding Polypeptides*

Utilizing the methods set forth above, biotinylated versions the KDR and VEGF/KDR complex binding polypeptides set forth in Table 3 were prepared. The letter "J" in the peptide sequences refers to a spacer or linker group, 8-amino-3,6-dioxaoctanoyl.

The ability of the biotinylated polypeptides (with the JJ spacer) to bind to KDR was assessed using the assay set forth in Example 5, following the procedures disclosed therein. Several biotinylated peptides bound well to the KDR-expressing cells: SEQ ID NO:66 ($K_D$ 1.81 nM +/- 0.27), SEQ ID NO:5 ($K_D$ 14.87+/- 5.0 nM, four experiment average), SEQ ID NO:31 + spacer ($K_D$ 10.00+/-2.36 nM, four experiment average),-SEQ ID NO:49 ($K_D$ 6.94+/-1.94 nM, one experiment), and SEQ ID NO:52 ($K_D$ 3.02+/-0.75 nM, one experiment).

**Table 3: KDR, VEGF/KDR Complex Binding Polypeptides**

| SEQ ID NO: | Structure (or) Sequence | Mol. Wt. | MS |
|---|---|---|---|
| 31 | Ac-GDSRV<u>CWEDSWGGEVC</u>FRYDPGGGK-NH$_2$ | 2801.98 | 1399.6 [M-H]$^-$ |
| 5 | Ac-AGPKW<u>CEEDWYYC</u>MITGTGGGK-NH$_2$ | 2361 | - |
| 7 | Ac-AGWVF<u>CWWKSGQC</u>YEFGTGGGK-NH$_2$ | 2474.06 | - |
| 44 | Ac-AQEGWFRNPQEIMGFGDSWDKPGGGK-NH$_2$ | 2934.35 | - |
| 31 | Ac-GDSRV<u>CWEDSWGGEVC</u>FRYDPGGGK(Biotin)-NH$_2$ | 3030.29 | 1512.4 [M$^{2-}$] |
| 45 | Ac-AQRGDYQEQYWHQQLVEQLK(iv-Dde)LLGGGK-NH$_2$ | 3318.71 | 1659.1 [M$^{2-}$] |
| 45 | Ac-AQRGDYQEQYWHQQLVEQLKLLGGGK-NH$_2$ | 3112.58 | - |
| 46 | Ac-AGWYW<u>CDYYGIGC</u>K(iv-Dde)WTGGGK-NH$_2$ | 2673.18 | |
| 47 | Ac-AGWYW<u>CDYYGIGC</u>KWTGTGGGK-NH$_2$ | 2467.05 | |
| 48 | Ac-AQWYYDWFHNQRKPPSDWIDNLGGGK-NH$_2$ | 3218.51 | - |
| 5 | Ac-AGPK(iv-Dde)W<u>CEEDWYYC</u>MITGTGGGK-NH$_2$ | 2698.11 | 2695.7 [M-H]$^-$; 1347.8 [M-2H]$^{2-}$/2 |
| 5 | Ac-AGPKW<u>CEEDWYYC</u>MITGTGGGK(Biotin)-NH$_2$ | 2718.13 | 2833.4 (M-H)$^-$ |
| 5 | Ac-AGPKW<u>CEEDWYYC</u>MITGTGGGK(Biotin-JJ-)-NH$_2$ | 3008.44 | 1502.6.4 (M-2H)$^{2-}$/2 |
| 5 | Ac-AGPKW<u>CEEDWYYC</u>MITGTGGGK (AcSCH$_2$C(=O)-)-NH$_2$ | 2608.96 | 1304, [M-2H]$^{2-}$/2 |
| 31 | Ac-GDSRV<u>CWEDSWGGEVC</u>FRYDPGGGK(Biotin-JJ-)-NH$_2$ | 3316.4 | 1657.8, [M-2H]$^{2-}$/2 |
| 31 | Ac-GDSRV<u>CWEDSWGGEVC</u>FRYDPGGGK (AcSCH$_2$C(=O)-)-NH$_2$ | 2917.15 | 1457.4, [M-2H]$^{2-}$/2 |
| 31 | Biotin-JJGDSRV<u>CWEDSWGGEVC</u>FRYDPGGGK-NH$_2$ | 3272.34 | 1636.7, [M-2H]$^{2-}$/2 |
| 5 | Ac-AGPKW<u>CEEDWYYC</u>MITGT-GGGK(AcSCH$_2$C(=O)-JJ-)-NH$_2$ | 2899.28 | 1449.2, [M-2H]$^{2-}$/2 |
| 17 | Ac-AGPTW<u>CEDDWYYC</u>WLFGTGGGK(Biotin-JJ-)-NH$_2$ | 3066.27 | 1532.8, [M-2H]$^{2-}$/2 |
| 49 | Ac-V<u>CWEDSWGGEVC</u>FRYDPGGGK(Biotin-JJ-)-NH$_2$ | 2903.24 | 1449.3, (M-2H)$^{2-}$/2; 965.8, (M-3H)$^{3-}$/3 |
| 50 | Ac-AGPTW<u>CEDDWYYC</u>WLFGTJK(Biotin-JJ-)-NH$_2$ | 3042.44 | 1519.7, (M-2H)$^{2-}$/2-; 1012.8 (M-3H)$^{3-}$/3 |
| 31 | Ac-GDSRV<u>CWEDSWGGEVC</u>FRYDPGGGK (AcSCH$_2$C(=O)-JJ-)-NH$_2$ | 3208.48 | 1602.6, [M-2H]$^{2-}$/2 |
| 17 | Ac-AGPTW<u>CEDDWYYC</u>WLFGTGGGK (AcSCH$_2$C(=O)-JJ-)-NH$_2$ | 2907.29 | 1453.1, [M-2H]$^{2-}$/2 |
| 51 | Ac-AQAHMPPWRPVAVDALFDWVEGG-GGGK(Biotin-JJ-)-NH$_2$ | 3404.64 | 1701.6, [M-2H]$^{2-}$/2 |
| 52 | Ac-AQAHMPPWWPLAVDAQEDWFEGGGGGK(Biotin-JJ-)-NH$_2$ | 3493.59 | 1746.2, [M-2H]$^{2-}$/2 |
| 52 | Ac-AQAQMPPWWPLAVDALFDWFEGGGGGK(Biotin-JJ-)-NH$_2$ | 3487.64 | 1743.2, [M-2H]$^{2-}$/2 |
| 53 | Ac-AQDWYWREWMPMHAQFLADDWGGGGGK(Biotin-JJ-)-NH$_2$ | 3751.64 | 1874.3, [M-2H]$^{2-}$/2 |
| 55 | Ac-AQPVTDWTPHHPK(iv-Dde)APDVWLFYTGGGGGK(Biotin-JJ-)-NH$_2$ | 3781.86 | 1890.4, [M-2H]$^{2-}$/2 |
| 56 | Ac-AQDALEAPK(iv-Dde)RDWYYDWFLNHSPGGGGGK(Biotin-JJ-)-NH$_2$ | 3897.85 | 1948.0, [M-2H]$^{2-}$/2 |
| 57 | Ac-KW<u>CEEDWYYC</u>MITGTGGGK(Biotin-JJ-)-NH$_2$ | 2781.2 | 1390.0, [M-2H]$^{2-}$/2 |
| 58 | Ac-AGPKW<u>CEEDWYYC</u>MIGGGK(Biotin-JJ-)-NH$_2$ | 2747.15 | 1373.5, [M-2H]$^{2-}$/2 |
| 59 | Ac-KW<u>CEEDWYYC</u>MIGGGK(Biotin-JJ-)-NH$_2$ | 2522.04 | 1260.8, [M-2H]$^{2-}$/2 |

(continued)

| Table 3: KDR, VEGF/KDR Complex Binding Polypeptides | | | |
|---|---|---|---|
| SEQ ID NO: | Structure (or) Sequence | Mol. Wt. | MS |
| 60 | Ac-AQPDNWK(iv-Dde)EFYESGWK(iv-Dde)-YPSLYK(iv-Dde)PLGGGGGK(Biotin-JJ-)-NH$_2$ | 4377.2 | 2188.4, [M-2H]$^{2-}$/2 |
| 61 | Ac-AQMPPGFSYWEQVVLHDDAQVLGG-GGGK(Biotin-JJ-)-NH$_2$ | 3499.7 | 1749.2, [M-2H]$^{2-}$/2 |
| 62 | Ac-AQARMGDDWEEAPPHEWGWADGGGGGK(Biotin-JJ-)-NH$_2$ | 3480.5 | 1740.2, [M-2H]$^{2-}$/2 |
| 63 | Ac-AQPEDSEAWYWLNYRPTMFHQLGG-GGGK(Biotin-JJ-)-NH$_2$ | 3751.7 | 1875.8, [M-2H]$^{2-}$/2 |
| 64 | Ac-AQSTNGDSFVYWEEVELVDHPGGGGGK(Biotin-JJ-)-NH$_2$ | 3554.6 | 1776.4, [M-2H]$^{2-}$/2 |
| 65 | Ac-AQWESDYWDQMRQQLK(iv-Dde)TAYMK(iv-Dde)VGGGGGK(Biotin-JJ-)-NH$_2$ | 4187.02 | 2093.0, [M-2H]$^{2-}$/2 |
| 66 | Ac-AQDWYYDEILSMADQLRHAFLSGGGGGK(Biotin-JJ-)-NH$_2$ | 3641.69 | 1820.9, [M-2H]$^{2-}$/2 |
| The putative disulfide constrained cyclic peptide is indicated by underlining. | | | |

*Example 5: Binding of KDR binding peptides/avidin HRP complex to KDR transfected 293H cells*

To determine the binding of peptides identified by phage display to KDR expressed in transiently-transfected 293H cells, a novel assay that measures the binding of biotinylated peptides complexed with neutravidin HRP to KDR on the surface of the transfected cells was developed. This assay was used to screen the biotinylated peptides set forth in Example 4. Neutravidin HRP was used instead of streptavidin or avidin because it has lower non-specific binding to molecules other than biotin due to the absence of lectin binding carbohydrate moieties and also due to the absence of the cell adhesion receptor-binding RYD domain in neutravidin.

In the experiments described herein, tetrameric complexes of KDR-binding peptides SEQ ID NO:31, SEQ ID NO:5, SEQ ID NO:17 and SEQ ID NO:66 and a control peptide, which does not bind to KDR, were prepared and tested for their ability to bind 293H cells that were transiently-transfected with KDR. All four tetrameric complexes of KDR-binding peptides were niotinylated and contained the JJ spacer, and bound to the KDR-expressing cells; however, SEQ ID NO:66 exhibited the best K$_D$ (1.81nM). The tetrameric complexes of KDR-binding peptides SEQ ID NO:31, SEQ ID NO:5 exhibited improved binding over monomers of the same peptides. Moreover, inclusion of a spacer between the KDR-binding peptide and the biotin was shown to improve binding in Experiment B.

In Experiment C, it was shown that this assay can be used to assess the effect of serum on binding of peptides as used in the invention to KDR and VEGF/KDR complex. The binding of SEQ ID NO:5, SEQ ID NO:31, and SEQ ID NO:66 was not significantly affected by the presence of serum, while the binding of SEQ ID NO:17 was reduced more than 50% in the presence of serum.

In Experiment D, it was shown that this assay is useful in evaluating distinct combinations of KDR and VEGF/KDR complex binding polypeptides for use in multimeric targeting constructs which contain more than one KDR and VEGF/KDR complex binding polypeptide. Moreover, Experiments D and E establish that tetrameric constructs including two or more KDR binding peptides which bind to different epitopes exhibited superior binding to "pure" tetrameric constructs of the targeting peptides alone.

Experiment A

Preparation of m-RNA & 5' RACE ready cDNA library

HUVEC cells were grown to almost 80% confluence in 175 cm$^2$ tissue culture flasks (Becton Dickinson, Biocoat, cat # 6478) and then 10 ng/ml of bFGF (Oncogene, cat # PF003) was added for 24 h to induce expression of KDR. mRNA was isolated using the micro-fast track 2.0 kit from Invitrogen (cat. # K1520-02). 12 μg of mRNA (measured by absorbance at 260 nM) was obtained from two flasks (about 30 million cells) following the kit instructions. Reverse transcription to generate cDNA was performed with 2 μg of mRNA, oligo dT primer (5'-(T)$_{25}$GC-3') and/or smart II oligo (5'AAGCAGTGGTAACAACGCAGAGTACGCGGG-3') (SEQ ID NO:67) using Moloney Murine Leukemia Virus (MMLV) reverse transcriptase. The reaction was performed in a total volume of 20 μl and the reaction mix contained 2 μl of RNA,

1 μl smart II oligo, 1 μl of oligo dT primer, 4 μl of 5X first-strand buffer (250 mM Tris HCl pH 8.3, 375 mM KCl, 30 mM MgCl$_2$) 1 μl DTT (20 mM, also supplied with reverse transcriptase), 1 μl dNTP mix (10 mM each of dATP, dCTP, dGTP, and dTTP in ddH$_2$O, Stratagene, cat. # 200415), 9 μl ddH$_2$O and 1 μl MMLV reverse transcriptase (Clonetech, cat #8460-1). The reverse transcription reaction was performed for 90 minutes at 42°C, and the reaction was stopped by adding 250 μl of tricine-EDTA buffer (10 mM tricine, 1.0 mM EDTA). The reverse transcription product, a 5' RACE ready cDNA library, can be stored for 3 months at -20°C. Note that all water used for DNA and RNA application was DNAse and RNAse free from USB (cat. # 70783).

Cloning of s-KDR into TOPOII Vector

In order to clone s-KDR, a 5' oligo (G ATG GAG AGC AAG GTG CTG CTG G) (SEQ ID NO:68) and a 3' oligo (C CAA GTT CGT CTT TTC CTG GGC A) (SEQ ID NO:69) were used. These were designed to amplify the complete extracellular domain of KDR (~2.2 kbps) from the 5' RACE ready cDNA library (prepared above) using polymerase chain reaction (PCR) with pfu polymerase (Stratagene, cat. # 600135). The PCR reaction was done in total volume of 50 μl and the reaction mix contained 2 μl 5' RACE ready cDNA library, 1 μl 5' oligo (10 μM), 1 μl 3' oligo (10 μM), 5 μl 10X PCR buffer [PCR buffer (200 mM Tris-HCl pH 8.8, 20 mM MgSO$_4$, 100 mM KCl, 100 mM (NH$_4$)$_2$SO$_4$) supplied with pfu enzyme plus 1% DMSO and 8% glycerol], 1 μl dNTP mix (10 mM) and 40 μl ddH$_2$0. The PCR reaction was performed by using a program set for 40 cycles of 1 minute at 94C, 1 minute at 68C and 4 minutes at 72C. The PCR product was purified by extraction with 1 volume of phenol, followed by extraction with 1 volume of chloroform and precipitated using 3 volume of ethanol and 1/10 volume of 3M sodium acetate. The PCR product was resuspended in 17 μl of ddH$_2$O, the 2 μl of 10X Taq polymerase buffer (100 mM Tris-HCl pH 8.8, 500 mM KCl, 15 mM MgCl$_2$, 0.01% gelatin) and 1 μl of *Taq* polymerase (Stratagene, cat. # 600131) was added to generate an A overhang to each end of the product. After incubating for 1 hour at 72C the modified product was cloned directly into a TOPOII vector (InVitrogen, Carlsbad, CA) following the manufacturer's protocol to give TOPO-sKDR. The TOPO vector allows easy cloning of PCR products because of the A-overhang in *Taq* (PCR enzyme)-treated PCR products.

Cloning the transmembrane and cytoplasmic domains of KDR into TOPO II Vector

In order to clone the transmembrane and cytoplasmic domains of KDR, a 5' oligo (TCC CCC GGG ATC ATT ATT CTA GTA GGC ACG GCG CTG) (SEQ ID NO:70) and a 3' oligo (C AGG AGG AGA GCT CAG TGT GGT C) (SEQ ID NO:71) were used. These were designed to amplify the complete transmembrane and cytoplasmic domains of KDR (~1.8 kbps) from the 5' RACE ready cDNA library (described above) using polymerase chain reaction (PCR) with pfu polymerase. PCR reaction conditions and the program were exactly the same as described above for s-KDR. Just as with the s-KDR sequence, the PCR product was purified using phenol chloroform extraction, treated with Taq polymerase and cloned into TOPOII vector from Invitrogen to give TOPO-CYTO.

Cloning of full-length KDR into pcDNA6 Vector

To create the full-length receptor, the extra-cellular domain and the cytoplasmic domain (with trans-membrane domain) were amplified by PCR separately from TOPO-sKDR and TOPO-CYTO respectively and ligated later to create the full-length receptor. An oligo with a Not1 site at the 5' end of the extracellular domain (A TAA GAA TGC GGC CGC AGG ATG GAG AGC AAG GTG CTG CTG G) (SEQ ID NO:72) and an oligo complimentary to the 3' end of the extracellular domain (TTC CAA GTT CGT CTT TTC CTG GGC ACC) (SEQ ID NO:73) were used to amplify by PCR the extracellular domain from TOPO-sKDR. Similarly, the 5' oligo (ATC ATT ATT CTA GTA GGC ACG GCG GTG) (SEQ ID NO:74) and the 3' oligo, with a *Not*1 site (A TAA GAA TGC GGC CGC AAC AGG AGG AGA GCT CAG TGT GGT C) (SEQ ID NO:75), were used to amplify by PCR the cytoplasmic domain of KDR (with transmembrane domain) from TOPO-CYTO. Both PCR products were digested with Not1 and ligated together to create the full-length receptor. The cDNA encoding the full-length receptor was purified on an agarose gel and ligated into the Not I site of the pcDNA6/V5-HisC vector. Purification of DNA and ligation was done as described earlier for psKDR. The ligation reaction was used to transform a culture of DH5α bacteria and a number of individual clones were analyzed for the presence and orientation of insert by restriction analysis of purified plasmid from each clone with EcoRI enzyme.

Cell Culture

293H cells were obtained from Invitrogen (cat. # 11631) and grown as monolayer culture in their recommended media plus 1ml/L pen/strep (Invitrogen, cat. # 15140-148). All the cells were grown in presence of antibiotic for everyday culture but were split into antibiotic free media for 16-20 hours prior to transfection.

Preparation of DNA for Transfection

*E. coli* bacteria DH5α containing pf-KDR was streaked onto LB with 50 μg/ml ampicillin (LB agar from US biologicals, cat. # 75851 and ampicillin from Sigma, cat. #A2804) plates from a glycerol stock and plates were left in a 37°C incubator to grow overnight. Next morning, a single colony was picked from the plate and grown in 3 ml of LB/ampicillin media (LB from US biologicals, cat. # US75852) at 37°C. After 8 hours, 100 μl of bacterial culture from the 3 ml tube was transferred to 250 ml of LB/ampicillin media for overnight incubation at 37°C. Bacteria were grown up with circular agitation in a 500 ml bottle (Beckman, cat. # 355605) at 220 rpm in a Lab-Line incubator shaker. The next day, the bacterial culture was processed using maxi-prep kit (QIAGEN, cat. # 12163). Generally, about 1mg of plasmid DNA (as quantitated by absorbance at 260 nm) was obtained from 250 ml of bacterial culture.

Transfection of 293H cells in 96 well plate

Transfection was done as recommended in the lipofectamine 2000 protocol (Invitrogen, cat# 11668-019) using a poly-D-lysine-coated 96 well plate. 320 ng of KDR DNA (pc-DNA6-fKDR)/per well in 0.1 ml was used for 96 well transfection. Transfection was done in serum-containing media, the transfection reagent mix was removed from cells after 6-8 hours and replaced with regular serum-containing medium. Transfection was done in black/clear 96-well plates (Becton Dickinson, cat. # 354640). The left half of the plate (48 wells) were mock-transfected (with no DNA) and the right half of the plate was transfected with KDR cDNA. The cells were 80-90% confluent at the time of transfection and completely confluent next day, at the time of the assay, otherwise the assay was aborted.

Preparation of M199 media

In order to prepare M199 media for the assay, one M199 medium packet (GIBCO, cat. # 31100-035), 20 ml of 1 mM HEPES (GIBCO, cat. #15630-080) and 2 gm of DIFCO Gelatin (DIFCO, cat. # 0143-15-1) were added to 950 ml of ddH$_2$O and the pH of the solution was adjusted to 7.4 by adding approximately 4 ml of 1N NaOH. After pH adjustment, the M199 media was warmed to 37°C in a water bath for 2 hours to dissolve the gelatin, then filter sterilized using 0.2 μm filters (Corning, cat. # 43109), and stored at 4°C to be used later in the assay.

Preparation of SoftLink soft release avidin-sepharose

SoftLink soft release avidin-sepharose was prepared by centrifuging the sepharose obtained from Promega (cat. # V2011) at 12,000 rpm for 2 minutes, washing twice with ice cold water (centrifuging in-between the washes) and resuspending the pellet in ice cold water to make a 50% slurry in ddH$_2$O. A fresh 50% slurry of avidin-sepharose was prepared for each experiment.

Preparation of peptide/neutravidin HRP solution

Biotinylated peptides SEQ ID NOS:294, 264, 277, 356, and the non-binding biotinylated control peptide were used to prepare 250 μM stock solutions in 50% DMSO and a 33 μM stock solution of neutravidin-HRP was prepared by dissolving 2 mg of neutravidin-HRP (Pierce, cat. # 31001) in 1 mL of ddH$_2$O (all polypeptides contained the JJ spacer). Peptide stock solutions were stored at -20°C, whereas the Neutravidin HRP stock solution was stored at -80°C. To prepare peptide/neutravidin-HRP complexes, 10 μl of 250 μM biotinylated peptide stock solution and 10 μl of 33 μM neutravidin-HRP were added to 1 ml of M199 medium. This mixture was incubated on a rotator at 4°C for 60 minutes, followed by addition of 50 μl of soft release avidin-sepharose (50% slurry in ddH$_2$0) to remove excess peptides and another incubation for 30 minutes on a rotator at 4°C. Finally, the soft release avidin-sepharose was pelleted by centrifuging at 12,000 rpm for 5 minutes at room temperature, and the resulting supernatant was used for the assays. Fresh peptide/neutravidin-HRP complexes were prepared for each experiment.

Preparation of peptide/neutravidin HRP dilutions for the assay

For saturation binding experiments, 120 μl, 60 μl, 20 μl, 10 μl, 8 μl, 6 μl, 4 μl, and 1 μl of peptide/neutravidin HRP complex were added to 1.2 ml aliquots of M199 medium to create dilutions with final concentrations of 33.33 nM, 16.65 nM, 5.55 nM, 2.78 nM, 1.67 nM, 1.11 nM and 0.28 nM complex, respectively.

Preparation of blocking solution for transfected 293H cells

Blocking solution was prepared by adding 20 ml of M199 medium to 10 mg of lyophilized unlabeled neutravidin (Pierce, cat. # 31000). Fresh blocking solution was used for each experiment.

Assay to detect the binding of peptide/neutravidin-HRP

24 hours after transfection, each well of the 293H cells was washed once with 100 $\mu$l of M199 medium and incubated with 80 $\mu$l of blocking solution at 37°C. After one hour, cells were washed twice with 100 $\mu$l of M199 media and incubated with 70 $\mu$l of peptide/neutravidin-HRP dilutions of control peptide, SEQ ID NO:5, SEQ ID NO:31, SEQ ID NO:17, and SEQ ID NO:66 for two and half hours at room temperature. Each dilution was added to three separate wells of mock as well as KDR-transfected 293H cells (two plates were used for each saturation binding experiment). After incubation at room temperature, plates were transferred to 4°C for another half-hour incubation. Subsequently, cells were washed 5 times with ice-cold M199 media and once with ice-cold PBS (in that order). After the final wash, 100 $\mu$l of ice cold TMB solution (KPL, cat. # 50-76-00) was added to each well and each plate was incubated for 30 minutes at 37°C in an air incubator. Finally, the HRP enzyme reaction was stopped by adding 50 $\mu$l of 1N phosphoric acid to each well, and binding was quantitated by measuring absorbance at 450 nm using a microplate reader (BioRad Model 3550).

Binding of peptide/neutravidin HRP to KDR-transfected cells

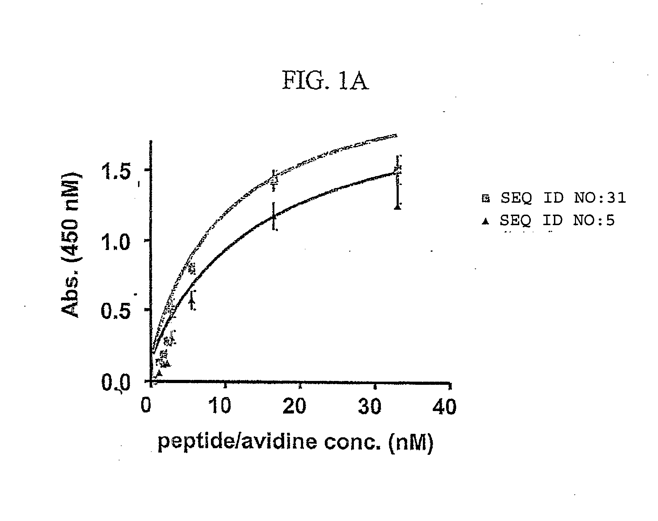

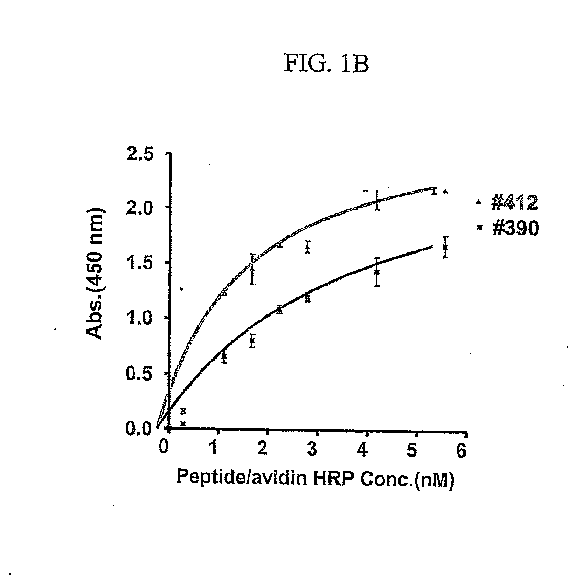

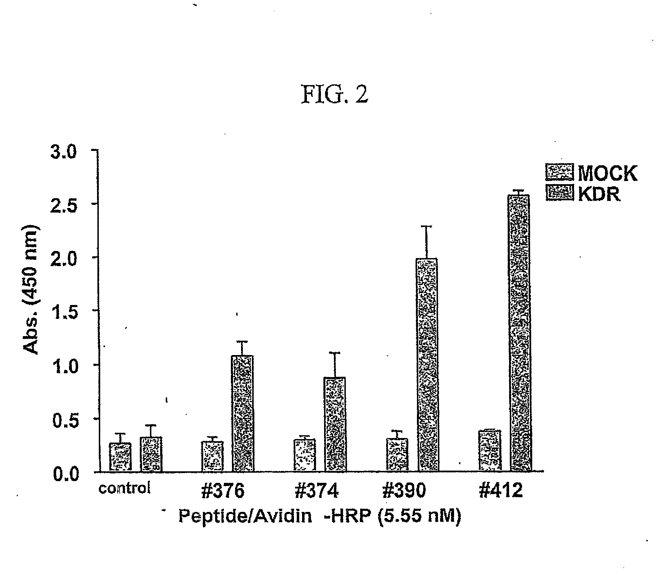

In this assay, complexes of control peptide, SEQ ID NO:5, SEQ ID NO:31, SEQ ID NO:17, and SEQ ID NO:66 peptides, each biotinylated with the JJ spacer and conjugated with neutravidin-HRP, were prepared as described above and tested for their ability to bind 293H cells that were transiently-transfected with KDR. During the peptide/neutravidin complex preparation, a 7.5-fold excess of biotinylated peptides over neutravidin-HRP was used to make sure that all four biotin binding sites on neutravidin were occupied. After complex formation, the excess of free biotinylated peptides was removed using soft release avidin-sepharose to avoid any competition between free biotinylated peptides and neutravidin HRP-complexed biotinylated peptides. The experiment was performed at several different concentrations of peptide/neutravidin-HRP, from 0.28 nM to 33.33 nM, to generate saturation binding curves for SEQ ID NO:5 and SEQ ID NO:31 (FIG. 1A) and 0.28 to 5.55 nM to generate saturation binding curve for SEQ ID NO:17 and SEQ ID NO:66 (FIG. 1B). In order to draw the saturation binding curve, the background binding to mock-transfected cells was subtracted from the binding to KDR-transfected cells for each distinct peptide/neutravidin HRP complex at each concentration tested. Therefore, absorbance on the Y-axis of FIG. 1 (below) is differential absorbance (KDR minus mock) and not the absolute absorbance. Analysis of the saturation binding data in FIG. 1 using Graph Pad Prism software (version 3.0) yielded a $K_D$ of 10.00 nM (+/-2.36) for the tetrameric SEQ ID NO:31, 14.87 nM (+/- 5.066) for the tetrameric SEQ ID NO:5, 4.031 nM (+/- 0.86) for the tetrameric SEQ ID NO:17, and 1.814 nM (+/- 0.27) for the tetrameric SEQ ID NO:66 peptide complexes. These binding constants are, as expected, lower than those measured by FP against the KDRFc construct for the related monodentate peptides SEQ ID NO:31 (69 nM), SEQ ID NO:5 (280 nM), SEQ ID NO:42 (51 nM), but similar to monodentate peptide SEQ ID NO:17 (3 nM). As expected, no saturation of binding for the control (non-binder) peptide/neutravidin HRP-complex was observed. The binding of peptide/neutravidin HRP complexes (FIG. 2) at a single concentration (5.55 nM) was plotted to demonstrate that a single concentration experiment can be used to differentiate between a KDR binding peptide (SEQ ID NOS:5, 31 and 17) from a non-binding peptide.

Experiment B

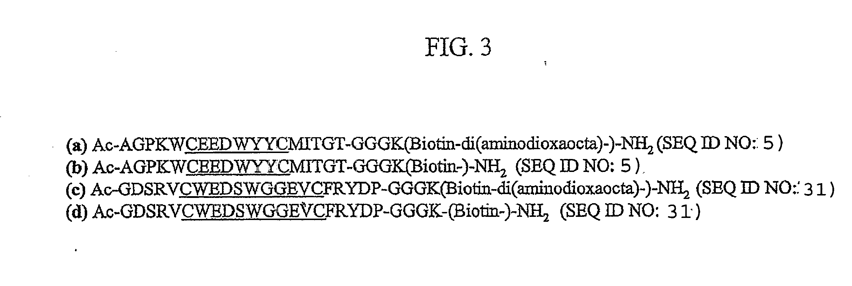

Experiment B was designed to look at the requirement of spacer (JJ, Table 3) between the KDR binding sequences (SEQ ID NOS:31 and 5) and biotin. In this experiment, biotinylated peptides with and without spacer JJ were tested (*e.g.*, biotinylated SEQ ID NO:5 with the JJ spacer, biotinylated SEQ ID NO:5 without the JJ spacer, SEQ ID NO:31 with a spacer, and biotinylated SEQ ID NO:31 without the spacer), and a non-KDR binding, biotinylated control peptide (with and without spacer, prepared as set forth above) was used as a control. The peptide structure of all the KDR-binding sequences tested in this experiment is shown in FIG. 3.

This experiment was performed as set forth in Experiment A described above, except that it was only done at a single concentration of 2.78 nM.

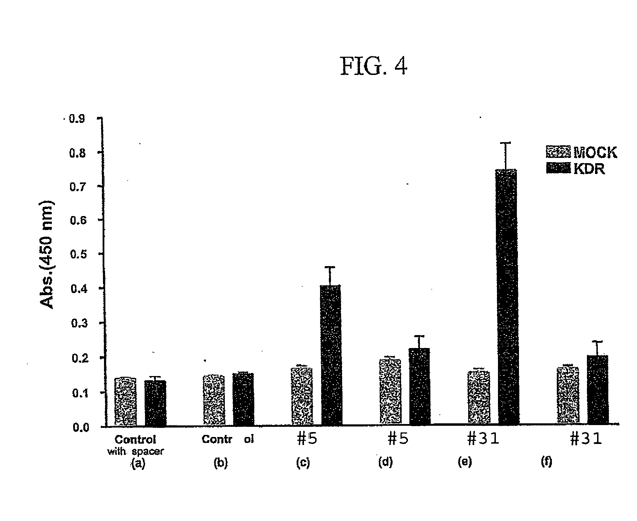

*Results:* It is evident from the results shown in the FIG. 4 that the spacer is enhances binding of SEQ ID NO:31 and SEQ ID NO:5. The spacer between the binding sequence and biotin can be helpful in enhancing binding to target molecule by multiple mechanisms. First, it could help reduce the steric hindrance between four biotinylated peptide after their binding to single avidin molecule. Second, it could provide extra length necessary to reach multiple binding sites available on a single cell.

Experiment C

Experiment C was designed to look at the serum effect on the binding of SEQ ID NOS: 31, 5, 17 and 66. In this procedure, biotinylated peptide/avidin HRP complexes of SEQ ID NOS:31, 5, 17 and 66 were tested in M199 media (as described above in Experiment A) with and without 40% rat serum. This experiment was performed as described for Experiment A except that it was only done at single concentration of 6.66 nM for SEQ ID NOS: 31 and 5, 3.33 nM for SEQ ID NO:17 and 2.22 nM for SEQ ID NO:66. Each of the polypeptides were biotinylated and had the JJ spacer.

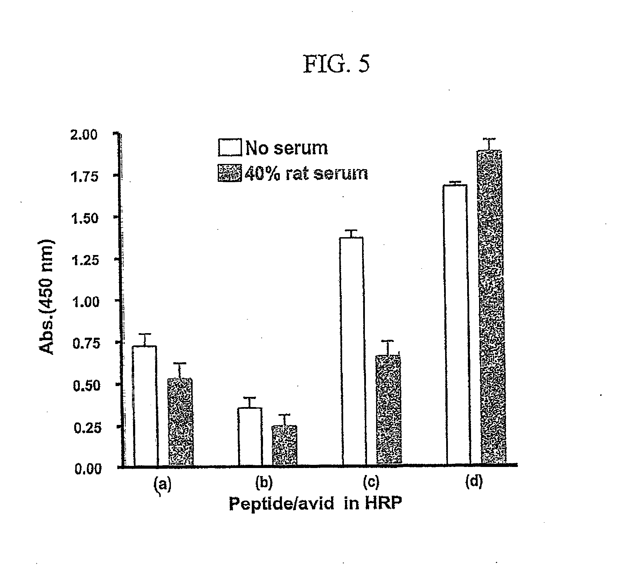

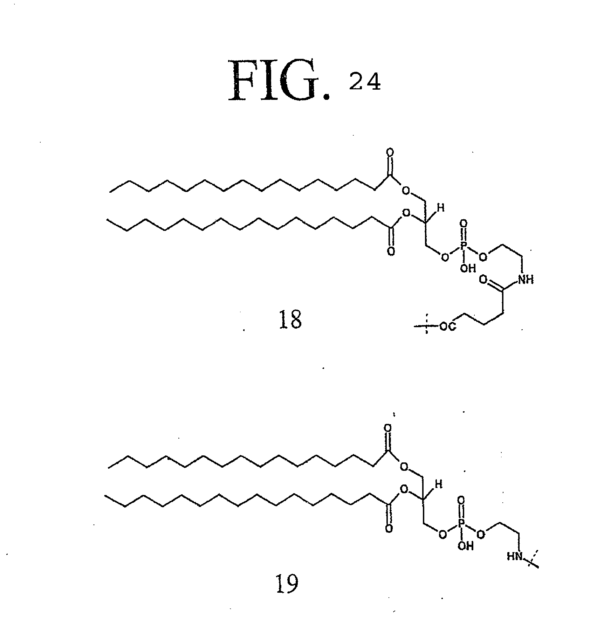

*Results:* Results in FIG. 5 indicate that binding of SEQ ID NO:5, SEQ ID NO:31, and SEQ ID NO:66 was not significantly affected by 40% rat serum, whereas binding of SEQ ID NO:17 was more than 50% lower in presence of 40% rat serum. More than an 80% drop in the binding of Tc-labeled SEQ ID NO:17 with Tc-chelate was observed in the presence of 40% rat serum (FIG. 24). Since the serum effect on the binding of Tc-labeled SEQ ID NO:17 is mimicked in the avidin HRP assay disclosed herein, this assay may be used to rapidly evaluate the serum effect on the binding of peptide(s) to KDR.

Experiment D

Experiment D was designed to evaluate the binding of tetrameric complexes of KDR and VEGF/KDR complex-binding polypeptides SEQ ID NO:31 and SEQ ID NO:5, particularly where the constructs included at least two KDR binding polypeptides. The KDR binding peptides and control binding peptide were prepared as described above. This experiment was performed using the protocol set forth for Experiment A, except the procedures set forth below were unique to this experiment.

*Preparation of Peptide/Neutravidin Solutions:* 250 $\mu$M stock solutions of biotinylated peptides SEQ ID NOs:5, 31 and control peptide were prepared in 50% DMSO and a 33 $\mu$M stock solution of Neutravidin HRP was prepared by dissolving 2 mg of Neutravidin HRP (Pierce, cat. # 31001) in 1 mL of ddH$_2$O. Peptide stock solutions were stored at -20C, whereas the Neutravidin HRP stock solution was stored at -80C. The sequences of the biotinylated peptides are shown above. To prepare peptide/neutravidin HRP complexes, a total 5.36 $\mu$L of 250 $\mu$M biotinylated peptide stock solution (or a mixture of peptide solutions, to give peptide molecules four times the number of avidin HRP molecules) and 10 $\mu$L of 33 $\mu$M Neutravidin HRP were added to 1 mL of M199 medium. This mixture was incubated on a rotator at 4C for 60 minutes, followed by addition of 50 $\mu$L of soft release avidin-sepharose (50% slurry in ddH$_2$0) to remove excess peptides and another incubation for 30 minutes on a rotator at 4C. Finally, the soft release avidin-sepharose was pelleted by centrifuging at 12,000 rpm for 5 minutes at room temperature, and the resulting supernatant was used for the assays. Fresh peptide/neutravidin HRP complexes were prepared for each experiment.

*Assay to Detect the Binding of Peptide/Neutravidin HRP:* 24 hours after transfection, each well of the 293H cells was washed once with 100 $\mu$L of M199 medium and incubated with 80 $\mu$L of blocking solution at 37C. After one hour, cells were washed twice with 100 $\mu$L of M199 media and incubated with 70 $\mu$L of 3.33 nM peptide (or peptide mix)/neutravidin HRP solutions (prepared by adding 10 $\mu$L of stock prepared earlier to 1 mL of M199 media) for two and half hours at room temperature. Each dilution was added to three separate wells of mock as well as KDR-transfected 293H cells. After incubation at room temperature, plates were transferred to 4C for another half-hour incubation. Subsequently, cells were washed five times with ice-cold M199 media and once with ice-cold PBS (in that order). After the final wash, 100 $\mu$L of ice cold TMB solution (KPL, Gaithersburg, MD) was added to each well and each plate was incubated for 30 minutes at 37C in an air incubator. Finally, the HRP enzyme reaction was stopped by adding 50 $\mu$L of 1N phosphoric acid to each well, and binding was quantitated by measuring absorbance at 450 nm using a microplate reader (BioRad Model 3550).

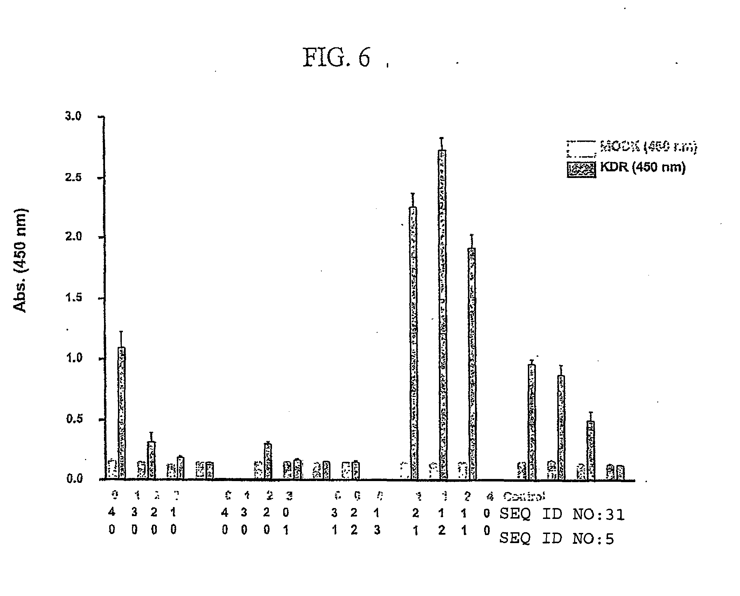

*Results:* This experiment establishes that SEQ ID NO:31 and SEQ ID NO:5 bind to KDR in multimeric fashion, and cooperate with each other for binding to KDR in 293H transfected cells. A biotinylated control peptide that does not bind to KDR was used. As expected, a tetrameric complex of the control peptide with avidin-HRP did not show enhanced binding to KDR-transfected cells. Tetrameric complexes of SEQ ID NO:31 and SEQ ID NO:5 bound to KDR-transfected cells significantly better than to mock-transfected cells (see FIG. 6). SEQ ID NO:31 tetramers, however, bound much better than SEQ ID NO:5 tetramers. If the control peptide was added to the peptide mixture used to form the tetrameric complexes, the binding to the KDR-transfected cells decreased. The ratio of specific binding of tetramer to monomer, dimer and trimer was calculated by dividing the specific binding (obtained by subtracting the binding to mock transfected cells from KDR transfected cells) of tetramer, trimer and dimer with that of monomer. Results indicate that there is co-operative effect of multimerization of SEQ ID NOS:5, 31 and 67 on the binding to KDR-transfected cells.

|  | Tetramer | Trimer | Dimer |
|---|---|---|---|
| SEQ ID NO: 5 | 45.4 | 5 | 4.3 |
| SEQ ID NO:31* | 38.6 | 7.1 | 2.7 |

(continued)

| | Tetramer | Trimer | Dimer |
|---|---|---|---|
| SEQ ID NO:17 | 1 | 1.1 | 1.1 |
| SEQ ID NO:66 | 16 | 5.7 | 2.3 |

*monomeric peptide binding at 2.22 nM was zero, therefore ratios were calculated using binding at 5.55 nM.

A mixture of 25% non-binding control peptide with 75% SEQ ID NO:5 did not bind significantly over background to KDR-transfected cells, indicating that multivalent binding is critical for the SEQ ID NO:5/avidn-HRP complex to remain bound to KDR throughout the assay. This phenomenon also held true for SEQ ID NO:31, where substituting 50% of the peptide with control peptide in the tetrameric complex abolished almost all binding to KDR on the transfected cells.

Surprisingly, a peptide mixture composed of 50% control peptide with 25% SEQ ID NO:31 and 25% SEQ ID NO:5 bound quite well to KDR-transfected cells relative to mock-transfected cells, indicating that there is a great advantage to targeting two sites or epitopes on the same target molecule. Furthermore, it was noted that tetrameric complexes containing different ratios of SEQ ID NO:31 and SEQ ID NO:5 (3:1, 2:2, and 1:3) all bound much better to KDR-transfected cells than pure tetramers of either peptide, in agreement with the idea that targeting two distinct sites on a single target molecule is superior to multimeric binding to a single site. This may be because multimeric binding to a single target requires that the multimeric binding entity span two or more separate target molecules which are close enough together for it to bind them simultaneously, whereas a multimeric binder which can bind two or more distinct sites on a single target molecule does not depend on finding another target molecule within its reach to achieve multimeric binding.

Experiment E

Experiment E was designed to confirm that SEQ ID NO:31 and SEQ ID NO:5 bind to distinct sites (epitopes) on KDR. If these peptides bind to the same site on KDR, then they should be able to compete with each other; however, if they bind to different sites they should not compete. This experiment was performed using a single concentration of SEQ ID NO:5/avidin HRP (3.33 nM) solution in each well and adding a varying concentration (0-2.5 $\mu$M) of biotinylated control peptide with spacer, SEQ ID NO:5 and SEQ ID NO:31, none of which were complexed with avidin.

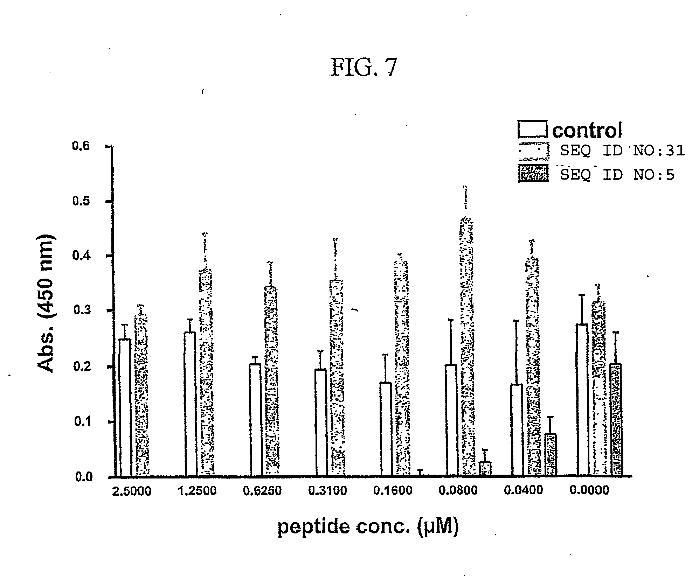

*Results:* It is evident from FIG. 7 that SEQ ID NO:5 does compete with SEQ ID NO:5/avidin HRP solution for binding to KDR transfected cells whereas control peptide and SEQ ID NO:31 do not compete with SEQ ID NO:5/avidin HRP solution for binding to KDR transfected cells. Thus, SEQ ID NO:5 and SEQ ID NO:31 bind to distinct and complementary sites on KDR.

*Example 6: Binding of Analogs of a KDR-binding Peptide to KDR-expressing Cells*

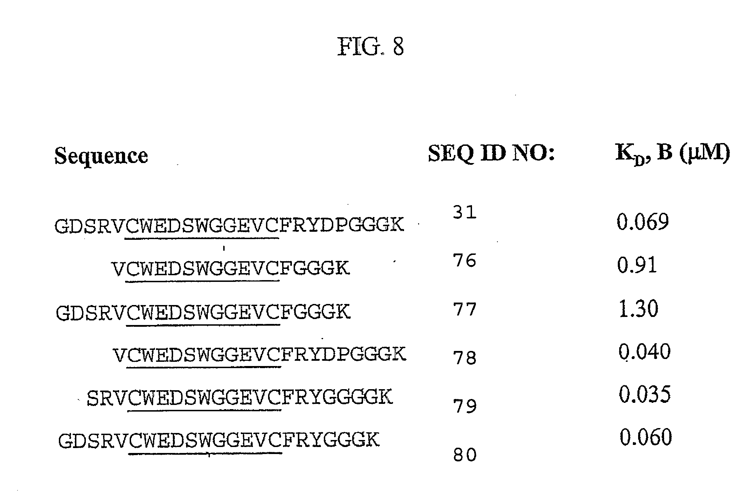

N-terminal and C-terminal truncations of a KDR binding polypeptide were made and the truncated polypeptides tested for binding to KDR-expressing cells. The synthesized polypeptides are shown in FIG. 8. Binding of the polypeptides to KDR-expressing cells was determined following the procedures of Example 3.

All of the peptides were N-terminally acetylated and fluoresceinated for determining apparent $K_D$ according to the method described above (Example 3). The results indicate that, for the SEQ ID NO:31 (FIG. 8) polypeptide, the C-terminal residues outside the disulfide-constrained loop contribute to KDR binding.

*Example 7: Bead-binding Assay to Confirm Ability of Peptides Identified by Phage Display to Bind KDR-expressing Cells*

The following procedures were performed to assess the ability of KDR-binding peptides to bind to KDR-expressing cells. In this procedure, KDR-binding peptides containing SEQ ID NOS:5, and 49 were conjugated to fluorescent beads and their ability to bind to KDR-expressing 293H cells was assessed. The experiments show these peptides can be used to bind particles such as beads to KDR-expressing sites. The results indicate that the binding of both KDR binding sequences improved with the addition of a spacer.

Protocol

*Biotinylation of an anti-KDR antibody:* Anti-KDR from Sigma (V-9134), as ascites fluid, was biotinylated using a kit from Molecular Probes (F-6347) according to the manufacturer's instructions.

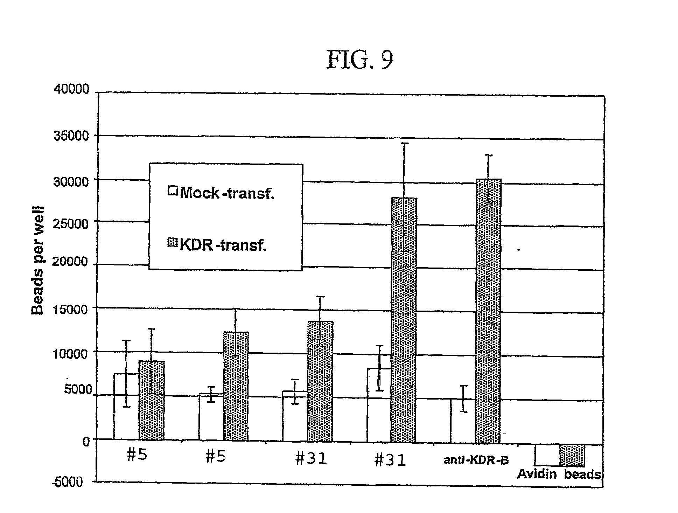

*Preparation of peptide-conjugated fluorescent beads:* 0.1 mL of a 0.2 mM stock solution of each biotinylated peptide (prepared as set forth above, in 50% DMSO) was incubated with 0.1 ml of Neutravidin-coated red fluorescent microspheres (2 micron diameter, custom-ordered from Molecular Probes) and 0.2 ml of 50mM MES (Sigma M-8250) buffer, pH 6.0 for 1 hour at room temperature on a rotator. As a positive control, biotinylated anti-KDR antibody was incubated with the Neutravidin-coated beads as above, except that 0.03 mg of the biotinylated antibody preparation in PBS (Gibco #14190-136) was used instead of peptide solution. Beads can be stored at 4°C until needed for up to 1 week.
*Binding Assay:* From the above bead preparations, 0.12 mL was spun for 10 minutes at 2000 rpm in a microcentrifuge at room temperature. The supernatant was removed and 0.06 ml of MES pH 6.0 was added. Each bead solution was then vortexed and sonicated in a water bath 15 min. To 1.47 ml of DMEM, high glucose (GIBCO #11965-084) with 1X MEM Non-Essential Amino Acids Solution (NEAA) (GIBCO 11140-050) and 40% FBS (Hyclone SH30070.02) 0.03 ml of the sonicated bead preparations was added. 96-well plates seeded with 293H cells which have been mock-transfected in columns 1 to 6, and KDR-transfected in columns 7 to 12 (as in Example 5), were drained and washed once with DMEM, high glucose with 1X NEAA and 40% FBS. To each well 0.1 ml of bead solution was added, six wells per bead preparation. After incubating at room temperature for 30 minutes, the wells were drained by inverting the plates and washed four times with 0.1 ml PBS with $Ca^{++}Mg^{++}$ (GIBCO #14040-117) with shaking at room temperature for 5 minutes each wash. After draining, 0.1 ml of PBS was added per well. The plates were then read on a Packard FluoroCount fluorometer at excitation 550nm/emission 620nm. Unconjugated neutravidin beads were used as a negative control while beads conjugated with a biotinylated anti-KDR antibody were used as the positive control for the assay.
To calculate the number of beads bound per well, a standard curve with increasing numbers of the same fluorescent beads was included in each assay plate. The standard curve was used to calculate the number of beads bound per well based on the fluorescence intensity of each well.
*Results:* The positive control beads with anti-KDR attached clearly bound preferentially to the KDR-expressing cells while avidin beads with nothing attached did not bind to either cell type (FIG. 9). Biotinylated SEQ ID NO:5 beads did not bind to the KDR-transfected cells significantly more than to mock-transfected cells, but adding a hydrophilic spacer between the peptide moiety and the biotin group (biotinylated SEQ ID NO:5 with a JJ spacer beads) enhanced binding to KDR cells without increasing the binding to mock-transfected cells. Biotinylated SEQ ID NO:31 beads showed greater binding to KDR-transfected cells, and adding a hydrophilic spacer between the peptide portion and the biotin of the molecule (biotinylated SEQ ID NO:31 with the JJ spacer) significantly improved the specific binding to KDR in the transfected cells. Thus, the peptide sequences of both SEQ ID NO:5 and SEQ ID NO:31 can be used to bind particles such as beads to KDR expressing sites. Addition of a hydrophilic spacer between the peptide and the group used for attachment to the particle should routinely be tested with new targeting molecules as it improved the binding for both of the peptides evaluated here.

*Example 8: Competition of KDR binding peptides and $^{125}$I-labeled VEGF for binding to KDR-transfected 293H cells*

KDR-binding polypeptides were next assessed for their ability to compete with $^{125}$I-labelled VEGF for binding to KDR expressed by transfected 293H cells. The results indicate that KDR-binding polypeptide SEQ ID NO:4 (Ac-AGDSWCSTEYTYCEMIGTGGGK-NH$_2$) did not compete significantly with $^{125}$I-labeled VEGF, and SEQ ID NOS:31, 5, and SEQ ID NO:17 competed very well with $^{125}$I-labeled VEGF, inhibiting 96.29±2.97% and 104.48±2.074% of $^{125}$I-labeled VEGF binding.
*Transfection of 293H cells:* 293H cells were transfected using the protocol described in Example 5. Transfection was done in black/clear 96-well plates (Becton Dickinson, cat. # 354640). The left half of the plates (48 wells) were mock-transfected (with no DNA) and the right half of the plates were transfected with KDR cDNA. The cells were 80-90% confluent at the time of transfection and completely confluent the next day, at the time of the assay; otherwise the assay was aborted.

*Preparation of M199 media:* M199 medium was prepared as described in Example 5.

*Preparation of peptide solutions:* 3 mM stock solutions of peptides SEQ ID NO:31, SEQ ID NO:4, SEQ ID NO:5 and SEQ ID NO:17 were prepared as described above in 50% DMSO.

*Preparation of $^{125}$I-labeled VEGF solution for the assay:* 25 μCi of lyophilized $^{125}$I-labeled VEGF (Amersham, cat. # IM274) was reconstituted with 250 μl of ddH$_2$O to create a stock solution, which was stored at -80C for later use.

For each assay, a 300 pM solution of $^{121}$I-labeled VEGF was made fresh by diluting the above stock solution in M199 medium. The concentration of $^{125}$I-labeled VEGF was calculated daily based on the specific activity of the material on that day.

*Preparation of 30 μM and 0.3 μM peptide solution in 300 pM $^{125}$I-labeled VEGF:* For each 96 well plate, 10 ml of 300 pM $^{125}$I-labeled VEGF in M199 medium was prepared at 4°C. Each peptide solution (3 mM, prepared as described above) was diluted 1:100 and 1:10000 in 300 μl of M199 media with 300 pM $^{125}$I-labeled VEGF to prepare 30 μM and 0.3 μM peptide solutions containing 300 μM of $^{125}$I-labeled VEGF. Once prepared, the solutions were kept on ice until ready to use. The dilution of peptides in M199 media containing 300 μM $^{125}$I-labelled VEGF was done freshly for each experiment.

*Assay to detect competition with $^{125}$I-labeled VEGF in 293H cells:* Cells were used 24 hours after transfection, and to prepare the cells for the assay, they were washed 3 times with room temperature M199 medium and placed in the refrigerator. After 15 minutes, the M199 medium was removed from the plate and replaced with 75 μl of 300 pM $^{125}$I-labeled VEGF in M199 medium (prepared as above) with the polypeptides. Each dilution was added to three separate wells of mock and KDR transfected cells. After incubating at 4°C for 2 hours, the plates were washed 5 times with cold binding buffer, gently blotted dry and checked under a microscope for cell loss. 100 μl of solubilizing solution (2% Triton X-100, 10% Glycerol, 0.1% BSA) was added to each well and the plates were incubated at room temperature for 30 minutes. The solubilizing solution in each well was mixed by pipeting up and down, and transferred to 1.2 ml tubes. Each well was washed twice with 100 μl of solubilizing solution and the washes were added to the corresponding 1.2 ml tube. Each 1.2 ml tube was then transferred to a 15.7 × 100 cm tube to be counted in an LKB Gamma Counter using program 54 ($^{125}$I window for 1 minute).

*Competition of peptides with $^{125}$I-labeled VEGF in 293H cells:* The ability of KDR-binding peptides SEQ ID NO:31, SEQ ID NO:4, SEQ ID NO:5 and SEQ ID NO:17 to specifically block $^{125}$I-labeled VEGF binding to KDR was assessed in mock-transfected and KDR-transfected cells. SEQ ID NO:4 was used in the assay as a negative control, as it exhibited poor binding to KDR in the FP assays described herein and would therefore not be expected to displace or compete with VEGF. To calculate the specific binding to KDR, the binding of $^{125}$I-labeled VEGF to mock-transfected cells was subtracted from KDR-transfected cells. Therefore, the binding of $^{125}$I-labeled VEGF to sites other than KDR (which may or may not be present in 293H cells) is not included when calculating the inhibition of $^{125}$I-labeled VEGF binding to 293H cells by KDR-binding peptides. Percentage inhibition was calculated using formula [(Y1-Y2)*100/Y1], where Y1 is specific binding to KDR-transfected 293H cells in the absence peptides, and Y2 is specific binding to KDR-transfected 293H cells in the presence of peptides or DMSO. Specific binding to KDR-transfected 293H cells was calculated by subtracting binding to mock-transfected 293H cells from binding to KDR-transfected 293H cells.

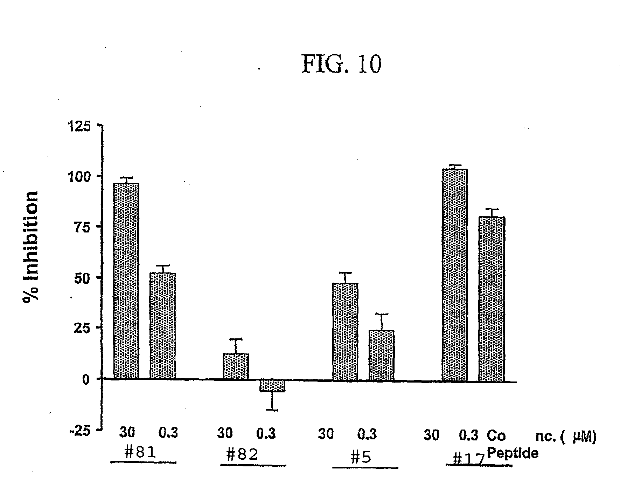

As shown in FIG. 10, in 293 cells, SEQ ID NO:4, which due to its relatively high $K_d$ (>2 μM) was used as a negative control, did not compete significantly with $^{125}$I-labeled VEGF, 12.69+7.18% at 30 μM and -5.45+9.37% at 0.3 μM (FIG. 10). At the same time, SEQ ID NOS:294 and 277 competed very well with $^{125}$I-labeled VEGF, inhibiting 96.29±2.97% and 104.48±2.074% of $^{125}$I-labeled VEGF binding at 30 μM and 52.27±3.78% and 80.96±3.8% at 0.3 μM (FIG. 10) respectively. The percentage inhibition with SEQ ID NO:5 was 47.95±5.09% of $^{125}$I-labelled VEGF binding at 30μM and 24.41±8.43% at 0.3 μM (FIG. 10). Thus, the three strongly KDR-binding polypeptides did compete with VEGF, and their potency increased with their binding affinity. This assay will be useful for identifying peptides that bind tightly to KDR but do not compete with VEGF, a feature that may be useful for imaging KDR in tumors, where there is frequently a high local concentration of VEGF that would otherwise block the binding of KDR-targeting molecules.

*Example 9: Inhibition of VEGF-induced KDR receptor activation by peptides identified by phage display*

The ability of KDR-binding peptides identified by phage display to inhibit VEGF induced activation (phosphorylation) of KDR was assessed using the following assay. A number of peptides of the invention were shown to inhibit activation of KDR in monomeric and/or tetrameric constructs. As discussed *supra,* peptides that inhibit activation of KDR may be useful as anti-angiogenic agents.

Protocol

Human umbilical vein endothelial cells (HUVECs) (Biowhittaker Catalog #CC-2519) were obtained frozen on dry ice and stored in liquid nitrogen until thawing. These cells were thawed, passaged, and maintained as described by the manufacturer in EGM-MV medium (Biowhittaker Catalog #CC-3125). Cells seeded into 100 mm dishes were allowed to become confluent, then cultured overnight in basal EBM medium lacking serum (Biowhittaker Catalog #CC-3121). The next morning, the medium in the dishes was replaced with 10 ml fresh EBM medium at 37C containing either no additives (negative control), 5 ng/ml VEGF (Calbiochem Catalog #676472 or Peprotech Catalog #100-20) (positive control), or 5 ng/ml VEGF plus the indicated concentration of the KDR-binding peptide (prepared as described above). In some cases, a neutralizing anti-KDR antibody (Catalog #AF357, R&D Systems) was used as a positive control inhibitor of activation. In such cases, the antibody was pre-incubated with the test cells for 30 min at 37°C prior to the addition of fresh medium containing both VEGF and the antibody. After incubating the dishes 5 min. in a 37°C tissue culture incubator they were washed three times with ice-cold D-PBS containing calcium and magnesium and placed on ice without removing the last 10 ml of Delbecco's phosphate buffered saline (D-PBS). The first dish of a set was drained and 0.5 ml of Triton lysis buffer was added (20 mM Tris base pH 8.0, 137 mM NaCl, 10% glycerol, 1% Triton X-100, 2 mM EDTA (ethylenediaminetetraacetic acid), 1 mM PMSF(phenylmethylsulfonylfluoride), 1 mM sodium orthovanadate, 100 mM NaF, 50 mM sodium pyrophosphate, 10 μg/ml leupeptin, 10 μg/ml aprotinin). The cells were quickly scraped into the lysis buffer using a cell scraper (Falcon, Cat No. #353087), dispersed by pipeting up and down briefly, and the resulting lysate was transferred to the second drained dish of the pair. Another 0.5 ml of lysis buffer was used to rinse out the first dish then transferred to the second dish, which was then also scraped and dispersed. The pooled lysate from the two dishes was transferred to a 1.5 ml Eppindorf tube. The above procedure was repeated for each of the controls and test samples (KDR-binding peptides), one at a time. The lysates were stored on ice until all the samples had been processed. At this point samples were either stored at -70° C or processed to the end of the assay without interruption.

The lysates, freshly prepared or frozen and thawed, were precleared by adding 20 μl of protein A-sepharose beads (Sigma 3391, preswollen in D-PBS, washed three times with a large excess of D-PBS, and reconstituted with 6 ml D-PBS to generate a 50% slurry) and rocking at 4° C for 30 min. The beads were pelleted by centrifugation for 2 min. in a Picofuge (Stratgene, Catalog #400550) at 2000 ×g and the supernatants transferred to new 1.5 ml tubes. Twenty μg of anti-Flk-1 antibody (Santa Cruz Biotechnology, Catalog #sc-504) was added to each tube, and the tubes were incubated overnight (16-18 hr.) at 4C on a rotator to immunoprecipitate KDR. The next day 40 μl of protein A-sepharose beads were added to the tubes which were then incubated 4C for 1 hr. on a rotator. The beads in each tube were subsequently washed three times by centrifuging for 2 min. in a Picofuge, discarding the supernatant, and dispersing the beads in 1 ml freshly added TBST buffer (20 mM Tris base pH 7.5, 137 mM NaCl, and 0.1% Tween 20). After centrifuging and removing the liquid from the last wash, 40 μl of Laemmli SDS-PAGE sample buffer (Bio-Rad, Catalog #161-0737) was added to each tube and the tubes were capped and boiled for 5 min. After cooling, the beads in each tube were pelleted by centrifuging and the supernatants containing the immunoprecipitated KDR were transferred to new tubes and used immediately or frozen and stored at -70C for later analysis.

Detection of phosphorylated KDR as well as total KDR in the immunoprecipitates was carried out by immunoblot analysis. Half (20 μL) of each immunoprecipitate was resolved on a 7.5% precast Ready Gel (Bio-Rad, Catalog #161-1154) by SDS-PAGE according to the method of Laemmli (Nature, 227:680-685 (1970)).

Using a Bio-Rad mini-Protean 3 apparatus (Catalog #165-3302). The resolved proteins in each gel were electroblotted to a PVDF membrane (Bio-Rad, Cat. No. 162-0174) in a Bio-Rad mini Trans-Blot cell (Catalog #170-3930) in CAPS buffer (10 mM CAPS, Sigma Catalog #C-6070, 1% ACS grade methanol, pH 11.0) for 2 hr. at 140 mA according to the method of Matsudaira (J. Biol. Chem., 262:10035-10038 (1987)). Blots were blocked at room temperature in 5% Blotto-TBS (Pierce Catalog #37530) pre-warmed to 37° C for 2 hr. The blots were first probed with an anti-phosphotyrosine antibody (Transduction Labs, Catalog #P11120), diluted 1:200 in 5% Blotto-TBS with 0.1% Tween 20 added for 2 hr. at room temp. The unbound antibody was removed by washing the blots four times with D-PBS containing 0.1% Tween 20 (D-PBST), 5 min. per wash. Subsequently, blots were probed with an HRP-conjugated sheep anti-mouse antibody (Amersham Biosciences Catalog #NA931) diluted 1:25,000 in 5% Blotto-TBS with 0.1% Tween 20 added for 1 hr. at room temp., and washed four times with D-PBST. Finally, the blots were incubated with 2 ml of a chemiluminescent substrate (ECL Plus, Amersham Catalog #RPN2132) spread on top for 2 min., drip-drained well, placed in plastic sheet protector (C-Line Products, Catalog #62038), and exposed to X-ray film (Kodak BioMax ML, Cat No. 1139435) for varying lengths of time to achieve optimal contrast.

To confirm that similar amounts of KDR were compared in the assay, the blots were stripped by incubating for 30 min. at 37° C in TBST with its pH adjusted to 2.4 with HCl, blocked for 1 hr. at room temp. with 5% Blotto-TBS with 0.1% Tween 20 (Blotto-TBST), and reprobed with an anti-Flk-1 polyclonal antibody (Catalog #sc-315 from Santa Cruz Biotech), 1:200 in 5% Blotto-TBST with 1% normal goat serum (Life Tech Catalog #16210064) for 2 hr. at room temp. The unbound antibody was removed by washing the blots four times with D-PBST, 5 min. per wash. Subsequently, the blots were probed with an HRP-conjugated donkey anti-rabbit secondary antibody (Amersham Biosciences Catalog #NA934) diluted 1:10,000 in 5% Blotto-TBST for 1 hr. at room temp., and washed four times with D-PBST. Finally, the blots were incubated with 2 ml of chemiluminescent substrate and exposed to X-ray film as described above.

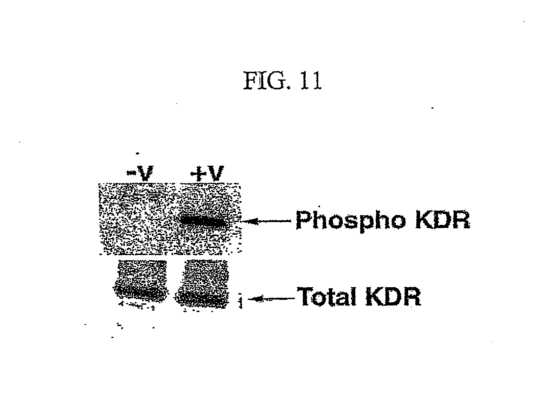

*Results:* Immunoblots of KDR immunoprecipitates prepared from HUVECs with and without prior VEGF stimulation demonstrated that activated (phosphorylated) KDR could be detected when the HUVECs were stimulated with VEGF. An anti-phosphotyrosine antibody (PY-20) detected no phosphorylated proteins close to the migration position of KDR from unstimulated HUVECs on the blots, but after five minutes of VEGF stimulation, an intense band was consistently observed at the expected location (FIG. 11, upper panel). When the blots were stripped of bound antibodies by incubation in acidic solution then reprobed with an anti-KDR antibody (sc-315), the identity of the phosphorylated protein band was confirmed to be KDR. Moreover, it was observed that immunoprecipitates from unstimulated HUVECs contained about as much total KDR as immunoprecipates from VEGF-stimulation HUVECs (FIG. 11, lower panel).

The foregoing results indicate that the phosphorylated KDR detected was formed from pre-existing KDR through autophosphorylation of KDR dimers resulting from VEGF binding, as five minutes is not enough time to synthesize and process a large glycosylated cell-surface receptor such as KDR.

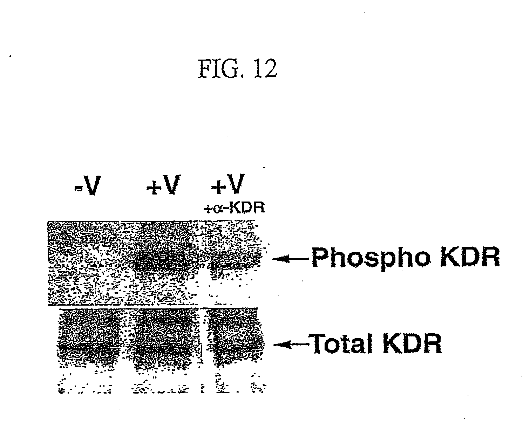

The ability of this assay to detect agents capable of blocking the VEGF activation of KDR was assessed by adding a series of compounds to HUVECs in combination with VEGF and measuring KDR phosphorylation with the immunoblot assay described above. As negative and positive controls, immunoprecipitates from unstimulated HUVECs and from HUVECs stimulated with VEGF in the absence of any test compounds were also tested in every assay. When a neutralizing anti-KDR antibody (Catalog #AF-357 from R&D Systems) was combined with the VEGF, the extent of KDR phosphorylation was greatly reduced (FIG. 12, upper panel), indicating that the antibody was able to interfere with the ability of VEGF to bind to and activate KDR. This result was expected since the ability of the antibody to block VEGF-induced DNA synthesis is part of the manufacturer's quality control testing of the antibody. Re-probing the blot with an anti-KDR antibody (FIG. 12, lower panel) indicated that slightly less total KDR was present in the VEGF+antibody-treated lane (+V+α-KDR) relative to the VEGF-only-treated lane (+V), but the difference was not great enough to account for the much lower abundance of phosphorylated KDR in the antibody-treated lane.

Repeating the foregoing assay, the following polypeptides demonstrated at least a 50% inhibition of VEGF-induced KDR phosphorylation at 10 μM:

Ac-AGWIE<u>CYHPDGIC</u>YHFGTGGGK-NH$_2$ (SEQ ID NO:9)
Ac-GDSRV<u>CWEDSWGGEVC</u>FRYDPGGGK-NH$_2$ (SEQ ID NO:31)
Ac-AQEPEGYAYWEVITLYHEEDGDGGK-NH$_2$ (SEQ ID NO:36)
Ac-AQAFPRFGGDDYWIQQYLRYTDGGK-NH$_2$ (SEQ ID NO:37)
Ac-AQGDYVYWEIIELTGATDHTPPGGK-NH$_2$ (SEQ ID NO:38)

SEQ ID NOS: 9 and 31 were the most potent compounds in the assay, producing at least a 50% inhibition of VEGF-induced KDR phosphorylation at 1 μM.

The following peptides were tested in the assay and did not produce significant inhibition of KDR activation at 10 μM:

Ac-AGPK(ivDde)W<u>CEEDWYYC</u>MITGTGGGK-NH$_2$ (SEQ ID NO:5)
Ac-AQRGDYQEQYWHQQLVEQLK(ivDde)LLGGGK-NH$_2$ (SEQ ID NO:45)

In addition, tetrameric complexes of biotinylated derivatives of SEQ ID NOS:31 and 17 (prepared as described above) produced at least a 50% inhibition of VEGF-induced KDR phosphorylation at 10 nM.

*Example 11: Preparation of peptides and dimeric peptide construction*

The following methods were used for the preparation of individual peptides and dimeric peptide constructs described in the following Examples (11-15).

Automated Peptide Synthesis

Peptide synthesis was carried out on a ABI-433A Synthesizer (Applied Biosystems Inc., Foster City, CA) on a 0.25 mmol scale using the FastMoc protocol. In each cycle of this protocol preactivation was accomplished by dissolution of 1.0 mmol of the requisite dry N$^α$-Fmoc side-chain protected amino acid in a cartridge with a solution of 0.9 mmol of HBTU, 2 mmol of DIEA, and 0.9 mmol of HOBt in a DMF-NMP mixture. The peptides were assembled on NovaSyn TGR (Rink amide) resin (substitution level 0.2 mmol/g). Coupling was conducted for 21 min. Fmoc deprotection was carried out with 20% piperidine in NMP. At the end of the last cycle, the N-terminal Fmoc group was removed and the fully protected resin-bound peptide was acetylated using acetic anhydride / DIEA / HOBt / NMP.

Cleavage, Side-chain Deprotection and Isolation of Crude Peptides

Cleavage of the peptides from the resin and side-chain deprotection was accomplished using Reagent B for 4.5h at ambient temperature. The cleavage solutions were collected and the resins were washed with an additional aliquot of Reagant B. The combined solutions were concentrated to dryness. Diethyl ether was added to the residue with swirling or stirring to precipitate the peptides. The liquid phase was decanted, and solid was collected. This procedure was repeated 2-3 times to remove impurities and residual cleavage cocktail components.

Cyclization of Di-cysteine Peptides

The crude ether-precipitated linear di-cysteine containing peptides were cyclized by dissolution in water, mixtures of aqueous acetonitrile (0.1% TFA), aqueous DMSO or 100% DMSO and adjustment of the pH of the solution to 7.5 - 8.5 by addition of aqueous ammonia, aqueous ammonium carbonate, aqueous ammonium bicarbonate solution or DIEA. The mixture was stirred in air for 16-48 h, acidified to pH 2 with aqueous trifluoroacetic acid and then purified by preparative reverse phase HPLC employing a gradient of acetonitrile into water. Fractions containing the desired material were pooled and the purified peptides were isolated by lyophilization.

Preparation of Peptides Containing Linkers

In a typical experiment, 400 mg of the resin-bound peptide bearing an ivDde-protected lysine) was treated with 10% hydrazine in DMF (2 x 20 mL). The resin was washed with DMF (2 x 20 mL) and DCM (1 x 20 mL). The resin was resuspended in DMF (10 mL) and treated with Fmoc-8-amino-3,6-dioxaoctanoic acid (0.4 mmol), HOBt (0.4 mmol), DIC (0.4 mmol) and DIEA (0.8 mmol) with mixing for 4 h. After the reaction, the resin was washed with DMF (2 × 10 mL) and with DCM (1 × 10 mL). The resin was then treated with 20% piperidine in DMF (2 × 15 mL) for 10 min each time. The resin was washed and the coupling with Fmoc-8-amino-3,6-dioxaoctanoic acid and Fmoc protecting group removal were repeated once more.
The resulting resin-bound peptide with a free amino group was washed and dried and then treated with reagent B (20 mL) for 4 h. The mixture was filtered and the filtrate concentrated to dryness. The residue was stirred with ether to produce a solid, which was washed with ether and dried. The solid was dissolved in anhydrous DMSO and the pH adjusted to 7.5 with DIEA. The mixture was stirred for 16h to effect the disulfide cyclization and the reaction was monitored by analytical HPLC. After completion of the cyclization, the reaction mixture was diluted with 25% acetonitrile in water and applied directly to a reverse phase C-18 column. Purification was effected using a gradient of acetonitrile into water (both containing 0.1% TFA). Fractions were analyzed by HPLC and those containing the pure product were combined and lyophilized to provide the required peptide.

Preparation of Biotinylated Peptides Containing Linkers

In a typical experiment, 400 mg of the resin-bound peptide bearing an ivDde-protected lysine, was treated with 10% hydrazine in DMF (2 x 20 mL). The resin was washed with DMF (2 x 20 mL) and DCM (1 x 20 mL). The resin was resuspended in DMF (10 mL) and treated with Fmoc-8-amino-3,6-dioxaoctanoic acid (0.4 mmol), HOBt (0.4 mmol), DIC (0.4 mmol) and DIEA (0.8 mmol) with mixing for 4 h. After the reaction, the resin was washed with DMF (2x10 mL) and with DCM (1x 10 mL). The resin was then treated with 20% piperidine in DMF (2 x 15 mL) for 10 min each time. The resin was washed and the coupling with Fmoc-8-amino-3,6-dioxaoctanoic acid and removal of the Fmoc protecting group were repeated once more.
The resulting resin-bound peptide with a free amino group was treated with a solution of Biotin-NHS ester (0.4 mmol, 5 equiv.) and DIEA (0.4 mmol, 5 equiv.) in DMF for 2 h. The resin was washed and dried as described previously and then treated with Reagent B (20 mL) for 4 h. The mixture was filtered and the filtrate concentrated to dryness. The residue was stirred with ether to produce a solid that was collected, washed with ether, and dried. The solid was dissolved in anhydrous DMSO and the pH adjusted to 7.5 with DIEA. The mixture was stirred for 4-6 h to effect the disulfide cyclization which was monitored by HPLC. Upon completion of the cyclization, the reaction mixture was diluted with 25% acetonitrile in water and applied directly to a reverse phase C-18 column. Purification was effected using a gradient of acetonitrile into water (both containing 0.1% TFA). Fractions were analyzed by HPLC and those containing the pure product were collected and lyophilized to provide the required biotinylated peptide.

Preparation of DOTA-Conjugated Peptides for Labeling with Selected Gadolinium or Indium Isotopes

In a typical experiment, 400 mg of the resin-bound peptide bearing an N$^{\varepsilon}$-ivDde-protected lysine moiety was treated with 10% hydrazine in DMF (2 × 20 mL). The resin was washed with DMF (2 × 20 mL) and DCM (1 × 20 mL). The resin was resuspended in DMF (10 mL) and treated with Fmoc-8-amino-3,6-dioxaoctanoic acid (0.4 mmol), HOBt (0.4 mmol), DIC (0.4 mmol), DIEA (0.8 mmol) with mixing for 4 h. After the reaction, the resin was washed with DMF (2 × 10 mL) and with DCM (1 × 10 mL). The resin was then treated with 20% piperidine in DMF (2 × 15 mL) for 10 min each time. The resin was washed and the coupling with Fmoc-8-amino-3,6-dioxaoctanoic acid and removal of the Fmoc protecting group were repeated once. The resulting resin-bound peptide with a free amino group was resuspended in DMF (10 mL) and treated with a solution of 1,4,7,10-tetraazacyclododecane-1,4,7,10-tetraacetic acid,-1,4,7-tris-t-butyl ester (DOTA-tris-t-butyl ester, 0.4 mmol, 5 equiv.), HOBt (0.4 mmol), DIC (0.4 mmol) and DIEA (0.8 mmol) in DMF (10 mL) with mixing for 4 h. Upon completion of the reaction, the resin was washed with DMF (2 × 10 mL) and with DCM (1 x 10 mL) and treated with Reagent B (20 mL) for 4 h. The mixture was filtered and the filtrate concentrated to dryness. The residue was stirred in ether to produce a solid that was collected, washed with ether, and dried. The solid was dissolved in anhydrous DMSO and the pH adjusted to 7.5 with DIEA. The mixture was stirred for 16 h to effect the disulfide cyclization, which was monitored by HPLC. Upon completion of the cyclization, the mixture was diluted with 25% acetonitrile in water and applied directly to a reverse phase C-18 HPLC column. Purification was effected using a gradient of acetonitrile into water (both containing 0.1% TFA). Fractions were analyzed by HPLC and those containing the pure product were combined and lyophilized to provide the required biotinylated peptide.

The following monomeric peptides of Table 4 were prepared by the above methods, "PnAO6", as used herein, refers to 3-(2-amino-3-(2-hydroxyimino-1,1-dimethyl-propylamino)-propylamino)-3-methyl-butan-2-one oxime.

Table 4. Sequence or Structure of Monomeric Peptides and Peptide Derivatives

| Structure or Sequence | SEQ. ID NO: or dimer |
|---|---|
| Ac-AGPTWCEDDWYYCWLFGTGGGK(BiotinJJ-$\underline{K}$)-NH$_2$ | 17 |
| Ac-VCWEDSWGGEVCFRYDPGGGK(Biotin-JJ$\underline{K}$)-NH$_2$ | 49 |
| Ac-VCWEDSWGGEVCFRYDPGGGK(JJ)-NH$_2$ | 49 |
| Ac-AQDWYYDEILSMADQLRHAFLSGGGGGK(J)-NH$_2$ Seq 12 derivative | 66 |
| Ac-GDSRVCWEDSWGGEVCFRYDPGGGK(JJ)-NH$_2$ Seq 5 derivative | 31 |
| Ac-GDSRVCWEDSWGGEVCFRYDPGGGK(JJ)-NH$_2$ Seq 5 deriv | 31/D10 |
| Ac-AGPTWCEDDWYYCWLFGTGGGK[(PnAO6-C(=O)(CH$_2$)$_3$C(=O)-K]-NH$_2$ A Seq 11 derivative | 17/D10 |
| Ac-AGPTWCEDDWYYCWLFGTGGGK[(DOTA-JJK(iV-Dde)]-NH$_2$ A Seq 11 derivative | 17/D11 |
| Ac-VCWEDSWGGEVCFRYDPGGGK-NH$_2$ A Seq 5 derivative specifically: Seq 5 residues 5-25 | 49/D12 |
| Ac-AGPTWCEDDWYYCWLFGTGGGK[K(BOA)]-NH$_2$ Seq 11 derivative | 17/D13 |
| Ac-AQDWYYDEILSMADQLRHAFLSGGGGGK[PnAO6-C(=O)(CH$_2$)$_3$C(=O)-K(iV-Dde)]-NH$_2$ Application seq 12 derivative | 66/D 14 |
| Ac-GDSRVCWEDSWGGEVCFRYDPGGGK(JJ)-NH$_2$ Seq 5 deriv linker = Glut | 31/D15 |
| Ac-AGPTWCEDDWYYCWLFGTGGGK-[PnAO6-C(=O)(CH$_2$)$_3$C(=O)-K]-NH$_2$ A Seq 11 derivative, new sequence | 17/D16 |
| Ac-AGPTWCEDDWYYCWLFGTGGGK(Biotin-K)-NH$_2$, A Seq 11 derivative | 31/D19 |
| JJAGPTWCEDDWYYCWLFGTGGGK(iV-Dde)-NH$_2$ (SEQ ID NO:$\underline{17}$) | 17/D20 |
| JJAGPTWCEDDWYYCWLFGTGGGK(iV-Dde)-NH$_2$ | 17/D21 |
| Ac-GDSRVCWEDSWGGEVCFRYDPGGGK(JJ)-NH$_2$ | 31/D24 |
| Ac-VCWEDSWGGEVCFRYDPGGGK(NH$_2$-Ser(GalNAc(Ac)$_3$-alpha-D)-Gly-Ser(GalNAc(Ac)$_3$-alpha-D)-NH$_2$ | 49/D26 |

*Example 12: Preparation of homodimeric and heterodimeric constructs*

The purified peptide monomers mentioned above in Example 8 were used in the preparation of various homodimeric and heterodimeric constructs.

Preparation ofHomodimer-Containing Constructs

To prepare homodimeric compounds, half of the peptide needed to prepare the dimer was dissolved in DMF and treated with 10 equivalents of glutaric acid bis-N-hydroxysuccinimidyl ester. The progress of the reaction was monitored by HPLC analysis and mass spectroscopy. At completion of the reaction, the volatiles were removed *in vacuo* and the residue was washed with ethyl acetate to remove the unreacted bis-NHS ester. The residue was dried, redissolved in anhydrous DMF and treated with another half portion of the peptide in the presence of 2 equivalents of DIEA. The reaction was allowed to proceed for 24 h. This mixture was applied directly to a YMC reverse phase HPLC column and purified by elution with a linear gradient of acetonitrile into water (both containing 0.1% TFA).

Preparation of Heterodimer-Containing Constructs

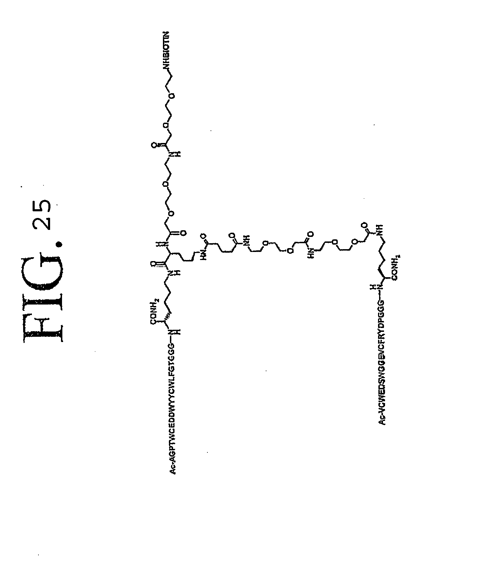

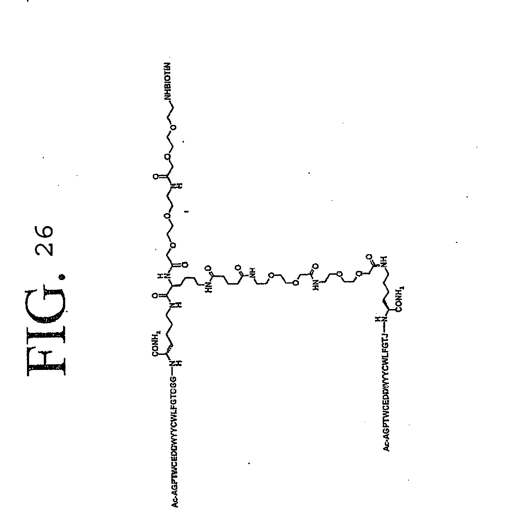

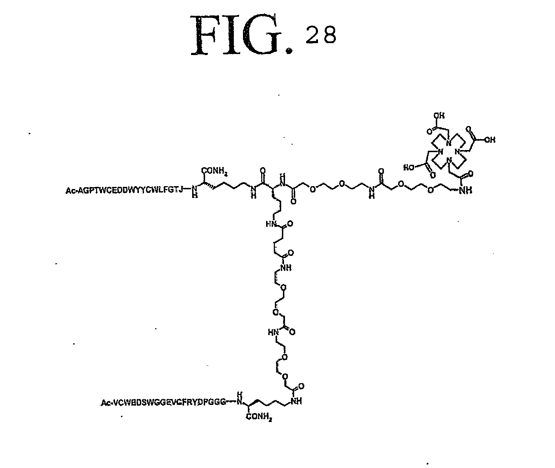

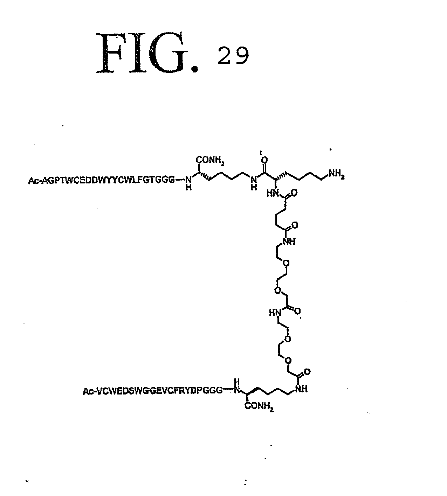

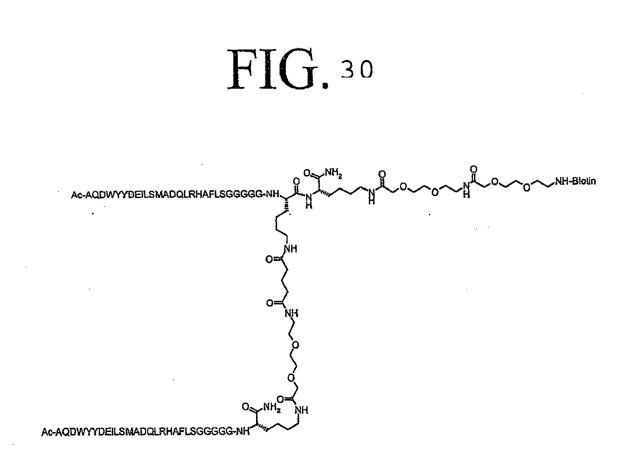

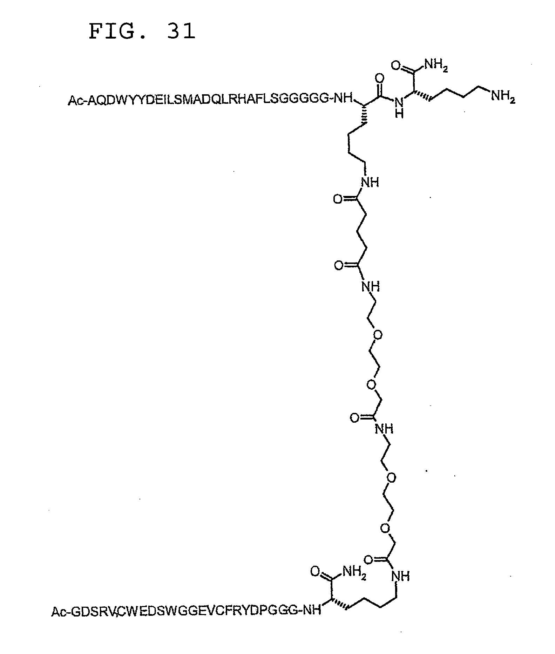

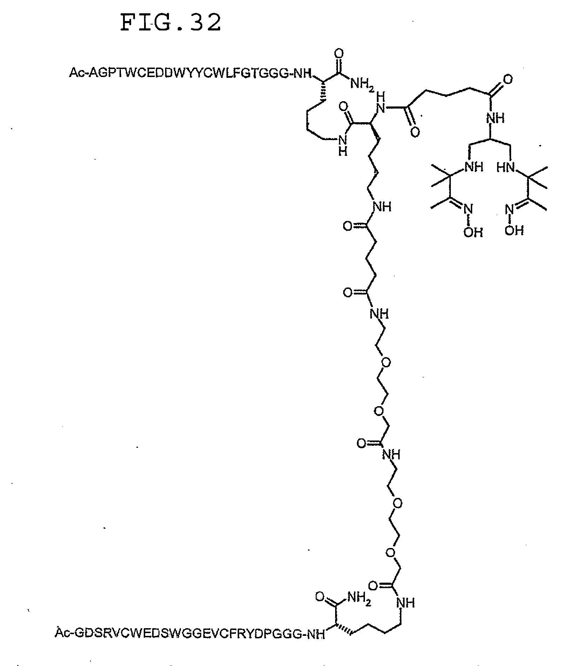

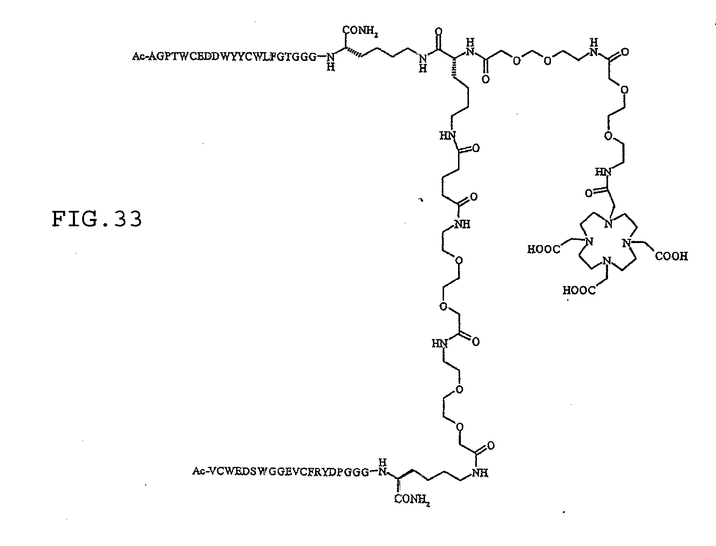

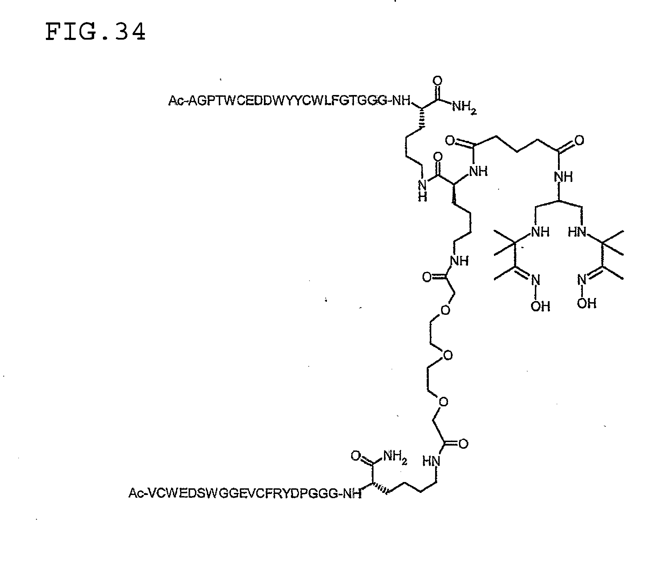

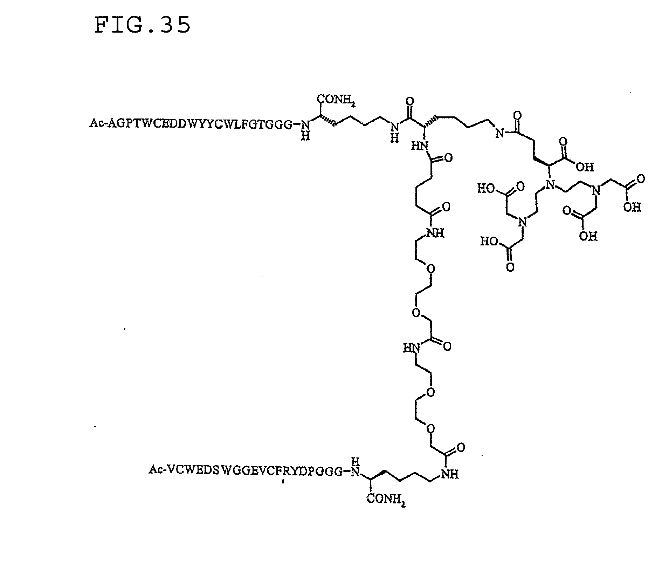

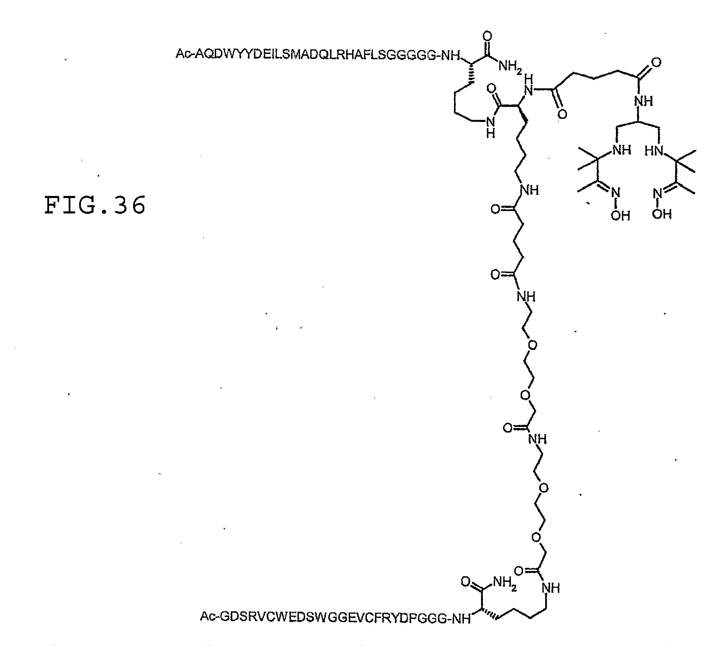

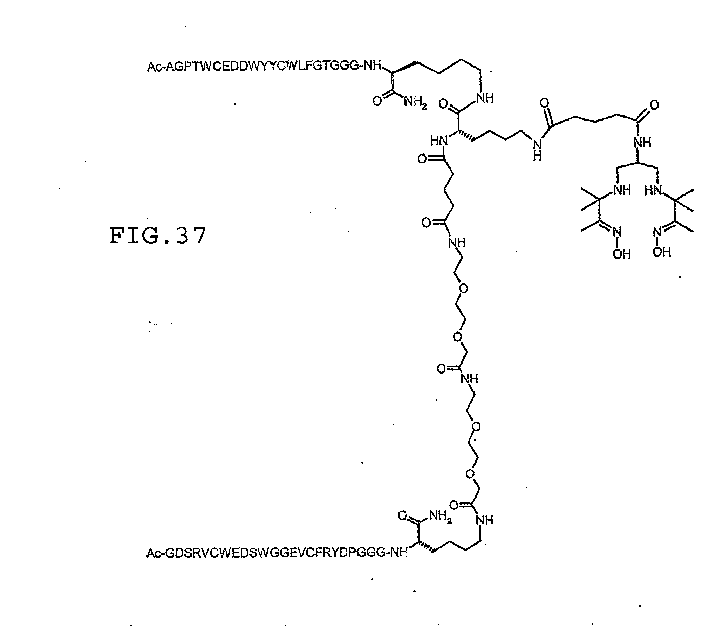

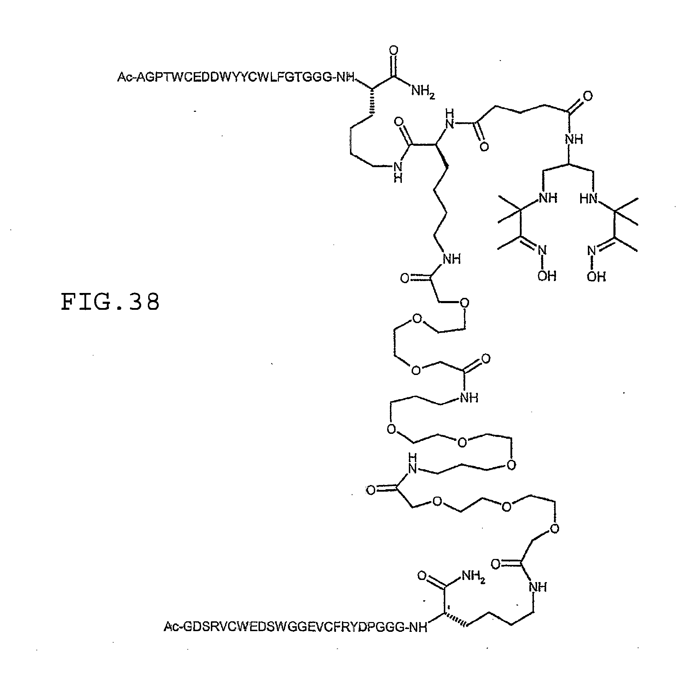

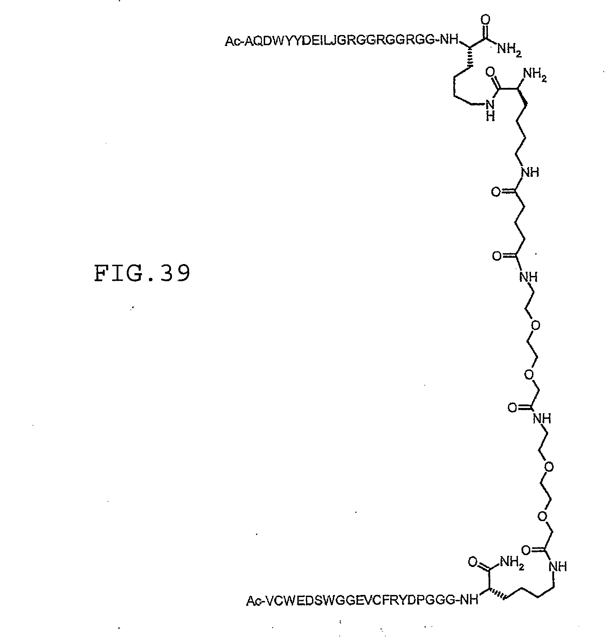

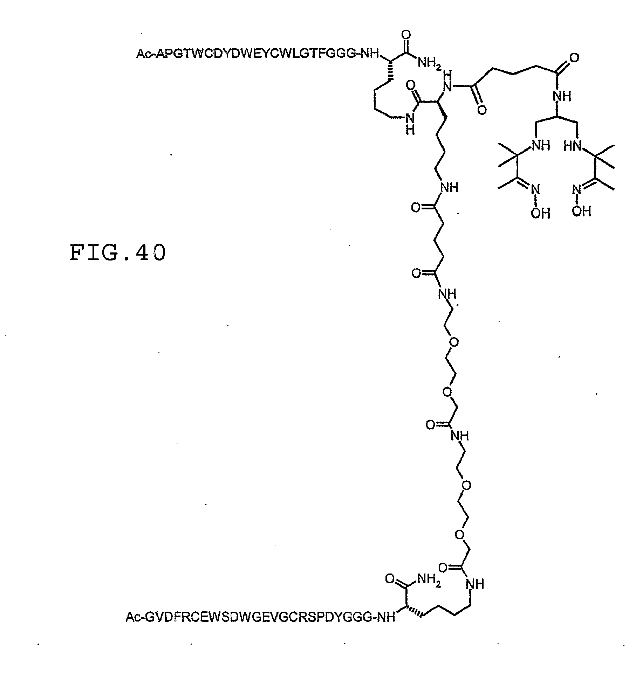

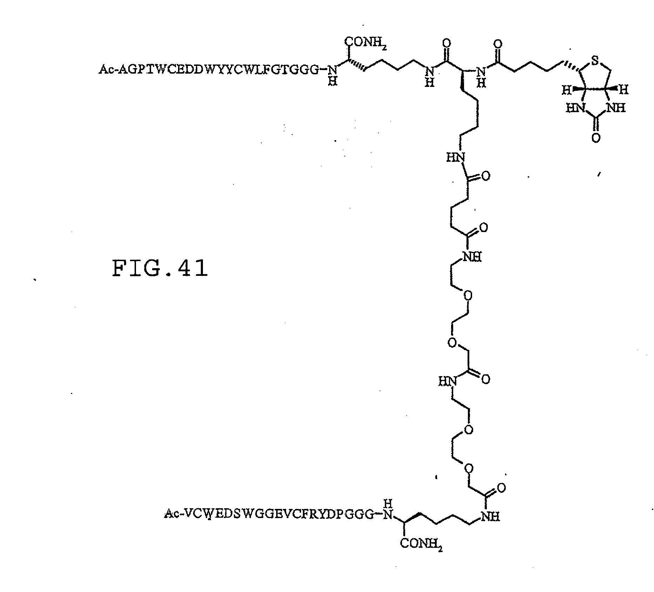

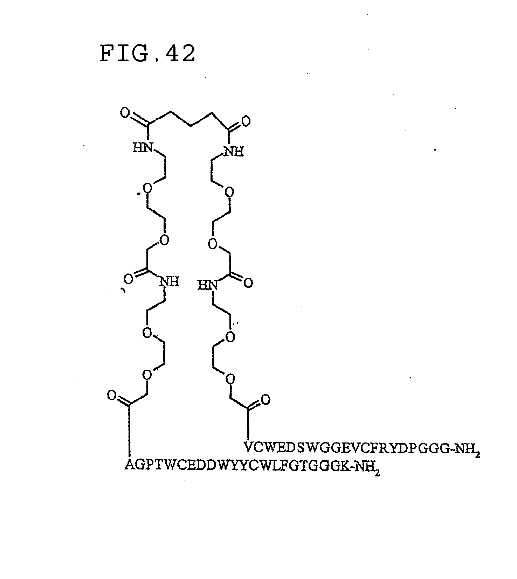

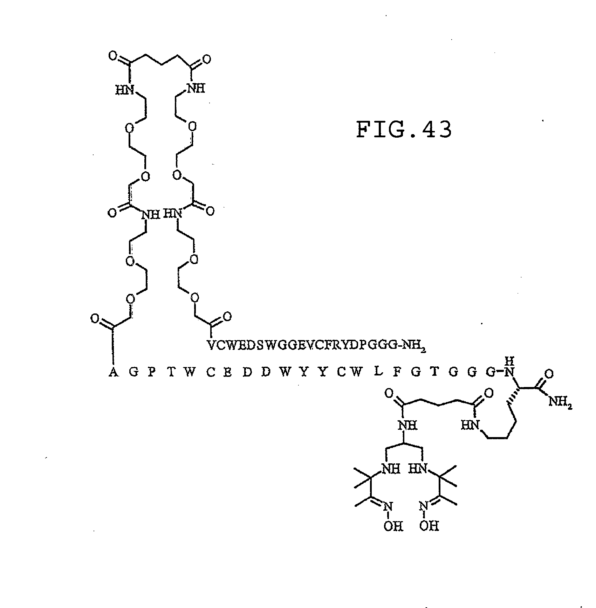

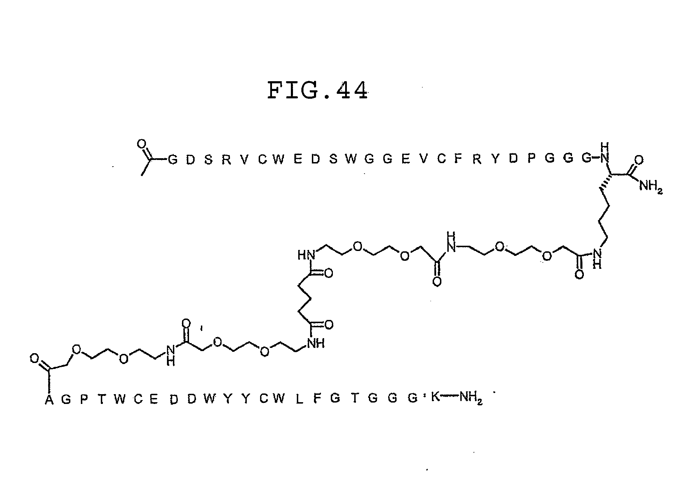

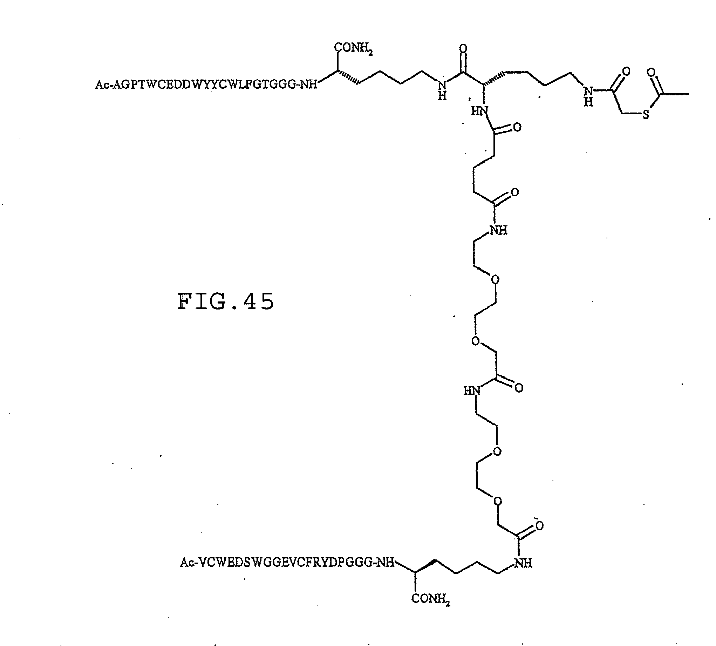

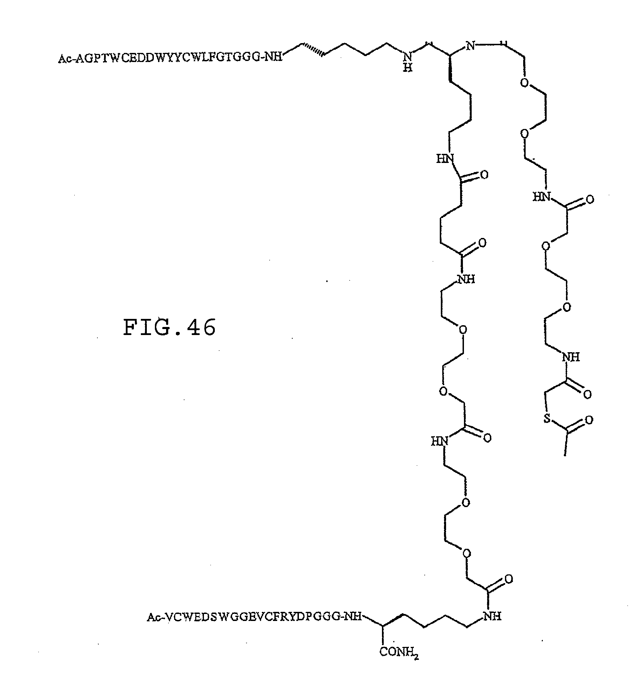

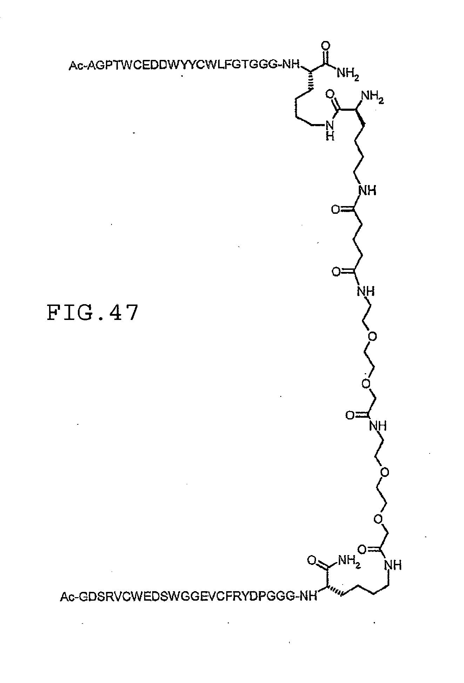

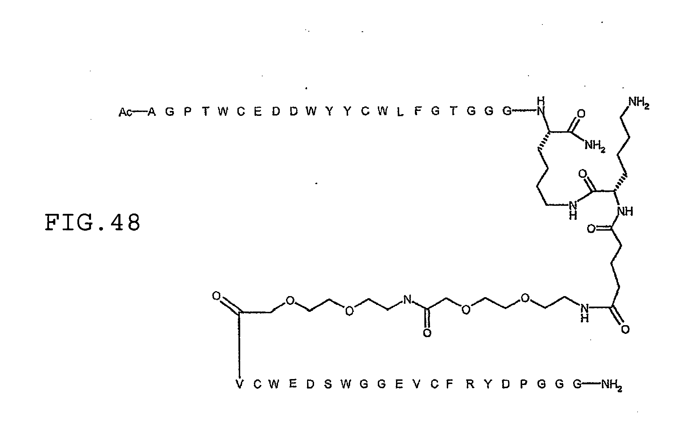

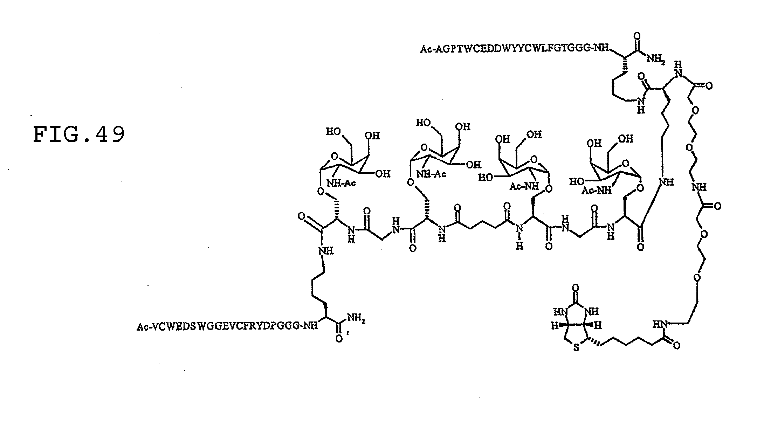

In the case of heterodimers, one of the monomers ("A") was reacted with the bis-NHS ester of glutaric acid and after washing off the excess of bis-NHS ester (as described for the homodimeric compounds), the second monomer ("B") was added in the presence of DIEA. After the reaction the mixture was purified by preparative HPLC. Typically, to a solution of glutaric acid bis N-hydroxysuccinimidyl ester (0.02 mmol, 10 equivalents) in DMF (0.3 mL) was added a solution of peptide "A" and DIEA (2 equiv) in DMF (0.5mL) and the mixture was stirred for 2 h. The progress of the reaction was monitored by HPLC analysis and mass spectroscopy. At completion of the reaction, the volatiles were removed *in vacuo* and the residue was washed with ethyl acetate (3 x 1.0 mL) to remove the unreacted bis-NHS ester. The residue was dried, re-dissolved in anhydrous DMF (0.5 mL) and treated with a solution of peptide "B" and DIEA (2 equiv) in DMF (0.5 mL) for 24 h. The mixture was diluted with water (1:1,v/v and applied directly to a YMC C-18 reverse phase HPLC column and purified by elution with a linear gradient of acetonitrile into water (both containing 0.1% TFA). Fractions were analyzed by analytical HPLC and those containing the pure product were combined and lyophilized to obtain the required dimer. The dimers depicted in FIGS. 25-49 were prepared by this method (structure, name, compound reference number as described in the "Brief Description of the Drawings").

For the preparation of the dimer D5, after the coupling reaction of the individual peptides, 50 μL of hydrazine was added to the reaction mixture (to expose the, lysine N$^ε$-amino group) and the solution was stirred for 2 min. The reaction mixture was diluted with water (1.0 mL) and the pH was adjusted to 2 with TFA. This was then purified by the method described above.

The HPLC analysis data and mass spectral data for the dimeric peptides are given in Table 5 below.

Table 5. Analytical Data for Homodimeric and Heterodimeric Peptide Constructs HPLC Analysis System

|  | Retention Time (System) | Mass Spectral data (API-ES, - ion) |
|---|---|---|
| D1 | 8.98 min. (A) | 1987.7 (M-3H)/3, 1490.6 (M-4H)/4, 1192.3 (M-5H)/5 |
| D2 | 16.17 min (B) | 2035.3 (M-3H)/3, 1526.1 (M-4H)/4, 1220.7 (M-5H)/5 |
| D3 | 8.74 min (C) | 1933.6 (M-3H)/3, 1449.9 (M-4H)/4, 1159.4 (M-5H)/5 |
| D4 | 10.96 min (D) | 2032.8 (M-3H)/3 |
| D5 | 6.57 min (E) | 1816.2 (M-3H)/3, 1361.8 (M-4H)/4, 1089.4 (M-5H)/5, 907.7 (M-6H)/6 |
| D8 | 4.96 min; (F) | 2379.3 [M-3H]/3 |
| D9 | 5.49 min; (G) | 2146.4 [M-3H]/3 |
| D10 | 5.44 min; (H) | 2082.7 [M-3H]/3, 1561.7 [M-4H]/4, 1249.1 [M-5H]/5, 1040.7 [M-6H]/6 |
| D11 | 7.23 min; (E) | 2041.8 [M-3H]/3, 1531.1 [M-4H]/4, 1224.6 [M-5H]/5 |
| D12 | 5.84 min; (H) | 1877.1 [M-3H]/3, 1407.6 [M-4H]/4, 1125.9 [M-5H]/5, 938.1 [M-6H]/6. |
| D13 | 5.367 min; (E) | 1965.3 [M-3H]/3, 1473.8 [M-4H]/4, 1178.8 [M-5H]/5, 982.2 [M-6H]/6 |
| D14 | 4.78 min; (I) | 2275.0 [M-3H]/3, 1362.8 [M-5H]/5 |
| D15 | 5.41 min; (H) | 1561.3 [M-4H]/4, 1249.1 [M-5H]/5, 1040.8 [M-6H]/6, 891.8 [M-7H]/7. |
| D16 | 5.44 min; (J) | 2150.8 [M-3H]/3, 1613.1 [M-4H]/4, 1289.9 [M-5H]/5, 1074.8 [M-6H]/6, 920.9 [M-7H]/7. |
| D17 | 4.78 min; (K) | 1789.4 [M-3H]/3, 1347.7 [M-4H]/4. |
| D18 | 4.74 min; (L) | 2083.1 [M-3H]/3, 1562.7 [M-4H]/4, 1249.5 [M-5H]/5. |
| D19 | 7.13 min; (O) | 1891.9 [M-3H]/3, 1418.4 [M-4H]/4, 1134.8 [M-5H]/5, 945.5 [M-6H]/6. |

(continued)

|  | Retention Time (System) | Mass Spectral data (API-ES, - ion) |
|---|---|---|
| D20 | 9.7 min; (P) | 2700.4 [M-2H]/2, 1799.3[M-3H]/3 |
| D21 | 6.1 min; (P) | 2891.3 [M-2H]/2, 1927.2[M-3H]/3, 1445.1 [M-4H]/4, 1155.8 [M-5H]/5. |
| D22 | 6.23 min; (Q) | 1994.4 [M-3H]/3, 1495.7 [M-4H]/4, 1196.3 [M-5H]/5 |
| D23 | 7.58 min; (J) | 1854.4 [M-3H]/3, 1390.8 [M-4H]/4, 1112.7 [M-5H]/5, 927 [M-6H]/6 |
| D24 | 8.913 min; (R) | 1952.1 [M-3H]/3, 1463.4 [M-4H]/4, 1171.1 [M-5H]/5, 975.3 [M-6H]/6 |
| D25 | 5.95 min; (E) | 1954.9 [M-3H]/3, 1466.1 [M-4H]/4, 1172.4 [M-5H]/5, 976.8 [M-6H]/6. |
| D26 | 6.957 min; (S) | 1759.1 [M-3H]/3, 1319.6 [M-4H]/4, 1055.1 [M-5H]/5 |
| D27 | 5.5 min; (M) | 2317.6 [M-3H]/3, 1737.2[M-4H]/4, 1389.3[M-5H]/5, 1157.7 [M-6H]/6. |

Table 6: Dimer sequences and linkers

| Dimer # | Sequence |
|---|---|
| D8 | Ac-AQDWYYDEILSMADQLRHAFLSGGGGGK{Ac-AQDWYYDEILSMADQLRHAFLSGGGGGK(J-Glut-)-NH$_2$}K(Biotin-JJ)-NH$_2$ |
| D9 | Ac-AQDWYYDEILSMADQLRHAFLSGGGGGK{[Ac-GDSRVCWEDSWGGEVCFRYDPGGGK(JJ-Glut-)]-NH$_2$}K-NH$_2$ |
| D10 | Ac-AGPTWCEDDWYYCWLFGTGGGK{[Ac-GDSRVCWEDSWGGEVCFRYDPGGGK(JJ-Glut-NH(CH$_2$)$_4$-(S)-CH(PnAO6-Glut-NH)(C=O-)]-NH$_2$}-NH$_2$ |
| D11 | Ac-AGPTWCEDDWYYCWLFGTGGGK{Ac-VCWEDSWEDSWGGEVCFRYDPGGGK[JJ-Glut-NH(CH$_2$)$_4$-(S)-CH(DOTA-JJ-NH-)(C=O)-]-NH$_2$}-NH$_2$ |
| D12 | Ac-AGPTWCEDDYCWLFGTGGGK{[PnAO6-Glut-K(Ac-VCWEDSWGGEVCFRYDPGGGK(-C(=O)CH$_2$(OCH$_2$CH$_2$)$_2$OCH$_2$C(=O)-)-NH$_2$]}-NH$_2$ |
| D13 | Ac-AGPTWCEDDWYYCWLFGTGGGK{Ac-VCWEDSWGGEVCFRYDPGGGK[JJ-Glut-K(BOA)]-NH$_2$}-NH$_2$: Dimer 13 (D13) |
| D14 | Ac-AQDWYYDEILSMADQLRHAFLSGGGGGK{PnAO6-Glut-K[Ac-GSDRVCWEDSWGGEVCFRYDPGGGK(JJ-Glut)-NH$_2$]}-NH$_2$ |
| D15 | Ac-AGPTWCEDDWYYCWLFGTGGGK{[[Ac-GDSRVCWEDSWGGEVCFRYDPGGGKJJ-Glut]-NH$_2$]-K(PnAO6-Glut)}-NH$_2$ |
| D16 | Ac-AGPTWCEDDWYYCWLFGTGGGK{PnAO6-Glut-K[Ac-GDSRVCWEDSWGGEVCFRYDPGGGK[-C(=O)CH$_2$O(CH$_2$CH$_2$O)$_2$CH$_2$C(=O)NH(CH$_2$)$_3$O(CH$_2$CH$_2$O)$_2$(CH$_2$)$_3$NHC(=O)CH$_2$O(CH$_2$CH$_2$O)$_2$CH$_2$C(=O)-]-NH$_2$]}-NH$_2$ |
| D17 | Ac-AQDWYYDEILJGRGGRGGRGGK{K[Ac-VCWEDSWGGEVCFRYDPGGGK(JJ-Glut)-NH$_2$]}-NH$_2$ |
| D18 | Ac-AGPTWCDYDWEYCWLGTFGGGK{PnAO6-Glut-K[Ac-GVDFRCEWSDWGEVGCRSPDYGGGK(JJ-Glut)-NH$_2$]}-NH$_2$ |

(continued)

| Dimer # | Sequence |
|---|---|
| D19 | Ac-AGPTWCEDDWYYCWLFGTGGGK{Biotin-K[Ac-VCWEDSWGGEVCFRYDPGGGK(JJ-Glut)-NH$_2$]}-NH$_2$ |
| D20 | (-JJAGPTWCEDDWYYCWLFGTGGGGK-NH$_2$)-Glut-VCWEDSWGGEVCFRYDPGGG-NH$_2$ |
| D21 | [-JJAGPTWCEDDWYYCWLFGTGGGGK(PnAO6-Glut)-NH$_2$]-Glut-VCWEDSWGGEVCFRYDPGGG-NH$_2$ |
| D22 | Ac-GDSRVCWEDSWGGEVCFRYDPGGGK{JJ-Glut-JJ-AGPTWCEDDWYYCWLFTGGGK-NH$_2$}-NH$_2$ |
| D23 | Ac-AGPTWCEDDWYYCWLFGTGGGK{Ac-VCWEDSWGGEVCFRYDPGGGK[JJ-Glut-K(SATA)]-NH$_2$}-NH$_2$ |
| D24 | Ac-AGPTWCEDDWYYCWLFGTGGGK{SATA-JJK[Ac-VCWEDSWGGEVCFRYDPGGGK(JJ-Glut)-NH$_2$]}-NH$_2$ |
| D25 | Ac-AGPTWCEDDWYYCWLFGTGGGK{Ac-GDSRVCWEDSWGGEVCFRYDPGGGK[JJ-Glut-NH(CH$_2$)4-(S)-CH(NH$_2$)C(=O)-]-NH$_2$}-NH$_2$ |
| D26 | AGPTWCEDDWYYCWLFGTGGGGK{(-Glut-JJ-VCWEDSWGGEVCFRYDPGGG-NH$_2$)-K}-NH$_2$ |
| D27 | Ac-AGPTWCEDDWYYCWLFGTGGGGK{Ac-VCWEDSWGGEVCFRYDPGGGK[S(GalNAc(Ac)$_3$-alpha-D)-G-S(GalNAc(Ac)$_3$-alpha-D)-Glut-S(GalNAc(Ac)$_3$-alpha-D)-G-S(GalNAc(Ac)$_3$-alpha-D)-NH(CH$_2$)$_4$-(S)-CH(Biotin-JJNH-)C(=O)-]-NH$_2$}-NH$_2$ |

HPLC Analysis Systems

System A: Column: YMC C-4 (4.6 x 250 mm); Eluents: A: Water (0.1% TFA), B: ACN (0.1% TFA); Elution: initial condition, 25% B, linear gradient 25-60% B in 10 min; flow rate: 2.0 ml/ min; detection: UV @ 220 nm.

System B: Column: YMC C-4 (4.6 x 250 mm); Eluents: A: water (0.1% TFA), B: ACN (0.1% TFA); Elution: initial condition, 25 % B, linear gradient 25-60% B in 20 min; flow rate: 2.0 mL/min; detection: UV @ 220 nm.

System C: Column: YMC C-4 (4.6 x 250 mm); Eluents: A: water (0.1% TFA), B: ACN (0.1% TFA); Elution: initial condition, 30% B, linear gradient 30-60% B in 10 min; flow rate: 2. 0 mL/ min; detection: UV @ 220 nm.

System D: Column: YMC C-4 (4.6 x 250 mm); Eluents: A: water (0.1% TFA), B: ACN (0.1% TFA); Elution: initial condition, 20% B, linear gradient 20-60% B in 10 min; flow rate: 2. 0 mL/ min; Detection: UV @ 220 nm.

System E: Column: Waters XTerra, 4.6 x 50 mm; Eluents:A: water (0.1%TFA), B: ACN (0.1%TFA); Elution: initial condition, 10 % B, linear gradient 10-60 % B in 10 min; flow rate: 3.0 mL/min; detection: UV @ 220 nm.

System F: Column: Waters XTerra, 4.6 x 50 mm; Eluents:A: water (0.1%TFA), B: Acetonitrile (0.1%TFA); Elution: Initial condition, 30 % B, Linear Gradient 30-70 % B in 10 min; Flow rate: 3.0 mL/min; Detection: UV @ 220 nm.

System G: Column: Waters XTerra, 4.6 x 50 mm; Eluents:A: water (0.1%TFA), B: ACN (0.1%TFA); Elution: initial condition, 30 % B, linear gradient 30-75 % B in 10 min; flow rate: 3.0 mL/min; detection: UV @ 220 nm.

System H: Column: Waters XTerra, 4.6 x 50 mm; Eluents:A: water (0.1%TFA), B: ACN (0.1%TFA); Elution: initial condition, 20 % B, linear gradient 20-52 % B in 10 min; flow rate: 3.0 mL/min; detection: UV @ 220 nm.

System I: Column: Waters XTerra, 4.6 x 50 mm; Eluents:A: water (0.1%TFA), B: ACN (0.1%TFA); Elution: initial condition, 10 % B, linear gradient 10-65 % B in 10 min; flow rate: 3.0 mL/min; detection: UV @ 220 nm.

System J: Column: Waters XTerra, 4.6 x 50 mm; Eluents:A: water (0.1%TFA), B: ACN (0.1%TFA); Elution: initial condition, 20 % B, linear gradient 20-60 % B in 10 min; flow rate: 3.0 mL/min; detection: UV @ 220 nm.

System K: Column: Waters XTerra, 4.6 x 50 mm; Eluents:A: water (0.1%TFA), B: ACN (0.1%TFA); Elution: initial condition, 5 % B, linear gradient 5-60 % B in 10 min; flow rate: 3.0 mL/min; detection: UV @ 220 nm.

System L: Column: Waters XTerra, 4.6 x 50 mm; Eluents:A: water (0.1%TFA), B: ACN (0.1%TFA); Elution: initial condition, 5 % B, linear gradient 5-65 % B in 10 min; flow rate: 3.0 mL/min; detection: UV @ 220 nm.

System M: Column: Waters XTerra, 4.6 x 50 mm; Eluents:A: water (0.1%TFA), B: ACN (0.1%TFA); Elution:

initial condition, 15 % B, linear gradient 15-50 % B in 10 min; flow rate: 3.0 mL/min; detection: UV @ 220 nm.

System N: Column: Waters XTerra, 4.6 x 50 mm; Eluents:A: water (0.1%TFA), B: ACN (0.1%TFA); Elution: initial condition, 10 % B, linear gradient 20-80 % B in 10 min; flow rate: 3.0 mL/min; detection: UV @ 220 nm.

System O: Column: YMC-C18, 4.6 x 250 mm; Eluents:A: water (0.1%TFA), B: ACN (0.1%TFA); Elution: initial condition, 30 % B, linear gradient 30-60 % B in 10 min; flow rate: 2.0 mL/min; detection: UV @ 220 nm.

System P: Column: YMC-C18, 4.6 x 250 mm; Eluents:A: water (0.1%TFA), B: ACN (0.1%TFA); Elution: initial condition, 20 % B, linear gradient 20-80 % B in 20 min; flow rate: 2.0 mL/min; detection: UV @ 220 nm.

System Q: Column: YMC-C18, 4.6 x 250 mm; Eluents:A: water (0.1%TFA), B: ACN (0.1%TFA); Elution: initial condition, 20 % B, linear gradient 20-60 % B in 6 min; flow rate: 2.0 mL/min; detection: UV @ 220 nm.

System R: Column: YMC-C18, 4.6 x 250 mm; Eluents:A: water (0.1%TFA), B: ACN (0.1%TFA); Elution: initial condition, 25 % B, linear gradient 25-60 % B in 10 min; flow rate: 2.0 mL/min; detection: UV @ 220 nm.

System S: Column: YMC-C18, 4.6 x 100 mm; Eluents:A: water (0.1%TFA), B: ACN (0.1%TFA); Elution: initial condition, 10 % B, linear gradient 10-60 % B in 10 min; flow rate: 3.0 mL/min; detection: UV @ 220 nm.

*Example 13: Competition with $^{125}$I-VEGF for binding to KDR on HUVECs and KDR-transfected cells*

The following experiment assessed the ability of KDR-binding peptides to compete with $^{125}$I-labeled VEGF for binding to KDR expressed by transfected 293H cells.

Protocol:

293H cells were transfected with the KDR cDNA or mock-transfected by standard techniques. The cells were incubated with $^{125}$I-VEGF in the presence or absence of competing compounds (at 10 $\mu$M, 0.3 $\mu$M, and 0.03 $\mu$M). After washing the cells, the bound radioactivity was quantitated on a gamma counter. The percentage inhibition of VEGF binding was calculated using the formula [(Y1-Y2) $\times$ 100/Y1], where Y1 is specific binding to KDR-transfected 293H cells in the absence peptides, and Y2 is specific binding to KDR-transfected 293H cells in the presence of peptide competitors. Specific binding to KDR-transfected 293H cells was calculated by subtracting the binding to mock-transfected 293H cells from the binding to KDR-transfected 293H cells.

Results

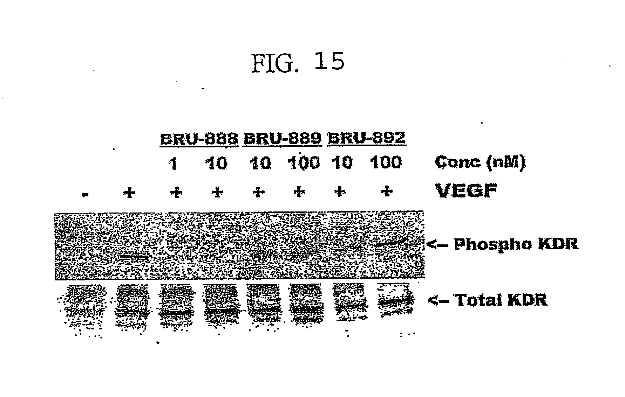

As shown in FIG. 15, all of the KDR-binding peptides assayed were able to compete with $^{125}$I-VEGF for binding to KDR-transfected cells. The heterodimer (D1) was clearly the most effective at competing with $^{125}$I-VEGF, even over the two homodimers (D2 and D3), confirming the superior binding of D1.

*Example 14. Receptor Activation Assay*

The ability of KDR-binding peptides to inhibit VEGF induced activation (phosphorylation) of KDR was assessed using the following assay.

Protocol

Dishes of nearly confluent HUVECs were placed in basal medium lacking serum or growth factors overnight. The dishes in group (c), below were then pretreated for 15 min in basal medium with a KDR-binding peptide, and then the cells in the dishes in groups (a), (b), and (c) were placed in fresh basal medium containing:

(a) no additives (negative control),
(b) 5 ng/ mL VEGF (positive control), or
(c) 5 ng/ mL VEGF plus the putative competing/inhibiting peptide.

After 5 min of treatment, lysates were prepared from each set of dishes. KDR was immunoprecipitated from the lysates was analyzed sequentially by immunoblotting for phosphorylation with an anti-phosphotyrosine antibody, and for total KDR with an anti-KDR antibody (to control for sample loading).

Results

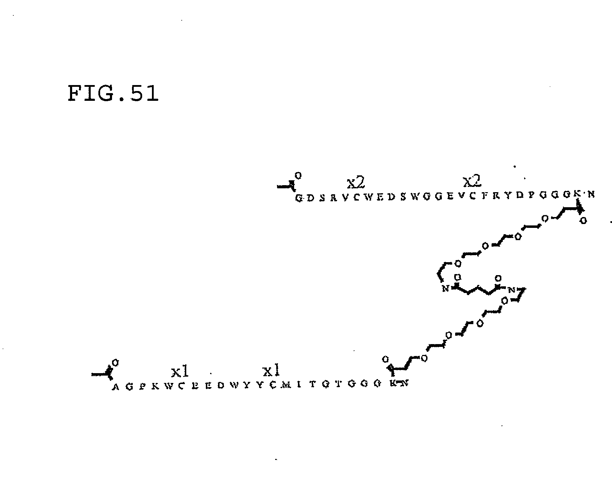

As shown in FIG. 15, D1 was able to completely block the VEGF-induced phosphorylation of KDR in HUVECs at 10 nM. More than half of the phosphorylation was inhibited by the compound at 1 nM. Homodimers D2 and D3, made up of the two individual binding moieties that are contained in D1, had no effect on phosphorylation at up to 100 nM, demonstrating the benefit of heterodimer constructs in blocking a receptor-ligand interaction. In multiple experiments, the $IC_{50}$ for D 1 in this assay varied between 0.5 and 1 nM. A different heterodimer containing unrelated binding sequences, a tail-to-tail heterodimer comprising the polypeptides of SEQ ID NO:36 and SEQ ID NO:37 (FIG. 51), had no effect on phosphorylation at 100 nM in spite of it's high binding affinity (11 nM for KDR by SPR), suggesting that the choice of KDR-binding moieties is important when constructing a multimer to compete with VEGF for binding to KDR. One of ordinary skill in the art would be able to construct suitable heteromultimers using the binding polypeptides provided herein and routine screening assays.

Even though the affinity of D1 for KDR is 10-fold higher than that of D2 (by SPR analysis), the $IC_{50}$ of D1 in the activation assay is at least 100-fold lower. Without wishing to be bound by theory, this suggests that targeting two distinct epitopes on KDR with a single binding molecule can generate greater steric hindrance than a molecule with similar affinity that only binds to a single epitope on KDR and, therefore, improve the ability to inhibit VEGF induced KDR activity. Similarly, it should be pointed out that the two KDR-binding moieties within D1, when tested as monomeric free peptides (SEQ ID NO:17 and SEQ ID NO:49 in the receptor activation assay, had $IC_{50}$s of 0.1 and 1 micromolar, respectively. The $IC_{50}$ for the monomeric free peptides were 100 to 1000-fold higher than the $IC_{50}$ for D1 in the assay and 14 to 30-fold higher than the $K_D$S for the fluoresceinated derivatives of the monomeric peptides. Thus, creating a dimer containing two peptides with weak VEGF-blocking activity has resulted in a molecule with very potent VEGF-blocking activity that goes well beyond the increased binding affinity of D1.

*Example 15: Migration Assay*

The following experiment assessed the ability of D1 to block the VEGF-induced migration of HUVECs in culture.

Protocol

Serum-starved HUVECs were placed, 100,000 cells per well, into the upper chambers of BD Matrigel-coated FluoroBlok 24-well insert plates (#354141). Basal medium, containing either nothing or different attractants such as VEGF (10 ng/mL) or serum (5% FBS) in the presence or absence of potential VEGF-blocking/inhibiting compounds, was added to the lower chamber of the wells. After 22 hours, quantitation of cell migration/invasion was achieved by post-labeling cells in the insert plates with a fluorescent dye and measuring the fluorescence of the invading/migrating cells in a fluorescent plate reader. The VEGF-induced migration was calculated by subtracting the migration that occurred when only basal medium was placed in the lower chamber of the wells.

Results:

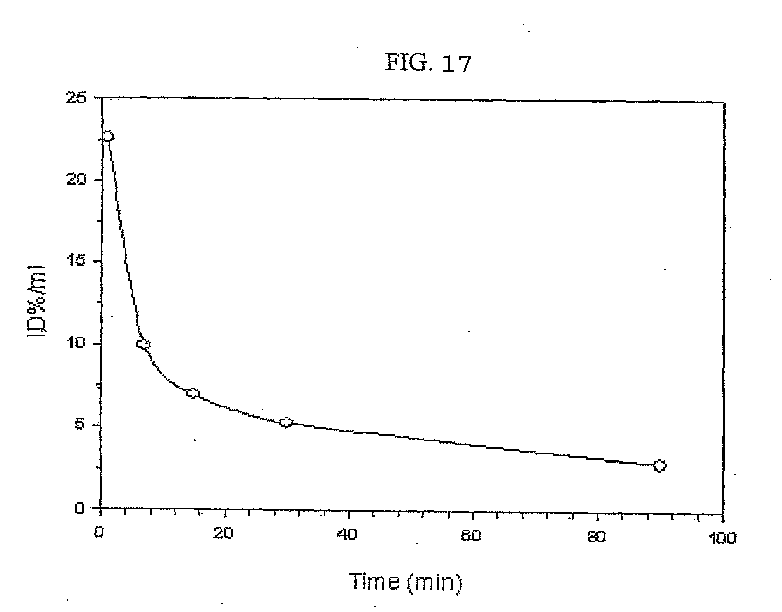

VEGF induced a large increase in endothelial cell migration in the assay, which was potently blocked by D1. At 5 nM D1, the VEGF-stimulated endothelial cell migration was 84% blocked (see FIG. 17). At 25 nM D1, this migration was almost completely blocked. In other experiments, a known KDR inhibitor, SU-1498 (Strawn, L. et al., 1996, Cancer Res., 56:3540-3545) was tested in the assay. SU-1498 at 3 micromolar did not block the VEGF-induced migration as well as D1 (47% blocked at 3 micromolar). D6 (structure shown below in Example 18), at 50 nM, also produced essentially complete inhibition of the migration stimulated by VEGF. Serum was a very powerful attractant in the assay when used in place of VEGF, but its effect was not significantly diminished by D1, indicating that D1 specifically inhibits endothelial migration induced by VEGF.

*Example 18: Biacore analysis of heterodimer binding to KDR-Fc and determination of affinity constant*

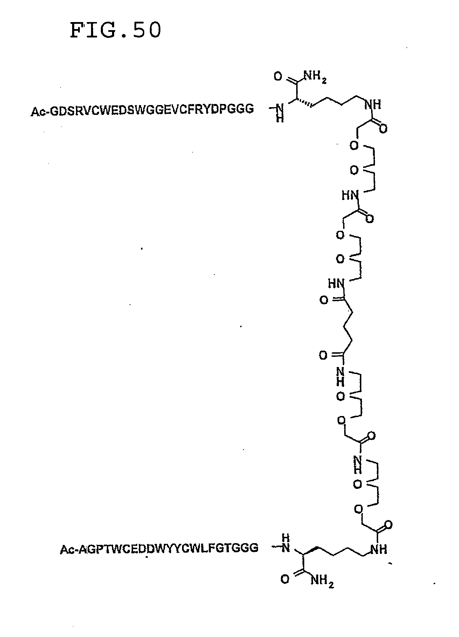

A peptide heterodimer (FIG. 50) composed of SEQ ID NO:17 and SEQ ID NO:31 was prepared as previously described in Example 12 using glutaric acid bis N-hydoxysuccinimidyl ester. The heterodimer was tested for binding to KDR-Fc using Biacore and an affinity constant was determined as follows.

Three densities of KDR-Fc were cross-linked to the dextran surface of a CM5 sensor chip by the standard amine coupling procedure (0.5 mg/mL solution diluted 1:100 or 1:50 with 50 mM acetate, pH 6.0). Flow cell 1 was activated and then blocked to serve as a reference subtraction. Final immobilization levels achieved:

$R_L$ Fc 2 KDR-Fc = 1607
$R_L$ Fc 3 KDR-Fc = 3001
$R_L$ Fc 4 KDR-Fc = 6319

Experiments were performed in PBS (5.5 mM phosphate, pH 7.65, 0.15 M NaCl) + 0.005% P-20 (v/v)). D6 was diluted to 250 nM in PBS and serial dilutions were performed to produce 125, 62.5, 31.3 15.6, 7.8, and 3.9 nM solutions. All samples were injected in duplicate. For association, peptides were injected at 20 $\mu$L/min for 12.5 minutes using the kinject program. Following a 10 minute dissociation, any remaining peptide was stripped from the KDR surface with a quickinject of 50 mM NaOH +1 M NaCl for 12 s at 75 $\mu$L/min. Sensorgrams were analyzed using BIAevaluation software 3.1 and a hyperbolic double rectangular regression equation in SigmaPlot 6.0. Heterodimer steady state binding affinities ($K_{DAV}$) were determined at all three KDR immobilization densities (Table 7).

Table 7. Summary of Parameters

|  |  | $K_{D1}$ (nM) | $R_{max1}$ | $K_{DAV}$(nM) | $R_{maxAV}$ | $R^{2*}$ |
|---|---|---|---|---|---|---|
|  | Vs. 1600RU | 46 | 13.1 | 1.5 | 12.6 | 0.995 |
| D6 | Vs. 3000RU | 25.5 | 21.2 | 0.665 | 22.7 | 0.991 |
|  | Vs. 6000RU | 17 | 61.3 | 0.662 | 62.2 | 0.993 |

From this data, it appears that at the higher immobilization densities, the heterodimer binds KDR with a sub-nanomolar affinity (~0.6 nM).

To assess the *in vivo* clearance of this peptide heterodimer, a small amount of material was iodinated using iodogen and Na[125]I according to standard protocols (Pierce). One tube coated with the iodogen reagent was pre-wet with 1 mL of 25 mM Tris, 0.4M NaCl, pH 7.5. This was discarded and 100 $\mu$l of the same buffer added. Using a Hamilton syringe 11 $\mu$L of the $^{125}$I-NaI was transferred to the reaction tube. Based on original estimates of the No[125]I concentration of 143.555 mCi/ml, the 11 $\mu$L should contain about 1.5 mCi. After addition, the sample was swirled and set in a lead pig to incubate for 6min with a swirl every 30 sec. After 6 min, the entire sample was transferred to the protein that was in an Eppendorf tube. The sample was swirled and set to incubate for 8 min, with a swirl every 30 sec. After 8 min the reaction was quenched (terminated) with tyrosine (10mg/mL, a saturated solution), allowed to sit for 5 min, and then 2 $\mu$L was removed for a standard.

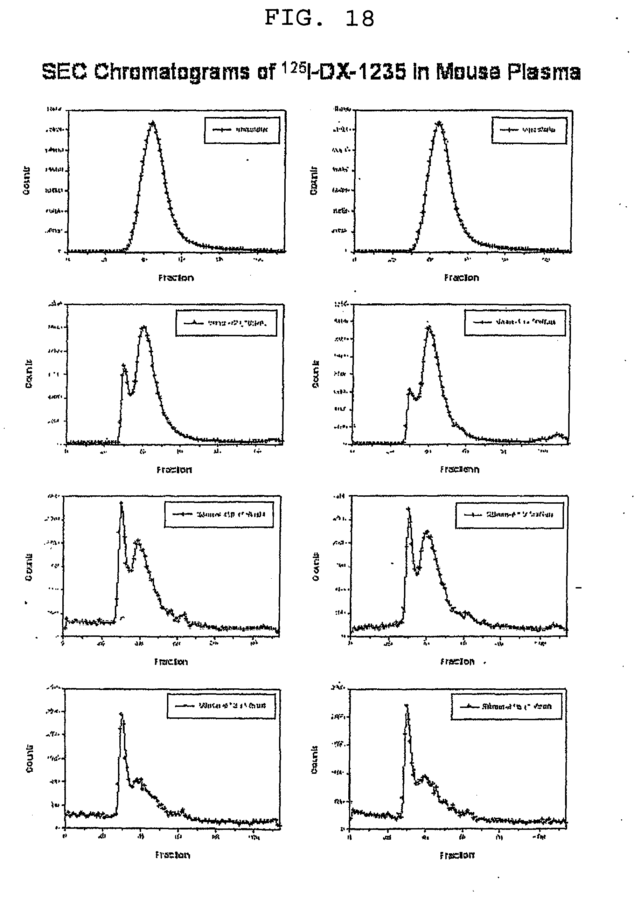

For purification a 10 mL column of the D-salt polyacrylamide 1800 was used to separate the labeled peptide from labeled tyrosine. The column was first washed with 10 mL saline, then 5 mL of 25 mM Tris, 0.4M NaCl, pH 7.5 containing 2.5% HSA to block non-specific sites. After the HSA buffer wash, the column was eluted with 60mL of the 25 mM Tris, 0.4 M NaCl buffer, and the column was stored overnight at 4°C. The labeled sample contained 1.355 mCi, as determined by the dose calibrator. The 2 $\mu$l sample that was removed as a standard contained 8.8 $\mu$Ci. The peptide sample was applied to the D-salt 1800 column and eluted with the Tris/NaCl buffer, pH 7.5. The flow was controlled by applying single 0.5ml aliquots for each fraction, #1-14, and then 1.0 mL for fractions 25-43. FIG. 18 shows the elution profile of activity versus fraction number. The peak of activity in fractions # 9, 10, and 11, was assumed to be the peptide. The radioactivity in 24 through ~40 is likely the labeled tyrosine. From this purification, fractions #9-12 were pooled together and used for the subsequent clearance study (concentration of $^{125}$I-D6 in pool is 7.023 $\mu$g/mL; 100 $\mu$L = 0.702$\mu$g with 8.6$\mu$Ci).

A total of 15 mice were injected with 100 $\mu$L $^{125}$I-D6 and mice (in sets of 3) were sacrificed at the following time points: 0, 7, 15, 30, 90 minutes. After injection more than 2 $\mu$Ci was found remaining in the syringe, so actual activity injected was about 6 $\mu$Ci. With 6 $\mu$Ci injected, the corresponding protein administered was ~ 0.5 $\mu$g per animal. Once sacrificed, the counts were determined in a 50 $\mu$L plasma sample from each animal. For each set of three animals at each time point, the counts were averaged, converted to % injected dose/ml plasma (ID%/mL), and then plotted to assess the rate of clearance (FIG. 18). This data was fit to either a 4 or 5 parameter equation to determine the biphasic half life of this molecule. The 4 parameter fit resulted in a $T_{1/2\alpha}$ of 2.55 minutes and a $T_{1/2\beta}$ of 64.66 minutes. The 5 parameter fit resulted in a $T_{1/2\alpha}$ of 2.13 minutes and a $T_{1/2\beta}$ of 23.26 minutes.

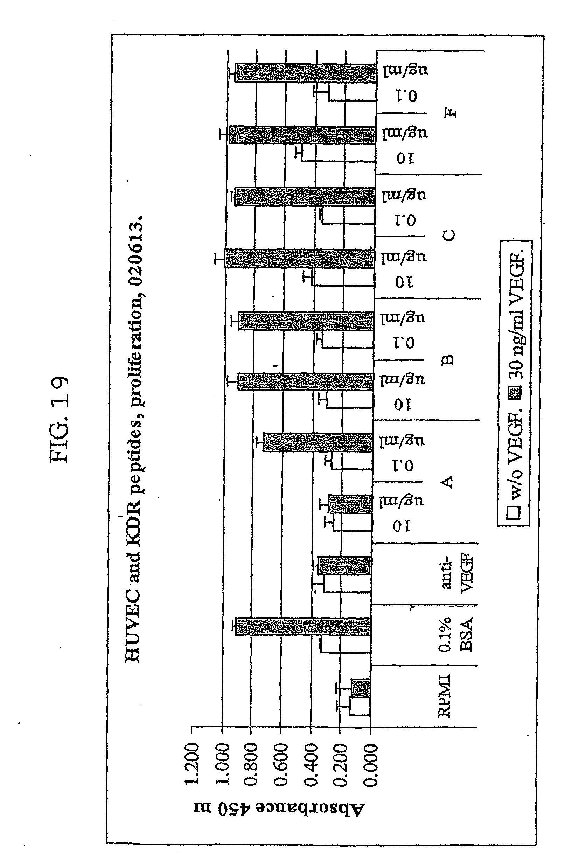

Larger volumes of plasma were also taken from mice sacrificed at the 0, 30, and 90 minute time points. These samples were injected onto a Superdex peptide column (Pharmacia) coupled to a radioactivity detector to assess the association of the peptide with serum proteins (FIG. 19). As shown, the labeled peptide does associate with higher MW proteins, which could explain its biphasic half life clearance behavior.

To help assess the potency of the peptide as an anti-angiogenesis inhibitor, D6 was tested in an endothelial cell proliferation assay using HUVECs and BrdU detection. Briefly, freshly isolated HUVECs (between p3 - 6) were cultured in RPMI + 10% FCS +1% antibiotics + 1% L-glutamine + 0.4% BBE (bovine brain extract) and seeded per well, 5000-10000/well in 100 $\mu$L. The cells were allowed to recover for 24 hrs prior to use. Then the cells were washed with PBS twice and treated for 48 hrs with anti-VEGF antibody (positive control) or peptides A, B and C (0.1 and 10 ug/mL) in RPMI + 0.1% BSA + 1% L-glutamine. The following 6 variables were tested in 2 series (n=4):

Series I: w/o VEGF

Series II: w/ VEGF (30 ng/mL)

1. Standard medium: RPMI + 10% FCS +1% antibiotics + 1% L-glutamine + 0.4% BBE
2. Negative control 1: RPMI (true starvation)
3. Negative control 2: RPMI + 0.1% BSA + 1% L-glutamine
4. Positive control: anti-VEGF 10 μg/ml in RPMI + 0.1% BSA + 1% L-glutamine
5. 0.1 μg/ml KDR peptides in RPMI + 0.1% BSA + 1% L-glutamine
6. 10 μg/ml KDR peptides in RPMI + 0.1 % BSA + 1% L-glutamine

Protocol:

1) cells are incubated for 48 hours under various conditions
2) 10μL BrdU dilution (1:100 in EBM) is added to each well at 24 hours
3) incubate for another 24 hours (total 48 hrs)
4) aspirate the culture medium
5) add 100μL FixDenat (Roche Applied Science, Indianapolis, IN) to each well, incubate at room temperature for 30 min.
6) Discard FixDenat solution
7) 100 μL antibody-solution (PBS 1% BSA and anti-BrdU PO) added to each well.
8) incubate at RT for 90 minutes.
9) wash 3 times with PBS, 200 μL/well, 5 min.
10) add substrate solution (TMB), incubate for 10-30 minutes
11) transfer all to a flexible plate
12) stop the reaction by adding 2 M H$_2$SO$_4$, 25 μL/well
13) read absorbance at 450 nm within 5 minutes after stopping the reaction.

Background binding was determined by omitting the anti-BrdU antibody in 4 wells with control cells (cultured in complete medium; EBM + BulletKit (Clonetics, BioWhittaker, Inc., MD) and by complete labeling of cells that was not exposed to BrdU.

Of the two KDR binding peptide tested (D6 and SEQ ID NO:17) as shown in FIG. 19, D6 completely inhibits HUVEC proliferation at 10 μg/mL in the presence of VEGF, similar to an anti-VEGF antibody (positive control). On the other hand, SEQ ID NO:17 (one of the peptides that make up the heterodimer) did not inhibit proliferation in this assay at the highest concentration tested (10 μg/mL). As a result, the heterodimer shows an enhanced ability to compete with VEGF in comparison with SEQ ID NO:17 alone.

*Example 19: BIAcare Analysis-murine KDR-Fc Binding of Peptide Dimers D1 and D7*

Using BIAcore, the binding constants of peptide dimers D1 (a heterodimer of SEQ ID NO:17 and SEQ ID NO:31 and D7 (a heterodimer of SEQ ID NO:5 and SEQ ID NO 31; see FIG. 51) for murine KDR-Fc were determined.

Procedure

Three densities of recombinant murine KDR-Fc were cross-linked to the dextran surface of a CM5 sensor chip by the standard amine coupling procedure (0.5 mg/mL solution diluted 1:100 or 1:40 with 50 mM acetate, pH 6.0). Flow cell 1 was activated and then blocked to serve as a reference subtraction. Final immobilization levels achieved:

R$_L$ Fc 2 KDR-Fc = 2770
R$_L$ Fc 3 KDR-Fc = 5085
R$_L$ Fc 4 KDR-Fc = 9265

Experiments were performed in PBS buffer (5.5 mM phosphate, pH 7.65, 0.15 M NaCl) + 0.005% P-20 (v/v)). SEQ ID NO:17, run as a control, was diluted to 125 nM in PBS. Serial dilutions were performed to produce 62.5, 31.3, 15.6, 7.8, and 3.9 nM solutions. D1 and D6 were diluted to 50 nM in PBS and serial dilutions were performed to produce 25, 12.5, 6.25, 3.13, 1.56, 0.78, and 0.39 nM solutions. All samples were injected in duplicate. For association, peptides were injected at 30 μL/min for 3 minutes using the kinject program. Following a 10 minute dissociation, any remaining peptide was stripped from the rmKDR-Fc surface with a quickinject of 50 mM NaOH + 1 M NaCl for 12s at 75 μL/min.

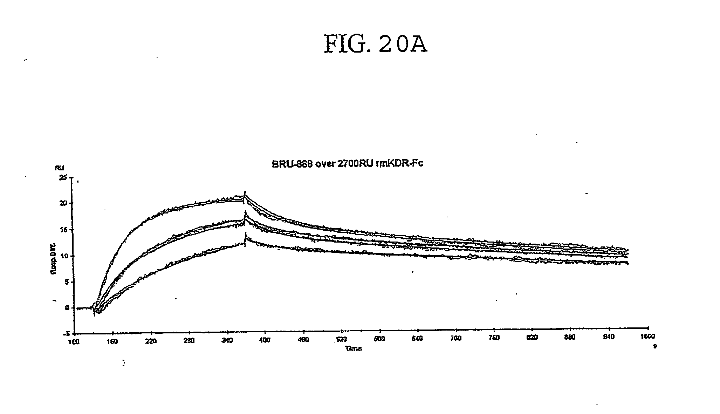

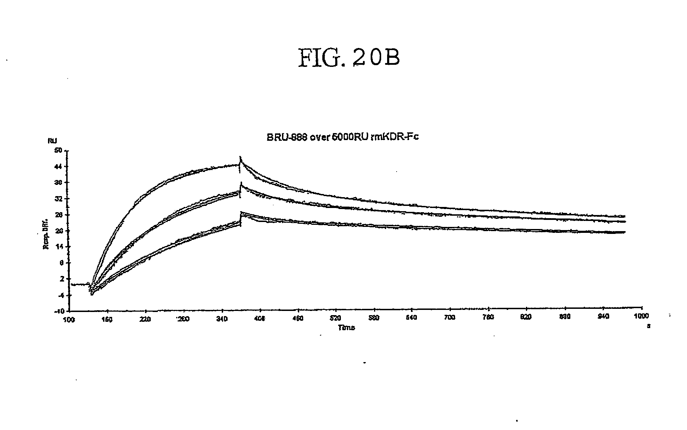

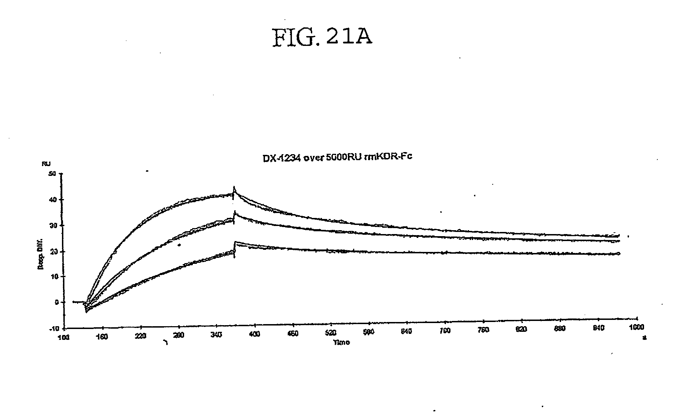

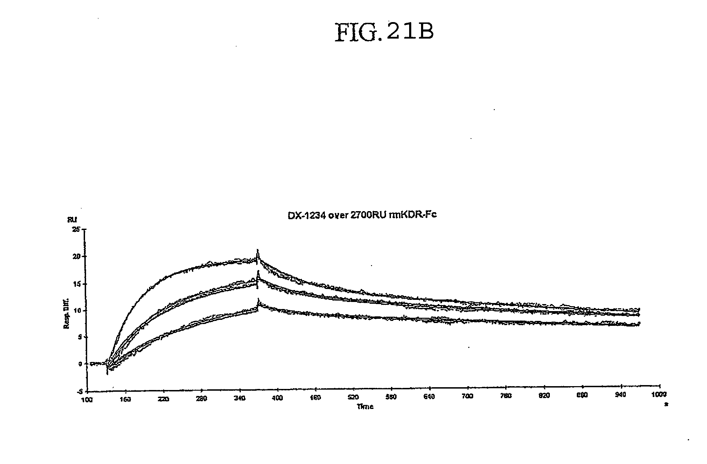

Sensorgrams were analyzed using the simultaneous $k_a/k_d$ fitting program in the BIAevaluation software 3.1. The results are shown in Table 8 and FIGS. 20-21. The fact that about the same $K_{D2}$ constant was achieved for both heterodimers even when the density of receptor on the sensor chip was reduced by half is consistent with multimeric binding of the heterodimers to individual receptors rather than cross-link-type binding between receptors.

Table 8. Summary of Kinetic Parameters.

| | | ka1 (1/Ms) | Kd1 (1/s) | ka2 (1/RUs) | kd2 (1/s) | KD1[#] (nM) | KD2[‡] (nM) | Chi²[*] |
|---|---|---|---|---|---|---|---|---|
| D1 | vs. 2700RU | 7.94E+05 | 0.0139 | 3.31E-04 | 5.96E-04 | 17.5 | 0.751 | 0.077 |
| | vs. 5000RU | 5.54E+05 | 8.88E-03 | 1.17E-04 | 4.57E-04 | 16.0 | 0.825 | 0.323 |
| D7 | vs. 2700RU | 7.59E+05 | 0.011 | 3.36E-04 | 6.44E-04 | 14.5 | 0.848 | 0.082 |
| | vs. 5000RU | 5.21E+05 | 7.39E-03 | 1.17E-04 | 4.68E-04 | 14.2 | 0.898 | 0.278 |
| Fluorescein SEQ ID NO:277 | vs. 2700RU | 1.02E+06 | 0.037 | - | - | 36.4 | - | 0.073 |
| | vs. 5000RU | 5.18E+05 | 0.0174 | - | - | 33.6 | - | 0.167 |

[#] $K_{D1}$ is a calculated $K_D$ based on $kd_1/ka_1$
[‡] $K_{D2}$ is a calculated $K_D$ based on $kd_2/ka_1$ (*i.e.*, avidity factor)
[*] The chi2 value is a standard statistical measure of the closeness of the fit. For good fitting to ideal data, chi2 is of the same order of magnitude as the instrument noise in RU (typically < 2).

*Example 20. In Vivo inhibition of tumor growth.*

Conditions are described providing methods for determining efficacy of three (3) concentrations for Test Article (binding peptide, D6) suspected of having anti-angiogenic activity on SW-480 human colon carcinoma cells using an *in vivo* xenograft tumor model.

Athymic nude mice are acceptable hosts for the growth of allogenic and heterogenic cells. Nude mice are required in Points to Consider in the Characterization of Cell Lines used to Produce Biologicals (Points to Consider in the Characterization of Cell Lines used to Produce Biologicals, FDA 1993).

D6 is a synthetic heterodimeric peptide suspected of having anti-angiogenic activity. This peptide binds to the human VEGF receptor 2 (KDR) with high affinity and competes with VEGF binding.

SW-480 Human Carcinoma Cells

Colon carcinoma, SW-480, cells (ATCC) were cultured in Dulbecco's Modified Eagles Medium (DMEM) supplemented with 4 mM L-glutamine, 0.1 mM non-essential amino acids, 50 mg/mL Gentamicin, 250 mg/mL Fungizone and 10% heat inactivated fetal bovine serum at 37°C in 95% air and 5% $CO_2$.

Exponentially growing cells were harvested, washed twice in phosphate buffered saline (PBS) to remove any traces of trypsin or serum. Cells were suspended in Hanks Balanced Salt Solution (HBSS) for injections.

Sterile phosphate buffered saline (BioWhittaker) was manufactured in accordance with cGMP regulations and was cell culture tested to assure compatibility; having a pH of 7.3-7.7 and an osmolarity of 271-287 mOsm/kg. PBS was the vehicle used to reconstitute Test Articles and for vehicle control injections.

Cisplatin (American Pharmaceutical Partners, Inc.; Los Angeles, CA) was prepared according to manufacture's specifications. Cisplatin was prepared in an aseptic fashion using a BL2 BioChem guard hood.

TEST SYSTEM

A. Species/Strain: *Mus musculus,* Crl:NU/NU-nuBR mice (nude mice)
B. Sex: Female
C. Age: 6-8 weeks at initiation of treatment
D. Weight Range: No weight requirement
E. Source: Animals were received from the Gnottobiotic Department at Charles River Laboratories, Wilmington, MA.
F. Number: A total of 115 animals were received and injected for this study, with 90 mice used on study.

G. Method of Identification:

Mice were uniquely numbered using an ear tag system. Additionally, cages were marked with cage cards minimally identifying group number, animal number, study number and IACUC protocol number.

H. Randomization:

Animals were randomly assigned to treatment groups using Microsoft® Excel 97 SR-1 program.

I. Humane Care of Animals:

Treatment and care of the animals were in accordance with the standard operating procedures of Charles River Laboratories, which adheres to the regulations outlined in the USDA Animal Welfare Act (9 CFR, Parts 1, 2, and 3) and the *Guide for the Care and Use of Laboratory Animals.*

This study protocol was covered under the Charles River Laboratories Institutional Animal Care and Use Committee (IACUC Protocol Number: P07182001I).

ANIMAL CARE

A. Diet and Drinking Water:

Mice were fed gamma-irradiated rodent chow *ad libitum.* Tap water was sterilized and supplied via bottle and sipper tube *ad libitum.*

B. Animal Environment:

Animals were housed by groups in semi-rigid isolators. Mice were housed in flat bottom caging containing five to ten animals. Cages contained gamma-irradiated contact bedding. The number of mice in each cage may have been altered due to the behavior of the mice, changes were noted in the isolator inventory. The housing conforms to the recommendations set forth in the Guide for the Care and Use of Laboratory Animals, National Academy Press, Washington, D.C., 1996 and all subsequent revisions.

Environmental controls were set to maintain a temperature of 16-26°C ($70 \pm 8$°F) with a relative humidity of 30-70. A 12:12 hour light: dark cycle was maintained.

C. Acclimation:

Once animals were received, they were allowed to acclimate to the laboratory environment for 24-hours prior to the study start. Mice were observed for signs of disease, unusual food and/or water consumption or other general signs of poor condition. At the time of animal receipt, animals were clinically observed and appeared to be healthy.

EXPERIMENTAL DESIGN

A. General Description:

Female athymic nude mice (Crl:NU/NU-*nu*BR) at 6-8 weeks of age were used in this study. A total of 115 mice were injected subcutaneously into the right lateral thorax with $5 \times 10^6$ SW-480, human colon carcinoma cells. When tumors reached a target window size of approximately $150 \pm 75$ mg, 90 tumor-bearing mice were randomly selected and distributed into one of nine groups. Test Articles and vehicle were administered intraperitoneally (IP), Cisplatin was administered intravenously (IV). Tumor measurements were recorded twice weekly using hand-held calipers. Mice were monitored daily for signs of toxicity and morbidity. At study termination, animals were euthanized by carbon dioxide overdose and necropsied for tissue collection.

B. Group Assignments:

A total of nine (9) groups were used in this study. Each group contained ten (10) tumor-bearing mice. Groups 1 and 2 contained untreated and vehicle treated negative control mice, respectively. Groups 3, 4, and 5 contained mice that received one of three different concentrations of the D6 anti-angiogenic peptide. Group 9 contained mice that received cisplatin, a standard chemotherapeutic compound as a positive control.

C. Dosing Levels and Regiment:

Dose levels for each group are provided in Table 9. Dosing began the same day that animals were randomly sorted into groups (Study Day 7). Each dose was removed from the dose vial using aseptic technique for each animal and the injection site was wiped with an alcohol swab prior to dose administration. Doses were administered with a 1.0 mL syringe and a 27-gauge x ½" needle for each mouse

The Test Article- and vehicle-treated mice received daily intraperitoneal (IP) injections for 15 days. Cisplatin was administered every other workday for a total of five (5) doses via an intravenous route.

Table 9. Study Treatment Groups

| Group | Test Article | Concentration mg/kg | Number of Animals |
|-------|--------------|---------------------|-------------------|
| 1 | Untreated | - | 10 |
| 2 | Vehicle | 0 | 10 |
| 3 | D6 | 0.05 | 10 |
| 4 | D6 | 0.5 | 10 |
| 5 | D6 | 5.0 | 10 |
| 9 | Cisplatin | 6.0 | 10 |

D. Clinical Observations of Animals:

Clinical Observations of each animal were performed and recorded at least once daily for toxicity, morbidity and mortality. Morbidity included signs of illness such as, but not limited to, emaciation, dehydration, lethargy, hunched posture, unkempt appearance, dyspnea and urine or fecal staining.

E. Tumor Measurements:

In accordance with the protocol tumor measurements were taken twice weekly throughout the study by measuring the length and width of tumors with calibrated calipers. Measurements occurred a minimum of 3-4 days apart, except when animals were euthanized and measurements were taken; this sometimes resulted in an interval of less than 3 days. Tumor weights were calculated using the following formula: $mg = (L \times W^2)/2$. Animals were euthanized either when mean tumor weight was $\geq$ 1000 mg per group over two (2) consecutive measurements, or if tumors became ulcerated, impaired the animal's ability to ambulate or obtain food and water.

F. Unscheduled Euthanasia and Unexpected Deaths:

1. Unscheduled Euthanasia:

None of the animals required unscheduled euthanasia while on study.

2. Unexpected Deaths:

None of the animals died while on study.

G. Necropsy:

1. Euthanasia and Necropsy Order:

All mice in groups 1, 2, 3, 4, and 5 (50 total) were submitted for necropsy when tumors reached a group mean target size of $\geq$ 1000 mg over two (2) consecutive measurements within a group. Animals were submitted for necropsy to the Charles River Laboratories Health Monitoring Laboratory (HM), Wilmington, MA. All animals were euthanized on Study Day 22, short of received the full 28 day treatment regiment with Test Articles because mean tumor size was $\geq$ 1000 mg in Test Article Treated Groups 3-8.

All animals were humanely euthanized by carbon dioxide ($CO_2$) inhalation.

2. Tissue Collection:

Tumors were dissected free of surrounding tissue and overlying skin. Additionally the kidneys were collected. Any abnormalities noted on the renal surfaces were noted.

Frozen blocks were made of tumors and kidneys for each animal. A representative section of the tissue (tumor, kidneys) was taken. Kidney sections included the cortex and medulla. Tissue sections were placed in the bottom of a labeled plastic-freezing mold. Tissue was embedded with OCT medium. Blocks were submerged into isopentane chilled with dry ice until frozen. Blocks were briefly examined for quality, and stored on dry ice.

Blocks were labeled with the animal number and a letter code corresponding to tissue (A = left kidney; B = right kidney; C = mass). Blocks from one animal were placed into a labeled bag.

RESULTS

A. In-Life Measurements and Observations:

1. Clinical Observations, Morbidity and Mortality Summary Statement:

All animals appeared healthy and were within normal limits throughout the study. D6 showed no signs of toxicity at the doses used in this study.

Animals were euthanized on Study Day 22. All mice, except Group 9 mice, were euthanized prior to completing Test Article administration, because mean tumor size was $\geq$ 1000 mg in Groups 1-8. Group 9, Cisplatin-treated animals were euthanized on Study Day 22 when mean tumor weight was 995 mg. No animals died while on study.

2. Mass Palpation Summary Statement:

Throughout the study palpable masses were detected in all mice, with tumors progressively growing for the duration of the study. As expected tumors in untreated and vehicle treated negative control mice (Groups 1 and 2) grew the fastest, reaching a mean tumor size of 1000 mg on or before Study Day 20. In addition, animals treated with Cisplatin (Group 9) developed tumors that grew the slowest reaching a mean tumor size of 995 mg at study termination (Day 22).

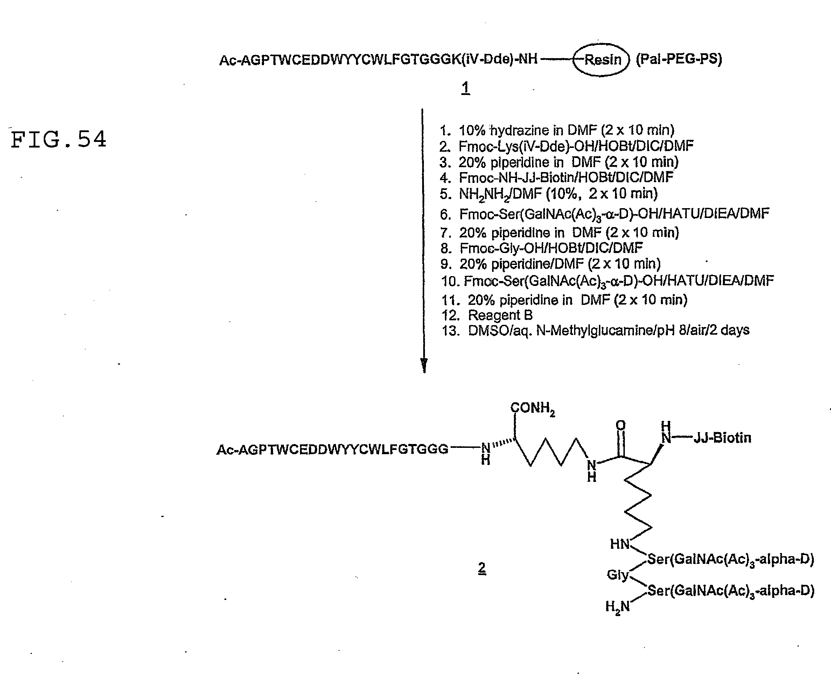

In general, except for Group 3 mice, all animals treated with Test Article resulted in slower tumor growth (FIG. 54). Animals in Group 3, which were treated with the low dose of D6 (0.05 mg/kg) had tumors that grew at approximately the same rate as the tumors in untreated and vehicle treated animals in Groups 1 and 2. Animals treated with either higher doses of D6 (Groups 4 and 5) had tumors that grew slower; reaching a mean

| Days After Cell Injection | Test Article | | | | | |
| | Untreated Control | Vehicle Control | D6 | | | Cisplatin |
| | | | 0.005 mg/kg | 0.05 mg/kg | 0.5 mg/kg | 6 mg/kg |
| 4 | 48 | 49 | 43 | 51 | 50 | 34 |
| 7 | 164 | 156 | 157 | 163 | 154 | 160 |
| 8 | 180 | 164 | 156 | 133 | 168 | 173 |
| 11 | 340 | 388 | 333 | 298 | 310 | 407 |
| 14 | 684 | 648 | 726 | 596 | 577 | 675 |
| 20 | 1064 | 986 | 973 | 857 | 978 | 635 |

Tumor Weights (mg)

tumor size of 1000 mg on Study Day 21. When compared to control Groups 1 and 2 mice,

Test Article treatment resulted in a delay of tumor growth of approximately 1 day.

B. Conclusions:

Data from this study validate the model used because tumor-bearing mice in negative control Groups 1 and 2 and positive control Group 9 responded as expected.

Throughout the study palpable masses were observed in all groups. In addition, all animals were healthy and within normal limits throughout the study. Furthermore, D6 did not adversely affect the animals. Therefore, these data would suggest that animals treated with D6 Test Article had tumors that grew slowly (approximately 1 day slower over the 22 day test period than controls). Also, since the Test Article did not show any significant toxic effects, higher concentrations of Test Article could also be used with potentially better tumor regression.

Table 10.

| | 21 | 1412 | 1571 | 1468 | 983 | 1056 | 839 | |
|---|---|---|---|---|---|---|---|---|
| | 22 | 1967 | 1863 | 2026 | 1474 | 1526 | 995 | |

*Example 21: In vitro cell proliferation assay*

Microvascular endothelial cells (MVECs, Cascade Biologics, Portland, OR) were used to assess the *in vitro* efficacy of D6 and related analogues for their ability to inhibit VEGF-stimulated proliferation. MVECs (passage 2) were grown to 90% confluency, trypsinized and plated in gelatin-coated 96-well microtiter plates at a density of 4-8 $\times$ $10^3$ cells/well. Sixteen to 24 hours after plating, the cells were washed one time (200μL/well) with media devoid of fetal bovine serum but containing 0.1% bovine serum albumin (BSA). Fresh BSA-containing media was added to each well and the cells were incubated for an additional 24 hours. After this 24 hour period of starvation, fresh BSA-containing media (containing 25 ng/mL VEGF) with or without D6 was added and the cells were incubated for an additional 48 hours at 37°C. To assess the dose reponse in this assay, multiple D6 concentrations were tested in duplicate wells. The media was removed and fresh BSA-containing media was added with or without BrdU and the cells were incubated for an additional 24 hours prior to determining the level of incorporation exactly as described by the manufacturer. Results are shown in FIG. 62.

*Example 22.*

The following experiment assessed the ability of D25 and D27 to block the VEGF-induced migration of HUVECs in culture and demonstrated that the added glycosylation and/or distinct spacer structure used in D27 enhanced its potency.

*Protocol:* Serum-starved HUVECs were placed, 100,000 cells per well, into the upper chambers of BD fibronectin-coated FluoroBlok 24-well insert plates. Basal medium, with or without VEGF (10 ng/mL) in the presence or absence of D25 or D27, was added to the lower chamber of the wells. After 22 hours, quantitation of cell migration/invasion was achieved by post-labeling cells in the insert plates with a fluorescent dye and measuring the fluorescence of the invading/migrating cells in a fluorescent plate reader. The VEGF-induced migration was calculated for each experimental condition by subtracting the amount of migration that occurred when only basal medium was added to the lower chamber of the wells.

*Results:* VEGF induced a large increase in endothelial cell migration in the assay, which

was potently blocked by both D25 and D27. D27 was ten-fold more potent than D25 ($IC_{50}$ 0.5 nM and 5 nM respectively), indicating that the glycosylation of D27 and/or its distinct spacer properties has enhanced its ability to bind KDR and block the effects of VEGF.

*Example 23.*

The following experiment assessed the ability of "Adjunct A" multimeric construct of TKPPR peptide (binds to NP-1, a VEGF receptor that enhances the effects of VEGF mediated by KDR),to enhance the inhibition of the VEGF-induced migration of HUVECs in culture produced by D6. Adjunct A = 5CF-Gly-N{[CH_2CH_2C(=O)-Gly-N(CH_2CH_2C(=O)-Adoa-Thr-Lys-Pro-Pro-Arg-OH]_2}_2 where Adoa = 3,6-dioxa-8-aminooctanoyl, 5CF = 5-carboxyfluoresceinyl.

*Protocol:* Serum-starved HUVECs were placed, 100,000 cells per well, into the upper chambers of BD fibronectin-coated FluoroBlok 24-well insert plates. Basal medium, containing with or without VEGF (10 ng/mL) in the presence or absence of varying concentrations of D6 , or varying concentrations of D6 in combination with a constant 100 nM Adjunct A (synthesized as described in WO 01/91805 A2), was added to the lower chamber of the wells. After 22 hours, quantitation of cell migration/invasion was achieved by post-labeling cells in the insert plates with a fluorescent dye and measuring the fluorescence of the invading/migrating cells in a fluorescent plate reader. VEGF-induced migration was calculated for each experimental condition by subtracting the amount of migration observed in the absence of VEGF.

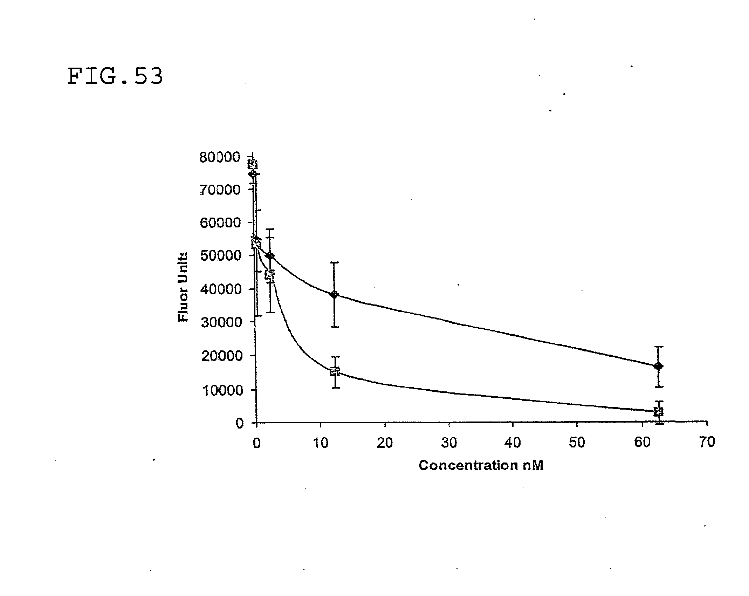

*Results:* VEGF induced a large increase in endothelial cell migration in the assay, which was potently blocked by D6 ($IC_{50}$ about 12.5 nM), but not by 100 nM Adjunct A alone (FIG. 53). Surprisingly however, Adjunct Awas able to enhance the potency of D6 by about ten-fold when used in the assay simultaneously with D6 ($IC_{50}$ about 2.5 nM). This indicates that compounds containing the TKPPR sequence (or similar) found in Adjunct A can be used to enhance the potency of certain compounds such as D6, which compete with VEGF for binding to KDR. In addition, a heteromultimer containing the peptide sequences found in D6 or similar) as well as the TKPPR sequence (or similar), in one or more repetitions, would likely possess enhanced activity in this assay.

*Example 24: Synthesis of D27*

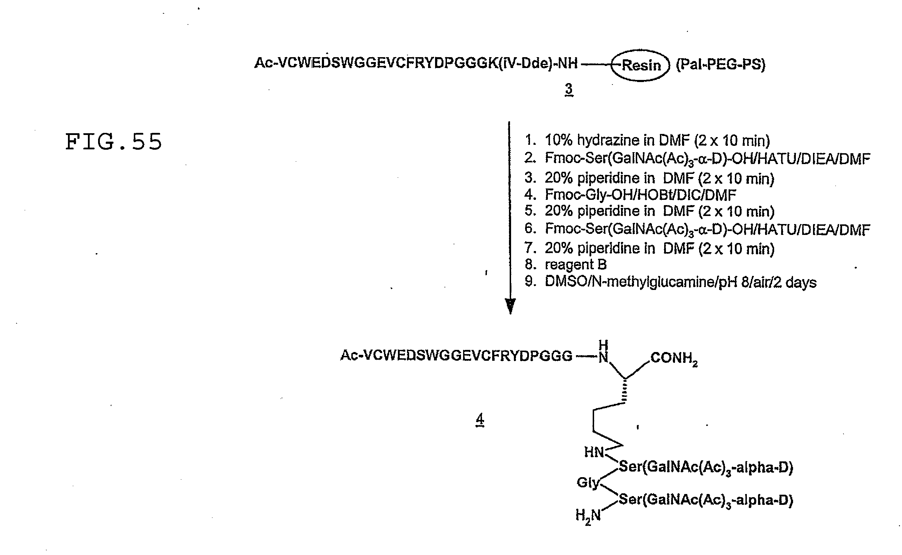

Synthesis of **1** and **3** (see FIGS. 54 and 55)

Synthesis of the monomers were carried out as described in Method 5 on a 0.25 mmol scale employing as the starting resin Fmoc-GGGK(iV-Dde)NH-PAL-PEG-PS resin. The peptide resin was washed and dried before cleavage or further derivatization by automated or manual methods.

Procedure Synthesis of Peptide **2** and Peptide **4** (see FIGS. 54 and 55)

Appendage of Biotin-JJ, Lysyl, Glycyl and Serinyl(GalNAc(Ac)$_3$-$\alpha$-D moieties onto **1** and **3** was done by manual SPPS such as described in Method 6 and Method 8. The coupling of amino acids was carried out in DMF using HOBt/DIC activation (except for Ser(GalNAc(Ac)$_3$-$\alpha$-D). Fmoc removal was carried out with 20% piperidine in DMF. All couplings were 5-16 hours duration. After each coupling, the completion was confirmed by the Kaiser test. In the case of Ser(Gal-NAc(Ac)$_3$-$\alpha$-D, the coupling was performed in DMF employing HATU/DIEA as the coupling agent. In cases where the Kaiser test indicated unreacted amino groups the coupling was repeated. Removal of the N-terminal Fmoc group and cleavage from resin was performed. The crude peptide was precipitated in ether and washed twice by ether and dried under vacuum. The linear crude peptide was directly cyclized by dissolving the peptide in DMSO (40 mg/mL). The pH of the solyution was adjusted to 8 by addition of aqueous N-methylglucamine.and the solution was was stirred in air for 48h at room temperature. The peptides were then purified employing gradient HPLC as described in Method 1 employing a Waters-YMC C-18 ODS preparative column (250 mm x 4.6 mm i.d.). The pure product-containing fractions were combined and lyophilized to provide the needed peptides.

Procedure: Synthesis of D27 - Compound **6**

To a solution of glutaric acid bis-NHS ester (0.122 mmol, Pierce Scientific Co.) in anhydrous DMF was added dropwise a solution of **4** in DMF (40 mg, 0.0122 mmol, DIEA was added to neutralize the trifluoroacetic acid bound to the peptide and N-hydroxysuccinimide formed during the reaction). This 0.7 mL solution was stirred for 4h. The reaction was monitored by HPLC and mass spectroscopy. DMF was removed under vacuum. The excess diester was removed by addition of ethyl acetate which precipitated the peptide-monoester **5** while dissolving glutaric acid bis-NHS ester. The mixture was centrifuged and the liquid portion decanted. This was repeated twice. The residue was kept under vacuum for 10 min. The residue was dissolved in DMF and mixed with a solution of **2** (37 mg, 0.009 mmol) in DMF (pH 7). It was stirred at ambient temperature for 16 h. The volatiles were removed under high vacuum and the acetate functions were removed by treatment of the residue with 1 mL of hydrazine/MeOH (15/85, v/v) solution with stirring for 2.5 h at ambient temperature. Acetone was added to quench the excess of hydrazine and the volatiles were removed under vacuum. The resulting residue was dissolved in DMSO and purified by preparative HPLC as described above to provide 9 mg of the pure material.

Sequence and Analytical Data for Peptides **2**, **4** and **6**

| Compound identifier | Sequence | HPLC Ret. time (System) | Mass Spectrum (ESI, neg. ion) |
|---|---|---|---|
| **Peptide 2 :** New Seq, a Seq 11 derivative | Ac-AGPTWCEDDWYYCWLFGTGGGK{Biotin-JJK[NH$_2$-Ser(GalNAc(Ac)$_3$-$\alpha$-D)-Gly-Ser(GalNAc(Ac)$_3$-$\alpha$-D]}-NH$_2$ | 7.4 min (T) | 2041.3 [M - 2H]/2 |
| **Peptide 4:** New Seq, a Seq 5 derivative | Ac-VCWEDSWGGEVCFRYDPGGGK(NH$_2$ Ser(GalNAc(Ac)$_3$-$\alpha$-D)-Gly-Ser(GalNAc(Ac)$_3$-$\alpha$-D)-NH$_2$ | 8.0 min (T) | 1636.3 [M - 2H]/2 |
| D27 | Ac-AGPTWCEDDWYYCWLFGTGGGGK{Ac-VCWEDSWGGEVCFRYDPGGGK[S(GalNAc-$\alpha$-D)-G-S(GalNAc-$\alpha$-D)-Glut-S(GalNAc-$\alpha$-D)-G-S(GalNAc-$\alpha$-D)-NH(CH$_2$)$_4$-(S)-CH(Biotin-JJNH-)C(=O)-]-NH$_2$}-NH$_2$ | 5.50 min (M) | 1737.2 (M - 4H)/4; 1389.3 (M – 5H)/5; 1157.7 [M – 6H]/6 |

System T: Column: Waters XTerra, 4.6 × 50 mm; Eluents:A: Water (0.1%TFA), B: Acetonitrile (0.1%TFA) : Elution: Initial condition, 15 % B, Linear Gradient 15-50 % B in 8 min; Flow rate: 3.0 mL/min; Detection: UV @ 220 nm.

*Example 41: Enhancing the serum residence of KDR-binding peptides*

It is known in the art that compounds that contain maleimide and other groups that can react with thiols react with thiols on serum proteins, especially serum albumin, when the compounds are injected. The adducts have serum life times similar to serum albumin, more than 14 days in humans for example.

Conjugation to maleimide

Methods are available that allow for the direct synthesis of maleimide-labeled linear peptides encompassed by the present invention (Holmes, D. et al., 2000. Bioconjug. Chem., 11:439-444).

Peptides that include disulfides can be derivatized with maleimide in one of several ways. For example, a third cysteine can be added at the carboxy terminus. The added cysteine is protected with protecting group that is orthogonal to the type of groups used for the cysteines that are to form the disulfide. The disulfide is formed by selectively deprotecting the intended cysteines and oxidizing the peptide. The final cysteine is then deprotected and the peptide reacted with a large molar excess of a bismaleimide. The resulting compound has one of the maleimides free to react with serum albumin or other thiol-containing serum proteins.

Alternatively, a cyclic peptide of the present invention is synthesized with a lysine-containing C-terminal extention, such as -GGGK. Lysines of the KDR-binding motif are protected with ivDde and the C-terminal lysine is deprotected. This lysine is reacted with a maleimide-contining compound, such as N-[e-maleimidocaproyloxy]succinimide ester (Pierce Biotechnology, Rockford, IL) or N-(a-Maleimidoacetoxy)succinimide ester (Pierce Biotechnology).

**Conjugation to a moiety that binds serum albumin non-covelently**

Polypeptides having a molecular weight less than 50-60 kDa are rapidly excreted. Many small molecules, such as fatty acids, bind to serum albumin. Fatty acids containing 10 to 20 carbon atoms have substantial affinity for serum albumin. Linear and branched fatty acids can be used. This binding in serum can reduce the rate of excretion. Using methods known in the art, serum-albumin-binding moieties can be conjugated to any one of the peptides herein disclosed. The serum-ablumin-binding moiety can be joined to the KDR-binding peptide through a linker. The linker can be peptidic or otherwise, such as PEG. Linkers of zero to about thirty atoms are preferred. It is preferred that the linker be hydrophilic. The serum-albumin-binding moiety can be conjugated to the KDR-binding peptide at either end or though a side group of an appended amino acid. Suitable side groups include lysine and cysteine. Such compounds can also comprise chelators for radionuclides, as discussed herein. A KDR-binding peptide joined to a serum-ablumin-binding moiety will bind KDR.

**Conjugation to PEG**

As is well known in the art, attachment of poly(ethyleneglycol) (PEG) to proteins and peptides enhances the serum residence of these molecules. Attachment of PEG (linear or branched) to a KDR-binding peptide is expected give substantial enhancement of serum residence time. The molecular weight of the PEG should be at least 10 kDA, more preferably at least 20 kDa, and most preferably 30 kDa or more. The PEG could be attached at the N- or C-terminus. Methods of attaching PEG to peptides are well known in the art (Roberts M. et al., 2002. Adv. Drug. Deliv. Rev., 54:459-476). PEG can be attached to reactive side groups such as lysine or cysteine.

**Fusion to serum protein**

It is known in the art that proteins comprising serum albumin (SA) and other proteins have enhanced serum residence times. The amino-acid sequence of human SA (hSA) is shown in Table 22. Table 23 shows a fusion protein comprising

AGDWWVECRVGTGLCYRYDTGTGGGK(SEQ ID NO:25)::

PGGSGGEGGSGGEGGRPGGSEGGTGG::mature hSA:: GGSGGEGGSGGEGGSGPGEGGEGSGGRP :: GDSRVCWEDSWGGEVCFRYDPGGGK(SEQ ID NO:31). The KDR-binding peptides are separated from mature hSA by linkers that are rich in glycine to allow flexible spacing. It is known in the art that one need not use all of hSA to obtain an injectable protein that will have an enhanced serum residence time. It is also known in the art that chemical groups, such as maleimide and alpha bromo carboxylates, react with the unpaired cysteine (residue 34) to form stable adducts. Thus, one could attach a single chelator to hSA fusion proteins so that the adduct will bind a radionuclide. One could prepare a chelator with a maleimide group and couple that to hSA or an hSA derivative. Alternatively, hSA or an hSA derivative could be reacted with a bismaleimide and a chelator carrying a reactive thiol could be reacted with the bismaleimide-derivatized hSA.

Construction of genes that encode a given amino-acid sequence are known in the art. Expression of HSA fusions in *Saccharomyces cerevisiae* is known in the art (Sleep, D et al., 1991. Biotechnology (NY), 9:183-187).

## Claims

1. A compound comprising an optical label conjugated directly or via a linker or spacer to at least one polypeptide having the ability to bind to KDR or VEGF/KDR complex comprising an amino acid sequence selected from:

   AGDSWCSTEYTYCEMIGTGGGK (SEQ ID NO: 4);
   AGPKWCEEDWYYCMITGTGGGK (SEQ ID NO: 5);
   AGVWECAKTFPFCHWFGTGGGK (SEQ ID NO: 6);
   AGWVECWWKSGQCYEFGTGGGK (SEQ ID NO: 7);
   AGWIQCNSITGHCTSGGTGGGK (SEQ ID NO: 8);
   AGWIECYHPDGICYHFGTGGGK (SEQ ID NO: 9);
   AGSDWCRVDWYYCWLMGTGGGK (SEQ ID NO: 10);
   AGANWCEEDWYYCFITGTGGGK (SEQ ID NO: 11);
   AGANWCEEDWYYCWITGTGGGK (SEQ ID NO: 12);
   AGPDWCEEDWYYCWITGTGGGK (SEQ ID NO: 13);
   AGSNWCEEDWYYCYITGTGGGK (SEQ ID NO: 14);
   AGPDWCAADWYYCYITGTGGGK (SEQ ID NO: 15);
   AGPEWCEVDWYYCWLLGTGGGK (SEQ ID NO: 16);
   AGPTWCEDDWYYCWLFGTGGGK (SEQ ID NO: 17);
   AGSKWCEQDWYYCWLLGTGGGK (SEQ ID NO: 18);
   AGRNWCEEDWYYCFITGTGGGK (SEQ ID NO: 19);
   AGVNWCEEDWYYCWITGTGGGK (SEQ ID NO: 20);
   AGANWCEEDWYYCYITGTGGGK (SEQ ID NO: 21);
   AGQAWVECYAETGYCWPRSWGTGGGK (SEQ ID NO: 22);
   AGQAWIECYAEDGYCWPRSWGTGGGK (SEQ ID NO: 23);
   AGVGWVECYQSTGFCYHSRDGTGGGK (SEQ ID NO: 24);
   AGDWWVECRVGTGLCYRYDTGTGGGK (SEQ ID NO: 25);
   AGDSWVECDAQTGFCYSFLYGTGGGK (SEQ ID NO: 26);
   AGERWVECRAETGFCYTWVSGTGGGK (SEQ ID NO: 27);
   AGGGWVECRAETGHCQEYRLGTGGGK (SEQ ID NO: 28);
   AGVAWVECYQTTGKCYTFRGGTGGGK (SEQ ID NO: 29);
   AGEGWVECFANTGACFTYPRGTGGGK (SEQ ID NO: 30);
   GDSRVCWEDSWGGEVCFRYDPGGGK (SEQ ID NO: 31);
   GDDSYCMMNEKGWWNCYLYDPGGGK (SEQ ID NO: 32);
   GDPAQCWESNYQGIFFCDNPDPGGGK (SEQ ID NO: 33);
   GDGWACAKWPWGGEICQPSDPGGGK (SEQ ID NO: 34);
   GDSSVCFEYSWGGEVCFRYDPGGGK (SEQ ID NO: 104);
   GDSRVCWEYSWGGQICLGYDPGGGK (SEQ ID NO: 105);
```

AQQVQYQFFLGTPRYEQWDLDKGGK (SEQ ID NO: 35);
AQEPEGYAYWEVITLYHEEDGDGGK (SEQ ID NO: 36);
AQAFPRFGGDDYWIQQYLRYTDGGK (SEQ ID NO: 37);
AQGDYVYWEIIELTGATDHTPPGGK (SEQ ID NO: 38);
AQRGDYQEQYWHQQLVEQLKLLGGK (SEQ ID NO: 39);
AQRSWYLGPPYYEEWDPIPNGGK (SEQ ID NO: 40);
AQDWYYDEILSMADQLRHAFLSGGGK (SEQ ID NO: 41);
AQAPAWTFGTNWRSIQRVDSLTGGGGGK (SEQ ID NO: 42);
AQEGWFRNPQEIMGFGDSWDKPGGGGGGK (SEQ ID NO: 43);
AQEGWFRNPQEIMGFGDSWDKPGGGK (SEQ ID NO: 44);
AQRGDYQEQYWHQQLVEQLKLLGGGK (SEQ ID NO: 45);
Ac-AGWYWCDYYGIGCK(ivDde)WTGGGK-NH$_2$(SEQ ID NO: 46);
Ac-AGWYWCDYYGIGCKWTGTGGGK-NH$_2$(SEQ ID NO: 47);
Ac-AQWYYDWFHNQRKPPSDWIDNLGGGK-NH$_2$(SEQ ID NO: 48);
Ac-VCWEDSWGGEVCFRYDPGGGK(biotin-JJ-)-NH$_2$ (SEQ ID NO: 49);
Ac-AGPTWCEDDWYYCWLFGTJK(biotin-JJ-)-NH$_2$ (SEQ ID NO: 50);
Ac-AQAHMPPWRPVAVDALFDWVEGG-GGGK(biotin-JJ-)-NH$_2$ (SEQ ID NO: 51);
Ac-AQAHMPPWWPLAVDAQEDWFEGG-GGGK(biotin-JJ-)-NH$_2$ (SEQ ID NO: 52);
Ac-AQAQMPPWWPLAVDALFDWFEGG-GGGK(biotin-JJ-)-NH$_2$ (SEQ ID NO: 53);
Ac-AQDWYWREWMPMHAQFLADDWGG-GGGK(biotin-JJ-)-NH$_2$ (SEQ ID NO: 54);
Ac-AQPVTDWTPHHPK(ivDde)APDVWLFYT-GGGGGK(biotin-JJ-)-NH$_2$ (SEQ ID NO: 55);
Ac-AQDALEAPK(ivDde)RDWYYDWFLNHSP-GGGGGK(biotin-JJ-)-NH$_2$ (SEQ ID NO: 56);
Ac-KWCEEDWYYCMITGTGGGK(biotin-JJ-)-NH$_2$ (SEQ ID NO: 57);
Ac-AGPKWCEEDWYYCMIGGGK(biotin-JJ-)-NH$_2$ (SEQ ID NO. 58);
Ac-KWCEEDWYYCMIGGGK(biotin-JJ-)-NH$_2$ (SEQ ID NO: 59);
Ac-AQPDNWK(ivDde)EFYESGWK(ivDde)-YPSLYK(ivDde)PLGGGGGK(biotin-JJ-)-NH$_2$ (SEQ ID NO: 60);
Ac-AQMPPGFSYWEQWLHDDAQVLGG-GGGK(biotin-JJ-)-NH$_2$ (SEQ ID NO: 61);
Ac-AQARMGDDWEEAPPHEWGWADGG-GGGK(biotin-JJ-)-NH$_2$ (SEQ ID NO: 62);
Ac-AQPEDSEAWYWLNYRPTMFHQLGG-GGGK(biotin-JJ-)-NH$_2$ (SEQ ID NO: 63);
Ac-AQSTNGDSFVYWEEVELVDHPGG-GGGK(biotin-JJ-)-NH$_2$ (SEQ ID NO: 64);
Ac-AQWESDYWDQMRQQLK(iv-Dde)TAYMK(iv-Dde)VGGGGGK(biotin-JJ-)-NH$_2$ (SEQ ID NO: 65);
Ac-AQDWYYDEILSMADQLRHAFLSGGGGGK(biotin-JJ-)-NH$_2$ (SEQ ID NO: 66);
VCWEDSWGGEVCFGGGK (SEQ ID NO: 76);
GDSRVCWEDSWGGEVCFGGGK (SEQ ID NO: 77);
SRVCWEDSWGGEVCFRYGGGGK (SEQ ID NO: 79);
GDSRVCWEDSWGGEVCFRYGGGK (SEQ ID NO: 80);
DWYYGGGK (SEQ ID NO: 95);
AQDWYYDEILGRGRGGRGG (SEQ ID NO: 96);
AGPTWEEDDWYYKWLFGTGGGK (SEQ ID NO: 90);
AGPTWKEDDWYYEWLFGTGGGK (SEQ ID NO: 91);
AGPTWDprEDDWYYDWLFGTGGGK (SEQ ID NO: 92);
AGPTWDEDDWYYDprWLFGTGGGK (SEQ ID NO: 93); and
AGPTWDEDDWYYKWLFGTGGGK (SEQ ID NO: 94), wherein J is the spacer or
linker group, 8-amino-3,6-dioxaoctanoyl, and iv-Dde is 1-(4,4-dimethyl-2,6-dioxocyclohex-1-ylidene)-3-methylbutyl.

2. The compound of claim 1, wherein the optical label is conjugated directly or via a linker or spacer to a dimeric or multimeric compound comprising two or more polypeptides having the ability to bind KDR or VEGF/KDR complex.

3. The compound of claim 1, wherein the compound comprises two or more polypeptides and the polypeptides have specificity for different epitopes on KDR, wherein the poly-peptides are preferably independently selected from the group consisting of:

AGPKWCEEDWYYCMITGTGGGK (SEQ ID NO: 5);
GDSRVCWEDSWGGEVCFRYDPGGGK (SEQ ID NO: 31);
AQDWYYDEILSMADQLRHAFLSGGK (SEQ ID NO: 41);
AGPTWCEDDWYYCWLFGTGGGK (SEQ ID NO: 17);
AGDWWVECRVGTGLCYRYDTGTGGGK (SEQ ID NO: 25); and

Ac-VCWEDSWGGEVCFRYDPGGGK(biotin-JJ-)-NH$_2$ (SEQ ID NO: 49).

4. The compound of any of claims 1 or 3, wherein one or more of the polypeptides do not include the C terminal GGGK extension in their amino acid sequence.

5. The compound of claim 3, wherein the polypeptides comprise any of the following combinations:

AGPTWCEDDWYYCWLFGTGGGK (SEQ ID NO: 17) and
Ac-VCWEDSWGGEVCFRYDPGGGK(biotin-JJ-)-NH$_2$ (SEQ ID NO: 49);
AGPTWCEDDWYYCWLFGTGGGK (SEQ ID NO: 17) and
GDSRVCWEDSWGGEVCFRYDPGGGK (SEQ ID NO: 31);
AGPKWCEEDWYYCMITGTGGGK (SEQ ID NO: 5) and
GDSRVCWEDSWGGEVCFRYDPGGGK (SEQ ID NO: 31); or
AQDWYYDEILSMADQLRHAFLSGGK (SEQ ID NO: 41) and
Ac-VCWEDSWGGEVCFRYDPGGGK(biotin-JJ-)-NH$_2$ (SEQ ID NO: 49).

6. The compound of claim 1, wherein the polypeptide comprises
AGPKWCEEDWYYCMITGTGGGK (SEQ ID NO: 5);
AGPTWCEDDWYYCWLFGTGGGK (SEQ ID NO: 17);
GDSRVCWEDSWGGEVCFRYDPGGGK (SEQ ID NO: 31);
AQDWYYDEILSMADQLRHAFLSGGK (SEQ ID NO: 41); or
Ac-VCWEDSWGGEVCFRYDPGGGK(biotin-JJ-)-NH$_2$ (SEQ ID NO: 49).

7. A compound comprising an optical label conjugated directly or via a linker or spacer to a dimer, the dimer comprising two polypeptides having the ability to bind KDR or VEGF/KDR complex, and wherein the dimer is selected from the group consisting of D1 (FIG.25), D2 (FIG.26), D3 (FIG.27), D5 (FIG.29), D6 (FIG.50), D7 (FIG.51), D8 (FIG.30), D9 (FIG.31), D17 (FIG.39), D19 (FIG.41), D20 (FIG.42), D22 (FIG.44), D23 (FIG.45), D24 (FIG.46), D25 (FIG.47), D26 (FIG.48), and D27 (FIG.49).

8. The compound of any one of the preceding claims, wherein the polypeptide is linked to the optical label via a linker or spacer, wherein said linker or spacer is preferably selected from the group consisting of a substituted alkyl chain, an unsubstituted alkyl chain, a polyethylene glycol derivative, an amino acid spacer, a sugar, an aliphatic spacer, an aromatic spacer, a lipid molecule, or combination thereof.

9. The compound of any one of the preceding claims, wherein the optical label comprises an optical dye, a bioluminescent molecule, a photoabsorption label or a fluorescent bead.

10. The compound of claim 9, wherein the optical label is an optical dye such as an organic chromophore or fluorophore, such as, for example, a fluorescent compound or wherein the optical label is a fluorescent bead.

11. The compound of claim 9, wherein the optical label comprises a bioluminescent molecule.

12. Use of a compound according to any one of claims 1 to 11 for producing a diagnostic composition for imaging, in particular for imaging important sites of angiogenesis, e.g., neoplastic tumors.

13. Use according to claim 12, wherein said imaging is for monitoring of a therapy with a tumorcidal agent or an anti-angiogenesis agent.

14. Use according to claim 12 or 13, wherein said label is detected by a parameter selected from transmitted radiation, absorption, fluorescent or phosphorescent emission, light reflection, changes in absorbance amplitude or maxima and elastically-scattered radiation.

**Patentansprüche**

1. Eine Verbindung umfassend ein optisches Label, das direkt oder über einen Linker oder Spacer konjugiert ist mit mindestens einem Polypeptid mit der Fähigkeit KDR oder den VEGF/KDR-Komplex zu binden, umfassend einer Aminosäuresequenz ausgesucht aus:

AGDSWCSTEYTYCEMIGTGGGK (SEQ ID Nr: 4);
AGPKWCEEDWYYCMITGTGGGK (SEQ ID Nr: 5);
AGVWECAKTFPFCHWFGTGGGK (SEQ ID Nr: 6);
AGWVECWWKSGQCYEFGTGGGK (SEQ ID Nr: 7);
AGWIQCNSITGHCTSGGTGGGK (SEQ ID Nr: 8);
AGWIECYHPDGICYHFGTGGGK (SEQ ID Nr: 9);
AGSDWCRVDWYYCWLMGTGGGK (SEQ ID Nr: 10);
AGANWCEEDWYYCFITGTGGGK (SEQ ID Nr: 11);
AGANWCEEDWYYCWITGTGGGK (SEQ ID Nr: 12);
AGPDWCEEDWYYCWITGTGGGK (SEQ ID Nr: 13);
AGSNWCEEDWYYCYITGTGGGK (SEQ ID Nr: 14);
AGPDWCAADWYYCYITGTGGGK (SEQ ID Nr: 15);
AGPEWCEVDWYYCWLLGTGGGK (SEQ ID Nr: 16);
AGPTWCEDDWYYCWLFGTGGGK (SEQ ID Nr: 17);
AGSKWCEQDWYYCWLLGTGGGK (SEQ ID Nr: 18);
AGRNWCEEDWYYCFITGTGGGK (SEQ ID Nr: 19);
AGVNWCEEDWYYCWITGTGGGK (SEQ ID Nr: 20);
AGANWCEEDWYYCYITGTGGGK (SEQ ID Nr: 21);
AGQAWVECYAETGYCWPRSWGTGGGK (SEQ ID Nr: 22);
AGQAWIECYAEDGYCWPRSWGTGGGK (SEQ ID Nr: 23);
AGVGWVECYQSTGFCYHSRDGTGGGK (SEQ ID Nr: 24);
AGDWWVECRVGTGLCYRYDTGTGGGK (SEQ ID Nr: 25);
AGDSWVECDAQTGFCYSFLYGTGGGK (SEQ ID Nr: 26);
AGERWVECRAETGFCYTWVSGTGGGK (SEQ ID Nr: 27);
AGGGWVECRAETGHCQEYRLGTGGGK (SEQ ID Nr: 28);
AGVAWVECYQTTGKCYTFRGGTGGGK (SEQ ID Nr: 29);
AGEGWVECFANTGACFTYPRGTGGGK (SEQ ID Nr: 30);
GDSRVCWEDSWGGEVCFRYDPGGGK (SEQ ID Nr: 31);
GDDSYCMMNEKGWWNCYLYDPGGGK (SEQ ID Nr: 32);
GDPAQCWESNYQGIFFCDNPDGGGK (SEQ ID Nr: 33);
GDGWACAKWPWGGEICQPSDGGGK (SEQ ID Nr: 34);
GDSSVCFEYSWGGEVCFRYDPGGGK (SEQ ID Nr: 104);
GDSRVCWEYSWGGQICLGYDPGGGK (SEQ ID Nr: 105);
AQQVQYQFFLGTPRYEQWDLDKGGK (SEQ ID Nr: 35);
AQEPEGYAYWEVITLYHEEDGDGGK (SEQ ID Nr: 36);
AQAFPRFGGDDYWIQQYLRYTDGGK (SEQ ID Nr: 37);
AQGDYVYWEIIELTGATDHTPPGGK (SEQ ID Nr: 38);
AQRGDYQEQYWHQQLVEQLKLLGGK (SEQ ID Nr: 39);
AQRSWYLGPPYYEEWDPIPNGGK (SEQ ID Nr: 40);
AQDWYYDEILSMADQLRHAFLSGGGK (SEQ ID Nr: 41);
AQAPAWTFGTNWRSIQRVDSLTGGGGGK (SEQ ID Nr: 42);
AQEGWFRNPQEIMGFGDSWDKPGGGGGK (SEQ ID Nr: 43);
AQEGWFRNPQEIMGFGDSWDKPGGGK (SEQ ID Nr: 44);
AQRGDYQEQYWHQQLVEQLKLLGGGK (SEQ ID Nr: 45);
Ac-AGWYWCDYYGIGCK(ivDde)WTGGGK-NH$_2$(SEQ ID NR: 46);
Ac-AGWYWCDYYGIGCKWTGTGGGK-NH$_2$(SEQ ID NR: 47);
Ac-AQWYYDWFHNQRKPPSDWIDNLGGGK-NH$_2$(SEQ ID NR: 48);
Ac-VCWEDSWGGEVCFRYDPGGGK(Biotin-JJ-)-NH$_2$ (SEQ ID NR: 49);
Ac-AGPTWCEDDWYYCWLFGTJK(Biotin-JJ-)-NH$_2$ (SEQ ID NR: 50);
Ac-AQAHMPPWRPVAVDALFDWVEGG-GGGK(Biotin-JJ-)-NH$_2$ (SEQ ID NR: 51);
Ac-AQAHMPPWWPLAVDAQEDWFEGG-GGGK(Biotin-JJ-)-NH$_2$ (SEQ ID NR: 52);
Ac-AQAQMPPWWPLAVDALFDWFEGG-GGGK(Biotin-JJ-)-NH$_2$ (SEQ ID NR: 53);
Ac-AQDWYWREWMPMHAQFLADDWGG-GGGK(Biotin-JJ-)-NH$_2$ (SEQ ID NR: 54);
Ac-AQPVTDWTPHHPK(ivDde)APDVWLFYT-GGGGGK(Biotin-JJ-)-NH$_2$ (SEQ ID NR: 55);
Ac-AQDALEAPK(ivDde)RDWYYDWFLNHSP-GGGGGK(Biotin-JJ-)-NH$_2$ (SEQ ID NR: 56);
Ac-KWCEEDWYYCMITGTGGGK(Biotin-JJ-)-NH$_2$ (SEQ ID NR: 57);
Ac-AGPKWCEEDWYYCMIGGGK(Biotin-JJ-)-NH$_2$ (SEQ ID NR. 58);
Ac-KWCEEDWYYCMIGGGK(Biotin-JJ-)-NH$_2$ (SEQ ID NR: 59);

Ac-AQPDNWK(ivDde)EFYESGWK(ivDde)-YPSLYK(ivDde)PLGGGGGK(Biotin-JJ-)-NH$_2$ (SEQ ID NR: 60);
Ac-AQMPPGFSYWEQVVLHDDAQVLGG-GGGK(Biotin-JJ-)-NH$_2$ (SEQ ID NR: 61);
Ac-AQARMGDDWEEAPPHEWGWADGG-GGGK(Biotin-JJ-)-NH$_2$ (SEQ ID NR: 62);
Ac-AQPEDSEAWYWLNYRPTMFHQLGG-GGGK(Biotin-JJ-)-NH$_2$ (SEQ ID NR: 63);
Ac-AQSTNGDSFVYWEEVELVDHPGG-GGGK(Biotin-JJ-)-NH$_2$ (SEQ ID NR: 64);
Ac-AQWESDYWDQMRQQLK(iv-Dde)TAYMK(iv-Dde)VGGGGGK(Biotin-JJ-)-NH$_2$ (SEQ ID NR: 65);
Ac-AQDWYYDEILSMADQLRHAFLSGGGGGK(Biotin-JJ-)-NH$_2$ (SEQ ID NR: 66);
VCWEDSWGGEVCFGGGK (SEQ ID Nr: 76);
GDSRVCWEDSWGGEVCFGGGK (SEQ ID Nr: 77);
SRVCWEDSWGGEVCFRYGGGGK (SEQ ID Nr: 79);
GDSRVCWEDSWGGEVCFRYGGGK (SEQ ID Nr: 80);
DWYYGGGK (SEQ ID Nr: 95);
AQDWYYDEILGRGRGGRGG (SEQ ID Nr: 96);
AGPTWEEDDWYYKWLFGTGGGK (SEQ ID Nr: 90);
AGPTWKEDDWYYEWLFGTGGGK (SEQ ID Nr: 91);
AGPTWDprEDDWYYDWLFGTGGGK (SEQ ID Nr: 92);
AGPTWDEDDWYYDprWLFGTGGGK (SEQ ID Nr: 93); und
AGPTWDEDDWYYKWLFGTGGGK (SEQ ID Nr: 94), wobei J die Spacer oder Linker Gruppe ist, 8-Amino-3,6-Dioxaoctanoyl, und iv-Dde 1-(4,4-Dimethyl-2,6-Dioxocyclohex-1-Yliden)-3-Methylbutyl ist.

2. Die Verbindung nach Anspruch 1, wobei das optische Label direkt oder über einen Linker oder Spacer konjugiert ist mit einer dimeren- oder multimeren Verbindung umfassend zwei oder mehr Polypeptide mit der Fähigkeit KDR oder den VEGF/KDR-Komplex zu binden

3. Verbindung nach Anspruch 1, wobei die Verbindung zwei oder mehr Polypeptide umfasst und die Polypeptide Spezifität für unterschiedliche Epitope auf KDR haben, wobei die Polypeptide vorzugsweise unabhängig ausgewählt werden aus der Gruppe bestehend aus:

AGPKWCEEDWYYCMITGTGGGK (SEQ ID Nr: 5);
GDSRVCWEDSWGGEVCFRYDPGGGK (SEQ ID Nr: 31);
AQDWYYDEILSMADQLRHAFLSGGK (SEQ ID Nr: 41);
AGPTWCEDDWYYCWLFGTGGGK (SEQ ID Nr: 17);
AGDWWVECRVGTGLCYRYDTGTGGGK (SEQ ID Nr: 25); und
Ac-VCWEDSWGGEVCFRYDPGGGK(Biotin-JJ-)-NH$_2$ (SEQ ID Nr: 49).

4. Verbindung nach einem der Ansprüche 1 oder 3, wobei ein oder mehrere der Polypeptide nicht die C-terminale Verlängerung GGGK in ihrer Aminosäuresequenz enthalten.

5. Die Verbindung nach Anspruch 3, wobei die Polypeptide eine der folgenden Kombinationen umfassen:

AGPTWCEDDWYYCWLFGTGGGK (SEQ ID Nr: 17) und
Ac-VCWEDSWGGEVCFRYDPGGGK(Biotin-JJ-)-NH$_2$ (SEQ ID NR: 49);
AGPTWCEDDWYYCWLFGTGGGK (SEQ ID Nr: 17) und
GDSRVCWEDSWGGEVCFRYDPGGGK (SEQ ID Nr: 31);
AGPKWCEEDWYYCMITGTGGGK (SEQ ID Nr: 5) und
GDSRVCWEDSWGGEVCFRYDPGGGK (SEQ ID Nr: 31); oder
AQDWYYDEILSMADQLRHAFLSGGK (SEQ ID Nr: 41) und
Ac-VCWEDSWGGEVCFRYDPGGGK(Biotin-JJ-)-NH$_2$ (SEQ ID NR: 49).

6. Verbindung nach Anspruch 1, wobei das Polypeptid umfasst:

AGPKWCEEDWYYCMITGTGGGK (SEQ ID Nr: 5);
AGPTWCEDDWYYCWLFGTGGGK (SEQ ID Nr: 17);
GDSRVCWEDSWGGEVCFRYDPGGGK (SEQ ID Nr: 31);
AQDWYYDEILSMADQLRHAFLSGGK (SEQ ID Nr: 41); oder
Ac-VCWEDSWGGEVCFRYDPGGGK(Biotin-JJ-)-NH$_2$ (SEQ ID NR: 49).

7. Eine Verbindung umfassend ein optisches Label, das direkt oder über einen Linker oder Spacer mit einem Dimer konjugiert ist, das Dimer umfassend zwei Polypeptide mit der Fähigkeit KDR oder den VEGF/KDR-Komplex zu binden, und wobei das Dimer ausgesucht wird aus der Gruppe bestehend aus D1 (FIG.25), D2 (FIG.26), D3 (FIG.27), D5 (FIG.29), D6 (FIG.50), D7 (FIG.51), D8 (FIG.30), D9 (FIG.31), D17 (FIG.39), D19 (FIG.41), D20 (FIG.42), D22 (FIG.44), D23 (FIG.45), D24 (FIG.46), D25 (FIG.47), D26 (FIG.48), und D27 (FIG.49).

8. Verbindung nach einem der vorhergehenden Ansprüche, wobei das Polypeptid an ein optisches Label über einen Linker oder Spacer gebunden ist, wobei der Linker oder Spacer vorzugsweise aus der Gruppe ausgewählt wird bestehend aus einer substituierten AlkylKette, eine unsubstituierte Alkylkette, einem Polyethylen-Glykolderivat, ein Aminosäure-Spacer, ein Zucker, ein aliphatischer Spacer, ein aromatischer Spacer, ein Lipidmolekül oder eine Kombination davon.

9. Die Verbindung nach einem der vorhergehenden Ansprüche, wobei das optische Label ein optischer Farbstoff, ein biolumineszierendes Molekül, ein Fotoabsorptions-Label oder ein fluoreszierendes Bead ist.

10. Verbindung nach Anspruch 9, wobei das optische Label ein optischer Farbstoff ist, wie ein organisches Chromophor oder Fluorophor, wie beispielsweise eine fluoreszierende Verbindung, oder wobei das optische Label ein fluoreszierendes Bead ist.

11. Die Verbindung nach Anspruch 9, wobei das optische Label ein biolumineszentes Molekül umfasst.

12. Verwendung einer Verbindung nach einem der Ansprüche 1 bis 11 zum Herstellen einer diagnostischen Zusammensetzung zur Bildgebung, insbesondere zur Bildgebung wichtiger Stellen der Angiogenese, z.B. neoplastische Tumore.

13. Verwendung nach Anspruch 12, wobei genannte Bildgebung zur Überwachung einer Therapie mit einem tumorizidalen Mittel oder einem Anti-Angiogenese-Mittel ist.

14. Verwendung nach Anspruch 12 oder 13, wobei das Label durch einen Parameter detektiert wird ausgesucht aus übertragener Strahlung, Absorption, Fluoreszenz-oder Phosphoreszenz-Emissions, Lichtreflexion, Änderungen der Absorption oder Amplitudenmaxima und elastisch gestreute Strahlung.


**Revendications**

1. Composé comprenant un marqueur optique conjugué directement ou par l'intermédiaire d'un lieur ou d'un espaceur à au moins un polypeptide ayant la capacité de se lier à KDR ou à un complexe VEGF/KDR comprenant une séquence d'acides aminés choisie parmi :

    AGDSWCSTEYTYCEMIGTGGGK (SEQ ID NO : 4) ;
    AGPKWCEEDWYYCMITGTGGGK (SEQ ID NO : 5) ;
    AGVWECAKTFPFCHWFGTGGGK (SEQ ID NO : 6) ;
    AGWVECWWKSGQCYEFGTGGGK (SEQ ID NO : 7) ;
    AGWIQCNSITGHCTSGGTGGGK (SEQ ID NO : 8) ;
    AGWIECYHPDGICYHFGTGGGK (SEQ ID NO : 9) ;
    AGSDWCRVDWYYCWLMGTGGGK (SEQ ID NO : 10) ;
    AGANWCEEDWYYCFITGTGGGK (SEQ ID NO : 11) ;
    AGANWCEEDWYYCWITGTGGGK (SEQ ID NO : 12) ;
    AGPDWCEEDWYYCWITGTGGGK (SEQ ID NO : 13) ;
    AGSNWCEEDWYYCYITGTGGGK (SEQ ID NO : 14) ;
    AGPDWCAADWYYCYITGTGGGK (SEQ ID NO : 15) ;
    AGPEWCEVDWYYCWLLGTGGGK (SEQ ID NO : 16) ;
    AGPTWCEDDWYYCWLFGTGGGK (SEQ ID NO : 17) ;
    AGSKWCEQDWYYCWLLGTGGGK (SEQ ID NO : 18) ;
    AGRNWCEEDWYYCFITGTGGGK (SEQ ID NO : 19) ;
    AGVNWCEEDWYYCWITGTGGGK (SEQ ID NO : 20) ;
    AGANWCEEDWYYCYITGTGGGK (SEQ ID NO : 21) ;
    AGQAWVECYAETGYCWPRSWGTGGGK (SEQ ID NO : 22) ;
    AGQAWIECYAEDGYCWPRSWGTGGGK (SEQ ID NO : 23) ;

AGVGWVECYQSTGFCYHSRDGTGGGK (SEQ ID NO : 24) ;
AGDWWVECRVGTGLCYRYDTGTGGGK (SEQ ID NO : 25) ;
AGDSWVECDAQTGFCYSFLYGTGGGK (SEQ ID NO : 26) ;
AGERWVECRAETGFCYTWVSGTGGGK (SEQ ID NO : 27) ;
AGGGWVECRAETGHCQEYRLGTGGGK (SEQ ID NO : 28) ;
AGVAWVECYQTTGKCYTFRGGTGGGK (SEQ ID NO : 29) ;
AGEGWVECFANTGACFTYPRGTGGGK (SEQ ID NO : 30) ;
GDSRVCWEDSWGGEVCFRYDPGGGK (SEQ ID NO : 31) ;
GDDSYCMMNEKGWWNCYLYDPGGGK (SEQ ID NO : 32) ;
GDPAQCWESNYQGIFFCDNPDPGGGK (SEQ ID NO : 33) ;
GDGWACAKWPWGGEICQPSDPGGGK (SEQ ID NO : 34) ;
GDSSVCFEYSWGGEVCFRYDPGGGK (SEQ ID NO : 104) ;
GDSRVCWEYSWGGQICLGYDPGGGK (SEQ ID NO : 105) ;
AQQVQYQFFLGTPRYEQWDLDKGGK (SEQ ID NO : 35) ;
AQEPEGYAYWEVITLYHEEDGDGGK (SEQ ID NO : 36) ;
AQAFPRFGGDDYWIQQYLRYTDGGK (SEQ ID NO : 37) ;
AQGDYVYWEIIELTGATDHTPPGGK (SEQ ID NO : 38) ;
AQRGDYQEQYWHQQLVEQLKLLGGK (SEQ ID NO : 39) ;
AQRSWYLGPPYYEEWDPIPNGGK (SEQ ID NO : 40) ;
AQDWYYDEILSMADQLRHAFLSGGGK (SEQ ID NO : 41) ;
AQAPAWTFGTNWRSIQRVDSLTGGGGGK (SEQ ID NO : 42) ;
AQEGWFRNPQEIMGFGDSWDKPGGGGGK (SEQ ID NO : 43) ;
AQEGWFRNPQEIMGFGDSWDKPGGGK (SEQ ID NO : 44) ;
AQRGDYQEQYWHQQLVEQLKLLGGGK (SEQ ID NO : 45) ;
Ac-AGWYWCDYYGIGCK(ivDde)WTGGGK-$NH_2$ (SEQ ID NO : 46) ;
Ac-AGWYWCDYYGIGCKWTGTGGGK-$NH_2$ (SEQ ID NO : 47) ;
Ac-AQWYYDWFHNQRKPPSDWIDNLGGGK-$NH_2$ (SEQ ID NO : 48) ;
Ac-VCWEDSWGGEVCFRYDPGGGK(biotine-JJ-)-$NH_2$ (SEQ ID NO : 49) ;
Ac-AGPTWCEDDWYYCWLFGTJK(biotine-JJ-)-$NH_2$ (SEQ ID NO : 50) ;
Ac-AQAHMPPWRPVAVDALFDWVEGG-GGGK(biotine-JJ-)-$NH_2$ (SEQ ID NO : 51) ;
Ac-AQAHMPPWWPLAVDAQEDWFEGG-GGGK(biotine-JJ-)-$NH_2$ (SEQ ID NO : 52) ;
Ac-AQAQMPPWWPLAVDALFDWFEGG-GGGK(biotine-JJ-)-$NH_2$ (SEQ ID NO : 53) ;
Ac-AQDWYWREWMPMHAQFLADDWGG-GGGK(biotine-JJ-)-$NH_2$ (SEQ ID NO : 54) ;
Ac-AQPVTDWTPHHPK(ivDde)-APDVWLFYT-GGGGGK(biotine-JJ-)-$NH_2$ (SEQ ID NO : 55) ;
Ac-AQDALEAPK(ivDde)RDWYYDWFLNHSP-GGGGGK(biotine-JJ-)-$NH_2$ (SEQ ID NO : 56) ;
Ac-KWCEEDWYYCMITGTGGGK(biotine-JJ-)-$NH_2$ (SEQ ID NO : 57) ;
Ac-AGPKWCEEDWYYCMIGGGK(biotine-JJ-)-$NH_2$ (SEQ ID NO : 58) ;
Ac-KWCEEDWYYCMIGGGK(biotine-JJ-)-$NH_2$ (SEQ ID NO : 59);
Ac-AQPDNWK(ivDde)EFYESGWK(ivDde)-YPSLYK(ivDde) PLGGGGGK(biotine-JJ-)-$NH_2$ (SEQ ID NO : 60) ;
Ac-AQMPPGFSYWEQVVLHDDAQVLGG-GGGK(biotine-JJ-)-$NH_2$ (SEQ ID NO : 61) ;
Ac-AQARMGDDWEEAPPHEWGWADGG-GGGK(biotine-JJ-)-$NH_2$ (SEQ ID NO : 62) ;
Ac-AQPEDSEAWYWLNYRPTMFHQLGG-GGGK(biotine-JJ-)-$NH_2$ (SEQ ID NO : 63) ;
Ac-AQSTNGDSFVYWEEVELVDHPGG-GGGK(biotine-JJ-)-$NH_2$ (SEQ ID NO : 64) ;
Ac-AQWESDYWDQMRQQLK(iv-Dde)TAYMK(iv-Dde)VGGGGK (biotine-JJ-)-$NH_2$ (SEQ ID NO : 65) ;
Ac-AQDWYYDEILSMADQLRHAFLSGGGGGK(biotine-JJ-)-$NH_2$ (SEQ ID NO : 66) ;
VCWEDSWGGEVCFGGGK (SEQ ID NO : 76) ;
GDSRVCWEDSWGGEVCFGGGK (SEQ ID NO : 77) ;
SRVCWEDSWGGEVCFRYGGGK (SEQ ID NO : 79) ;
GDSRVCWEDSWGGEVCFRYGGGK (SEQ ID NO : 80) ;
DWYYGGGK (SEQ ID NO : 95) ;
AQDWYYDEILGRGRGGRGG (SEQ ID NO : 96) ;
AGPTWEEDDWYYKWLFGTGGGK (SEQ ID NO : 90) ;
AGPTWKEDDWYYEWLFGTGGGK (SEQ ID NO : 91) ;
AGPTWDprEDDWYYDWLFGTGGGK (SEQ ID NO : 92) ;
AGPTWDEDDWYYDprWLFGTGGGK (SEQ ID NO : 93) ; et
AGPTWDEDDWYYKWLFGTGGGK (SEQ ID NO : 94), où J représente le groupe espaceur ou lieur, 8-amino-3,6-dioxaoctanoyle, et iv-Dde représente le 1-(4,4-diméthyl-2,6-dioxocyclohex-1-ylidène)-3-méthylbutyle.

**2.** Composé de la revendication 1, dans lequel le marqueur optique est conjugué directement ou par l'intermédiaire d'un lieur ou d'un espaceur à un composé dimère ou multimère comprenant deux polypeptides ou plus ayant la capacité de lier KDR ou un complexe VEGF/KDR.

**3.** Composé de la revendication 1, dans lequel le composé comprend deux polypeptides ou plus et les polypeptides ont une spécificité pour des épitopes différents sur KDR, où les polypeptides sont de préférence choisis indépendamment dans le groupe constitué de :

AGPKWCEEDWYYCMITGTGGGK (SEQ ID NO : 5) ;
GDSRVCWEDSWGGEVCFRYDPGGGK (SEQ ID NO : 31) ;
AQDWYYDEILSMADQLRHAFLSGGK (SEQ ID NO : 41) ;
AGPTWCEDDWYYCWLFGTGGGK (SEQ ID NO : 17) ;
AGDWWVECRVGTGLCYRYDTGTGGGK (SEQ ID NO : 25) ; et
Ac-VCWEDSWGGEVCFRYDPGGGK(biotine-JJ-)-NH$_2$ (SEQ ID NO : 49).

**4.** Composé de l'une des revendications 1 ou 3, dans lequel un ou plusieurs polypeptide(s) parmi les polypeptides ne comporte/comportent pas l'extension GGGK C terminale dans sa/leurs séquence(s) d'acides aminés.

**5.** Composé de la revendication 3, dans lequel les polypeptides comprennent l'une des combinaisons suivantes :

AGPTWCEDDWYYCWLFGTGGGK (SEQ ID NO : 17) et
Ac-VCWEDSWGGEVCFRYDPGGGK(biotine-JJ-)-NH$_2$ (SEQ ID NO : 49) ;
AGPTWCEDDWYYCWLFGTGGGK (SEQ ID NO : 17) et
GDSRVCWEDSWGGEVCFRYDPGGGK (SEQ ID NO : 31) ;
AGPKWCEEDWYYCMITGTGGGK (SEQ ID NO : 5) et
GDSRVCWEDSWGGEVCFRYDPGGGK (SEQ ID NO : 31) ; ou
AQDWYYDEILSMADQLRHAFLSGGK (SEQ ID NO : 41) et
Ac-VCWEDSWGGEVCFRYDPGGGK(biotine-JJ-)-NH$_2$ (SEQ ID NO : 49).

**6.** Composé de la revendication 1, dans lequel le polypeptide comprend
AGPKWCEEDWYYCMITGTGGGK (SEQ ID NO : 5) ;
AGPTWCEDDWYYCWLFGTGGGK (SEQ ID NO : 17) ;
GDSRVCWEDSWGGEVCFRYDPGGGK (SEQ ID NO : 31) ;
AQDWYYDEILSMADQLRHAFLSGGK (SEQ ID NO : 41) ; ou
Ac-VCWEDSWGGEVCFRYDPGGGK(biotine-JJ-)-NH$_2$ (SEQ ID NO : 49).

**7.** Composé comprenant un marqueur optique conjugué directement ou par l'intermédiaire d'un lieur ou d'un espaceur à un dimère, le dimère comprenant deux polypeptides ayant la capacité de lier KDR ou un complexe VEGF/KDR, et où le dimère est choisi dans le groupe constitué de D1 (figure 25), D2 (figure 26), D3 (figure 27), D5 (figure 29), D6 (figure 50), D7 (figure 51), D8 (figure 30), D9 (figure 31), D17 (figure 39), D19 (figure 41), D20 (figure 42), D22 (figure 44), D23 (figure 45), D24 (figure 46), D25 (figure 47), D26 (figure 48), et D27 (figure 49).

**8.** Composé de l'une quelconque des revendications précédentes, dans lequel le polypeptide est lié au marqueur optique par l'intermédiaire d'un lieur ou d'un espaceur, où ledit lieur ou espaceur est de préférence choisi dans le groupe constitué d'une chaîne alkyle substituée, d'une chaîne alkyle non substituée, d'un dérivé de polyéthylène glycol, d'un espaceur d'acides aminés, d'un sucre, d'un espaceur aliphatique, d'un espaceur aromatique, d'une molécule lipidique, ou d'une combinaison de ceux-ci.

**9.** Composé de l'une quelconque des revendications précédentes, dans lequel le marqueur optique comprend un colorant optique, une molécule bioluminescente, un marqueur de photoabsorption ou une bille fluorescente.

**10.** Composé de la revendication 9, dans lequel le marqueur optique est un colorant optique tel qu'un chromophore ou un fluorophore organique, comme, par exemple, un composé fluorescent ou dans lequel le marqueur optique est une bille fluorescente.

**11.** Composé de la revendication 9, dans lequel le marqueur optique comprend une molécule bioluminescente.

**12.** Utilisation d'un composé selon l'une quelconque des revendications 1 à 11 pour la production d'une composition de diagnostic pour l'imagerie, en particulier pour l'imagerie de sites importants de l'angiogenèse, par exemple, des tumeurs néoplasiques.

13. Utilisation selon la revendication 12, dans laquelle ladite imagerie est destinée à la surveillance d'une thérapie avec un agent tumoricide ou un agent anti-angiogenèse.

14. Utilisation selon la revendication 12 ou 13, dans laquelle ledit marqueur est détecté par un paramètre choisi parmi un rayonnement transmis, une absorption, une émission fluorescente ou phosphorescente, une réflexion de lumière, des changements de maximums ou d'amplitude d'absorbance et un rayonnement diffusé élastiquement.

## FIG. 1A

FIG. 1B

FIG. 2

# FIG. 3

(a) Ac-AGPKW<u>CEEDWYYC</u>MITGT-GGGK(Biotin-di(aminodioxaocta)-)-NH$_2$ (SEQ ID NO: 5)

(b) Ac-AGPKW<u>CEEDWYYC</u>MITGT-GGGK(Biotin-)-NH$_2$ (SEQ ID NO: 5)

(c) Ac-GDSRV<u>CWEDSWGGEVC</u>FRYDP-GGGK(Biotin-di(aminodioxaocta)-)-NH$_2$ (SEQ ID NO: 31)

(d) Ac-GDSRV<u>CWEDSWGGEVC</u>FRYDP-GGGK-(Biotin-)-NH$_2$ (SEQ ID NO: 31)

## FIG. 4

FIG. 5

FIG. 6

FIG. 7

EP 2 301 587 B1

FIG. 8

| Sequence | SEQ ID NO: | $K_D$, B ($\mu$M) |
|---|---|---|
| GDSRVCWEDSWGGEVCFRYDPGGGK | 31 | 0.069 |
| VCWEDSWGGEVCFGGGK | 76 | 0.91 |
| GDSRVCWEDSWGGEVCFGGGK | 77 | 1.30 |
| VCWEDSWGGEVCFRYDPGGGK | 78 | 0.040 |
| SRVCWEDSWGGEVCFRYGGGGK | 79 | 0.035 |
| GDSRVCWEDSWGGEVCFRYGGGK | 80 | 0.060 |

EP 2 301 587 B1

# FIG. 9

FIG. 10

FIG. 11

FIG. 12

EP 2 301 587 B1

EP 2 301 587 B1

FIG. 13

-V  +V  +V
+ SEQ ID NO:38

◄— Phospho KDR

◄— Total KDR

FIG. 14

FIG. 15

FIG. 16

FIG. 17

## FIG. 18

**SEC Chromatograms of $^{125}$I-DX-1235 in Mouse Plasma**

FIG. 19

HUVEC and KDR peptides, proliferation, 020613.

EP 2 301 587 B1

FIG. 20A

BRLI-888 over 2700RU rmKDR-Fc

FIG. 20B

BRU-888 over 6000RU rmKDR-Fc

## FIG. 21A

DX-1234 over 5000RU rmKDR-Fc

## FIG.21B

DX-1234 over 2700RU rmKDR-Fc

FIG. 22A

DX-955 over 5000RU mKDR-Fc

FIG. 22B

DX-955 over 2700RU mkDR-Fc

# FIG. 23

Fmoc-Dap(Boc-Ser(t-Bu))-OH

4

Fmoc-Dap(Trt-Aoa)-OH

5

# FIG. 24

18

19

EP 2 301 587 B1

# FIG. 25

# FIG. 26

EP 2 301 587 B1

FIG. 27

# FIG. 28

EP 2 301 587 B1

EP 2 301 587 B1

# FIG. 29

FIG. 30

FIG. 31

Ac-AQDWYYDEILSMADQLRHAFLSGGGGG-NH

Ac-GDSRVCWEDSWGGEVCFRYDPGGG-NH

EP 2 301 587 B1

FIG.32

Ac-AGPTWCEDDWYYCWLFGTGGG-NH

Ac-GDSRVCWEDSWGGEVCFRYDPGGG-NH

FIG.33

EP 2 301 587 B1

FIG.34

EP 2 301 587 B1

FIG.35

EP 2 301 587 B1

FIG.36

EP 2 301 587 B1

FIG.37

EP 2 301 587 B1

Ac-AGPTWCEDDWYYCWLFGTGGG-NH

Ac-GDSRVCWEDSWGGEVCFRYDPGGG-NH

FIG.38

FIG.39

FIG.40

FIG.41

FIG. 42

VCWEDSWGGEVCFRYDPGGG-NH₂

AGPTWCEDDWYYCWLRGTGGGK-NH₂

# FIG.43

FIG.44

FIG. 45

FIG. 46

FIG. 47

FIG.48

FIG.49

FIG.50

FIG.51

FIG.52

FIG.53

EP 2 301 587 B1

Ac-AGPTWCEDDWYYCWLFGTGGGK(iV-Dde)-NH─────(Resin) (Pal-PEG-PS)

**1**

EP 2 301 587 B1

FIG.54

1. 10% hydrazine in DMF (2 x 10 min)
2. Fmoc-Lys(iV-Dde)-OH/HOBt/DIC/DMF
3. 20% piperidine in DMF (2 x 10 min)
4. Fmoc-NH-JJ-Biotin/HOBt/DIC/DMF
5. NH₂NH₂/DMF (10%, 2 x 10 min)
6. Fmoc-Ser(GalNAc(Ac)₃-α-D)-OH/HATU/DIEA/DMF
7. 20% piperidine in DMF (2 x 10 min)
8. Fmoc-Gly-OH/HOBt/DIC/DMF
9. 20% piperidine/DMF (2 x 10 min)
10. Fmoc-Ser(GalNAc(Ac)₃-α-D)-OH/HATU/DIEA/DMF
11. 20% piperidine in DMF (2 x 10 min)
12. Reagent B
13. DMSO/aq. N-Methylglucamine/pH 8/air/2 days

Ac-AGPTWCEDDWYYCWLFGTGGG

**2**

Ac-VCWEDSWGGEVCFRYDPGGGK(iV-Dde)-NH———(Resin) (Pal-PEG-PS)

**3**

**FIG.55**

1. 10% hydrazine in DMF (2 x 10 min)
2. Fmoc-Ser(GalNAc(Ac)$_3$-α-D)-OH/HATU/DIEA/DMF
3. 20% piperidine in DMF (2 x 10 min)
4. Fmoc-Gly-OH/HOBt/DIC/DMF
5. 20% piperidine in DMF (2 x 10 min)
6. Fmoc-Ser(GalNAc(Ac)$_3$-α-D)-OH/HATU/DIEA/DMF
7. 20% piperidine in DMF (2 x 10 min)
8. reagent B
9. DMSO/N-methylglucamine/pH 8/air/2 days

Ac-VCWEDSWGGEVCFRYDPGGG———N(H)—CONH$_2$

**4**

HN—Ser(GalNAc(Ac)$_3$-alpha-D)

Gly—Ser(GalNAc(Ac)$_3$-alpha-D)

H$_2$N

DMF/DIEA

**4** +

Ac-VCWEDSWGGEVCFRYDPGGG

CONH₂

HN

Gly

HN

Ser(GalNAc(Ac)₃-alpha-D)

Ser(GalNAc(Ac)₃-alpha-D)

**5**

compound **2** / DIEA / DMF

NH₂NH₂/MeOH (15% v/v)

# FIG.56

CONH₂

Ac-AGPTWCEDDWYYCWLFGTGGG — N
H

N
H
—JJ-Biotin

Ac-VCWEDSWGGEVCFRYDPGGG

CONH₂

NH

(GalNAc-alpha-D)Ser

Gly

(GalNAc-alpha-D)Ser

HN

Gly

Ser(GalNAc-alpha-D)

Ser(GalNAc-alpha-D)

**6**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 9958162 A
- US 440201 P
- US 2004018974 A
- US 5223409 A, Ladner
- WO 9818497 A
- WO 9818496 A
- WO 9818495 A
- WO 9818498 A
- WO 9853857 A
- WO 9617628 A
- WO 9718841 A
- WO 9623524 A
- WO 9847538 A
- US 5171298 A
- WO 9857666 A
- US 6165458 A, Foldvari
- US 6274166 B1, Sintov
- US 6312665 B1, Pankaj Modi
- US 4391797 A, Folkman
- WO 0191805 A2
- EP 10185498 A
- US 60360851 B
- US 60440411 B

### Non-patent literature cited in the description

- **PEPPER, M. et al.** *Enzyme Protein,* 1996, vol. 49, 138-162
- **RISAU, W.** *Nature,* 1997, vol. 386, 671-674
- **KIM, K. et al.** *Nature,* 1993, vol. 362, 841-844
- **HANAHAN, D. ; FOLKMAN, J.** *Cell,* 1996, vol. 86, 353-364
- **NEUFELD, G. et al.** *FASEB J.,* 1999, vol. 13, 9-22
- **FUKUMURA, D. et al.** *Cell,* 1998, vol. 94, 715-725
- **BIKFALVI, A. et al.** *J. Cell. Physiol.,* 1991, vol. 149, 50-59
- **VEIKKOLA, T. et al.** *Cancer Res.,* 2000, vol. 60, 203-212
- **KAY et al.** Phage Display of Peptides and Proteins: A Laboratory Manual. Academic Press, Inc, 1996
- **STEWART et al.** Solid-Phase Peptide Synthesis. W. H. Freeman Co, 1989
- **MERRIFIELD.** *J. Am. Chem. Soc.,* 1963, vol. 85, 2149-2154
- **BODANSZKY ; BODANSZKY.** The Practice of Peptide Synthesis. Springer-Verlag, 1984
- **DAVIS et al.** *Basic Methods in Molecular Biology,* 1986
- **ANDERSON ; MILLER.** *Clin. Chem.,* 1988, vol. 34 (7), 1417-21
- **MALMBORG et al.** *J. Immunol. Methods,* 1996, vol. 198 (1), 51-7
- **SCHUCK.** *Current Opinion in Biotechnology,* 1997, vol. 8, 498-502
- **HUTCHINSON.** *Molec. Biote,* 1995, vol. 3, 47-54
- **HILLER et al.** *Biochem. J.,* 1987, vol. 248, 167-171
- **ALON et al.** *Biochem. Biophys. Res. Commun.,* 1990, vol. 170, 1236-41
- **KIHLBERG.** Glycopeptide synthesis. In: Fmoc Solid Phase Peptide Synthesis - A Practical Approach. Oxford University Press, January 2000, 195-213
- **WAHL ; MUTTER.** *Tetrahedron Lett.,* 1996, vol. 37, 6861-6864
- **BACKES et al.** *J. Am. Chem. Soc.,* 1996, vol. 118 (12), 3055-56
- Principles and Practice of Solid Phase Synthesis. **FIELDS et al.** Synthetic Peptides, A Users Guide. W.H. Freeman Co, 1992, 77-183
- Convergent Peptide Synthesis. **BARLOS et al.** Fmoc Solid Phase Peptide Synthesis. Oxford University Press, 2000, 215-228
- Synthesis of Modified Peptides. **MELLOR et al.** Fmoc Solid Phase Peptide Synthesis. Oxford University Press, 2000, 169-176
- **FALORNI et al.** *Tetrahedron Lett.,* 1998, vol. 39 (41), 7607-7610
- **JOHNSON et al.** *Tetrahedron Lett.,* 1998, vol. 54 (16), 4097-4106
- **STANKOVA et al.** *Mol. Diversity,* 1996, vol. 2, 75-80
- Remington: The Science and Practice of Pharmacy. Lippincott, Williams and Wilkins, 2000
- **FAIRBROTHER et al.** *Biochemistry,* 1998, vol. 37 (51), 17754-17764
- **LAEMMLI.** *Nature,* 1970, vol. 227, 680-685
- **MATSUDAIRA.** *J. Biol. Chem.,* 1987, vol. 262, 10035-10038

- **STRAWN, L. et al.** *Cancer Res.,* 1996, vol. 56, 3540-3545
- Points to Consider in the Characterization of Cell Lines used to Produce Biologicals. *Points to Consider in the Characterization of Cell Lines used to Produce Biologicals,* 1993
- Guide for the Care and Use of Laboratory Animals. National Academy Press, 1996
- **HOLMES, D. et al.** *Bioconjug. Chem.,* 2000, vol. 11, 439-444
- **ROBERTS M. et al.** *Adv. Drug. Deliv. Rev.,* 2002, vol. 54, 459-476
- **SLEEP, D et al.** *Biotechnology (NY),* 1991, vol. 9, 183-187

SEQUENCE LISTING

<110> Dyax Corp. BRACCO International B.V.

<120> KDR and VEGF/KDR Binding Peptides and Their Use in Diagnosis and Therapy

<130> FB14882/B

<140> EP 10185498.2
<141> 2003-03-03

<150> US 60/360,851
<151> 2002-03-01

<150> US 60/440,411
<151> 2003-01-15

<160> 107

<170> FastSEQ for Windows Version 4.0

<210> 1
<211> 14
<212> PRT
<213> Artificial Sequence

<220>

<223> Library Isolate

<400> 1

```
         Pro Lys Trp Cys Glu Glu Asp Trp Tyr Tyr Cys Met Ile Thr
          1               5               10
```

<210> 2
<211> 14
<212> PRT
<213> Artificial Sequence

<220>
<223> Library Isolate

<400> 2

```
         Trp Val Glu Cys Phe Met Asp Thr Gly Ala Cys Tyr Thr Phe
          1               5               10
```

<210> 3
<211> 17
<212> PRT
<213> Artificial Sequence

<220>
<223> Library Isolate

<400> 3

```
         Ser Arg Val Cys Trp Glu Asp Ser Trp Gly Gly Glu Val Cys Phe Arg
          1               5               10                  15


                                      Tyr
```

<210> 4
<211> 22
<212> PRT
<213> Artificial Sequence

<220>
<223> Synthesized KDR-Binding Polypeptide

<400> 4

```
         Ala Gly Asp Ser Trp Cys Ser Thr Glu Tyr Thr Tyr Cys Glu Met Ile
          1               5               10                  15
         Gly Thr Gly Gly Gly Lys
                     20
```

<210> 5
<211> 22
<212> PRT
<213> Artificial Sequence

<220>
<223> Synthesized KDR-Binding Polypeptide

<400> 5

```
Ala Gly Pro Lys Trp Cys Glu Glu Asp Trp Tyr Tyr Cys Met Ile Thr
1               5               10              15
Gly Thr Gly Gly Gly Lys
            20
```

<210> 6
<211> 22
<212> PRT
<213> Artificial Sequence

<220>
<223> Synthesized KDR-Binding Polypeptide

<400> 6

```
Ala Gly Val Trp Glu Cys Ala Lys Thr Phe Pro Phe Cys His Trp Phe
1               5               10              15
Gly Thr Gly Gly Gly Lys
            20
```

<210> 7
<211> 22
<212> PRT
<213> Artificial Sequence

<220>
<223> Synthesized KDR-Binding Polypeptide

<400> 7

```
Ala Gly Trp Val Glu Cys Trp Trp Lys Ser Gly Gln Cys Tyr Glu Phe
1               5               10              15
Gly Thr Gly Gly Gly Lys
```

20

<210> 8
<211> 22
<212> PRT
<213> Artificial Sequence

<220>
<223> Synthesized KDR-Binding Polypeptide

<400> 8

```
Ala Gly Trp Ile Gln Cys Asn Ser Ile Thr Gly His Cys Thr Ser Gly
1               5               10              15
Gly Thr Gly Gly Gly Lys
            20
```

<210> 9
<211> 22
<212> PRT
<213> Artificial Sequence

<220>
<223> Synthesized KDR-Binding Polypeptide

<400> 9

```
Ala Gly Trp Ile Glu Cys Tyr His Pro Asp Gly Ile Cys Tyr His Phe
1               5                   10                  15
Gly Thr Gly Gly Gly Lys
            20
```

<210> 10
<211> 22
<212> PRT
<213> Artificial Sequence

<220>
<223> Synthesized KDR-Binding Polypeptide

<400> 10

```
Ala Gly Ser Asp Trp Cys Arg Val Asp Trp Tyr Tyr Cys Trp Leu Met
1               5                   10                  15
Gly Thr Gly Gly Gly Lys
            20
```

<210> 11
<211> 22
<212> PRT
<213> Artificial Sequence

<220>
<223> Synthesized KDR-Binding Polypeptide

<400> 11

```
Ala Gly Ala Asn Trp Cys Glu Glu Asp Trp Tyr Tyr Cys Phe Ile Thr
1               5                   10                  15
Gly Thr Gly Gly Gly Lys
            20
```

<210> 12
<211> 22
<212> PRT
<213> Artificial Sequence

<220>
<223> Synthesized KDR-Binding Polypeptide

<400> 12

```
Ala Gly Ala Asn Trp Cys Glu Glu Asp Trp Tyr Tyr Cys Trp Ile Thr
1                   5                   10                  15
Gly Thr Gly Gly Gly Lys
                20
```

<210> 13
<211> 22
<212> PRT
<213> Artificial Sequence

<220>
<223> Synthesized KDR-Binding Polypeptide

<400> 13

```
Ala Gly Pro Asp Trp Cys Glu Glu Asp Trp Tyr Tyr Cys Trp Ile Thr
1                   5                   10                  15
Gly Thr Gly Gly Gly Lys
                20
```

<210> 14
<211> 22
<212> PRT
<213> Artificial Sequence

<220>
<223> Synthesized KDR-Binding Polypeptide

<400> 14

```
Ala Gly Ser Asn Trp Cys Glu Glu Asp Trp Tyr Tyr Cys Tyr Ile Thr
1                   5                   10                  15
Gly Thr Gly Gly Gly Lys
                20
```

<210> 15
<211> 22
<212> PRT
<213> Artificial Sequence

<220>
<223> Synthesized KDR-Binding Polypeptide

<400> 15

```
Ala Gly Pro Asp Trp Cys Ala Ala Asp Trp Tyr Tyr Cys Tyr Ile Thr
1                   5                   10                  15
Gly Thr Gly Gly Gly Lys
                20
```

<210> 16
<211> 22
<212> PRT
<213> Artificial Sequence

<220>
<223> Synthesized KDR-Binding Polypeptide

<400> 16

```
    Ala Gly Pro Glu Trp Cys Glu Val Asp Trp Tyr Tyr Cys Trp Leu Leu
    1               5               10              15
    Gly Thr Gly Gly Gly Lys
                20
```

<210> 17
<211> 22
<212> PRT
<213> Artificial Sequence

<220>
<223> Synthesized KDR-Binding Polypeptide

<400> 17

```
    Ala Gly Pro Thr Trp Cys Glu Asp Asp Trp Tyr Tyr Cys Trp Leu Phe
    1               5               10              15
    Gly Thr Gly Gly Gly Lys
                20
```

<210> 18
<211> 22
<212> PRT
<213> Artificial Sequence

<220>
<223> Synthesized KDR-Binding Polypeptide

<400> 18

```
    Ala Gly Ser Lys Trp Cys Glu Gln Asp Trp Tyr Tyr Cys Trp Leu Leu
    1               5               10              15
    Gly Thr Gly Gly Gly Lys
                20
```

<210> 19
<211> 22
<212> PRT
<213> Artificial Sequence

<220>
<223> Synthesized KDR-Binding Polypeptide

<400> 19

```
    Ala Gly Arg Asn Trp Cys Glu Glu Asp Trp Tyr Tyr Cys Phe Ile Thr
    1               5               10              15
    Gly Thr Gly Gly Gly Lys
                20
```

<210> 20
<211> 22
<212> PRT
<213> Artificial Sequence

<220>

<223> Synthesized KDR-Binding Polypeptide

<400> 20

```
Ala Gly Val Asn Trp Cys Glu Glu Asp Trp Tyr Tyr Cys Trp Ile Thr
1               5                   10              15
Gly Thr Gly Gly Gly Lys
            20
```

<210> 21
<211> 22
<212> PRT
<213> Artificial Sequence

<220>
<223> Synthesized KDR-Binding Polypeptide

<400> 21

```
Ala Gly Ala Asn Trp Cys Glu Glu Asp Trp Tyr Tyr Cys Tyr Ile Thr
1               5                   10              15
Gly Thr Gly Gly Gly Lys
            20
```

<210> 22
<211> 26
<212> PRT
<213> Artificial Sequence

<220>
<223> Synthesized KDR-Binding Polypeptide

<400> 22

```
Ala Gly Gln Ala Trp Val Glu Cys Tyr Ala Glu Thr Gly Tyr Cys Trp
1               5                   10              15
Pro Arg Ser Trp Gly Thr Gly Gly Gly Lys
            20                  25
```

<210> 23
<211> 26
<212> PRT
<213> Artificial Sequence

<220>
<223> Synthesized KDR-Binding Polypeptide

<400> 23

```
Ala Gly Gln Ala Trp Ile Glu Cys Tyr Ala Glu Asp Gly Tyr Cys Trp
1               5                   10              15
Pro Arg Ser Trp Gly Thr Gly Gly Gly Lys
            20                  25
```

<210> 24
<211> 26
<212> PRT
<213> Artificial Sequence

<220>
<223> Synthesized KDR-Binding Polypeptide

<400> 24

```
Ala Gly Val Gly Trp Val Glu Cys Tyr Gln Ser Thr Gly Phe Cys Tyr
1               5                   10                  15
His Ser Arg Asp Gly Thr Gly Gly Lys
                20                  25
```

<210> 25
<211> 26
<212> PRT
<213> Artificial Sequence

<220>
<223> Synthesized KDR-Binding Polypeptide

<400> 25

```
Ala Gly Asp Trp Trp Val Glu Cys Arg Val Gly Thr Gly Leu Cys Tyr
1               5                   10                  15
Arg Tyr Asp Thr Gly Thr Gly Gly Gly Lys
                20                  25
```

<210> 26
<211> 26
<212> PRT
<213> Artificial Sequence

<220>
<223> Synthesized KDR-Binding Polypeptide

<400> 26

```
Ala Gly Asp Ser Trp Val Glu Cys Asp Ala Gln Thr Gly Phe Cys Tyr
1               5                   10                  15
Ser Phe Leu Tyr Gly Thr Gly Gly Gly Lys
                20                  25
```

<210> 27
<211> 26
<212> PRT
<213> Artificial Sequence

<220>
<223> Synthesized KDR-Binding Polypeptide

<400> 27

```
Ala Gly Glu Arg Trp Val Glu Cys Arg Ala Glu Thr Gly Phe Cys Tyr
1               5                   10                  15
Thr Trp Val Ser Gly Thr Gly Gly Gly Lys
                20                  25
```

<210> 28
<211> 26
<212> PRT

<213> Artificial Sequence

<220>
<223> Synthesized KDR-Binding Polypeptide

<400> 28

```
    Ala Gly Gly Gly Trp Val Glu Cys Arg Ala Glu Thr Gly His Cys Gln
    1               5                   10                  15
    Glu Tyr Arg Leu Gly Thr Gly Gly Gly Lys
                20                  25
```

<210> 29
<211> 26
<212> PRT
<213> Artificial Sequence

<220>
<223> Synthesized KDR-Binding Polypeptide

<400> 29

```
    Ala Gly Val Ala Trp Val Glu Cys Tyr Gln Thr Thr Gly Lys Cys Tyr
    1               5                   10                  15
    Thr Phe Arg Gly Gly Thr Gly Gly Gly Lys
                20                  25
```

<210> 30
<211> 26
<212> PRT
<213> Artificial Sequence

<220>
<223> Synthesized KDR-Binding Polypeptide

<400> 30

```
    Ala Gly Glu Gly Trp Val Glu Cys Phe Ala Asn Thr Gly Ala Cys Phe
    1               5                   10                  15
    Thr Tyr Pro Arg Gly Thr Gly Gly Gly Lys
                20                  25
```

<210> 31
<211> 25
<212> PRT
<213> Artificial Sequence

<220>
<223> Synthesized KDR-Binding Polypeptide

<400> 31

```
    Gly Asp Ser Arg Val Cys Trp Glu Asp Ser Trp Gly Gly Glu Val Cys
    1               5                   10                  15
    Phe Arg Tyr Asp Pro Gly Gly Gly Lys
                20                  25
```

<210> 32

<211> 25
<212> PRT
<213> Artificial Sequence

<220>
<223> Synthesized KDR-Binding Polypeptide

<400> 32

```
Gly Asp Asp Ser Tyr Cys Met Met Asn Glu Lys Gly Trp Trp Asn Cys
1               5                   10                  15
Tyr Leu Tyr Asp Pro Gly Gly Gly Lys
            20                  25
```

<210> 33
<211> 26
<212> PRT
<213> Artificial Sequence

<220>
<223> Synthesized KDR-Binding Polypeptide

<400> 33

```
Gly Asp Pro Ala Gln Cys Trp Glu Ser Asn Tyr Gln Gly Ile Phe Phe
1               5                   10                  15
Cys Asp Asn Pro Asp Pro Gly Gly Gly Lys
            20                  25
```

<210> 34
<211> 25
<212> PRT
<213> Artificial Sequence

<220>
<223> Synthesized KDR-Binding Polypeptide

<400> 34

```
Gly Asp Gly Trp Ala Cys Ala Lys Trp Pro Trp Gly Gly Glu Ile Cys
1               5                   10                  15
Gln Pro Ser Asp Pro Gly Gly Gly Lys
            20                  25
```

<210> 35
<211> 25
<212> PRT
<213> Artificial Sequence

<220>
<223> Synthesized KDR-Binding Polypeptide

<400> 35

```
Ala Gln Gln Val Gln Tyr Gln Phe Phe Leu Gly Thr Pro Arg Tyr Glu
1               5                   10                  15
Gln Trp Asp Leu Asp Lys Gly Gly Lys
            20                  25
```

<210> 36
<211> 25
<212> PRT
<213> Artificial Sequence

<220>
<223> Synthesized KDR-Binding Polypeptide

<400> 36

```
Ala Gln Glu Pro Glu Gly Tyr Ala Tyr Trp Glu Val Ile Thr Leu Tyr
1               5                   10                  15
His Glu Glu Asp Gly Asp Gly Gly Lys
                20                  25
```

<210> 37
<211> 25
<212> PRT
<213> Artificial Sequence

<220>
<223> Synthesized KDR-Binding Polypeptide

<400> 37

```
Ala Gln Ala Phe Pro Arg Phe Gly Gly Asp Asp Tyr Trp Ile Gln Gln
1               5                   10                  15
Tyr Leu Arg Tyr Thr Asp Gly Gly Lys
                20                  25
```

<210> 38
<211> 25
<212> PRT
<213> Artificial Sequence

<220>
<223> Synthesized KDR-Binding Polypeptide

<400> 38

```
Ala Gln Gly Asp Tyr Val Tyr Trp Glu Ile Ile Glu Leu Thr Gly Ala
1               5                   10                  15
Thr Asp His Thr Pro Pro Gly Gly Lys
                20                  25
```

<210> 39
<211> 25
<212> PRT
<213> Artificial Sequence

<220>
<223> Synthesized KDR-Binding Polypeptide

<400> 39

64

```
        Ala Gln Arg Gly Asp Tyr Gln Glu Gln Tyr Trp His Gln Gln Leu Val
        1                   5                   10                  15
        Glu Gln Leu Lys Leu Leu Gly Gly Lys
                        20                  25
```

<210> 40
<211> 23
<212> PRT
<213> Artificial Sequence

<220>
<223> Synthesized KDR-Binding Polypeptide

<400> 40

```
        Ala Gln Arg Ser Trp Tyr Leu Gly Pro Pro Tyr Tyr Glu Glu Trp Asp
        1                   5                   10                  15
        Pro Ile Pro Asn Gly Gly Lys
                        20
```

<210> 41
<211> 26
<212> PRT
<213> Artificial Sequence

<220>
<223> Synthesized KDR-Binding Polypeptide

<400> 41

```
        Ala Gln Asp Trp Tyr Tyr Asp Glu Ile Leu Ser Met Ala Asp Gln Leu
        1                   5                   10                  15
        Arg His Ala Phe Leu Ser Gly Gly Gly Lys
                        20                  25
```

<210> 42
<211> 28
<212> PRT
<213> Artificial Sequence

<220>
<223> KDR or KDR/VEGF Complex Binding Polypeptide

<400> 42

```
        Ala Gln Ala Pro Ala Trp Thr Phe Gly Thr Asn Trp Arg Ser Ile Gln
        1                   5                   10                  15
        Arg Val Asp Ser Leu Thr Gly Gly Gly Gly Lys
                        20                  25
```

<210> 43
<211> 28
<212> PRT
<213> Artificial Sequence

<220>
<223> KDR or KDR/VEGF Complex Binding Polypeptide

<400> 43

```
Ala Gln Glu Gly Trp Phe Arg Asn Pro Gln Glu Ile Met Gly Phe Gly
1               5                   10                  15
Asp Ser Trp Asp Lys Pro Gly Gly Gly Gly Lys
            20                  25
```

<210> 44
<211> 26
<212> PRT
<213> Artificial Sequence

<220>
<223> KDR or KDR/VEGF Complex Binding Polypeptide

<400> 44

```
Ala Gln Glu Gly Trp Phe Arg Asn Pro Gln Glu Ile Met Gly Phe Gly
1               5                   10                  15
Asp Ser Trp Asp Lys Pro Gly Gly Gly Lys
            20                  25
```

<210> 45
<211> 26
<212> PRT
<213> Artificial Sequence

<220>
<223> KDR or KDR/VEGF Complex Binding Polypeptide

<400> 45

```
Ala Gln Arg Gly Asp Tyr Gln Glu Gln Tyr Trp His Gln Gln Leu Val
1               5                   10                  15
Glu Gln Leu Lys Leu Leu Gly Gly Gly Lys
            20                  25
```

<210> 46
<211> 20
<212> PRT
<213> Artificial Sequence

<220>
<223> KDR or KDR/VEGF Complex Binding Polypeptide

<400> 46

```
Ala Gly Trp Tyr Trp Cys Asp Tyr Tyr Gly Ile Gly Cys Lys Trp Thr
1               5                   10                  15
Gly Gly Gly Lys
            20
```

<210> 47
<211> 22
<212> PRT
<213> Artificial Sequence

<220>

<223> KDR or KDR/VEGF Complex Binding Polypeptide

<400> 47

```
Ala Gly Trp Tyr Trp Cys Asp Tyr Tyr Gly Ile Gly Cys Lys Trp Thr
1               5                   10                  15
Gly Thr Gly Gly Gly Lys
            20
```

<210> 48
<211> 26
<212> PRT
<213> Artificial Sequence

<220>
<223> KDR or KDR/VEGF Complex Binding Polypeptide

<400> 48

```
Ala Gln Trp Tyr Tyr Asp Trp Phe His Asn Gln Arg Lys Pro Pro Ser
1               5                   10                  15
Asp Trp Ile Asp Asn Leu Gly Gly Gly Lys
            20                  25
```

<210> 49
<211> 21
<212> PRT
<213> Artificial Sequence

<220>
<223> KDR or KDR/VEGF Complex Binding Polypeptide

<400> 49

```
Val Cys Trp Glu Asp Ser Trp Gly Gly Glu Val Cys Phe Arg Tyr Asp
1               5                   10                  15
Pro Gly Gly Gly Lys
            20
```

<210> 50
<211> 19
<212> PRT
<213> Artificial Sequence

<220>
<223> KDR or KDR/VEGF Complex Binding Polypeptide

<400> 50

```
Ala Gly Pro Thr Trp Cys Glu Asp Asp Trp Tyr Tyr Cys Trp Leu Phe
1               5                   10                  15
Gly Thr Lys
```

<210> 51
<211> 27
<212> PRT
<213> Artificial Sequence

<220>
<223> KDR or KDR/VEGF Complex Binding Polypeptide

<400> 51

```
        Ala Gln Ala His Met Pro Pro Trp Arg Pro Val Ala Val Asp Ala Leu
        1               5               10              15
        Phe Asp Trp Val Glu Gly Gly Gly Gly Gly Lys
                    20              25
```

<210> 52
<211> 27
<212> PRT
<213> Artificial Sequence

<220>
<223> KDR or KDR/VEGF Complex Binding Polypeptide

<400> 52

```
        Ala Gln Ala His Met Pro Pro Trp Trp Pro Leu Ala Val Asp Ala Gln
        1               5               10              15
        Glu Asp Trp Phe Glu Gly Gly Gly Gly Gly Lys
                    20              25
```

<210> 53
<211> 27
<212> PRT
<213> Artificial Sequence

<220>
<223> KDR or KDR/VEGF Complex Binding Polypeptide

<400> 53

```
        Ala Gln Ala Gln Met Pro Pro Trp Trp Pro Leu Ala Val Asp Ala Leu
        1               5               10              15
        Phe Asp Trp Phe Glu Gly Gly Gly Gly Gly Lys
                    20              25
```

<210> 54
<211> 27
<212> PRT
<213> Artificial Sequence

<220>
<223> KDR or KDR/VEGF Complex Binding Polypeptide

<400> 54

```
        Ala Gln Asp Trp Tyr Trp Arg Glu Trp Met Pro Met His Ala Gln Phe
        1               5               10              15
        Leu Ala Asp Asp Trp Gly Gly Gly Gly Gly Lys
                    20              25
```

<210> 55
<211> 28
<212> PRT

<213> Artificial Sequence

<220>
<223> KDR or KDR/VEGF Complex Binding Polypeptide

<400> 55

```
Ala Gln Pro Val Thr Asp Trp Thr Pro His His Pro Lys Ala Pro Asp
1               5                10              15
Val Trp Leu Phe Tyr Thr Gly Gly Gly Gly Lys
            20              25
```

<210> 56
<211> 28
<212> PRT
<213> Artificial Sequence

<220>
<223> KDR or KDR/VEGF Complex Binding Polypeptide

<400> 56

```
Ala Gln Asp Ala Leu Glu Ala Pro Lys Arg Asp Trp Tyr Tyr Asp Trp
1               5                10              15
Phe Leu Asn His Ser Pro Gly Gly Gly Gly Gly Lys
            20              25
```

<210> 57
<211> 19
<212> PRT
<213> Artificial Sequence

<220>
<223> KDR or KDR/VEGF Complex Binding Polypeptide

<400> 57

```
Lys Trp Cys Glu Glu Asp Trp Tyr Tyr Cys Met Ile Thr Gly Thr Gly
1               5                10              15
Gly Gly Lys
```

<210> 58
<211> 19
<212> PRT
<213> Artificial Sequence

<220>
<223> KDR or KDR/VEGF Complex Binding Polypeptide

<400> 58

```
Ala Gly Pro Lys Trp Cys Glu Glu Asp Trp Tyr Tyr Cys Met Ile Gly
1               5                10              15
Gly Gly Lys
```

<210> 59

<211> 16
<212> PRT
<213> Artificial Sequence

<220>
<223> KDR or KDR/VEGF Complex Binding Polypeptide

<400> 59

```
Lys Trp Cys Glu Glu Asp Trp Tyr Tyr Cys Met Ile Gly Gly Gly Lys
1               5                   10                  15
```

<210> 60
<211> 29
<212> PRT
<213> Artificial Sequence

<220>
<223> KDR or KDR/VEGF Complex Binding Polypeptide

<400> 60

```
Ala Gln Pro Asp Asn Trp Lys Glu Phe Tyr Glu Ser Gly Trp Lys Tyr
1               5                   10                  15
Pro Ser Leu Tyr Lys Pro Leu Gly Gly Gly Gly Gly Lys
                20                  25
```

<210> 61
<211> 28
<212> PRT
<213> Artificial Sequence

<220>
<223> KDR or KDR/VEGF Complex Binding Polypeptide

<400> 61

```
Ala Gln Met Pro Pro Gly Phe Ser Tyr Trp Glu Gln Val Val Leu His
1               5                   10                  15
Asp Asp Ala Gln Val Leu Gly Gly Gly Gly Gly Lys
            20                  25
```

<210> 62
<211> 27
<212> PRT
<213> Artificial Sequence

<220>
<223> KDR or KDR/VEGF Complex Binding Polypeptide

<400> 62

```
Ala Gln Ala Arg Met Gly Asp Asp Trp Glu Glu Ala Pro Pro His Glu
1               5                   10              15
Trp Gly Trp Ala Asp Gly Gly Gly Gly Gly Lys
            20                  25
```

<210> 63
<211> 28
<212> PRT
<213> Artificial Sequence

<220>
<223> KDR or KDR/VEGF Complex Binding Polypeptide

<400> 63

```
Ala Gln Pro Glu Asp Ser Glu Ala Trp Tyr Trp Leu Asn Tyr Arg Pro
1               5                   10              15
Thr Met Phe His Gln Leu Gly Gly Gly Gly Gly Lys
            20                  25
```

<210> 64
<211> 27
<212> PRT
<213> Artificial Sequence

<220>
<223> KDR or KDR/VEGF Complex Binding Polypeptide

<400> 64

```
Ala Gln Ser Thr Asn Gly Asp Ser Phe Val Tyr Trp Glu Glu Val Glu
1               5                   10              15
Leu Val Asp His Pro Gly Gly Gly Gly Gly Lys
            20                  25
```

<210> 65
<211> 28
<212> PRT
<213> Artificial Sequence

<220>
<223> KDR or KDR/VEGF Complex Binding Polypeptide

<400> 65

```
Ala Gln Trp Glu Ser Asp Tyr Trp Asp Gln Met Arg Gln Gln Leu Lys
1               5                   10              15
Thr Ala Tyr Met Lys Val Gly Gly Gly Gly Gly Lys
            20                  25
```

<210> 66
<211> 28
<212> PRT
<213> Artificial Sequence

<220>
<223> KDR or KDR/VEGF Complex Binding Polypeptide

71

<400> 66

```
Ala Gln Asp Trp Tyr Tyr Asp Glu Ile Leu Ser Met Ala Asp Gln Leu
1               5                   10                  15
Arg His Ala Phe Leu Ser Gly Gly Gly Gly Gly Lys
            20                  25
```

<210> 67
<211> 30
<212> DNA
<213> Artificial Sequence

<220>
<223> Smart II Oligonucleotide

<400> 67
aagcagtggt aacaacgcag agtacgcggg          30

<210> 68
<211> 23
<212> DNA
<213> Artificial Sequence

<220>
<223> Oligonucleotide for Cloning

<400> 68
gatggagagc aaggtgctgc tgg          23

<210> 69
<211> 23
<212> DNA
<213> Artificial Sequence

<220>
<223> Oligonucleotide for Cloning

<400> 69
ccaagttcgt cttttcctgg gca          23

<210> 70
<211> 36
<212> DNA
<213> Artificial Sequence

<220>
<223> Oligonucleotide for Cloning

<400> 70
tcccccggga tcattattct agtaggcacg gcggtg          36

<210> 71
<211> 23
<212> DNA
<213> Artificial Sequence

<220>
<223> Oligonucleotide for Cloning

<400> 71
caggaggaga gctcagtgtg gtc        23


<210> 72
<211> 41
<212> DNA
<213> Artificial Sequence


<220>
<223> Oligonucleotide for Cloning


<400> 72
ataagaatgc ggccgcagga tggagagcaa ggtgctgctg g        41


<210> 73
<211> 27
<212> DNA
<213> Artificial Sequence


<220>
<223> Oligonucleotide for Cloning


<400> 73
ttccaagttc gtcttttcct gggcacc        27


<210> 74
<211> 27
<212> DNA
<213> Artificial Sequence


<220>
<223> Oligonucleotide for Cloning


<400> 74
atcattattc tagtaggcac ggcggtg        27


<210> 75
<211> 41
<212> DNA
<213> Artificial Sequence


<220>
<223> Oligonucleotide for Cloning


<400> 75
ataagaatgc ggccgcaaca ggaggagagc tcagtgtggt c        41


<210> 76
<211> 17
<212> PRT
<213> Artificial Sequence


<220>
<223> KDR-Binding Polypeptide


<400> 76

```
Val Cys Trp Glu Asp Ser Trp Gly Gly Glu Val Cys Phe Gly Gly Gly
1               5                   10              15
Lys
```

<210> 77
<211> 21
<212> PRT
<213> Artificial Sequence

<220>
<223> KDR-Binding Polypeptide

<400> 77

```
Gly Asp Ser Arg Val Cys Trp Glu Asp Ser Trp Gly Gly Glu Val Cys
1               5                   10              15
Phe Gly Gly Gly Lys
                20
```

<210> 78
<211> 20
<212> PRT
<213> Artificial Sequence

<220>
<223> KDR-Binding Polypeptide

<400> 78

```
Val Cys Trp Glu Asp Ser Trp Gly Gly Glu Val Cys Phe Arg Tyr Asp
1               5                   10              15

                          Pro Gly Gly Gly
                                  20
```

<210> 79
<211> 22
<212> PRT
<213> Artificial Sequence

<220>
<223> KDR-Binding Polypeptide

<400> 79

```
Ser Arg Val Cys Trp Glu Asp Ser Trp Gly Gly Glu Val Cys Phe Arg
1               5                   10              15
Tyr Gly Gly Gly Gly Lys
                20
```

<210> 80
<211> 23
<212> PRT
<213> Artificial Sequence

```
<220>
<223> KDR-Binding Polypeptide

<400> 80

        Gly Asp Ser Arg Val Cys Trp Glu Asp Ser Trp Gly Gly Glu Val Cys
        1               5                   10                  15
        Phe Arg Tyr Gly Gly Gly Lys
                    20

<210> 81
<211> 21
<212> PRT
<213> Artificial Sequence

<220>
<223> KDR-Binding Polypeptide

<400> 81

        Gly Asp Ser Arg Val Cys Trp Glu Asp Ser Trp Gly Gly Glu Val Cys
        1               5                   10                  15
        Phe Arg Tyr Asp Pro
                    20

<210> 82
<211> 18
<212> PRT
<213> Artificial Sequence

<220>
<223> KDR-Binding Polypeptide

<400> 82

        Ala Gly Asp Ser Trp Cys Ser Thr Glu Tyr Thr Tyr Cys Glu Met Ile
        1               5                   10                  15
        Gly Thr

<210> 83
<211> 24
<212> PRT
<213> Artificial Sequence

<220>
<223> KDR-Binding Polypeptide

<400> 83

        Ala Gln Asp Trp Tyr Tyr Asp Glu Ile Leu Ser Met Ala Asp Gln Leu
        1               5                   10                  15
        Arg His Ala Phe Leu Ser Gly Gly
                    20

<210> 84
<211> 26
<212> PRT
```

<213> Artificial Sequence

<220>
<223> Negative Control Polypeptide

<400> 84

```
        Ala Glu Gly Thr Gly Asp Leu His Cys Tyr Phe Pro Trp Val Cys Ser
        1               5                   10                  15
        Leu Asp Pro Gly Pro Glu Gly Gly Gly Lys
                    20                  25
```

<210> 85
<211> 22
<212> PRT
<213> Artificial Sequence

<220>
<223> KDR-Binding Polypeptide

<400> 85

```
        Ala Gly Pro Thr Trp Cys Glu Asp Asp Trp Tyr Tyr Cys Trp Leu Phe
        1               5                   10                  15
        Gly Thr Gly Gly Gly Lys
                    20
```

<210> 86
<211> 26
<212> PRT
<213> Artificial Sequence

<220>
<223> Library Isolate

<400> 86

```
        Gly Cys Lys Thr Lys Ile Ser Lys Val Lys Lys Lys Trp Asn Cys Tyr
        1               5                   10                  15
        Ser Asn Asn Lys Val Thr Gly Gly Gly Lys
                    20                  25
```

<210> 87
<211> 26
<212> PRT
<213> Artificial Sequence

<220>
<223> Library Isolate

<400> 87

```
        Lys Gln Phe Cys Glu Glu Asn Trp Glu Arg Gly Arg Asn His Tyr Tyr
        1               5                   10                  15
        Cys Leu Thr Thr Leu Ser Gly Gly Gly Lys
                    20                  25
```

<210> 88

<211> 17
<212> PRT
<213> Artificial Sequence

<220>
<223> Library Isolate

<400> 88

```
        Cys Glu Glu Asp Trp Tyr Tyr Cys Met Ile Thr Gly Thr Gly Gly Gly
        1               5               10              15
        Lys
```

<210> 89
<211> 18
<212> PRT
<213> Artificial Sequence

<220>
<223> Library Isolate

<400> 89

```
        Ala Gly Pro Lys Trp Cys Glu Glu Asp Trp Tyr Tyr Cys Met Ile Thr
        1               5               10              15
        Ala Thr
```

<210> 90
<211> 22
<212> PRT
<213> Artificial Sequence

<220>
<223> Library Isolate

<400> 90

```
        Ala Gly Pro Thr Trp Glu Glu Asp Asp Trp Tyr Tyr Lys Trp Leu Phe
        1               5               10              15
        Gly Thr Gly Gly Gly Lys
                        20
```

<210> 91
<211> 22
<212> PRT
<213> Artificial Sequence

<220>
<223> Library Isolate

<400> 91

```
        Ala Gly Pro Thr Trp Lys Glu Asp Asp Trp Tyr Tyr Glu Trp Leu Phe
        1               5               10              15
        Gly Thr Gly Gly Gly Lys
                        20
```

77

<210> 92
<211> 22
<212> PRT
<213> Artificial Sequence

<220>
<223> Library Isolate

<221> MOD_RES
<222> (6)...(6)
<223> Xaa = Dpr

<400> 92

```
Ala Gly Pro Thr Trp Xaa Glu Asp Asp Trp Tyr Tyr Cys Trp Leu Phe
1               5                   10              15
Gly Thr Gly Gly Gly Lys
            20
```

<210> 93
<211> 22
<212> PRT
<213> Artificial Sequence

<220>
<223> Library Isolate

<221> MOD_RES
<222> (13)...(13)
<223> Xaa = Dpr

<400> 93

```
Ala Gly Pro Thr Trp Asp Glu Asp Asp Trp Tyr Tyr Xaa Trp Leu Phe
1               5                   10              15
Gly Thr Gly Gly Gly Lys
            20
```

<210> 94
<211> 22
<212> PRT
<213> Artificial Sequence

<220>
<223> Library Isolate

<400> 94

```
Ala Gly Pro Thr Trp Asp Glu Asp Asp Trp Tyr Tyr Lys Trp Leu Phe
1               5                   10              15
Gly Thr Gly Gly Gly Lys
            20
```

<210> 95
<211> 8
<212> PRT
<213> Artificial Sequence

<220>
<223> Library Isolate

<400> 95

```
Asp Trp Tyr Tyr Gly Gly Gly Lys
1               5
```

<210> 96
<211> 19
<212> PRT
<213> Artificial Sequence

<220>
<223> Library Isolate

<400> 96

```
Ala Gln Asp Trp Tyr Tyr Asp Glu Ile Leu Gly Arg Gly Arg Gly Gly
1               5               10              15
Arg Gly Gly
```

<210> 97
<211> 25
<212> PRT
<213> Artificial Sequence

<220>
<223> Library isolate

<400> 97

```
Val Cys Trp Glu Asp Ser Trp Glu Asp Ser Trp Gly Gly Glu Val Cys
1               5               10              15
Phe Arg Tyr Asp Pro Gly Gly Gly Lys
            20              25
```

<210> 98
<211> 22
<212> PRT
<213> Artificial Sequence

<220>
<223> Library Isolate

<400> 98

```
Ala Gly Pro Thr Trp Cys Glu Asp Asp Trp Tyr Tyr Cys Trp Leu Phe
1               5               10              15
Gly Thr Gly Gly Gly Lys
        20
```

<210> 99
<211> 25
<212> PRT
<213> Artificial Sequence

<220>
<223> Library Isolate

<400> 99

```
        Gly Asp Ser Arg Val Cys Trp Glu Asp Ser Trp Gly Gly Glu Val Cys
        1               5               10              15
        Phe Arg Tyr Asp Pro Gly Gly Gly Lys
                    20              25
```

<210> 100
<211> 21
<212> PRT
<213> Artificial Sequence

<220>
<223> Synthesized KDR-Binding Polypeptide

<400> 100

```
        Ala Gln Asp Trp Tyr Tyr Asp Glu Ile Leu Gly Arg Gly Gly Arg Gly
        1               5               10              15
        Gly Arg Gly Gly Lys
                    20
```

210> 101

<211> 22
<212> PRT
<213> Artificial Sequence

<220>
<223> Library isolate

<400> 101

```
        Ala Pro Gly Thr Trp Cys Asp Tyr Asp Trp Glu Tyr Cys Trp Leu Gly
        1               5               10              15
        Thr Phe Gly Gly Gly Lys
                    20
```

<210> 102
<211> 23
<212> PRT
<213> Artificial Sequence

<220>
<223> Synthesized KDR-Binding Polypeptide

<400> 102

```
        Ala Gly Pro Thr Trp Cys Glu Asp Asp Trp Tyr Tyr Cys Trp Leu Phe

            1               5               10              15
        Gly Thr Gly Gly Gly Gly Lys
                    20
```

<210> 103
<211> 21
<212> PRT
<213> Artificial Sequence

<220>
<223> Synthesized KDR-Binding Polypeptide

<400> 103

```
Ala Gly Pro Thr Trp Cys Glu Asp Asp Trp Tyr Tyr Cys Trp Leu Phe
1               5                   10                  15
Thr Gly Gly Gly Lys
                20
```

<210> 104
<211> 25
<212> PRT
<213> Artificial Sequence

<220>
<223> Synthesized KDR-Binding Polypeptide

<400> 104

```
Gly Asp Ser Ser Val Cys Phe Glu Tyr Ser Trp Gly Gly Glu Val Cys
1               5                   10                  15
Phe Arg Tyr Asp Pro Gly Gly Gly Lys
                20                  25
```

<210> 105
<211> 25
<212> PRT
<213> Artificial Sequence

<220>
<223> Synthesized KDR-Binding Polypeptide

<400> 105

```
Gly Asp Ser Arg Val Cys Trp Glu Tyr Ser Trp Gly Gly Gln Ile Cys
1               5                   10                  15
Leu Gly Tyr Asp Pro Gly Gly Gly Lys
                20                  25
```

<210> 106
<211> 25
<212> PRT
<213> Artificial Sequence

<220>
<223> Library Isolate

<400> 106

```
Gly Val Asp Phe Arg Cys Glu Trp Ser Asp Trp Gly Glu Val Gly Cys
1               5                   10                  15
```

```
Arg Ser Pro Asp Tyr Gly Gly Gly Lys
         20              25
```

<210> 107
<211> 19
<212> PRT
<213> Artificial Sequence

<220>
<223> Mature HSA

<400> 107

```
Trp Gln Pro Cys Pro Trp Glu Ser Trp Thr Phe Cys Trp Asp Pro Gly
 1           5                  10                  15
Gly Gly Lys
```